# DEMANDE DE BREVET EUROPEEN

(11) **EP 1 798 231 A2**
(43) Date de publication de la demande: **20.06.2007**
(21) Numéro de dépôt: 07007308.5
(22) Date de dépôt: 04.06.2002
(51) Int. Cl.: C07D 471/08, C07D 471/18, C07D 491/18, C07D 495/18, C07D 215/38, C07D 217/22, C07D 217/26, C07D 471/04, C07D 495/04, C07D 513/04, C07D 513/18, C07D 498/04, C07D 498/18, C07D 491/04, A61K 31/551, A61P 31/04

(54) **Produits intermédiaires dans la préparation de composés anti-bactériens**

(30) Priorité: 08.06.2001 FR 0107520
(62) Demande divisionnaire de: 02778894.2
(71) Demandeur: NOVEXEL, 93230 Romainville (FR)
(72) Inventeur: Aszodi, Joseph, Tucson AZ 85750 (US); Lampilas, Maxime, 93230 Romainville (FR); Musicki, Branislav, 75019 Paris (FR); Rowlands, David Alan, 78300 Poissy (FR); Collette, Pascal, 06110 Le Canet (FR)
(74) Mandataire: Hirsch & Associés

(57) **Abrégé**

L'invention concerne de nouveaux composés hétérocycliques intermédiaires dans la synthèse des composés de formule générale (I) et de leurs sels avec une base ou un acide :

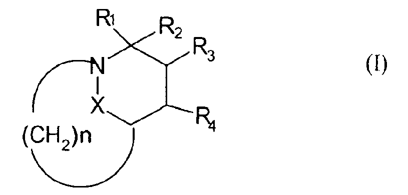

Les composés de formule (I) sont utiles comme médicaments, notamment comme anti-bactériens.

## Description

L'invention concerne de nouveaux composés hétérocycliques, utilisés comme intermédiaires dans la préparation de composés anti-bactériens.

Dans le journal J. Org. Chem., Vol. 37, No. 5, 1972, pages 697 à 699 est décrite notamment la préparation d'un dérivé bicyclique de formule brute C₁₀H₁₈N₂O.

Dans le journal J. Org. Chem., Vol. 45, No. 26, 1980, pages 5325-5326 est décrite notamment la préparation de dérivés bicycliques de formules brutes C₆H₉NO₂ et C₇H₁₁NO₂.

Dans la revue Chemical Reviews, 1983, vol. 83, No. 5, pages 549 à 555 est décrite notamment la préparation de dérivés bicycliques de formules brutes C₁₀H₁₈N₂O et C₇H₁₂N₂O.

Dans le journal Angew. Chem. Int. Ed. 2000, 39, n° 3, pages 625 à 628 est décrit notamment la préparation d'un composé de formule brute C₁₂H₁₂N₂O.

Aucune utilisation particulière dans le domaine thérapeutique de ces composés n'a été décrite dans ces documents.

WO-A-9518129 décrit des composés anti-bactériens dont notamment un dérivé de 1,6-diazabicyclo[3.2.1]octane.

L'invention a pour objet des composés intermédiaires utiles dans la préparation des composés répondant à la formule (I) suivante : 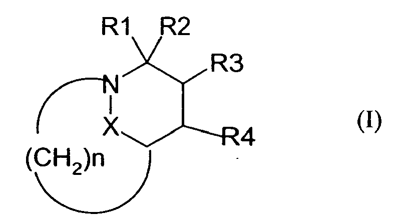 dans laquelle :
a) ou bien R1 représente un atome d'hydrogène, un radical COOH, CN, COOR, (CH₂)n'R₅, CONR₆R₇ ou 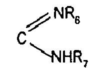 R est choisi dans le groupe constitué par un radical alkyle renfermant de 1 à 6 atomes de carbone, éventuellement substitué par un radical pyridyle, un radical - CH₂-alkényle renfermant au total de 3 à 9 atomes de carbone, un groupe (poly)alkoxyalkyle renfermant 1 à 4 atomes d'oxygène et 3 à 10 atomes de carbone, un radical aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
   R₅ est choisi dans le groupe constitué par un radical COOH, CN, OH, NH₂, CO-NR₆R₇, COOR, OR, OCOH, OCOR, OCOOR, OCONHR, OCONH₂,OSO₂R, NHR, NHCOR, NHCOH, NHSO₂R, NH-COOR, NH-CO-NHR ,NH-CO-NH₂,ou N₃, R étant défini comme ci-dessus,
   R₆ et R₇, identiques ou différents, sont choisis dans le groupe constitué par un atome d'hydrogène, un radical alkyle renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
   n' est égal à 1 ou 2,
   R₃ et R₄ forment ensemble un phényl ou un hétérocycle à caractère aromatique à 5 ou 6 sommets renfermant de 1 à 4 héréroatomes choisis parmi l'azote, l'oxygène et le soufre, et éventuellement substitué par un ou plusieurs groupements R', R' étant choisi dans le groupe constitué par un atome d'hydrogène et les radicaux alkyle renfermant de 1 à 6 atomes de carbone éventuellement substitué par un ou plusieurs radicaux hydroxy, oxo, halogène ou cyano ou par un radical nitro, alkényle renfermant de 2 à 6 atomes de carbone, halogèno, amino, OH, OH protégé, -OR, -NHCOH, -NHCOR, NHCOOR, COOH,
   -COOR, -C(C₆H₅)₃ et -CH₂-CH₂-S(O)m-R, R étant tel que défini précédemment et m étant égal à 0, 1 ou 2,
b) ou bien R₄ représente un atome d'hydrogène ou un groupement (CH₂)_{n'1}R₅, n' 1 étant égal à 0, 1 ou 2 et R₅ étant tel que défini ci-dessus,
   et R₁ et R₃ forment ensemble un phényle ou un hétérocycle éventuellement substitué, tel que défini ci-dessus,
   dans les deux cas a) et b)
   R₂ est choisi dans le groupe constitué par un atome d'hydrogène, un atome d'halogène et les radicaux R, S(O)ₘR, OR, NHCOR, NHCOOR et NHSO₂R, m et R étant tels que définis précédemment,
   X représente un groupement divalent -C(O)-B- relié à l'atome d'azote par l'atome de carbone,
   B représente un groupement divalent -O-(CH₂)ₙ"- lié au carbonyle par l'atome d'oxygène, un groupement -NR₈-(CH₂)_{n"}- ou -NR₈-O-relié au carbonyle par l'atome d'azote, n" est égal à 0 ou 1 et R₈ est choisi dans le groupe constitué par un atome d'hydrogène, un radical OH, R, OR, Y, OY, Y₁, OY₁, Y₂, OY₂, Y₃, O-CH₂-CH₂-S(O)m-R, SiRaRbRc et OSiRaRbRc, Ra, Rb et Rc représentant individuellement un radical alkyle linéaire ou ramifié renfermant de 1 à 6 atomes de carbone ou un radical aryle renfermant de 6 à 10 atomes de carbone, et R et m étant définis comme précédemment.

Y est choisi dans le groupe constitué par les radicaux COR, COOR, CONH₂, CONHR, CONHOH, CONHSO₂R, CH₂COOH, CH₂COOR, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂,
Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NH ou NR tétrazole, NH ou NR tétrazole substitué par le radical R, NHSO₂R et NRSO₂R, R étant défini comme ci-dessus,
n est égal à 1 ou 2.
On préfère une sous classe de composées de formule I telle que définit précédemment où R₈ dans -NR₈-(CH₂)ₙ" ne peut être méthyle lorsque par ailleurs n=1, R₄ est hydrogène et R₁ et R₃ forment ensemble un phényle.

L'invention a également pour objet les sels de ces composés qui peuvent être obtenus avec des bases ou des acides minéraux ou organiques.

Il est clair que les composés selon l'invention se distinguent structurellement des composés de l'art antérieur cités plus haut.

Les atomes de carbone asymétriques contenus dans les composés de formule (I) peuvent indépendamment les uns des autres présenter la configuration R, S ou RS et l'invention a donc également pour objet les composés de formule (I) se présentant sous la forme d'énantiomères purs ou de diastéréoisomères purs ou sous la forme d'un mélange d'énantiomères notamment de racémates, ou de mélanges de diastéréoisomères.

Il résulte de ce qui précède que les substituants R₁, R₂, ou R₄ pris individuellement d'une part et X d'autre part peuvent être en position cis et/ou trans par rapport au cycle sur lequel ils sont fixés et que l'invention a donc pour objet les composés de formule (I) se présentant sous la forme d'isomères cis ou d'isomères trans ou de mélanges.

Par radical alkyle renfermant de 1 à 6 atomes de carbone, on entend le radical méthyle, éthyle, propyle, isopropyle, ainsi que butyle, pentyle ou hexyle linéaire ou ramifié.

Par radical -CH₂-alkényle renfermant de 3 à 9 atomes de carbone, on entend par exemple le radical allyle, ou un radical butényle, pentényle ou hexényle.

Par radical aryle renfermant de 6 à 10 atomes de carbone, on entend un radical phényle ou naphtyle.

Par radical aralkyle renfermant de 7 à 11 atomes de carbone, on entend un radical benzyle, phénéthyle ou méthylnaphtyle.

Par radical alkoxy renfermant de 1 à 6 atomes de carbone, on entend notamment le radical méthoxy, éthoxy, propoxy, isopropoxy, butoxy, isobutoxy, sec-butoxy ou tert-butoxy.

Par radical halogéno ou par atome d'halogène, on entend fluor, chlore, brome ou iode.

Par radical squarate, on entend le radical de formule :

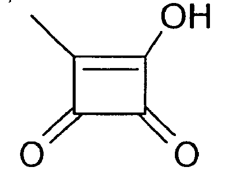

Par hétérocycle à caractère aromatique, on entend notamment ceux choisis dans la liste qui suit, les deux liaisons symbolisant la jonction avec le cycle azoté (R₃R₄ ou R₁R₃): 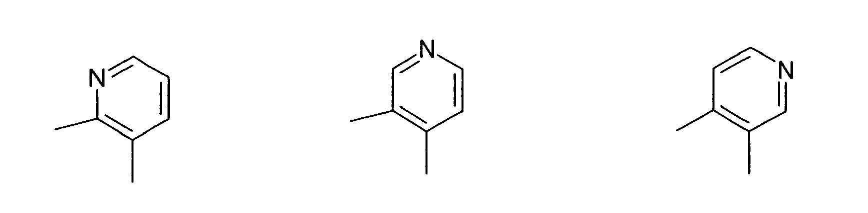 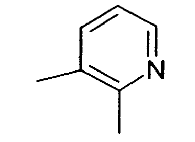 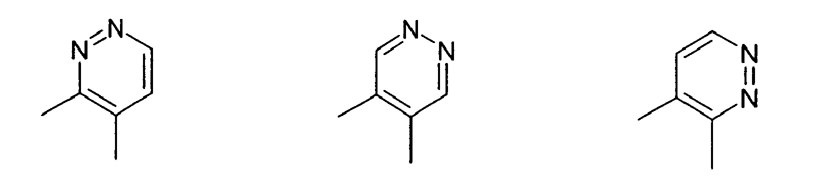 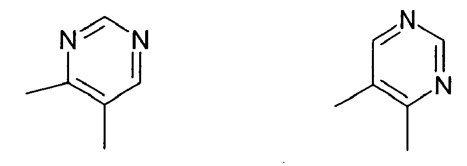 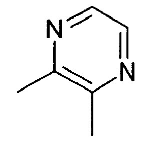 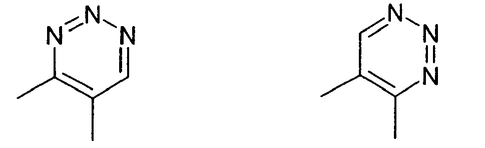 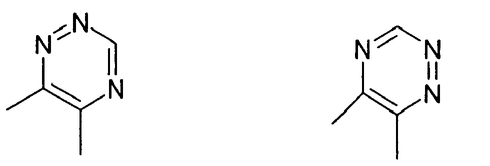 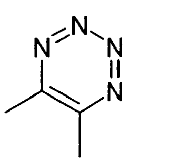 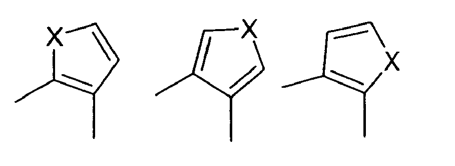 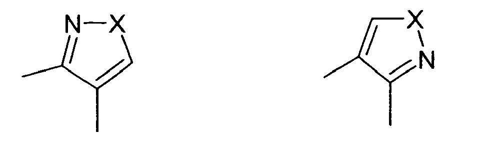 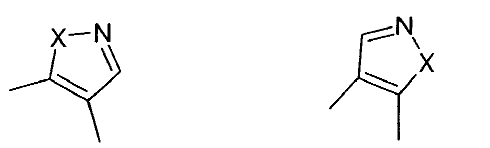 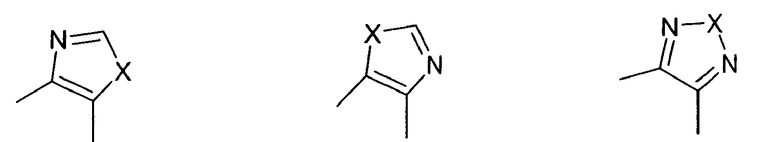 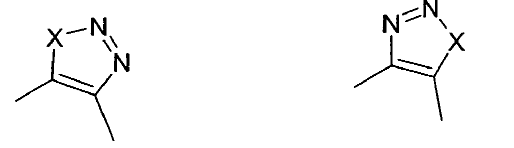 avec X = NR', S, O
Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkyl-phosphonium, les aryl-phosphoniums, les alkyl-aryl-phosphonium, les alkénylaryl-phosphonium ou les sels d'ammoniums quaternaires tels que le sel de tétra-n-butyl-ammonium.

Parmi les composés de formule (I), l'invention concerne notamment ceux dans lesquels n est égal à 1 ainsi que ceux dans lesquels R₂ est un atome d'hydrogène.

On préfère les composés de formule (I) dans lesquels R₃ et R₄ forment ensemble un phényl ou un hétérocycle, éventuellement substitué, tel que défini précédemment. Parmi, ces derniers, on cite en particulier les composés de formule (I) dans laquelle R₃ et R₄ forment ensemble un phényle ou un hétérocycle choisi dans le groupe constitué par thiényle, imidazolyle, furyle, pyrazolyle et triazolyle, éventuellement substitué.

Parmi les composés de formule (I), l'invention concerne notamment ceux dans lesquels R1 est choisi dans le groupe constitué par l'atome d'hydrogène et les groupements COOCH₃, COOC₂H₅, CONH₂, CONHCH₃, CONHCH₂-phényl et CONHCH₂-pyridyl.

Parmi les composés de formule (I), l'invention concerne encore notamment ceux dans lesquels X représente un groupement divalent -CO-B- dans lequel B représente un groupement -NR₈-(CH₂)ₙ" - tel que défini plus haut, dans lequel n" est égal à O.

Parmi ces derniers on peut citer en particulier, ceux dans lesquels R8 est un groupement Y₁ ou OY₁, dans lequel Y₁ est choisi parmi les groupements SO₂R, SO₂NHCOR, SO₂NHCOOR, SO₂NHCONHR et SO₃H et R tel que défini plus haut ; ou bien ceux dans lesquels le groupement R₈ est choisi dans le groupe constitué par l'atome d'hydrogène et les groupements hydroxy, CO-phényl, O-allyl, OPO₃H, OPO₃-benzyl, OCH₂COOH et O-benzyl.

Parmi les composés de formule (I), l'invention concerne tout particulièrement les composés dont les noms suivent :
- le sel de sodium de trans-3-oxo-4-(sulfooxy)-2,3,4,5-tétrahydro-2,5-méthano-1H-2,4-benzodiazépine-1-carboxamide ;
- le sel de sodium de 3-méthyl-5-(sulfooxy)-5,6,7,8-tétrahydro-4,7-méthano-4H-[1,2,3]-triazolo[4,5-e][1,3]diazépine-6(3H)-one ;
- le sel de sodium de trans-1-méthyl-6-oxo-5-(sulfooxy)-4,5,6,8-tétrahydro-4,7-méthano-1H-pyrazolo[3,4-e][1,3]diazépine-8(7H)-carboxamide ;
- le sel de sodium de trans-N-méthyl-3-oxo-4-(sulfooxy)-2,3,4,5-tétrahydro-2,5-méthano-1H-2,4-benzodiazépine-1-carboxamide ;
- le sel de sodium de 5-(sulfooxy)-5,6,7,8-tétrahydro-4,7-méthano-4H-furo[2,3-e] [1,3]diazépine-6-one ;
- le sel de pyridinium de 1-propyl-5-(sulfooxy)-4,5,7,8-tétrahydro-4,7-méthano-imidazo[4,5-e][1,3]diazépine-6(1H)-one ;
- le sel de sodium de trans-6-oxo-5-(sulfooxy)-5,6,7,8-tétrahydro-4,7-méthano-4H-furo[2,3-e][1,3]diazépine-8-carboxylate de méthyle ;
- le sel de sodium de 1-méthyl-5-(sulfooxy)-5,6,7,8-tétrahydro-4,7-méthano-4H-pyrazolo[3,4-e][1,3]diazépine-6( 1H)-one ;
- le sel de sodium de trans-3-oxo-N-(4-pyridinylméthyl)-4-(sulfooxy)-2,3,4,5-tétrahydro-2,5-méthano-1H-2,4-benzodiazépine-1-carboxamide ;
- le sel de sodium de trans-6-oxo-5-(sulfooxy)-5,6,7,8-tétrahydro-4,7-méthano-4H-thiéno[2,3-e][1,3]diazépine-8-carboxamide ;
- le sel de sodium de trans-6-oxo-5-(sulfooxy)-5,6,7,8-tétrahydro-4,7-méthano-4H-furo[2,3-e][1,3]diazépine-8-carboxamide.
- Le sel de sodium de trans-1,2,3,5-tétrahydro-8-hydroxy-3-oxo-2-(sulfooxy)-1,4-méthano-4*H*-2,4-benzodiazépine-5-carboxamide
- Le sel de sodium de trans-7-(acétylamino)-1,2,3,5-tétrahydro-8-hydroxy-3-oxo-2-(sulfooxy)-1,4-méthano-4*H*-2,4-benzodiazépine-5-carboxamide
- Le sel de sodium de trans-1,5-dihydro-5-(hydroxyméthyl)-2-(sulfooxy)-1,4-méthano-4*H*-2,4-benzodiazépin-3(2*H*)-one
- Le sel de sodium de trans-4,5,6,8-tétrahydro-N-méthyl-6-oxo-5-(sulfooxy)-4,7-méthano-7H-thiéno[2,3-e][1,3]diazépine-8-carboxamide
- Le sel de sodium de 7,8-dihydro-7-(sulfooxy)-5,8-méthano-5*H-*thiéno[2,3-*e*][1,3]diazépin-6(4*H*)-one
- Le sel de triéthylammonium de trans-2-bromo-4,5,6,8-tétrahydro-6-oxo-5-(sulfooxy)-4,7-méthano-7*H*-thiéno[2,3-*e*][1,3]diazépine-8-carboxamide

Le procédé de la préparation des composés de formule (I) est caractérisé en ce qu'il comporte :
a) une étape au cours de laquelle on fait réagir, avec un agent de carbonylation, le cas échéant en présence d'une base, un composé de formule (II) : 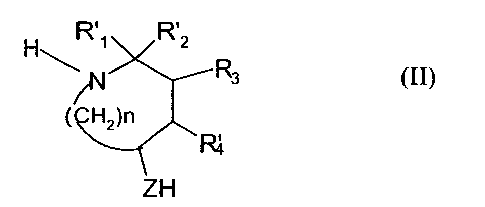 dans laquelle :
   a) ou bien R'₁ représente un atome d'hydrogène, un radical CN, COOH protégé, COOR", (CH₂)_{n'}R'₅, CONR₆R₇, ; 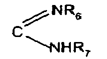 R" est choisi dans le groupe constitué par un radical alkyle renfermant de 1 à 6 atomes de carbone, éventuellement substitué par un radical pyridyle, un radical - CH₂-alkényle renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical NO₂, OH protégé, NH₂ protégé, alkyle renfermant de 1 à 6 atomes de carbone, alkoxy renfermant de 1 à 6 atomes de carbone ou par un ou plusieurs atomes d'halogène,
      R'₅ est choisi dans le groupe constitué par un radical OH protégé, CN, NH₂ protégé, CO-NR₆R₇, COOH protégé, COOR", OR", OCOH, OCOR", OCOOR", OCONH₂, OCONHR", NHR" protégé, NHCOR", NHSO₂R", NH-COOR", NH-CO-NHR" ou
      NH-CONH₂, R" étant défini comme ci-dessus,
      n', R₆, R₇ et R₃ sont tels que définis ci-dessus et R'₄ représente un radical R₄ tel que défini ci-dessus;
b) ou bien R'₄ représente un atome d'hydrogène ou un groupement (CH₂)ₙ'₁R'₅, n'1 étant égal à 0, 1 ou 2 et R'₅ étant tel que défini ci-dessus,
   et R'₁ et R₃ forment ensemble un phényle ou un hétérocycle éventuellement substitué, tel que défini ci-dessus pour R₃ et R₄,
   dans les deux cas a) et b)
   R'₂ est choisi dans le groupe constitué par un atome d'hydrogène, un atome d'halogène et les radicaux R", S(O)ₘR", OR", NHCOH, NHCOR", NHCOOR" et NHSO₂R", R" étant tel que défini précédemment,
   ZH représente un groupement HO-(CH₂)ₙ", HNR'₈-(CH₂)ₙ"-
   ou HNR'₈-O-, n" est tel que défini ci-dessus et R'₈ représente un atome d'hydrogène, un radical R", OH protégé, OR", Y', OY', Y'₁, OY'₁, Y'₂, OY'₂, Y'₃, O-CH₂-CH₂-S(O)ₘ-R", SiRaRbRc
   et OSiRaRbRc, Ra, Rb et Rc représentant individuellement un radical alkyle linéaire
   ou ramifié renfermant de 1 à 6 atomes de carbone ou un radical aryle renfermant de 6 à 10 atomes de carbone et R" et m étant défini comme précédemment ; avec de préférence les composés où R'₈ dans -NR'₈-(CH₂)ₙ" ne peut être méthyle lorsque par ailleurs n=1, R'₄ est hydrogène et R'₁ et R₃ forment ensemble un phényle,
   Y' est choisi dans le groupe constitué par les radicaux COH, COR", COOR", CONH₂, CONHR", CONHSO₂R", CH₂COOR", CH₂tétrazole protégé, CH₂SO₂R", CH₂PO(OR")₂, CONHOH protégé, CH₂COOH protégé, CH₂CONHOH protégé, CH₂SO₃ protégé, CH₂PO(OR)(OH) protégé, CH₂PO(R)(OH) protégé et CH₂PO(OH)₂ protégé,
   Y'₁ est choisi dans le groupe constitué par les radicaux SO₂R", SO₂NHCOH, SO₂NHCOR", SO₂NHCOOR", SO₂NHCONH₂, SO₂NHCONHR" et SO₃H protégé,
   Y'₂ est choisi dans le groupe constitué par les radicaux PO(OR")₂, PO(OH)₂ protégé, PO(OH)(OR) protégé et PO(OH)(R) protégé,
   Y'₃ est choisi dans le groupe constitué par les radicaux tétrazole protégé, tétrazole substitué par le radical R", squarate protégé, NH tétrazole protégé, NR" tétrazole protégé, NH protégé, NR" tétrazole substitué par le radical R", NHSO₂R" et NSO₂R", R" étant défini comme ci-dessus.
   n est tel que défini ci-dessus ;
   en vue d'obtenir un composé intermédiaire de formule : 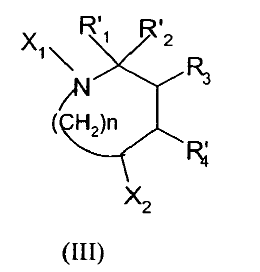 dans laquelle :
   R'₁, R'₂, R₃, R'₄ et n ont les mêmes significations que ci-dessus et soit X₁ est un atome d'hydrogène et X2 représente un groupement -Z-CO-X₃, X₃ représentant le reste de l'agent de carbonylation, soit X₂ est un groupement -ZH et X1 représente un groupement CO-X₃, X₃ étant défini comme ci-dessus ;
b) une étape au cours de laquelle on cyclise l'intermédiaire obtenu précédemment, en présence d'une base ;
   et en ce que :
c) le cas échéant, l'étape a) est précédée et/ou l'étape b) est suivie de l'une ou de plusieurs des réactions suivantes, dans un ordre approprié :
   - protection des fonctions réactives,
   - déprotection des fonctions réactives,
   - estérification
   - saponification,
   - sulfatation,
   - phosphatation
   - amidification,
   - acylation,
   - sulfonylation;
   - alkylation ;
   - formation d'un groupe urée ;
   - introduction d'un groupement tétrazole;
   - réduction d'acides carboxyliques ;
   - déshydratation d'amide en nitrile ;
   - salification ;
   - échange d'ions ;
   - dédoublement ou séparation de diastéréoisomères
   - oxydation de sulfure en sulfoxyde et/ou sulfone;
   - nitration ;
   - réduction d'un nitro en amino ;
   - halogénation ;
   - thioalkylation ;
   - carbamoylation ;
   - formation d'un groupe azido ;
   - réduction d'un azido en amine ;
   - réactions de couplage d'halogénures aromatiques avec des réactifs stannylés ;
   - hydrogénation de doubles liaisons ;
   - dihydroxylation de doubles liaisons ;
   - clivage de diols par oxydation ;
   - cyanuration.

Comme agent de carbonylation, on peut mettre en oeuvre un réactif tel que le phosgène, le diphosgène, le triphosgène, un chloroformiate d'aryle tel que le chloroformiate de phényle ou de p-nitrophényle, un chloroformiate d'aralkyle tel que chloroformiate de benzyle, un chloroformiate d'alkyle ou d'alkényle tel que le chloroformiate de méthyle ou d'allyle, un dicarbonate d'alkyle tel que le dicarbonate de tert-butyle, le carbonyl-diimidazole et leurs mélanges.

La réaction a lieu de préférence en présence d'une base ou d'un mélange de bases qui neutralise l'acide formé. Elle peut notamment être une amine telle que la triethylamine, la diisopropyléthylamine, la pyridine, la diméthylaminopyridine. Toutefois, on peut également opérer en utilisant le produit de départ de formule II comme base. On en utilise alors un excès.

Le cas échéant, le produit de formule II est mis en oeuvre sous la forme d'un sel d'acide, par exemple un chlorhydrate ou un trifluoroacétate.

Comme base dans l'étape b), on peut également utiliser les amines, ou encore les hydrures, les alcoolates, les amidures ou carbonates de métaux alcalins ou alcalino-terreux.

Les amines peuvent être choisies par exemple dans la liste ci-dessus.

Comme hydrure on peut notamment utiliser l'hydrure de sodium ou de potassium.

Comme alcoolate de métal alcalin, on utilise de préférence le t-butylate de potassium.

Comme amidure de métal alcalin on peut notamment utiliser le bis(triméthylsilyl) amidure de lithium.

Comme carbonate, on peut notamment utiliser le carbonate ou bicarbonate de sodium ou de potassium.

Le cas échéant, l'intermédiaire de formule III peut être obtenu sous forme d'un sel d'acide généré lors de la réaction de carbonylation et notamment un chlorhydrate. Il est ensuite mis en oeuvre dans la réaction de cyclisation sous cette forme.

Le cas échéant, la cyclisation peut être effectuée sans isolement de l'intermédiaire de formule III.

Les réactions mentionnées à l'étape c) sont d'une manière générale des réactions classiques, bien connues de l'homme du métier.

Les fonctions réactives qu'il convient, le cas échéant, de protéger sont les fonctions acides carboxyliques, amines, amides, hydroxy et hydroxylamines.

La protection de la fonction acide est notamment effectuée sous forme d'esters d'alkyle, d'esters allyliques, de benzyle, benzhydryle ou p-nitrobenzyle.

La déprotection est effectuée par saponification, hydrolyse acide, hydrogénolyse, ou encore clivage à l'aide de complexes solubles du Palladium O.

Des exemples de ces protections et déprotections sont fournis ci-après dans la partie expérimentale.

La protection des amines, des azotes hétérocycliques et des amides est notamment effectuée, selon les cas, sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, tri-phényl ou encore diphényl tertbutyl-silyle, ou de dérivés phénylsulfonylalkyle ou cyanoalkyle.

La déprotection est effectuée, selon la nature du groupement protecteur, par le sodium ou le lithium dans l'ammoniac liquide, par hydrogénolyse ou à l'aide de complexes solubles du Palladium O, par action d'un acide, ou par action du fluorure de tétrabutylammonium ou de bases fortes telles que l'hydrure de sodium ou le t.butylate de potassium.

Des exemples sont fournis ci-après dans la partie expérimentale.

La protection des hydroxylamines est effectuée notamment sous forme d'éthers de benzyle ou d'allyle.

Le clivage des éthers est effectué par hydrogénolyse ou à l'aide de complexes solubles du Palladium O.

Une illustration est fournie plus loin dans la partie expérimentale.

La protection des alcools et des phénols est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

Des exemples sont fournis dans la partie expérimentale.

La réaction de sulfatation est effectuée par action des complexes SO₃-amines tels que SO₃-pyridine ou SO₃-diméthylformamide en opérant dans la pyridine, le sel formé, par exemple le sel de pyridine, pouvant ensuite être échangé par exemple par un sel d'une autre amine, d'un ammonium quaternaire ou d'un métal alcalin. Des exemples sont fournis dans la partie expérimentale.

La réaction de phosphatation est effectuée par exemple par action d'un chlorophosphate tel que le diméthyl, dibenzyl ou diphényl chlorophosphate.

La réaction d'amidification est effectuée au départ de l'acide carboxylique à l'aide d'un agent d'activation tel qu'un chloroformiate d'alkyle, l'EDCI ou le BOP par action de l'ammoniaque ou d'une amine appropriée ou de leurs sels d'acides. Des exemples sont fournis ci-après dans la partie expérimentale.

Les réactions d'acylation et de sulfonylation sont effectuées sur les hydroxy-urées, les alcools, les amines ou les azotes hétérocycliques, par action selon les cas, d'un halogènure ou d'un anhydride d'acide carboxylique ou d'acide sulfonique approprié, le cas échéant en présence d'une base. Plusieurs exemples sont fournis ci-après dans la partie expérimentale.

La réaction d'alkylation est effectuée par action sur les dérivés hydroxylés, les énolates d'esters ou de cétones, les amines ou les azotes hétérocycliques, selon les cas, d'un sulfate d'alkyle ou d'un halogènure d'alkyle ou d'alkyle substitué, notamment par un radical carboxy libre ou estérifié. Des illustrations sont fournies ci-après dans la partie expérimentale.

La réduction d'acides en alcools peut être effectuée par action d'un borane ou via un anhydride mixte intermédiaire, par action d'un borohydrure alcalin. L'anhydride mixte est préparé par exemple à l'aide d'un chloroformiate d'alkyle. La réduction d'aldéhyde en alcool est de préférence effectuée par action de borohydrure de sodium. Des illustrations sont fournies dans la partie expérimentale.

La déshydratation d'amide en nitrile peut intervenir dans les conditions des réactions de carbonylation et cyclisation.

L'oxydation des sulfures en sulfoxyde et/ou sulfone peut être effectuée par action d'un peracide tel que l'acide métachloroperbenzoïque ou perphtalique ou de tout autre réactif connu de l'homme du métier.

La salification par les acides est le cas échéant réalisée par addition d'un acide en phase soluble au composé. La salification par les bases peut concerner les composés comportant une fonction acide et notamment les composés comportant une fonction carboxy, ceux comportant une fonction sulfooxy ou dérivée de l'acide phosphorique ou ceux comportant un hétérocycle à caractère acide.

Dans le cas d'une fonction carboxy, on opère par addition d'une base appropriée telle que celles citées précédemment. Dans le cas d'une fonction sulfooxy ou dérivée de l'acide phosphorique, on obtient directement le sel de pyridinium lors de l'action du complexe SO3-pyridine et on obtient les autres sels à partir de ce sel de pyridinium. Dans l'un ou l'autre cas, on peut encore opérer par échange d'ions sur résine. Des exemples de salifications par les acides ou par les bases et y compris d'hétérocycle à caractère acide, figurent ci-après dans la partie expérimentale.

La nitration peut être effectuée par l'acide nitrique ou par l'un de ses sels métalliques, en milieu acide.

La réduction d'un groupement nitro peut être effectuée par le dithionite de sodium ou encore par le zinc dans l'acide acétique.

Par halogénation on entend introduction d'un substituant halogéné à partir d'un hydroxy ou halogénation directe d'un cycle aromatique. Selon le cas , la réaction peut par exemple être mise en oeuvre par action d'iode ou en présence de triphénylphosphine, par action de brome dans l'acide acétique ou encore d'iode en présence de C₆H₅I(OCOCF₃)₂, ou encore par réaction d'un réactif halogéné électrophile tel que le N-fluorosulfonylimide en présence d'une base forte. De tels réactifs sont connus de l'homme du métier et des exemples figurent ci-après dans la partie expérimentale.

La réaction de thioalkylation peut être effectuée par la mise en oeuvre d'un réactif tel que le méthylthiosulfonate de méthyle en présence d'une base forte, donc par une réaction de nature électrophile.

La réaction de carbamoylation peut être réalisée par la mis en oeuvre d'un chloroformiate puis d'une amine ou, le cas échéant, d'ammoniac.

L'introduction d'un groupe azido peut être effectuée par exemple par action d 'azoture de sodium sur un intermédiaire de type mésylate.

La réduction d'un groupe azide peut être effectuée par action de trialkyl ou triarylphosphine.

La réaction de couplages d'halogènes aromatiques avec des dérivés de l'étain est effectuée par une méthode dite de Stille qui consiste à former au départ d'un halogénure aromatique un dérivé alkénylé puis à réduire l'alkényle en alkyle, par exemple par l'hydrogène en présence d'un catalyseur tel que le palladium sur charbon. Une illustration est donnée dans la partie expérimentale.

La dihydroxylation de double liaison carbone-carbone est effectuée notamment par action de tétroxide d'osmium.

Le clivage des diols est de préférence réalisé par le périodate de sodium.

L'introduction d'un cyano est réalisée par substitution nucléophile à l'aide d'un cyanure alcalin.

Des illustrations de ces réactions figurent ci-après dans la partie expérimentale.

La séparation des énantiomères et diastéréoisomères peut être réalisée selon les techniques connues de l'homme du métier, notamment la chromatographie.

Outre via les procédés décrits précédemment, des composés de formule (I) peuvent bien entendu être obtenus par des méthodes qui utilisent au départ un composé de formule (II) dans laquelle R'₁, R'₂, R₃, R₄ et HZ ont les valeurs qui conduisent directement (sans transformation) à celles des composés que l'on souhaite préparer. Le cas échéant, celles de ces valeurs qui renfermeraient des fonctions réactives telles que mentionnées plus haut sont alors protégées, la déprotection intervenant à l'issue de l'étape de cyclisation b ou à tout autre moment opportun dans la synthèse. Les protections et déprotections sont alors réalisées comme décrit ci-dessus.

De telles méthodes sont fournies ci-après dans la partie expérimentale.

Le composé de formule (II) dans laquelle ZH représente un groupement HO-(CH₂)ₙ"- ou HNR'₈-(CH₂)ₙ"- dans lequel n " est égal à O, ou un groupement HNR'₈-O-, peut être obtenu par un procédé selon lequel on traite un composé de formule (IV) : 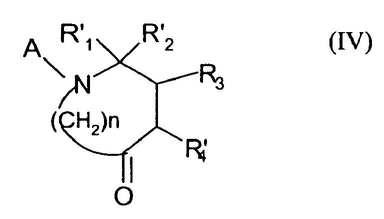 dans laquelle R'₁, R'₂, R₃, R'₄ et n sont définis comme précédemment, et A représente un atome d'hydrogène ou un groupement protecteur de l'azote, par un agent de réduction, pour obtenir un composé de formule (V) : 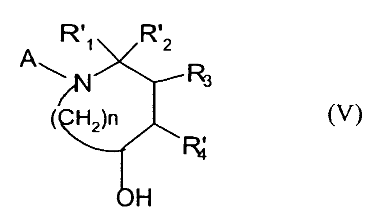 dans laquelle A, R'₁, R'₂, R₃, R'₄ et n conservent leur signification précitée, dans lequel, le cas échéant, l'on remplace le groupement OH par un groupe partant, pour obtenir un composé de formule (VI) : 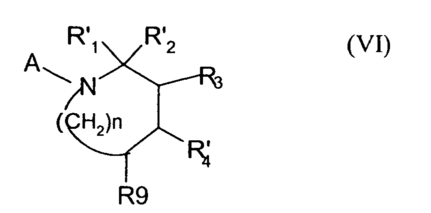 dans laquelle A, R'₁, R₃, R'₄ et n conservent leur signification précitée et R9 représente un groupe partant, que l'on traite par un composé de formule Z₁H₂ dans laquelle Z₁ représente un groupement divalent - NR'₈- ou -ONR'₈-, R'₈ conservant la signification précitée, puis, le cas échéant, par un agent de déprotection de l'atome d'azote approprié.

Le composé de formule (II) dans laquelle ZH représente un groupement NHR'₈-(CH₂)"ₙ- dans lequel n" est égal à 0 peut être obtenu par un procédé selon lequel on traite un composé de formule (IV) telle que définie précédemment, par un composé de formule H₂NR'₈, pour obtenir un composé de formule (VII) : 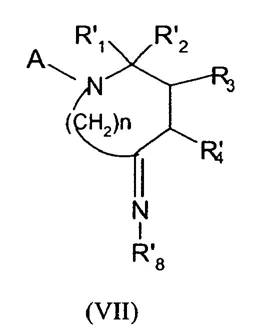 dans laquelle A, R'₁, R'₂, R₃, R'₄, n et R'₈ sont définis comme précédemment, que l'on fait réagir avec un agent de réduction pour obtenir un composé de formule (VIII) : 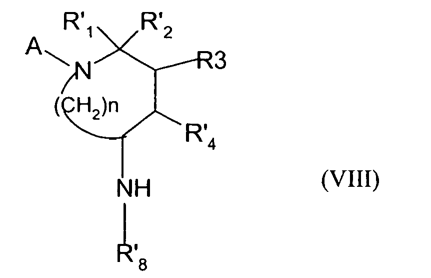 dans laquelle A, R'₁, R'₂, R₃, R'₄, n" et R'₈ sont définis comme précédemment, que l'on traite, le cas échéant, par un agent de déprotection de l'atome d'azote approprié.

Les composés de formule (II) dans laquelle ZH représente un groupement HO-(CH₂)ₙ" dans lequel n" est égal à 1 peuvent être obtenus selon les méthodes décrites par exemple par S. Shiotani et al. Chem. Pharm. Bull. 15(1)88-93 (1967) (composé "IV" p. 89) ou encore par N. Itoh. Chem. Pharm. Bull 16 (3)455-470 (1968) (composé "XVIII" p. 461) en utilisant un composé de départ approprié. Les composés de formule (II) dans laquelle ZH représente un groupement NHR'₈-(CH₂)ₙ" dans lequel n" est égal à 1 peuvent être obtenus au départ des composés ci-dessus par un procédé identique à celui qui est décrit plus haut pour la préparation des composés dans lesquels n" = 0.

Le groupement protecteur de l'azote est notamment l'un de ceux qui sont cités plus haut.

L'agent de réduction est notamment un borohydrure alcalin.

Le groupe partant est notamment un sulfonate, par exemple un mésylate ou un tosylate, obtenu par action de chlorure de sulfonyle correspondant en présence d'une base, ou un halogène, plus particulièrement un chlore, un brome ou un iode, obtenu par exemple par action du chlorure de thionyle ou de P(C₆H₅)₃CBr₄ ou PBr₃ ou, dans le cas d'un atome d'iode, par action d'un iodure alcalin sur un sulfonate.

L'agent de déprotection est notamment l'un de ceux mentionnés plus haut.

L'agent de réduction que l'on fait agir sur le composé de formule (VII) est notamment un cyano ou un acétoxyborohydrure de sodium.

Les produits de formule générale (I) possèdent une très bonne activité antibiotique sur les bactéries gram(+) telles que les staphylocoques. Leur efficacité sur les bactéries gram (-) notamment sur les entérobactéries est particulièrement notable.

Ces propriétés rendent aptes lesdits produits ainsi que leurs sels d'acides et de bases pharmaceutiquement acceptables à être utilisés comme médicaments et notamment comme médicaments antibiotiques dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

Parmi ces médicaments, on peut plus particulièrement citer les produits de formule (I) telle que décrite ci-dessus dans lesquels n est égal à 1 ainsi que ceux dans lesquels R₂ est un atome d'hydrogène, et tout particulièrement les produits de formule (I) dans lesquels R₃ et R₄ forment ensemble un phényle ou un hétérocycle, éventuellement substitué, tel que défini précédemment et notamment un phényle ou un hétérocycle choisi dans le groupe constitué par thiényle, imidazolyle, furyle, pyrazolyle et triazolyle, éventuellement substitué.

Parmi, ces derniers, on cite en particulier ceux dans lesquels R₁ est choisi dans le groupe constitué par l'atome d'hydrogène et les groupements COOCH₃, COOC₂H₅, CONH₂, CONHCH₃, CONHCH₂-phényl et CONHCH₂-pyridyl.

Parmi les composés de formule (I), on peut encore citer, à titre de médicaments, les produits de formule (I) dans lesquels X représente un groupement divalent -CO-B dans lequel B représente un groupement -NR₈-(CH2)ₙ"- tel que défini plus haut, dans lequel n" est égal à O.

Parmi ces derniers on peut citer en particulier, ceux dans lesquels R8 est un groupement Y₁ ou OY₁, dans lequel Y₁ est choisi parmi les groupements SO₂R, SO₂NHCOR, SO₂NHCOOR, SO₂NHCONHR et SO₃H et R tel que défini plus haut ; ou bien ceux dans lesquels le groupement R₈ est choisi dans le groupe constitué par l'atome d'hydrogène et les groupements hydroxy, CO-phényl, O-allyl, OPO₃H, OPO₃-benzyl, OCH₂COOH et O-benzyl.

Parmi les composés de formule (I), on peut tout particulièrement citer, à titre de médicaments, les composés dont les noms suivent:
- le sel de sodium de trans-3-oxo-4-(sulfooxy)-2,3,4,5-tétrahydro-2,5-méthano-1H-2,4-benzodiazépine-1-carboxamide;
- le sel de sodium de 3-méthyl-5-(sulfooxy)-5,6,7,8-tétrahydro-4,7-méthano-4H-[1,2,3]-triazolo[4,5-e][1,3]diazépine-6(3H)-one ;
- le sel de sodium de trans-1-méthyl-6-oxo-5-(sulfooxy)-4,5,6,8-tétrahydro-4,7-méthano-1H-pyrazolo[3,4-e][1,3]diazépine-8(7H)-carboxamide;
- le sel de sodium de trans-N-méthyl-3-oxo-4-(sulfooxy)-2,3,4,5-tétrahydro-2,5-méthano-1H-2,4-benzodiazépine-1-carboxamide;
- le sel de sodium de 5-(sulfooxy)-5,6,7,8-tétrahydro-4,7-méthano-4H-furo[2,3-e][1,3]diazépine-6-one;
- le sel de pyridinium de 1-propyl-5-(sulfooxy)-4,5,7,8-tétrahydro-4,7-méthano-imidazo[4,5-e][1,3]diazépine-6(1H)-one;
- le sel de sodium de trans-6-oxo-5-(sulfooxy)-5,6,7,8-tétrahydro-4,7-méthano-4H-furo[2,3-e][1,3]diazépine-8-carboxylate de méthyle ;
- le sel de sodium de 1-méthyl-5-(sulfooxy)-5,6,7,8-tétrahydro-4,7-méthano-4H-pyrazolo[3,4-e][1,3]diazépine-6(1H)-one;
- le sel de sodium de trans-3-oxo-N-(4-pyridinylméthyl)-4-(sulfooxy)-2,3,4,5-tétrahydro-2,5-méthano-1H-2,4-benzodiazépine-1-carboxamide;
- le sel de sodium de trans-6-oxo-5-(sulfooxy)-5,6,7,8-tétrahydro-4,7-méthano-4H-thiéno[2,3-e][1,3]diazépine-8-carboxamide;
- le sel de sodium de trans-6-oxo-5-(sulfooxy)-5,6,7,8-tétrahydro-4,7-méthano-4H-furo[2,3-e][1,3]diazépine-8-carboxamide.
- Le sel de sodium de trans-1,2,3,5-tétrahydro-8-hydroxy-3-oxo-2-(sulfooxy)-1,4-méthano-4*H*-2,4-benzodiazépine-5-carboxamide
- Le sel de sodium de trans-7-(acétylamino)-1,2,3,5-tétrahydro-8-hydroxy-3-oxo-2-(sulfooxy)-1,4-méthano-4*H*-2,4-benzodiazépine-5-carboxamide
- Le sel de sodium de trans-1,5-dihydro-5-(hydroxyméthyl)-2-(sulfooxy)-1,4-méthano-4*H*-2,4-benzodiazépin-3(2*H*)-one
- Le sel de sodium de trans-4,5,6,8-tétrahydro-N-méthyl-6-oxo-5-(sulfooxy)-4,7-méthano-7H-thiéno[2,3-e][1,3]diazépine-8-carboxamide
- Le sel de sodium de 7,8-dihydro-7-(sulfooxy)-5,8-méthano-5*H*-thiéno[2,3-*e*] [1,3]diazépin-6(4*H*)-one
- Le sel de triéthylammonium de trans-2-bromo-4,5,6,8-tétrahydro-6-oxo-5-(sulfooxy)-4,7-méthano-7*H*-thiéno[2,3-*e*][1,3]diazépine-8-carboxamide

Les composés de formule (I) peuvent être utilisés pour préparer des compositions pharmaceutiques renfermant comme principe actif, au moins un desdits composés tels que définis ci-dessus.

Ces compositions peuvent être administrées par voie buccale, rectale, parentérale, notamment intramusculaire, ou par voie locale en application topique sur la peau et les muqueuses.

Ces compositions peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1 ou encore comprise entre 0,25 g et 10 g par jour par voie intramusculaire ou intraveineuse.

Les produits de formule (I) peuvent également être utilisés comme désinfectants des instruments chirurgicaux.

L'invention a pour objet, à titre de produits industriels nouveaux et notamment à titre de produits intermédiaires nécessaires à la préparation des produits de formule (I):
- les produits de formule (III) telle que définie précédemment ainsi que leurs sels avec les acides et notamment leurs chlorhydrates et trifluoroacétates, à l'exclusion du composé dans lequel n=1, R'₄ représente un atome d'hydrogène R'₁ et R₃ forment ensemble un phényle, X₁ représente CO-X₃ avec X₃=O-tBu et X₂ représente HNR'₈-(CH₂)_{n"}- avec R'₈=CH₃ et n" = 0, notamment les produits de formule (III) telle que définie précédemment dans laquelle lorsque R'₄ représente un atome d'hydrogène et n=1 , R'₁ et R₃ forment ensemble un hétérocycle éventuellement substitué tel que définit pour R₃ et R₄ ainsi que leurs sels avec les acides et notamment leurs chlorhydrates et trifluoroacétates ;
- les produits de formule (II) telle que définie précédemment ainsi que leurs sels avec les acides et notamment leurs chlorhydrates et trifluoroacétates, ainsi que
- les produits de formules (IV), (V), (VI), (VII) et (VIII) telles que définies précédemment, ainsi que leurs sels avec un acide et notamment leurs chlorhydrates et trifluoroacétates.

Les produits de formule (IV) sont préparables par exemple selon des méthodes fournies ci-après dans la partie expérimentale.

Les exemples suivants illustrent l'invention, sans toutefois en limiter la portée.

### EXEMPLES

Dans la description qui précède ainsi que dans les exemples qui suivent les abréviations suivantes ont été &utilisées:
DEAD : azo-dicarboxylate de diéthyle
TEA : triéthylamine
DMAP : 4-diméthylamino-pyridine
EDCI : 1-(3-diméthylamino-propyl)-3-éthylcarbo-diimide chlorhydrate
THF : tétrahydrofuranne
AcOEt : acétate d'éthyle
DMF : N,N-diméthylformamide
AIBN : 2,2'-azo-bis-isobutyronitrile
M : masse molaire moléculaire
SM : spectrométrie de masse
EI: impact électronique
SIMS : secondary ion mass spectrometry
FAB : fast atom bombardement
BOP : benzotriazol-1-yloxytripyrolidino-phosphonium hexafluorophosphate
HOBt: 1-hydroxybenzotriazole hydrate
DBU : diazabicycloundécène
(BOC)₂O : dicarbonate de t-butyle
NaBH₃CN: cyanoborohydrure de sodium
DMSO : diméthylsulfoxyde
DIEA : diisopropylethyldiamine
CIMEM : chlorure de 2-méthoxyéthoxyméthyle
TMSCN : cyanure de triméthylsilyle
BOC-ON : 2-(terbutoxycarbonyloxyimino)-2-phénylacétonitrile

### Exemple 1

### 3-benzoyl-1,3,4,5-tétrahydro-1,4-méthano-2H-1,3-benzodiazépin-2-one

### Stade A

On dissout 50 mg (0,19 mmole) de N-(1,2,3,4-tétrahydro-3-quinoléinyl)-benzamide (décrit dans Chem. Pharm. Bull., 12(6), 647-651, (1964)) dans 2 ml de dichlorométhane et on ajoute 25 µl de TEA. On refroidit à 0°C sous azote, puis on ajoute 22 µl de diphosgène.

On dilue ensuite dans le dichlorométhane, puis on lave à l'acide tartrique à 10%, on récupère la phase organique et on évapore le solvant sous pression réduite.

On purifie par chromatographie sur silice, en éluant avec du dichlorométhane puis avec un mélange dichlorométhane/AcOEt 98/2.

On recueille ainsi 46 mg du produit de formule brute C₁₇H₁₆N₂O₂Cl (M = 315,5 g). Le rendement correspondant est de 76%.

### Stade B

On dissout sous atmosphère d'argon 119 mg (0,38 mmole) du composé obtenu au stade A dans 5 ml de THF anhydre et on refroidit sous argon à -78°C.

Puis, on ajoute 380 µl d'une solution de bis(triméthylsilyl) amidure de lithium 1 M dans le THF. On laisse réagir à -78°C pendant 15 minutes.

On dilue ensuite avec de l'AcOEt, on lave avec une solution aqueuse d'acide tartrique à 10%, on sèche sur sulfate de sodium, puis avec une solution saturée de chlorure de sodium.

On sépare la phase organique et on évapore le solvant sous pression réduite.

On obtient un produit brut que l'on chromatographie sur silice en éluant au dichlorométhane, puis avec un mélange dichlorométhane/AcOEt 98/2.

On recueille ainsi 94 mg de produit que l'on purifie à nouveau par chromatographie sur silice en éluant avec un mélange dichlorométhane/toluène 50/50 puis seulement avec du dichlorométhane.

On récupère ainsi 67 mg du produit attendu, de produit de formule brute C₁₇H₁₄N₂O₂ (M = 278,31 g).

Le rendement correspondant est de 63%.

### Spectre RMN du proton

Dans le CDCl₃, à 300 MHz, déplacements chimiques et multiplicité : 3,16 (dd), 3,21 (d), 3,49 (d) et 3,83 (ddd) : CH-CH₂-N et CH-CH₂-C=; 4,94 (td) : CH-N; 7,40 (d), 7,50 (tt) et 7,61 (d): CO-C₆H₅; 7,13 à 7,32 (m): C₆H₄ (aromatiques).
SM (EI) m/z : [M]⁺ = 278, 130, 105.

### Exemple 2

### 4-benzoyl-1,2,4,5-tétrahydro-2, 5-méthano-3 H-2,4-benzodiazépin-3-one

### Stade A

On fait réagir de manière analogue à ce qui est indiqué au stade A de l'Exemple 1, 288 mg (1 mmole) de chlorhydrate de N-(1,2,3,4-tétrahydro-4-isoquinoléinyl)-benzamide (décrit dans Yakugaku Zasshi, 87, 547, (1967)) avec 280µl de TEA et 60 µl de diphosgène.

On recueille ainsi 132 mg de chlorure de 4-(benzoylamino)-3,4-dihydro-2(1H)-isoquinoléinecarbonyle (M = 314,5 g). Le rendement correspondant est de 42%.

### Stade B

On fait réagir comme indiqué au stade B de l'Exemple 1, 127 mg (0,4 mmole) du composé obtenu au stade A avec 400 µl d'une solution de bis(triméthylsilyl) amidure de lithium.

On obtient 103 mg d'un produit brut que l'on chromatographie sur silice en éluant au dichlorométhane.

On récupère ainsi 41 mg de produit de formule brute C₁₇H₁₄N₂O₂ (M = 278,31 g).

Le rendement correspondant est de 37%.

### Spectre RMN du proton

Dans le CDCl₃, à 300 MHz, déplacements chimiques et multiplicité :

3,34 (d) et 3,90 (dd) : CO-CH₂-CH; 4,49 (AB) : CO-N-CH₂-C₆H₄; 5,24 (d): CO-CH₂-CH; 7,62 (m) : 2H aromatiques en ortho de CO ; 7,03 (dl) et 7,22 à 7,56 (m) : 7H aromatiques.
SM (SIMS) m/z : [M]⁺ = 279.

### Exemple 3

### Sel de 1-propényltriphénylphosphonium de trans-3-oxo-4-(sulfooxy)-2,3,4,5-tétra-hydro-2,5-méthano-1H-2,4-benzodiazépine-1-carboxylate de méthyle

### Stade A

Dans un ballon, on introduit 43,0 g de chlorhydrate de alpha-amino-benzèneacétate de méthyle C₉H₁₁ClNO₂ (M = 165,19 g, décrit dans J. Med. Chem., 26, 1267-1277, (1983)), 35,9 g de carbonate de potassium et 430 ml de DMF.

On ajoute ensuite 65,6 ml de bromoacétate de tertiobutyle et on chauffe pendant 5 heures 30 à 50°C.

On filtre l'insoluble et on évapore le solvant sous pression réduite.

On obtient ainsi 101,4 g d'une huile que l'on purifie par chromatographie sur silice en éluant avec un mélange dichlorométhane/AcOEt 95/5.

On recueille 60,6 g d'alpha-[[[(1,1-diméthyléthoxy)carbonyl]méthyl]amino]-benzèneacétate de méthyle de formule brute C₁₅H₂₁NO₄ (M = 279,34 g).

Le rendement correspondant est de 83,4%.

### Stade B

Dans un ballon placé sous atmosphère d'argon, on introduit 61,9 g (0,22 moles) du produit obtenu au stade A, 620 ml de THF anhydre, 50 ml de diisopropyléthylamine.

On refroidit vers 0-5°C, puis on ajoute 20,6 ml de chloroformiate de méthyle. On laisse en contact 1 heure 30 à 20°C.

On dilue ensuite à l'AcOEt, puis on lave avec une solution aqueuse d'acide tartrique à 10% et à l'eau déminéralisée.

On sèche ensuite la phase organique sur du sulfate de magnésium, on filtre et on évapore le solvant sous pression réduite.

On obtient ainsi 72,8 g de alpha-[[[(1,1-diméthyléthoxy)carbonyl]-méthyl](méthoxycarbonyl)amino]-benzèneacétate de méthyle de formule brute C₁₇H₂₃NO₆ (M = 337,37 g).

Le rendement correspondant est de 97%.

### Stade C

Dans un ballon, on introduit 72,8 g du produit obtenu au stade B puis on refroidit vers 0-5°C et on ajoute 730 ml de mélange acide trifluoroacétique/dichlorométhane 1/1.

On laisse en contact à 20°C pendant 1 heure.

On évapore les solvants sous pression réduite.

On obtient ainsi 60,7 g d' alpha-[(carboxyméthyl)(méthoxycarbonyl)amino]-benzèneacétate de méthyle de formule brute C₁₃H₁₅NO₆ (M = 281,27 g) sous la forme d'une huile. Le rendement est quantitatif.

### Stade D

Dans un ballon équipé d'une agitation magnétique, d'un réfrigérant et d'une garde de chlorure de calcium, on introduit 60,7 g de l'acide obtenu au stade C puis 730 ml de SOCl₂.

On chauffe à 70°C et on maintient pendant 4 heures.

On évapore à sec sous pression réduite.

On obtient ainsi 54,8 g de chlorhydrate de 2,5-dioxo-alpha-phényl-3-oxazolidineacétate de méthyle de formule brute C₁₂H₁₁NO₅ (M = 249 g). Le rendement est quantitatif.

### Stade E

Dans un ballon placé sous atmosphère d'azote, on introduit 54,8 g de chlorure d'acide obtenu au stade D (0,22 moles) et 500 ml de dichlorométhane.

On ajoute ensuite 105 g de chlorure d'aluminium.

On garde sous agitation pendant une nuit à 20°C, puis on dilue au dichlorométhane et amène à pH 8-9 par ajout de soude 2N en refroidissant. On dilue ensuite avec 1l d'eau et 1l de dichlorométhane.

On décante, on extrait à plusieurs reprises au dichlorométhane, on rassemble les phases organiques et on les sèche sur sulfate de sodium.

On évapore ensuite le solvant sous pression réduite.

On obtient ainsi 35,6 g de 4-oxo-1,2,3,4-tétrahydro-1-isoquinoléinecarboxylate de méthyle de formule brute C₁₁H₁₁NO₃ (M = 205,22 g).

Le rendement correspondant est de 88%.

### Stade F

Dans un ballon, on introduit 35,6 g du produit obtenu au stade E (0,173 moles) et 360 ml de THF.

On refroidit à 0°C, puis on ajoute 41,5 g de (BOC)₂O et on laisse réagir pendant 2 heures 30 à 20°C.

On dilue ensuite à l'AcOEt, lave avec une solution aqueuse d'acide tartrique à 10%, puis à l'eau déminéralisée.

On sèche ensuite la phase organique sur du sulfate de magnésium.

On évapore le solvant sous pression réduite et on purifie ensuite par chromatographie sur silice.

On obtient ainsi 48,9 g de 3,4-dihydro-4-oxo-1,2(1H)-isoquinoléinedicarboxylate de 2-(1,1-diméthyléthyle) et de 1-méthyle de formule brute C₁₆H₁₉NO₅ (M = 305,33 g)

Le rendement correspondant est de 92%.

### Stade G

Dans un ballon placé sous atmosphère d'azote et refroidi par un bain de glace, on introduit 20,5 g du produit obtenu au stade F (67,1 mmoles) et 40 ml de méthanol.

On ajoute ensuite 2,67 g de NaBH₄.

On agite tout en laissant revenir à 20°C pendant 30 minutes.

On dilue ensuite avec 200 ml de dichlorométhane, on lave avec une solution aqueuse d'acide tartrique à 10%, puis à l'eau déminéralisée, on sèche la phase organique sur du sulfate de sodium.

On évapore le solvant sous pression réduite.

On obtient ainsi 19,8 g de cis-3,4-dihydro-4-hydroxy-1,2(1 H)-isoquinoléinedicarboxylate de 2-(1,1-diméthyléthyle) et de 1-méthyle de formule C₁₆H₂₁NO₅ (M = 307,25 g).

Le rendement correspondant est de 96%.

### Stade H

Dans un ballon placé sous atmosphère d'argon, on introduit 19,8 g du produit obtenu au stade G (64,4 mmoles) et 300 ml de dichlorométhane.

On refroidit vers 0-5°C, puis on ajoute successivement 10,8 ml de TEA et 5,3 ml de chlorure de méthane sulfonyle. On laisse revenir la température à 20°C et on garde sous agitation pendant 1 h 20 à 20°C.

On dilue au dichlorométhane, puis on lave avec une solution aqueuse d'acide tartrique à 10% et à l'eau déminéralisée. On sèche la phase organique sur du sulfate de sodium.

On évapore ensuite le solvant sous pression réduite.

On obtient ainsi 23,7 g de cis-3,4-dihydro-4-[(méthylsulfonyl)oxy]-1,2(1H)-isoquinoléinedicarboxylate de 2-(1,1-diméthyléthyle) et de 1-méthyle de formule brute C₁₇H₂₃NO₇S (M = 385,44 g).

Le rendement correspondant est de 95%.

### Stade I

Au mésylate fraîchement préparé au stade H, on ajoute 24,5 ml de O-allylhydroxylamine, puis on laisse en contact à 0-5°C pendant 72 heures.

On dilue ensuite au dichlorométhane et on lave avec une solution aqueuse d'acide tartrique à 10% puis à l'eau déminéralisée.

On sèche la phase organique sur du sulfate de sodium et on évapore le solvant sous pression réduite.

L'extrait sec obtenu est purifié par chromatographie sur silice en éluant avec un mélange dichlorométhane/AcOEt 98/2.

On recueille 12,3 g de trans-3,4-dihydro-4-[(2-propényloxy)amino]-1,2(1H)-isoquinoléinedicarboxylate de 2-(1,1-diméthyléthyle) et de 1-méthyle de formule brute C₁₉H₂₆N₂O₅ (M = 362,43 g).

Le rendement correspondant est de 55,3%.

### Stade J

On dissout 12,3 g (33,9 mmoles) du produit obtenu au stade I dans 12 ml d'AcOEt et on refroidit à 0°C.

On ajoute 74 ml de solution de chlorure d'hydrogène dans l'AcOEt à 4,6 moles/l. On laisse en contact pendant 1 heure à 20°C.

On évapore le solvant sous pression réduite puis on cristallise le produit dans l'éther éthylique.

On obtient ainsi 11,2 g de chlorhydrate de trans-4-[(2-propényloxy)amino]-1,2,3,4-tétrahydro-1-isoquinoléinecarboxylate de méthyle de formule brute C₁₄H₂₀Cl₂N₂O₃ (M = 335,23 g).

Le rendement correspondant est de 98%.

### Stade K

On place les 11,2 g (33,4 mmoles) du produit obtenu au stade J en suspension dans 500 ml de dichlorométhane.

On ajoute 67 ml de soude 1N.

On décante, on lave à l'eau déminéralisée et on sèche la phase organique sur du sulfate de sodium.

On évapore ensuite le solvant sous pression réduite.

On obtient ainsi 7,86 g de trans-4-[(2-propényloxy)amino]-1,2,3,4-tétrahydro-1-isoquinoléinecarboxylate de méthyle de formule brute C₁₄H₁₈N₂O₃ (M = 262,31 g).

Le rendement correspondant est de 90%.

### Stade L

Dans un ballon placé sous atmosphère d'argon et refroidit par un bain de glace, on introduit 7,86 g (29,9 mmole) de la diamine obtenue au stade K, 300 ml d'acétonitrile et 8,3 ml de TEA.

On agite pendant 2 minutes et on introduit ensuite 1,8 ml (14,6 mmole) de diphosgène.

On agite la solution à 20°C pendant 1 heure.

On dilue à l'AcOEt et on lave avec une solution d'acide tartrique à 10% puis à l'eau.

On sèche la phase organique sur du sulfate de magnésium et on évapore le solvant sous pression réduite.

Le produit brut est dissout dans 500 ml de dichlorométhane avec 0,45 ml de DBU. Après 10 minutes de contact, le mélange réactionnel est lavé par une solution d'acide tartrique à 10% puis à l'eau. Après évaporation du solvant sous pression réduite, on obtient 8,6 g de produit brut qui est purifié par chromatographie pour donner 6,45 g de trans-3-oxo-4-(2-propényloxy)-2,3,4,5-tétrahydro-2,5-méthano-1H-2,4-benzodiazépine-1-carboxylate de méthyle de formule brute C₁₅H₁₆N₂O₄ (M = 288,30 g).

Le rendement correspondant est de 75%.

### Stade M

Dans un ballon placé sous atmosphère d'argon, on introduit 140 mg (0,486 mmole) du produit obtenu au stade L, 1,4 ml de dichlorométhane, puis on ajoute 56 µl d'acide acétique et 280 mg de Pd[P(C₆H₅)₃]₄.

Après 15 minutes de contact, on ajoute 232 mg de complexe SO₃-pyridine en solution dans 1,4 ml de pyridine.

On laisse sous agitation à 20°C pendant 1 heure 30, puis on évapore sans chauffer sous pression réduite.

On ajoute 50 ml de dichlorométhane, puis on lave avec de l'eau. On sèche la phase organique sur sulfate de sodium, on évapore à sec sous pression réduite.

L'extrait sec obtenu est purifié par chromatographie sur silice en éluant progressivement avec un mélange dichlorométhane/acétone contenant 0,1% en volume de TEA : 100/0 puis 80/20 et 50/50.

On obtient ainsi 132 mg de sel de 1-propényltriphénylphosphonium de trans-3-oxo-4-(sulfooxy)-2,3,4,5-tétrahydro-2,5-méthano-1H-2,4-benzodiazépine-1-carboxylate de méthyle de formule brute C₃₃H₃₁N₂O₇PS (M = 630,66 g).

Le rendement correspondant est de 43,1 %.
IR (CHCl₃) : 1753, 1638, 1611, 1603, 1587 cm⁻¹.
SM (Electrospray négatif) m/z : [M anion]⁻ = 327
SM (Electrospray positif) m/z : [M cation]⁺ = 303

### Exemple 4

### Sel de sodium de trans-3-oxo-4-(sulfooxy)-2,3,4,5-tétrahydro-2,5-méthano-1H-2,4-benzodiazépine-1-carboxylate de méthyle

On dissout 132 mg (0,209 mmole) du sel de phosphonium obtenu au stade M de l'Exemple 3, dans 0,5 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 58 mg du sel de sodium attendu, de formule brute C₁₂H₁₁N₂O₇SNa (M = 350,28 g).

Le rendement correspondant est de 79,1%.

### Exemple 5

### Sel de sodium de 1-méthyl-5-(sulfooxy)-5,6,7,8-tétrahydro-4,7-méthano-4H-pyrazolo[3,4-e][1,3]diazépin-6(1H)-one

### Stade A

On met en suspension 12,8 g (60 mmoles) de 3,5-dioxo-1-pipéridinecarboxylate de 1,1-diméthyléthyle de formule brute C₁₀H₁₅NO₄ (préparé par un procédé analogue à celui décrit dans Heterocycles, 22, 2769-2773, (1984), en remplaçant le chloroformiate de méthyle par (BOC)₂O) dans 128 ml de toluène).

On ajoute à température ambiante 12,65 ml de N,N-diméthylformamide diméthylacétal à 95%.

On agite pendant une demi-heure à 80°C puis pendant 3 heures à 50°C.

On évapore le solvant sous pression réduite.

On obtient ainsi 18,03 g de 4-[(diméthylamino)méthylène]-3,5-dioxo-1-pipéridinecarboxylate de 1,1-diméthyléthyle de formule brute C₁₃H₂₀N₂O₄.

### Stade B

On dissout 18,03 g de la résine obtenue au stade A dans 146 ml d'éthanol absolu, puis on ajoute 3,55 ml de méthylhydrazine.

On agite pendant 3 heures 30 à température ambiante.

On évapore le solvant sous pression réduite et on purifie par chromatographie sur silice en éluant avec un mélange dichlorométhane/AcOEt 1/1.

On recueille ainsi 12,47 g de 4,7-dihydro-1-méthyl-4-oxo-1H-pyrazolo[3,4-c]pyridine-6(5H)-carboxylate de 1,1-diméthyléthyle de formule brute C₁₂H₁₇N₃O₃ (M= 251,29 g).

Le rendement correspondant est de 83%.

### Stade C

On introduit dans un ballon sous atmosphère d'azote 2,99 g (11,9 mmoles) de produit préparé au stade B dans 30 ml d'éthanol, 1,32 g (12,1 mmoles) de O-allylhydroxylamine et 2,9 ml de pyridine.

On laisse sous agitation pendant 1 heure, puis on dilue le milieu réactionnel dans 250 ml de dichlorométhane, on lave avec une solution d'acide tartrique à 10%, puis à l'eau.

On sèche la phase organique sur sulfate de magnésium.

On évapore le solvant sous pression réduite.

On obtient 3,46 g de 4,7-dihydro-1-méthyl-4-[(2-propényloxy)lminol-1H-pyrazolo[3,4-c]pyridine-6(5H)-carboxylate de 1,1-diméthyléthyle, de formule brute C₁₅H₂₂N₄O₃ (M = 306,37 g).

Le rendement correspondant est de 95%.

### Stade D

Dans un ballon sous atmosphère d'azote et refroidi à 0°C, on introduit 3,02 g (9,86 mmole) du produit obtenu au stade C dans 30 ml d'éthanol, quelques grains de méthyl orange et en plusieurs fois 3,52 g (62,2 mmoles) de cyanure de sodium. A chaque ajout de cyanure de sodium, on ajoute aussi une solution aqueuse d'acide chlorhydrique 2N pour maintenir le pH à 4-5.

On laisser réagir pendant 5 heures puis on ajoute une solution aqueuse d'hydrogénocarbonate de sodium et on agite pendant 15 minutes.

On extrait au dichlorométhane et on lave à l'eau.

La phase organique est séchée sur du sulfate de magnésium.

Le solvant est évaporé sous pression réduite.

Le produit obtenu est purifié par chromatographie sur silice en éluant successivement avec un mélange dichlorométhane/AcOEt 9/1, puis 8/2.

On obtient ainsi 1,49 g de 4,7-dihydro-1-méthyl-4-[(2-propényloxy)amino]-1H-pyrazolo[3,4-c]pyridine-6(5H)-carboxylate de 1,1-diméthyléthyle (M = 308,38 g).

Le rendement correspondant est de 46%.

### Stade E

On introduit dans un ballon 1,4 g (4,5 mmoles) du produit obtenu au stade B et 12 ml d'une solution de chlorure d'hydrogène en solution à 140 g/l (0,046 mole) dans l'AcOEt.

On laisse réagir pendant 30 minutes à 0°C, puis on laisse revenir à température ambiante.

On évapore le solvant sous pression réduite pour obtenir 1,5 g de chlorhydrate de 1-méthyl-N-(2-propényloxy)-4,5,6,7-tétrahydro-1H-pyrazolo[3,4-c]pyridin-4-amine.

Le rendement correspondant est quantitatif.

### Stade F

On introduit dans un ballon 980 mg (3,08 mmole) du produit obtenu au stade E et 20 ml de dichlorométhane, on ajoute 1,62 ml (9,25 mmoles) de soude.

On laisse sous agitation pendant quelques minutes, puis on évapore la phase aqueuse sous pression réduite et on dissout le résidu dans 40ml d'un mélange dichlorométhane/éthanol 9/1.

Après filtration et rinçage au dichlorométhane, évaporation du solvant sous pression réduite, on obtient 741 mg de produit.

Le rendement correspondant est de 98%.

### Stade G

On introduit dans un ballon placé sous atmosphère d'azote et refroidit à 0°C, 100 mg (0,43 mmole) du produit obtenu au stade F dans 40 ml d'acétonitrile et 300 µl (2,08 mmoles) de TEA et 30 µl (0,24 mmole) de diphosgène.

On laisse revenir à la température ambiante et réagir pendant 30 minutes.

On dilue ensuite à l'AcOEt et on lave à l'eau.

On sèche la phase organique sur sulfate de magnésium.

On évapore le solvant sous pression réduite.

Le résidu est purifié par chromatographie sur silice en éluant avec un mélange dichlorométhane/AcOEt 1/1 contenant 0,1% en volume de TEA.

On recueille ainsi 63,7 mg de 1-méthyl-5-(propényloxy)-5,6,7,8-tétrahydro-4,7-méthano-4H-pyrazolo[3,4-e][1,3]diazépin-6-one.

Le rendement correspondant est de 66%.

### Stade H

On introduit dans un ballon placé sous atmosphère d'azote et à température ambiante, 86 mg (0,367 mmole) du produit obtenu au stade G dans 2 ml de dichlorométhane, 42 µl d'acide acétique et 225 mg de Pd[P(Ph)₃]₄.

Après 15 minutes de réaction, on ajoute 1 ml de pyridine, puis 275 mg (1,723 mmole) de complexe SO₃-pyridine.

On laisse réagir pendant 4 heures, puis dilue au dichlorométhane.

On évapore le solvant sous pression réduite, on reprend au dichlorométhane, on lave à l'eau.

On sèche la phase organique sur du sulfate de magnésium.

On évapore le solvant sous pression réduite. On purifie ensuite le résidu par chromatographie sur silice en éluant avec un mélange dichlorométhane/acétone 3/7 contenant 0,1 % de TEA.

On obtient ainsi 99 mg de sel de 1-propényltriphénylphosphonium de 1-méthyl-5-(sulfooxy)-5,6,7,8-tétrahydro-4,7-méthano-4H-pyrazolo[3,4-e] [1,3]diazépin-6(1H)-one.

Le rendement correspondant est de 46%.

### Stade I

On dissout le produit obtenu au stade H dans 1 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 42 mg de sel de sodium attendu, de formule brute C₈H₉N₄O₅SNa (M = 296,24 g).

Le rendement correspondant est de 84%.

### Spectre RMN du proton

Dans D₂O, à 300 MHz, déplacements chimiques et multiplicité :
3,33 (d) et 3,78 (dd) : N-CH₂-CH ; 3,69 (s) : NCH₃; 4,52 (s) : N-CH₂-C=; 4,93 (d) : N-CH-C=; 7,58 (s) : N=CH.

### Exemple 6

### Trans-3-oxo-4-(phénylméthoxy)-2,3,4,5-tétrahydro-2, 5-méthano-1 H-2,4-benzodiazépine-1-carboxylate de méthyle

### Stade A

Dans un ballon, on introduit 50,0 g (0,163 moles) de la cétone obtenue au stade F de l'Exemple 3, de formule brute C₁₆H₁₉NO₅, et 500 ml d'éthanol.

On ajoute ensuite 28,7 g de O-benzylhydroxylamine puis 40 ml de pyridine.

On agite pendant 1 heure à 20°C.

On évapore ensuite le solvant sous pression réduite puis on dilue avec du dichlorométhane, on lave avec une solution aqueuse d'acide tartrique à 10%, puis à l'eau déminéralisée.

On sèche ensuite la phase organique sur du sulfate de sodium et on évapore le solvant sous pression réduite.

Le produit brut obtenu est ensuite chromatographié sur silice en éluant avec un mélange dichlorométhane/AcOEt 95/5 pour donner 65,4 g de 3,4-dihydro-4-[(phénylméthoxy)imino]-1,2(1H)-isoquinoléinedicarboxylate de 2-(1,1-diméthyléthyle) et de 1-méthyle de formule brute C₂₃H₂₆N₂O₅ (M = 410,47 g).

Le rendement correspondant est de 97,3%.

### Stade B

On introduit 65,4 g (0,160 mole) du produit obtenu au stade A dans 670 ml de méthanol. 1.

On refroidit vers 0-5°C, puis on ajoute 108 g de NaBH₃CN et 103 ml d'éthérate de trifluorure de bore dans l'éther éthylique.

On agite pendant 30 minutes en maintenant à 0-5°C, puis on laisse la température revenir à 20°C et on agite à cette température pendant 30 minutes.

On verse ensuite le milieu réactionnel dans de l'eau saturée en hydrogénocarbonate de sodium. On agite pendant 45 minutes, puis on décante, on lave la phase organique à l'eau déminéralisée et on la sèche sur du sulfate de sodium.

On évapore ensuite le solvant sous pression réduite pour obtenir 67,9 g de produit brut que l'on purifie par chromatographie sur silice en éluant avec du dichlorométhane contenant 2% d'acétone.

On recueille ainsi 8,62 g de produit de formule brute C₂₃H₂₈N₂O₅ (M = 412,49 g) et 54,5 g d'un mélange de produit de départ et du produit attendu. Cette dernière fraction est à nouveau traitée dans les mêmes conditions que précédemment avec 50 g de cyanoborohydrure de sodium. On obtient ainsi au total 52,0 g de 3,4-dihydro-4-[(phénylméthoxy)amino]-1,2(1H)-isoquinoléinedicarboxylate de 2-(1,1-diméthyléthyle) et de 1-méthyle.

Le rendement correspondant est de 77,4%.

### Stade C

Dans une solution contenant 52 g de l'amine obtenu au stade B et 150 ml d'AcOEt, que l'on a refroidit à 0°C, on introduit lentement 300 ml d'AcOEt saturé par du HCl gazeux. On laisse revenir à la température ambiante et on poursuit l'agitation pendant 1 heure. On évapore les solvants sous pression réduite, puis on dilue à l'eau, on neutralise par 40 ml d'ammoniaque et on extrait au dichlorométhane après avoir saturé la phase aqueuse par du chlorure de sodium. On récupère ainsi 38,97 g d'une huile jaune que l'on purifie par chromatographie sur silice, en éluant avec un mélange dichlorométhane/AcOEt 95/5.

On obtient 34,48 g de 3,4-dihydro-4-[(phénylméthoxy)amino]-1,2(1H)-isoquinoléinecarboxylate de méthyle de formule brute C₁₈H₂₀N₂O₃ (M = 312,37 g).

Le rendement correspondant est de 87,5%.

### Stade D

On refroidit à 0°C une solution contenant 34,48 g du produit obtenu au stade C, 1,3 litre d'acétonitrile, 32 ml de TEA, puis on introduit en 10 minutes, 6,6 ml (0,055 mole) de diphosgène.

On laisse revenir à température ambiante et on agite pendant 1 heure. On dilue le mélange réactionnel par 1 1 d'acétate d'éthyle puis on lave la phase organique trois fois avec 500 ml d'eau.

On évapore les solvants sous pression réduite, puis on reprend le résidu dans 200 ml de dichlorométhane et 17 ml de DBU.

On agite pendant 45 minutes et on lave 3 fois à l'eau la phase organique.

On évapore sous pression réduite.

On obtient ainsi une mousse beige que l'on purifie par chromatographie. La fraction pure est reprise dans l'éther. Les liqueurs mères obtenues sont repurifiées par chromatographie.

On obtient 21,975 g de cristaux blancs du produit attendu de formule brute C₁₉H₁₈N₂O₄(M = 338,37 g).

Le rendement correspondant est de 68%.
Spectre RMN du proton

Dans CDCl₃, à 300 MHz, déplacements chimiques et multiplicité :
3,51 (dd) et 3,64 (d) : N-CH₂-CH ; 3,79 (d) : N-CH₂-CH ; 3,82(s) : CH₃-OC(O)-CH ; 5,15 (s) : CH₃-O-C(O)-CH; 4,87 et 4,99 (AB) : O-CH₂-C₆H₅ 6,98 (dl), 7,21 (td) et de 7,30 à 7,50 (m) : 9 H aromatiques.

### Exemple 7

### Sel de sodium de trans-3-oxo-N-(phénylméthyl)-4-(sulfooxy)-2,3,4,5-tétrahydro-2,5-méthano-1 H-2,4-benzodiazépine-1-carboxamide

### Stade A

Dans un ballon équipé d'un agitateur magnétique, on introduit 2,7 g (9,36 mmoles) de l'ester méthylique obtenu au stade L de l'Exemple 3 et 54 ml de mélange dioxane/eau 50/50.

On ajoute ensuite 9,4 ml de soude 1N et on agite pendant une heure à 20°C.

On dilue ensuite à l'AcOEt et on acidifie avec une solution aqueuse saturée en NaH₂P0₄. On décante et on reextrait à l'AcOEt. On rassemble les phases organiques, on les lave à l'eau et on les sèche sur du sulfate de magnésium.

On évapore le solvant sous pression réduite.

On obtient ainsi 2,11 g d'acide trans-3-oxo-4-(2-propényloxy)-2,3,4,5-tétrahydro-2,5-méthano-1H-2,4-benzodiazépine-1-carboxylique de formule brute C₁₄H₁₄N₂O₄ (M = 274,28 g).

Le rendement correspondant est de 82,2%.

### Stade B

Dans un ballon placé sous atmosphère d'argon, on mélange 152 mg (0,554 mmoles) de l'acide obtenu à l'étape A et 5 ml de dichlorométhane.

On refroidit à -20°C et on ajoute 71 µl de 2,6-lutidine et 78 µl de chloroformiate d'isobutyle. Après 5 minutes de contact, on ajoute 67 µl de benzylamine et on laisse réagir pendant 30 minutes à -20°C, puis on laisser remonter la température jusqu'à 20°C. On continue à agiter pendant 1 heure et demie, puis on dilue avec du dichlorométhane, on lave avec une solution aqueuse d'acide tartrique à 10%, puis avec un tampon phosphate à pH 7, enfin avec une solution de chlorure de sodium saturée.

On sèche la phase organique sur sulfate de magnésium et on évapore le solvant sous pression réduite.

On obtient ainsi 215 mg de produit brut que l'on purifie sur silice en éluant avec un mélange dichlorométhane/AcOEt 98/2 contenant 0,1% de TEA, puis avec un mélange dichlorométhane/ AcOEt 95/5 contenant également 0,1% de TEA.

On recueille 146 mg de trans-3-oxo-N-(phénylméthyl)-4-(2-propényloxy)-2,3,4,5-tétrahydro-2,5-méthano-1H-2,4-benzodiazépine-1-carboxamide de formule brute C₂₁H₂₁N₃O₃ (M = 363,42 g).

Le rendement correspondant est de 72%.

### Stade C

Dans un ballon placé sous atmosphère d'argon, on dissout 146 mg (0,4 mmole) du produit obtenu au stade B dans 1,5 ml de dichlorométhane.

On ajoute à 20°C, 46 µl d'acide acétique et 232 mg de Pd[P(C₆H₅)₃]₄. On agite pendant 15 minutes à 20°C.

On ajoute ensuite 200 mg de complexe SO₃-pyridine en solution dans 1,4 ml de pyridine. On laisse sous agitation à 20°C pendant 5 heures.

On évapore le solvant sous pression réduite et on reprend avec 50 ml de dichlorométhane. On lave à l'eau et on sèche la phase organique sur du sulfate de magnésium.

On évapore le solvant sous pression réduite.

On obtient ainsi 400 mg du produit brut que l'on purifie par chromatographie sur colonne de silice en éluant progressivement avec un mélange de dichlorométhane/acétone contenant 0,1% de TEA : 100/0 puis 80/20 et 50/50.

On recueille le sel de 1-propényltriphénylphosphonium de trans-3-oxo-N-(phénylméthyl)-4-sulfooxy)-2,3,4,5-tétrahydro-2,5-méthano- 1H-2,4-benzodiazépine-1-carboxamide que l'on dissout dans 1 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 89 mg de sel de sodium de trans-3-oxo-N-(phénylméthyl)-4-(sulfooxy)-2,3,4,5-tétrahydro-2,5-méthano-1 H-2,4-benzodiazépine-1-carboxamide, de formule brute C₁₈H₁₆N₃O₆SNa (M = 425,40 g).

Le rendement global du stade C est de 52%.

### Spectre RMN du proton

Dans le CDCl₃, à 300 MHz, déplacements chimiques et multiplicité :
3,52 (dl) et 3,79 (dl) : OC-N-CH₂ ; 4,50 (AB) : N-CH₂-C₆H₅; 5,21 (s) : N-CH-CO-N; 4,94 : CH₂-CHN; 7,30 à 7,75 : H aromatiques.
IR (CHCl₃): 1754, 1670, 1515, 1496 cm⁻¹.
SM (Electrospray négatif) m/z : [M]⁻ = 402.

### Exemple 8

### Sel de sodium de trans-3-oxo-N-(4-pyridinylméthyl)-4-(sulfooxy)-2,3,4,5-tétrahydro-2,5-méthano-1H-2,4-benzodiazépine-1-carboxamide

### Stade A

On procède comme indiqué au stade B de l'Exemple 7 avec 142,5 mg (0,52 mmole) du produit de formule brute C₁₄H₁₄N₂O₄ (M = 274,28 g) obtenu au stade A de l'Exemple 7, 67 µl de 2,6-lutidine, 72 µl de chloroformiate d'isobutyle et 58 µl d'aminométhylpyridine.

On obtient ainsi 75 mg de trans-3-oxo-4-(2-propényloxy)-N-[(4-pyridinyl)méthyl]-2,3,4,5-tétrahydro-2,5-méthano-1 H-2,4-benzodiazépine-1-carboxamide de formule brute C₂₀H₂₀N₄O₃ (M = 364,41 g).

Le rendement correspondant est de 40%.

### Stade B

Dans un ballon placé sous atmosphère d'argon, on dissout 75 mg (0,205 mmole) du produit obtenu au stade A dans 1 ml de dichlorométhane.

On ajoute à 20°C, 23 µl d'acide acétique et 120 mg de Pd[P(C₆H₅)₃]₄. On agite pendant 1 heure à 20°C.

L'hydroxyurée intermédiaire précipite. On filtre le précipité, puis on le redissout dans 4 ml de pyridine et on ajoute 100 mg du complexe SO₃-pyridine. On laisse sous agitation à 20°C pendant 4 heures.

Le milieu réactionnel est ensuite concentré sous vide.

On obtient ainsi 250 mg de sel de pyridinium brut que l'on dissout dans 12 ml d'une solution aqueuse de KH₂PO₄.

On agite pendant 15 minutes à 20°c, puis on lave trois fois avec 5 ml d'AcOEt.

On traite ensuite par 60 mg de n-Bu₄N⁺HSO₄⁻, on ajuste le pH à 5 par une solution saturée d'hydrogénocarbonate de sodium et on extrait avec 8 fois 5 ml d'AcOEt et on réunit les phases extraites, on les sèche et on évapore le solvant sous vide.

On sèche sur sulfate de magnésium et évapore le solvant sous vide.

On purifie sur silice avec comme éluant un mélange dichlorométhane/acétone 10/90 contenant 2% de TEA.

On obtient ainsi 16,8 mg de sel de 1-propényltriphénylphosphonium de trans-3-oxo-N-[(4-pyridinyl)méthyl]-4-(sulfooxy)-2,3,4,5-tétrahydro-2,5-méthano-1 H-2,4-benzodiazépine-1-carboxamide de formule brute C₃₃H₅₁N₅O₆S (M = 645,867 g).

Le sel obtenu est échangé sur 4,5 g de résine DOWEX 50WX8 sous forme Na⁺, pour donner, après lyophilisation, 7,5 mg de sel de sodium de trans-3-oxo-N-(4-pyridinylméthyl)-4-(sulfooxy)-2,3,4,5-tétrahydro-2,5-méthano-1 H-2,4-benzodiazépine-1-carboxamide de formule brute C₁₇H₁₅N₄NaO₆S (M = 426,39 g).

Le rendement correspondant est de 8,5%.
SM (Electrospray négatif) m/z : [M]⁻ = 403.

### Exemple 9

### Sel de sodium de trans-3-oxo-4-(sulfooxy)-2,3,4,5-tétrahydro-2,5-méthano-1H-2,4-benzodiazépine-1-carboxamide

A partir de 110 mg (0,40 mmole) du produit de formule brute C₁₄H₁₄N₂O₄ (M = 274,28 g) obtenu au stade A de l'Exemple 7, on procède de façon similaire à ce qui indiqué dans les stades B et C de l'Exemple 7, sauf qu'au stade B, au lieu d'utiliser de la benzylamine, on utilise 335 µl d'ammoniaque concentrée.

On obtient un sel de 1-propényltriphénylphosphonium de trans-3-oxo-4-(sulfooxy)-2,3,4,5-tétrahydro-2,5-méthano-1H-2,4-benzodiazépine-1-carboxamide, que l'on lyophilise.

On obient ainsi après lyophilisation 46 mg du sel de sodium attendu, de formule brute C₁₁H₁₀N₃O₆SNa (M = 335,27 g).

Le rendement correspondant est de 70,1%.

### Spectre RMN du proton

Dans D₂O, à 300 MHz, déplacements chimiques et multiplicité:
3,56 (dl) et 3,81 (dl) : OC-N-CH₂ ; 4,83 : CH₂-CHN; 5,19 (s) : N-ÇH-CO-N; 7,30 à 7,52 : H aromatiques.
IR (CHCl₃) : 1749, 1680, 1585 cm⁻¹.
SM (Electrospray négatif) m/z : [M]⁻ = 312.

### Exemple 10

### Trans-4-hydroxy- 3-oxo-2,3,4,5-tétrahydro-2,5-méthano-1H-2,4-benzodiazépine-1-carboxamide

### Stade A

On procède comme au stade A de l'Exemple 7 avec 676 mg (2 mmoles) de trans-3-oxo-4-(phénylméthoxy)-2,3,4,5-tétrahydro-2,5-méthano-1H-2,4-benzodiazépine-1-carboxylate de méthyle de formule brute C₁₉H₁₈N₂O₄ (M = 338,37) obtenu au stade D de l'Exemple 6.

On obtient ainsi 620 mg d'acide trans-3-oxo-4-(phénylméthoxy)-2,3,4,5-tétrahydro-2,5-méthano-1H-2,4-benzodiazépine-1-carboxylique de formule C₁₈H₁₆N₂O₄ (M = 324,34 g).

Le rendement correspondant est de 95%.

### Stade B

Dans un ballon équipé d'une agitation magnétique et sous atmosphère d'azote, on introduit les 620 mg de produit brut obtenu au stade A dans 10 ml de DMF.

On ajoute 1,27 g de BOP, puis 0,387 g de HOBt, 0,204 g de NH₄Cl et 1,33 ml de N,N diisopropyléthylamine.

Après réaction, on dilue avec de l'AcOEt et on lave avec 10 ml de HCl 0,1 N, puis avec 10 ml d'une solution saturée d'hydrogénocarbonate de sodium.

On rassemble les phases organiques, on les sèche sur du sulfate de sodium, on filtre et on élimine le solvant par évaporation sous pression réduite.

On obtient ainsi 800 mg d'un solide que l'on cristallise dans un mélange d'éther éthylique et d'acétone, puis on sèche sous pression réduite.

On récupère ainsi, 249,6 mg de trans-3-oxo-4-(phénylméthoxy)-2,3,4,5-tétrahydro-2,5-méthano-1H-2,4-benzodiazépine-1-carboxamide sous la forme d'un solide incolore de point de fusion 104-106°C, de formule brute C₁₈H₁₇N₃O₃ (M = 323,35 g).

Le rendement correspondant est de 40%.

### Stade C

Dans un ballon équipé d'une agitation magnétique, on introduit 763 mg (2,36 mmoles) du produit obtenu au stade B, 35 ml d'éthanol, 35 ml de THF et 80 mg de catalyseur Pd/C à 10% en poids.

On agite sous atmosphère d'hydrogène à une pression de 1,9 bar pendant une nuit.

On filtre ensuite le catalyseur, on lave à l'éthanol et on évapore les solvants sous pression réduite.

On cristallise le produit dans l'éther.

On obtient ainsi 550 mg du produit attendu de formule brute C₁₁H₁₁N₃O₃ (M = 233,23 g).

Le rendement correspondant est quantitatif.

### Spectre RMN du proton

Dans le DMSO d6, à 300 MHz, déplacements chimiques et multiplicité :
3,43 (dd) et 3,75 (d) : N-CH₂-OH; 4,26 (d) : N-CH₂-OH ; 4,77 (s) CH-CO-NH₂; 7,49 et 7,92 : CH-CO-NH₂ ; de 7,16 à 7,38 (m) : 4H aromatiques ; 9,73 (s1) : N-OH.
IR (CHCl₃) : 1749, 1680, 1585 cm⁻¹.
SM (Electrospray positif) m/z : [2M+Na]⁺= 489 ; [M+H]⁺ = 234, 189.

### Exemple 11

### Sel de disodium de l'acide trans-3-oxo-4-(sulfooxy)-2,3,4,5-tétrahydro-2,5-méthano-1 H-2,4-benzodiazépine-1-carboxylique

### Stade A

Dans un ballon, on introduit 155 mg d'acide trans-3-oxo-4-(phénylméthoxy)-2,3,4,5-tétrahydro-2,5-méthano-1H-2,4-benzodiazépine-1-carboxylique, de formule brute C₁₈H₁₆N₂O₄ (0,5 mmole) obtenu au terme du stade A de l'Exemple 10 en solution dans 2 ml d'éthanol, on introduit 55 mg de Pd/C à 10% en poids et on place sous atmosphère d'hydrogène à pression normale.

On laisse réagir pendant 3 heures 30.

On filtre ensuite le catalyseur et on évapore le solvant sous pression réduite.

On obtient ainsi 98 mg d'acide trans-4-hydroxy-3-oxo-2,3,4,5-tétrahydro-2,5-méthano-1H-2,4-benzodiazépine-1-carboxylique de formule brute C₁₁H₁₀N₂O₃ (M = 218 g).

Le rendement correspondant est de 89%.

### Stade B

Dans un ballon placé sous atmosphère inerte, on introduit 96 mg (0,44 mmole) du produit obtenu au stade A, 209 mg du complexe SO₃-pyridine et 2 ml de pyridine.

On laisse réagit pendant une nuit, puis on filtre et on évapore le solvant sous pression réduite.

On obtient ainsi 370 mg d'acide trans-3-oxo-4-(sulfooxy)-2,3,4,5-tétrahydro-2,5-méthano-1H-2,4-benzodiazépine-1-carboxylique, que l'on transforme en sel de sodium par passage sur résine DOWEX 50WX8 sous forme Na⁺.

Il est ensuite purifié sur résine Diaion HP20 en éluant à l'eau pour donner 79 mg du sel de disodium attendu, de formule brute C₁₁H₈Na₂N₂O₇S (M = 358,24 g).

Le rendement correspondant est de 50%.

### Spectre RMN du proton

Dans le DMSO d6, à 300 MHz, déplacements chimiques et multiplicité :
3,30 et 4,07 (d) : N-CH₂-CH-N; 4,46 (s1) : CH-CO₂; 4,51 (d) : N-CH₂-CH-N; 7,01 (dl), 7,09 (tl), 7,22 (td) et 7,52 (dl), : H aromatiques.

SM (Electrospray négatif) m/z : [M]⁻ = 313, 269.

### Exemple 12

### Sel de disodium de trans-3-oxo-4-(phosphonooxy)-2,3,4,5-tétrahydro-2,5-méthano-1H-2,4-benzodiazépine-1-carboxate de méthyle

### Stade A

On procède comme indiqué au stade A de l'Exemple 11, avec 1 g de produit obtenu au stade D de l'Exemple 6 de formule brute C₁₉H₁₈N₂O₄ (M = 338,37) en utilisant un mélange d'éthanol, de THF et 150 mg de catalyseur Pd/C à 10% en poids.

On recueille ainsi 0,75 g de trans-4-hydroxy-3-oxo-2,3,4,5-tétrahydro-2,5-méthano-1H-2,4-benzodiazépine-1-carboxylate de méthyle de formule brute C₁₂H₁₂N₂O₄ (M = 248,24 g).

Le rendement correspondant est quantitatif.

### Stade B

Dans un ballon placé sous atmosphère inerte, on dissout 350 mg du produit obtenu au stade A dans 12 ml d'acétonitrile.

On refroidit à 0°C et on ajoute 1,3 ml de N,N diisopropyléthyamine puis 34 mg de DMAP, 153 ml de tétrachlorure de carbone et 1,5 ml de dibenzylphosphite.

On laisse revenir à la température ambiante.

On verse ensuite dans un mélange heptane/AcOEt 33/66 et on lave la phase organique avec une solution aqueuse de NaH₂PO₄ 1 M et on la sèche sur du sulfate de magnésium.

On obtient ainsi 0,8 g de produit brut que l'on purifie sur silice en éluant d'abord avec un mélange AcOEt/dichlorométhane 7/93, puis avec un mélange heptane/AcOEt 1/1.

On recueille ainsi 0,140 mg de trans-4-[[di(phénylméthoxy)phosphinyl]oxy]-3-oxo-2,3,4,5-tétrahydro-2,5-méthano-1H-2,4-benzodiazépine-1-carboxylate de méthyle de formule brute C₂₆H₂₅N₂O₇P. (M= 508,47 g).

Le rendement correspondant est de 20%.

### Stade C

On dissout 140 mg du produit obtenu au stade B dans 3 ml d'éthanol.

On ajoute 46 mg d'hydrogénocarbonate de sodium, on introduit 15 mg de catalyseur Pd/C à 10% en poids et on place sous atmosphère d'hydrogène.

On laisse réagir pendant 2 heures, puis on filtre, on lave le catalyseur avec 5 ml d'éthanol, puis avec 2 ml d'eau.

On évapore à sec en entraînant au toluène.

On empâte le résidu dans de l'éther, on filtre et on évapore le solvant sous pression réduite.

On obtient ainsi 63 mg du sel de disodium attendu de formule brute C₁₂H₁₁N₂O₇PNa₂ (M = 372 g).

Le rendement correspondant est de 58%.
IR(CHCl₃) : 1746, 1622 à 1695 cm⁻¹.
SM (Electrospray négatif) m/z : [M²⁻+H]⁻ = 327,1.

### Exemple 13

### Trans-4-[[hydroxy(phénylméthoxy)phosphinyl]oxy]-3-oxo-2,3,4,5-tétrahydro-2,5-méthano-1H-2,4-benzodiazépine-1-carboxylate de méthyle

On dissout 150 mg de trans-4-[[di(phénylméthoxy)phosphinyl]oxy]-3-oxo-2,3,4,5-tétrahydro-2,5-méthano-1H-2,4-benzodiazépine-1-carboxylate de méthyle obtenu au stade B de l'exemple 12 dans 3 ml d'acétonitrile.

On ajoute 77 mg de bromure de lithium. On chauffe la suspension pendant 2 heures à reflux.

On évapore ensuite l'acétonitrile sous pression réduite, et on dissout le résidu dans l'AcOEt. On lave la solution avec une solution aqueuse de NaH₂PO₄ 1M, on sèche la phase organique sur du sulfate de magnésium et on évapore à sec sous pression réduite.

On reprend dans un mélange ether/pentane. Puis, on filtre la suspension.

On obtient ainsi 55 mg de cristaux beiges du produit attendu de formule brute C₁₉H₁₉N₂O₇P (M = 418,34 g).

Le rendement correspondant est de 42%.
SM Electrospray négatif) m/z : [M]⁻ = 417, 187, 79.

### Exemple 14

### Sel de sodium de trans-N-méthyl-3-oxo-4-(sulfooxy)-2,3,4,5-tétrahydro-2,5-méthano-1H-2,4-benzodiazépine-1-carboxamide

### Stade A

Dans un ballon placé sous atmosphère d'azote, on introduit 109 mg (0,4 mmole) du produit de formule brute C₁₄H₁₄N₂O₄ obtenu au stade A de l'Exemple 7 et 2 ml de THF.

On refroidit la solution à -8°C et on ajoute 0,83 ml de TEA en solution dans 1 ml de dichlorométhane.

On ajoute 0,3 ml d'une solution de méthylamine 2 M dans le THF, puis une solution de 62 mg de HOBt dans 2 ml de THF et 114,70 mg de EDCI.

On maintient sous agitation à 0°C pendant 2 heures.

On verse dans de l'eau, on extrait à l'AcOEt, on lave à l'eau et on sèche sur du sulfate de magnésium. Après évaporation du solvant sous pression réduite, on obtient environ 300 mg d'une huile que l'on purifie par chromatographie sur silice en éluant à l'AcOEt.

On recueille 91 mg de trans-N-méthyl-3-oxo-4-(2-propényloxy)-2,3,4,5-tétrahydro-2,5-méthano-1H-2,4-benzodiazépine-1-carboxamide de formule brute C₁₅H₁₇N₃O₃ (M = 287,321 g).

Le rendement correspondant est de 79%.

### Stade B

On procède comme indiqué au stade C de l'Exemple 7 avec 83 mg (0,288 mmole) du produit obtenu au stade A, 2,5 ml de dichlorométhane, 33 µl d'acide acétique, 166 mg de Pd[P(C₆H₅)₃]₄ et 137 mg du complexe SO₃-pyridine.

On obtient ainsi d'abord 100 mg de sel de 1-propényltriphénylphosphonium de trans-N-méthyl-3-oxo-4-(sulfooxy)-2,3,4,5-tétrahydro-2,5-méthano-1H-2,4-benzodiazépine-1-carboxamide de formule brute C₃₃H₃₂N₃O₆PS (M = 629,68 g, le rendement correspondant est de 55%), puis, après passage sur colonne DOWEX 50WX8 sous forme Na⁺ puis lyophilisation, 40 mg de sel de sodium attendu de formule brute C₁₂H₁₂N₃NaO₆S (M = 349,30 g) et qui se présente sous la forme d'un solide amorphe jaune. Le rendement correspondant à l'opération d'échange est de 72%.

### Spectre RMN du proton

Dans le DMSO d6, à 300 MHz, déplacements chimiques et multiplicité en ppm:
2,67 (d) : CH₃-NH-C=O ; 8,46 (ql) : CH₃-NH-C=O ; 3,43 (dd) et 3,79 (d) : N-CH₂-CH ; 4,64 (d) : N-CH₂-CH ; 4,80 (s) : CH-CO-NHCH₃; 7,12 (dl), 7,23 (m) et 7,33 (td) : H aromatiques.
SM (Electrospray négatif) m/z : [M]⁻ = 326

### Exemple 15

### Trans-4-(2-hydroxy-2-oxoéthoxy)-3-oxo-2,3,4,5-tétrahydro-2,5-méthano-1H-2,4-benzodiazépine-1-carboxylate de méthyle

### Stade A

Dans un ballon placé sous atmosphère d'argon, on introduit 92 mg (0,319 mmole) du produit obtenu au stade L de l'Exemple 3, 1,5 ml de THF, puis on ajoute à 20°C 36 µl d'acide acétique et 184 mg de Pd[P(C₆H₅)₃]₄.

On laisse sous agitation à 20°C pendant 1 heure, puis on rajoute 1 ml de dichlorométhane et on agite à température ambiante pendant 2 heures.

On évapore le solvant sous pression réduite.

On dissout ensuite le résidu dans 1,5 ml de THF, puis on ajoute 132 mg de carbonate de potassium, 2,5 ml d'eau et 152 µl de bromoacétate de benzyle.

On laisse sous agitation pendant 4 heures à 20°C. On dilue à l'AcOEt, et on lave avec une solution aqueuse d'acide tartrique à 10%, puis avec un tampon phosphate à pH 7 et avec une solution aqueuse saturée de chlorure de sodium.

On sèche la phase organique sur du sulfate de magnésium et évapore le solvant sous pression réduite.

On obtient ainsi 490 mg de produit brut que l'on purifie sur silice en éluant au dichlorométhane contenant 0,1% de TEA, puis avec un mélange dichlorométhane/AcOEt 95/5 contenant 0,1% de TEA.

On recueille 62 mg de trans-3-oxo-4-[[(phénylméthoxy)carbonyl]méthoxy]-2,3,4,5-tétrahydro-2,5-méthano-1H-2,4-benzodiazépine-1-carboxylate de méthyle de formule brute C₂₁H₂₀N₂O₆ (M = 396,402 g).

Le rendement correspondant est de 49%.

### Stade B

Dans un ballon, on introduit 30 mg (7,56 mmoles) du produit du stade A dans 1,5 ml d'AcOEt.

On ajoute 15 mg de catalyseur Pd/C à 10% en poids. On place sous atmosphère d'hydrogène à pression normale et on laisse réagir à température ambiante pendant 30 minutes.

On filtre et on rince le catalyseur à l'AcOEt.

On évapore le solvant sous pression réduite pour recueillir 25 mg de produit que l'on lave avec 0,5 ml d'éther éthylique.

On évapore l'éther sous pression réduite pour obtenir 20,3 mg du produit du produit attendu, de formule brute C₁₄H₁₄N₂O₆ (M = 306,277 g).

Le rendement correspondant est de 87%.

### Spectre RMN du proton

Dans le CDCl₃, à 300 MHz, déplacements chimiques et multiplicité :
3,58 (dd) et 3,88 (d) : N-CH₂ -CH; 3,87 (s) : O-CH₃; 4,56 (d) : N-CH-CH₂ ; 4,59 (AB) : O-CH₂-CO ; 5,22 (s) : N-CH-C=O ; 7,27 et 7,42 : H aromatiques.
IR (CHCl₃) : 1749, 1600 cm⁻¹.
SM (Electrospray négatif) m/z : [2M-H]- = 611 ; [M]⁻= 305, 231, 199, 171.

### Exemple 16

### Sel de sodium de trans-3-oxo-4-sulfo-2,3,4,5-tétrahydro-2,5-méthano-1H-2,4-benzodiazépine-1-carboxylate de méthyle

### Stade A

Dans un ballon placé sous atmosphère d'argon, on introduit 5,98 g (15,51 mmoles) du produit obtenu au stade H de l'Exemple 3 de formule brute C₁₇H₂₃NO₇S (M = 385,44 g) et 40 ml de DMSO.

On ajoute à 20°C 1,1 g de d'azoture de sodium et on laisse sous agitation à 20°C pendant 18 heures.

On dilue le milieu réactionnel à l'AcOEt et on le lave avec une solution diluée d'hydrogénocarbonate de sodium, puis 3 fois à l'eau, puis avec une solution de chlorure de sodium saturée.

On sèche la phase organique sur du sulfate de magnésium et on évapore le solvant sous pression réduite.

On obtient ainsi 6 g de produit brut sous forme d'huile, que l'on purifie sur silice, en éluant avec un mélange dichlorométhane/AcOEt 99/1.

On recueille 4,15 g de trans-4-azido-3,4-dihydro-1,2(1H)-isoquinoléine-dicarboxylate de 2-(1,1-diméthyléthyle) et de 1-méthyle de formule brute C₁₆H₂₀N₄O₄ (M = 332,362 g).

Le rendement correspondant est de 80%.

### Stade B

Dans un ballon équipé d'une agitation magnétique, on dissout 4,15 g (12,48 mmoles) de l'azide obtenu au stade A dans 110 ml de méthanol.

On ajoute 470 mg de catalyseur Pd/C à 10% en poids, on place sous atmosphère d'hydrogène à pression normale et on laisse réagir à température ambiante pendant 30 minutes.

On filtre et on rince le catalyseur au méthanol.

On évapore le solvant du filtrat pour recueillir 4 g d'une huile que l'on reprend avec du dichlorométhane, on filtre à nouveau et on évapore le solvant sous pression réduite.

On obtient ainsi 3,65 g de trans-4-amino-3,4-dihydro-1,2(1H)-isoquinoléinedicarboxylate de 2-(1,1-diméthyléthyle) et de 1-méthyle de formule brute C₁₆H₂₂N₂O₄ (M = 306,364 g).

Le rendement correspondant est de 95%.

### Stade C

Dans un ballon équipé placé sous atmosphère d'argon, on dissout 2,57 g (8,39 mmoles) de l'amine obtenue au stade B dans 25 ml de dichlorométhane.

Ensuite, on ajoute successivement, à 5°C, 0,85 ml de pyridine, 1,5 ml de chloroformiate de benzyle, puis on laisse réagir à 20°C pendant 2 heures.

On dilue avec du dichlorométhane, on lave avec une solution aqueuse d'acide tartrique à 10%, puis à l'hydrogénocarbonate de sodium dilué, puis à l'eau.

On sèche la phase organique sur du sulfate de magnésium et on évapore le solvant sous pression réduite.

On obtient ainsi 4 g d'une huile que l'on purifie sur silice en éluant avec un mélange dichlorométhane/AcOEt 9/1.

On recueille 2,98 g de trans-3,4-dihydro-4-[[(phénylméthoxy)carbonyl]-amino]-1,2(1H)-isoquinoléinedicarboxylate de 2-(1,1-diméthyléthyle) et de 1-méthyle de formule C₂₄H₂₈N₂O₆ (M= 440,5 g).

Le rendement correspondant est de 80%.

### Stade D

Dans un ballon refroidi par un bain de glace, on dissout 2,96 g (6,71 mmoles) du carbamate obtenu au stade C dans 4 ml d'AcOEt.

On refroidit vers 0-5°C et on ajoute à cette température 14,6 ml d'une solution de chlorure d'hydrogène dans de l'AcOEt à 4,6 mol/l.

On laisse la température remonter à 20°C pendant une heure, puis on évapore le solvant sous pression réduite. On cristallise le chlorhydrate dans de l'éther éthylique.

On obtient ainsi 2,55 g de chlorhydrate de trans-4-[[(phénylméthoxy)-carbonyl]amino]-1,2,3,4-tétrahydro-1-isoquinoléinecarboxylate de méthyle de formule brute C₁₉H₂₁N₂O₄Cl (M = 376,843 g).

Le rendement correspondant est quantitatif.

### Stade E

On procède comme indiqué au stade A de l'Exemple 1, avec 2,62 g (6,68 mmoles) du produit obtenu au stade D, 1,08 ml de pyridine et 443 µl de diphosgène.

On obtient ainsi 2,29 g de trans-2-(chlorocarbonyl)-4-[[(phényl-méthoxy)carbonyl]amino]-1,2,3,4-tétrahydro-1-isoquinoléinecarboxylate de méthyle de formule brute C₂₀H₁₉N₂O₅Cl (M = 402,837 g).

Le rendement correspondant est de 85%.

### Stade F

On procède comme indiqué au stade B de l'Exemple 1, avec 1,79 g (4,438 mmoles) du produit obtenu au stade E et 5,8 ml d'une solution de bis(triméthylsilyl) amidure de lithium 1 M dans le THF.

On obtient ainsi 373 mg de produit après purification.

Le rendement correspondant est de 22,9%.

On dissout ces 373 mg (1,02 mmole) dans 20 ml de dichlorométhane. On ajoute 30 µl de DBU et on agite à 20°C pendant 30 minutes.

On dilue au dichlorométhane, on lave avec une solution aqueuse d'acide tartrique à 10%, puis avec un tampon phosphate à pH 7 et avec une solution saturée de chlorure de sodium.

On sèche la phase organique sur du sulfate de magnésium et on évapore le solvant sous pression réduite.

On obtient ainsi 358 mg de trans-3-oxo-2,3-dihydro-2,5-méthano-1H-2,4-benzodiazépine-1,4(5H)-dicarboxylate de 1-méthyle et de 4-phénylméthyle de formule brute C₂₀H₁₈N₂O₅ (M = 366,376 g).

Le rendement correspondant à cette étape est de 96%.

### Stade G

On dissout 357 mg (0,974 mmoles) du carbamate obtenu au stade F dans 30 ml d'AcOEt.

On ajoute 212 mg de catalyseur Pd/C à 10% en poids. On laisse réagir à température ambiante pendant 1 heure 45 minutes, sous une pression d'hydrogène de 1 atmosphère.

On filtre et on rince le catalyseur à l'AcOEt.

On évapore le solvant sous pression réduite pour recueillir 223 mg de trans-3-oxo-2,3,4,5-tétrahydro-2,5-méthano-1H-2,4-benzodiazépine-1-carboxylate de méthyle de formule brute C₁₂H₁₂N₂O₃ (M = 232,241 g).

Le rendement correspondant est de 98,6%.

### Stade H

Dans un ballon placé sous atmosphère d'argon, on dissout 180 mg (0,775 mmoles) du produit obtenu au stade G dans 2 ml de DMF.

On ajoute à 20°C, 7 ml d'une solution de complexe SO₃-pyridine dans le DMF 0,33 M.

On laisse sous agitation à 20°C pendant 6 heures.

On évapore ensuite le solvant sous pression réduite pour obtenir 672 mg d'un sel de pyridinium de formule brute C₁₇H₁₇N₃O₆S (M = 391,40 g).

Ces 672 mg (0,775 mmoles) sont dissous dans 45 ml d'une solution 0,5 M de KH₂PO₄ dans l'eau.

La phase aqueuse est lavée avec 3 fois 15 ml d'AcOEt.

On ajoute ensuite 237 mg de HSO₄⁻NBu₄⁺ à la phase aqueuse, puis on extrait par 10 fois 15 ml d'AcOEt, on sèche les phases organiques réunies sur du sulfate de magnésium et on évapore le solvant sous pression réduite.

On obtient ainsi 259 mg d'un produit que l'on purifie sur silice en éluant au dichlorométhane, puis avec un mélange dichlorométhane/acétone 6/4 contenant 0, 1 % de TEA.

On récupère 170 mg d'un produit que l'on fait passer sur une colonne de résine DOWEX 50WX8 sous forme Na⁺, en éluant avec de l'eau contenant 10% de THF.

On lyophilise le produit recueilli.

On obtient 91 mg de sel de sodium de trans-3-oxo-4-sulfo-2,3,4,5-tétrahydro-2,5-méthano-1H-2,4-benzodiazépine-1-carboxylate de méthyle, de formule brute C₁₂H₁₁N₂O₆SNa (M = 334,29 g).

Le rendement correspondant est de 35%.

### Spectre RMN du proton

Dans D₂O, à 300 MHz, déplacements chimiques et multiplicité :
3,60 (d) et 3,76 (dd) : NCH₂-CH; 3,87 (s) : CO₂CH₃; 5,00 (d) : NCH₂-CH; 5,32 (s) : NCH-C=O ; 7,37 et 7,48 : H aromatiques.
IR (nujol) : 1738, 1634 cm⁻¹.
SM (Electrospray négatif) m/z : [M]- = 311, 279, 171.

### Exemple 17

### Sel de sodium de 1-propyl-5-(sulfooxy)-5,6,7,8-tétrahydro-4,7-méthano-4H-pyrazolo[3,4-e][1,3]diazépin-6-one

### Stade A

Dans un ballon placé sous atmosphère inerte, on dissout 50 g de BOC-NH-NH₂ dans 250 ml de DMF anhydre. On refroidit à -10°C, puis on ajoute par petites fractions 16,5 g d'hydrure de sodium à 50% dans l'huile.

On ajoute ensuite du bromure de propylène et on laisse sous agitation pendant une nuit à température ambiante. Puis, on ajoute lentement de l'eau et une solution à 1M d'hydrogénophosphate de sodium, puis 200 ml d'un mélange AcOEt/heptane 2/1 puis on extrait et on sèche la phase organique sur du sulfate de magnésium.

On filtre et on évapore ensuite le solvant sous pression réduite. On purifie le produit brut obtenu sur silice en éluant avec un mélange dichlorométhane/AcOEt 95/5.

On recueille ainsi 24 g de 2-(2-propényl)-hydrazinecarboxylate de 1,1-diméthyléthyle pur.

Le rendement correspondant est de 76%.

### Stade B

On dissout 24 g du produit obtenu au stade A dans 80 ml d'AcOEt.

On refroidit à 0°C, puis on ajoute 332 ml d'une solution d'acide chlorhydrique 5,5 N dans l'AcOEt. On agite pendant 1 heure 30 à température ambiante, puis on filtre et on lave à l'éther.

On obtient ainsi 15 g de (2-propényl)-hydrazine de formule brute C₃H₈N₂,2HCl sous la forme de cristaux blancs.

Le rendement correspondant est de 84%.

### Stade C

On dissout 11 g du produit de formule brute C₁₄H₂₁N₃O₄ obtenu au stade A de l'Exemple 5 dans 130 ml d'éthanol.

On ajoute 6,51 g du produit obtenu au stade B et 11,33 g de carbonate de potassium.

On agite la suspension pendant 45 minutes, puis on évapore l'éthanol sous pression réduite. On solubilise le résidu dans l'AcOEt, puis on lave la phase organique à l'eau, on la sèche ensuite sur du sulfate de magnésium, on filtre et on évapore le solvant sous pression réduite.

On obtient ainsi 10,8 g de 3,5-dioxo-4-[[2-(2-propényl)hydrazino]-méthylène]-1-pipéridinecarboxylate de 1,1-diméthyléthyle de formule brute C₁₄H₂₁N₃O₃ (M = 295,34 g).

Le rendement correspondant est de 80%.

### Stade D

On dissout 10,8 g du produit obtenu au stade C dans 120 ml de toluène.

On ajoute 1 g d'acide p-toluène sulfonique monohydraté et on chauffe à reflux pendant une heure.

On laisse refroidir, on verse dans de l'AcOEt, on lave la phase organique à l'eau et on la sèche sur du sulfate de magnésium. On évapore le solvant sous pression réduite.

On obtient ainsi 8,5 g de produit brut que l'on purifie par chromatographie sur silice en éluant avec un mélange heptane/AcOEt 2/1.

On recueille ainsi 7,5 g de 4,7-dihydro-4-oxo-1-(2-propényl)-1H-pyrazolo-[3,4-c]pyridine-6(5H)-carboxylate de 1,1-diméthyléthyle de formule brute C₁₄H₁₉N₃O₃ (M = 277,33 g).

Le rendement correspondant est de 74%.

### Stade E

On procéde comme indiqué au stade A de l'Exemple 6, avec 7,5 g du produit obtenu au stade D dans 150 ml de pyridine, et 4,74 g de O-benzylhydroxylamine.

On obtient ainsi 9,72 g de 4,7-dihydro-4-[(phénylméthoxy)imino]-1-(2-propényl)-1H-pyrazolo[3,4-c]pyridine-6(5H)-carboxylate de 1,1-diméthyléthyle de formule brute C₂₁H₂₆N₄O₃ (M = 382,47 g).

Le rendement correspondant est de 90%.

### Stade F

On procède comme indiqué au stade B de l'Exemple 6 avec 9,2 g du produit obtenu au stade E dans 750 ml de méthanol, 24,2 g de NaBH₃CN et 36,51 ml d'éthérate de trifluorure de bore.

On obtient ainsi 5,3 g de 4,7-dihydro-4-[(phénylméthoxy)amino]-1-(2-propényl)-1H-pyrazolo[3,4-c]pyridine-6(5H)-carboxylate de 1,1-diméthyléthyle de formule brute C₂₁H₂₈N₄O₃ (M= 384,48 g).

Le rendement correspondant est de 60%.

### Stade G

On procède comme indiqué aux stades J et K de l'Exemple 3 avec 5,25 g du produit obtenu au stade F et une solution de chlorure d'hydrogène 5,5 N.

On obtient ainsi 3,95 g de N-(phénylméthoxy)-1-(2-propényl)-4,5,6,7-tétrahydro-1H-pyrazolo[3,4-c]pyridin-4-amine de formule brute C₁₆H₂₀N₄O (M = 284,36 g).

Le rendement correspondant est de 90%.

### Stade H

On procède comme indiqué au stade L de l'Exemple 3 avec 3,8 g du produit obtenu au stade G, 4,2 ml de TEA et 0,8 ml de diphosgène.

On obtient ainsi 2,5 g de 5-(phénylméthoxy)- 1-(2-propényl)-4,5,7,8-tétrahydro-4,7-méthano-pyrazolo[3,4-e][1,3]diazépin-6(1H)-one de formule brute C₁₇H₁₈N₄O₂ (M = 310,36 g).

Le rendement correspondant est de 68%.

### Stade I

On procède comme au stade A de l'Exemple 11 avec 0,1 g du produit obtenu au stade H et 20 mg de catalyseur Pd/C à 10% en poids.

On obtient ainsi 75 mg de 5-hydroxy-1-propyl-4,5,7,8-tétrahydro-4,7-méthano-pyrazolo[3,4-e][1,3]diazépin-6(1H)-one de formule brute C₁₀H₁₄N₄O₂ (M = 222,25 g).

Le rendement correspondant est quantitatif.

### Stade J

Dans un ballon placé sous atmosphère inerte, on dissout 75 mg du produit obtenu au stade I dans 3 ml de pyridine. On ajoute 0,161 g du complexe SO₃-pyridine et on laisse réagir pendant une nuit à température ambiante.

On évapore le solvant sous pression réduite et on passe le résidu sur une colonne de résine DOWEX 50WX8 sous forme Na⁺, en éluant avec de l'eau contenant 10% de THF.

Après évaporation du solvant sous pression réduite, le produit est solubilisé dans l'acétone et le sulfate de sodium est éliminé par filtration. Le solvant est ensuite évaporé sous pression réduite et le produit cristallisé dans l'éther éthylique.

On obtient ainsi 80 mg du sel de sodium attendu, de formule brute C₁₀H₁₃N₄O₅SNa (M = 301,30 g).

Le rendement correspondant est de 80%.

### Spectre RMN du proton

Dans le DMSO d6, à 300 MHz, déplacements chimiques et multiplicité :
0,80 (t) : CH₃-CH₂-CH₂-N; 1,69 (m) : CH₃-CH₂-CH₂-N; 3,86 (m) : CH₃-CH₂-CH₂-N; 3,12 (d) et 3,47 (dd) : N-CH₂-CH; 4,67 (s1) : N-CH₂-CH ; 4,33 (s1) : N-CH₂-C= ;7,36 (s) : N=CH
IR CHCl₃) : 1752, 1644, 1540, 1505 cm⁻¹.
SM (Electrospray négatif) m/z : [M]⁻ = 301

### Exemple 18

### Sel de sodium de trans-1-méthyl-6-oxo-5-(sulfooxy)-5,6,7,8-tétrahydro-4,7-méthano-4H-pyrazolo[3,4-e][1,3]diazépine-8-carboxylate de méthyle

### Stade A

Dans un ballon placé sous atmosphère d'azote, on dissout 12,05 g de la cétone obtenue au stade B de l'exemple 5 dans 157 ml de méthanol, puis on refoidit au bain de glace et on introduit par petites fractions, en 20 minutes, 1,82 g de borohydrure de sodium.

On laisse revenir à la température ambiante en deux heures, puis on ajoute successivement 1 litre de dichlorométhane et 354 ml de solution aqueuse d'acide tartrique à 10%.

On agite vigoureusement puis on décante et on réextrait au dichlorométhane.

On réunit les phases organiques et on les sèche sur du sulfate de magnésium, on filtre et on évapore le solvant du filtrat sous pression réduite.

On reprend le résidu dans environ 500 ml de dichlorométhane, on filtre et on évapore à nouveau le solvant sous pression réduite.

On obtient ainsi 11,36 g de 4,7-dihydro-4-hydroxy-1-méthyl-1H-pyrazolo[3,4-c]pyridine-6(5H)-carboxylate de 1,1-diméthyléthyle de formule brute C₁₂H₁₉N₃O₃ (M= 253,3 g).

Le rendement correspondant est de 94%.

### Stade B

Dans un ballon placé sous atmosphère d'azote, on dissout 3,03 g (12 mmoles) de l'alcool obtenu au stade A dans 52 ml de THF, puis on refoidit à -78°C et on introduit 18 ml de terbutyllithium en solution 1,7 M dans le pentane.

On laisse réagir 15 minutes à -78°C, puis on introduit du gaz carbonique en excès pendant 10 minutes, puis on laisse revenir à la température ambiante.

On ajoute ensuite 95 ml d'AcOEt, puis acidifié à pH = 4 par 31 ml de HCl 1N.

On sature avec du chlorure de sodium et on réextrait à l'AcOEt. On sèche la phase organique sur sulfate de magnésium, on filtre puis on évapore le solvant sous pression réduite.

On obtient ainsi 3,37 g de 4,7-dihydro-4-hydroxy-1-méthyl-1H-pyrazolo[3,4-c]pyridine-6(5H),7-dicarboxylate de 6-(1,1-diméthyléthyle).

Le rendement correspondant est de 95%.

### Stade C

On dissout ensuite l'acide brut obtenu au stade B dans 10 ml de dichlorométhane, puis on ajoute un excès de diazométhane en solution dans le dichlorométhane, on laisse réagir pendant 20 minutes, puis on évapore le solvant sous pression réduite.

On obtient ainsi 3,34 g de 4,7-dihydro-4-hydroxy-1-méthyl-1H-pyrazolo[3,4-c]pyridine-6(5H),7-dicarboxylate de 6-(1,1-diméthyléthyle) et de 7-méthyle de formule brute C₁₄H₂₁O₅N₃ (M = 311,34 g).

Le rendement correspondant est de 90%.

### Stade D

Dans un ballon, on introduit 3,30 g de chlorochromate de pyridinium, 6,6 g de tamis moléculaire de 4 Å en poudre et 60 ml de dichlorométhane.

On agite pendant 40 minutes, puis on ajoute une solution comprenant 3,34 g du produit obtenu au stade C et 40 ml de dichlorométhane.

On agite pendant 1 heure 20. Ensuite on filtre le mélange réactionnel et on évapore le solvant sous pression réduite.

On reprend dans de l'AcOEt, on refiltre et on évapore le solvant sous pression réduite. On purifie le résidu par chromatographie sur silice en éluant avec du dichlorométhane contenant 15% d'AcOEt.

On recueille ainsi 1,33 g de 4,7-dihydro-1-méthyl-4-oxo-1H-pyrazolo[3,4-c]pyridine-6(5H),7-dicarboxylate de 6-(1,1-diméthyléthyle) et de 7-méthyle de formule brute C₁₄H₁₉N₃O₅ (M = 309,32 g).

Le rendement correspondant est de 36%.

### Stade E

On procède comme indiqué au stade A de l'Exemple 6 avec 2,63 g (8,5 mmoles) du produit obtenu au stade D, 26,3 ml d'éthanol, 6,2 ml de pyridine et 2,85 g (25,5 mmoles) de chlorhydrate d'hydroxylamine.

On obtient ainsi 1,68 g de 4,7-dihydro-1-méthyl-4-[(2-propényloxy)imino]-1H-pyrazolo[3,4-c] pyridine-6(5H),7-dicarboxylate de 6-(1,1-diméthyléthyle) et de 7-méthyle de formule brute C₁₇H₂₄N₄O₅ (M = 364,4 g).

Le rendement correspondant est de 54%.

### Stade F

Dans un ballon équipé d'une agitation magnétique, on introduit 1,34 g (3,68 mmoles) du produit obtenu au stade E, 13,4 ml d'acide acétique et 3 ml de dichlorométhane.

On abaisse la température à 10°C, puis on additionne 1,04 g de NaBH₃CN et on agite à température ambiante pendant 7 heures.

On verse ensuite sur un mélange d'une solution saturée d'hydrogénocarbonate de sodium dans l'eau et d'AcOEt.

On agite pendant 40 minutes jusqu'à ce qu'il n'y ait plus de dégagement gazeux.

On décante et on extrait 4 fois à l'AcOEt.

On sèche les phases organiques sur du sulfate de sodium, puis on filtre et on évapore le solvant sous pression réduite.

On obtient ainsi 1,4 g d'un produit que l'on purifie sur silice en éluant avec un mélange dichlorométhane/AcOEt 50/50.

On recueille 0,493 g de cis-4,7-dihydro-1-méthyl-4-[(2-propényloxy)amino]-1H-pyrazolo[3,4-c]pyridine-6(5H),7-dicarboxylate de 6-(1,1-diméthyléthyle) et de 7-méthyle de formule brute C₁₇H₂₆N₄O₅ (M = 366,48 g).

Le rendement correspondant est de 36%.

### Stade G

On procède comme indiqué aux stades J et K de l'Exemple 3, avec 0,704 g (1,92 mmoles) du produit obtenu au stade F, 15 ml d'une solution de chlorure d'hydrogène à 143 g/l dans l'AcOEt et en remplaçant la soude par l'ammoniaque.

On obtient ainsi 0,519 g de cis-1-méthyl-4-[(2-propényloxy)amino]-4,5,6,7-tétrahydro-1H-pyrazolo[3,4-c]pyridine-7-carboxylate de méthyle de formule brute C₁₂H₁₈N₄O₃(M=266,3 g).

Le rendement correspondant est quantitatif.

### Stade H

On procède comme indiqué au stade L de l'Exemple 3 avec 0,519 g (1,92 mmoles) du produit obtenu au stade G, 1,33 ml de TEA et 131 µl de diphosgène.

On obtient 0,557 g de trans-1-méthyl-6-oxo-5-(2-propényloxy)-4,5,6,8-tétrahydro-4,7-méthano-1H-pyrazolo[3,4-e][1,3]diazépine-8(7H)-carboxylate de méthyle de formule brute C₁₃H₁₆N₄O₄ (M = 292,30 g).

Le rendement correspondant est quantitatif.

On dissout ensuite 0,540 g (1,85 mmoles) du produit obtenu dans 35,7 ml de dichlorométhane.

On ajoute 55.3 µl de DBU et on laisse sous agitation pendant une heure.

On lave avec une solution aqueuse d'acide tartrique à 10%, puis avec de l'eau déminéralisée.

On sèche la phase organique sur du sulfate de magnésium, on rince puis on évapore le solvant sous pression réduite.

On obtient ainsi 472,3 mg de produit que l'on purifie par chromatographie sur silice en éluant avec un mélange dichlorométhane/AcOEt 80/20.

On recueille ainsi 0,194 g de trans-1-méthyl-6-oxo-5-(2-propényloxy)-4,5,6,8-tétrahydro-4,7-méthano-1H-pyrazolo[3,4-e][1,3]diazépine-8(7H)-carboxylate de méthyle purifié de formule brute C₁₃H₁₆N₄O₄ (M = 292,30 g).

Le rendement correspondant est de 48%.

### Stade I

On procède comme indiqué au stade C de l'exemple 7 avec 80 mg (0,274 mmoles) du produit obtenu au stade H, 50 µl d'acide acétique, 158,3 mg de Pd[P(C₆H₅)₃]₄, 2,4 ml de pyridine et 126 mg du complexe pyridine-SO₃.

On obtient ainsi 0,246 g d'un produit brut que l'on purifie par chromatographie sur silice en éluant avec un mélange dichlorométhane/méthanol 94/6, puis avec un mélange dichlorométhane/acétone/TEA 1/1/2%.

On recueille 9,8 mg de sel de 1-propényltriphénylphosphonium de trans-1-méthyl-6-oxo-5-(sulfooxy)-4,5,6,8-tétrahydro-4,7-méthano- 1H-pyrazolo[3,4-e][1,3]diazépine-8(7H)-carboxylate de méthyle de formule brute C₃₁H₃₁N₄O₇PS (M = 634,65 g).

Le rendement correspondant est de 6%.

Après passage sur une colonne de résine DOWEX 50WX8 sous forme Na⁺ et lyophilisation on récupère 3,5 mg du sel de sodium attendu de formule brute C₁₀H₁₁N₄O₇SNa (M = 331 g).

Le rendement correspondant à cette opération d'échange est de 64%.
SM (Electrospray négatif) m/z : [M]⁻ = 331

### Exemple 19

### Sel de sodium de trans-1-méthyl-6-oxo-5-(sulfooxy)-4,5,6,8-tétrahydro-4,7-méthano-1H-pyrazolo[3,4-e][1,3]diazépine-8(7H)-carboxamide

### Stade A

On procède comme indiqué au stade A de l'Exemple 7 avec 189 mg (0,646 mmole) du produit obtenu au stade H de l'exemple 18.

On obtient 17,6 mg d'acide trans-1-méthyl-6-oxo-5-(2-propényloxy)-4,5,6,8-tétrahydro-4,7-méthano- H-pyrazoto[3,4-e][1,3]diazépine-8(7H)-carboxylique de formule brute C₁₂H₁₄N₄O₄ (M = 278,27 g).

Le rendement correspondant est de 84%.

### Stade B

On procède comme indiqué au stade B de l'Exemple 10 avec 149,3 mg (0,536 mmoles) du produit obtenu au stade A, 355,6 mg de BOP, 111 mg de HOBT, 57,3 mg (1,07 mmole) de chlorure d'ammonium et 0,373 ml de DIPEA.

On récupère ainsi 81,1 mg de trans-1-méthyl-6-oxo-5-(2-propényloxy)-4,5,6,8-tétrahydro-4,7-méthano-1H-pyrazolo[3,4-e][1,3]diazépine-8(7H)-carboxamide de formule brute C₁₂H₁₅N₅O₃ (M = 277,29 g).

Le rendement correspondant est de 55%.

### Stade C

On procède comme indiqué au stade C de l'Exemple 7 avec 80 mg (0,288 mmoles) du produit obtenu au stade B, 35,2 µl d'acide acétique et 44 mg de Pd[P(C₆H₅)₃]₄.

On obtient ainsi 17,4 mg de sel de 1-propényltriphénylphosphonium de trans-1-méthyl-6-oxo-5-(sulfooxy)-4,5,6,8-tétrahydro-4,7-méthano-1H-pyrazolo[3,4-e][1,3]diazépine-8(7H)-carboxamide de formule brute C₃₀H₃₀N₅O₆PS (M = 619,64 g).

Le rendement correspondant est de 10 %.

Après passage sur colonne de résine DOWEX 50WX8 sous forme Na⁺ et lyophilisation, on recueille 6,8 mg du sel de sodium attendu, de formule brute C₉H₁₀N₅O₆SNa (M = 339,26 g).

Le rendement correspondant à cette étape d'échange est de 78%.

### Spectre RMN du proton

Dans D₂O, à 300 MHz, déplacements chimiques et multiplicité :
3,32 (d) et 3,75 (dd) : O=C-NCH₂-CH; 4,99 (d) : O=C-NCH₂-CH; 5,50 (s) : N-CH-C=O; 7,67 (s) : N=CH; 3,81 (s) : CH₃-N

### Exemple 20

### Sel de sodium de 6-oxo-7-(sulfooxy)-5,6,7,8-tétrahydro-5,8-méthano-4H-thiazolo[4,5-e][1,3]diazépine-2-carbamate de phénylméthyle

### Stade A

Dans un ballon placé sous atmosphère inerte et refroidi par un bain de glace, on introduit 21,3 g (100 mmole) de 3,5-dioxo-1-pipéridinecarboxylate de 1,1-diméthyléthyle de formule brute C₁₀H₁₅NO₄ (M = 213,24 g) utilisé au stade A de l'exemple 5 et 532 ml de tétrachlorure de carbone.

On ajoute en refroidissant 21,35 g de N-bromosuccinimide (120 mmoles), et 1,6 g d'AIBN.

On agite le mélange pendant 1 heure 30 à une température de 10-15°C.

Ensuite, on évapore le solvant sous pression réduite.

On additionne 40 ml d'AcOEt puis 300 ml d'eau, on extrait, on lave la phase organique à l'eau, on la sèche sur sulfate de sodium et on évapore le solvant sous pression réduite.

On obtient ainsi 30 g de 4-bromo-3,5-dioxo-1-pipéridinecarboxylate de 1,1-diméthyléthyle de formule brute C₁₀H₁₄BrNO₄ (M = 292,13 g).

Le rendement correspondant est quantitatif.

### Stade B

On introduit 30 g (100 mmole) du produit obtenu au stade A, 300 ml d'éthanol et 15,2 g de thiourée (200 mmoles).

La solution est chauffée à reflux pendant 5 heures. Le mélange est refroidi à la température ambiante et le précipité qui s'est formé est lavé à l'éthanol, filtré et séché sous pression réduite.

On obtient ainsi 15 g de bromhydrate de 2-amino-5,6-dihydro-thiazolo[4,5-c]pyridin-7(4H)-one de formule brute C₆H₈BrN₃OS (M = 250 g).

Le rendement correspondant est de 60%.

### Stade C

Dans un ballon placé sous atmosphère inerte et refroidi par un bain de glace, on introduit 15 g du produit obtenu au stade B et 150 ml de DMF.

On ajoute ensuite 19 ml de TEA et 16,2 g de (BOC)₂O, on agite pendant 2 heures à température ambiante.

On verse ensuite le mélange réactionne sur 600 ml d'eau, puis on essore le précipité formé, on le lave à l'eau et on le sèche sous pression réduite.

On obtient ainsi 16,5 g de 2-amino-6,7-dihydro-7-oxo-thiazolo[4,5-c]pyridine-5(4H)-carboxylate de 1,1-diméthyléthyle de formule brute C₁₁H₁₅N₃O₃ (M = 269,32 g).

Le rendement est correspondant est quantitatif.

### Stade D

Dans un ballon placé sous atmosphère d'azote, on introduit 6,9 g du produit obtenu au stade C et 140 ml de THF.

On refroidit à -20°C, puis on ajoute 6,1 g (50 mmoles) de 4-diméthylaminopyridine et 7,11 ml (50 mmoles) de chloroformiate de benzyle en solution dans 35 ml de THF.

On laisse ensuite revenir à la température ambiante en une heure.

On verse dans 300 ml d'eau contenant de la glace, on extrait à l'AcOEt, on lave 3 fois à l'eau, puis on réunit les phases organiques et on sèche sur du sulfate de sodium. On évapore le solvant sous pression réduite.

On obtient ainsi 11 g de 6,7-dihydro-7-oxo-2-[[(phénylméthoxy)carbonyl]-amino]-thiazolo[4,5-c]pyridine-5(4H)-carboxylate de 1,1-diméthyléthyle de formule C₁₉H₂₁N₃O₅S (M = 403,46 g).

Le rendement correspondant est quantitatif.

### Stade E

On procède comme indiqué au stade A de l'Exemple 6, avec 11 g du produit obtenu au stade D, 18 ml de pyridine et 8,2 g de chlorhydrate de O-allylhydroxylamine.

On obtient ainsi 7,9 g de 6,7-dihydro-2-[[(phénylméthoxy)carbonyl]amino]-7-[(2-propényloxy)imino]-thiazolo[4,5-c]pyridine-5(4H)-carboxylate de 1,1-diméthyléthyle.

Le rendement correspondant est de 69%.

### Stade F

On procède comme indiqué au stade B de l'Exemple 6 avec 2,29 g du produit obtenu au stade E, 5,02 g de cyanoborohydrure de sodium et 7,3 ml d'éthérate de trifluorure de bore.

On obtient ainsi 1,2 g de 6,7-dihydro-2-[[(phénylméthoxy)carbonyl]amino]-7-[(2-propényloxy)amino]-thiazolo[4,5-c]pyridine-5(4H)-carboxylate de 1,1-diméthyléthyle de formule brute C₂₂H₂₈N₄O₅S (M = 460,56 g).

Le rendement correspondant est de 52%.

### Stade G

On procède comme indiqué aux stades J et K de l'Exemple 3 avec 1,2 g du produit obtenu au stade F et 12 ml de chlorure d'hydrogène et en remplaçant la soude par de l'ammoniaque.

On obtient ainsi 800 mg de [7-[(2-propényloxy)amino]-4,5,6,7-tétrahydrothiazolo[4,5-c]pyridin-2-yl]-carbamate de phénylméthyle de formule C₁₇H₂₀N₄O₃S (M = 360,44 g).

Le rendement correspondant est de 85%.

### Stade H

Dans un ballon placé sous atmosphère inerte, on introduit 720 mg (2 mmole) du produit obtenu au stade G et 240 ml d'acétonitrile.

On refroidit la solution à 0°C et on ajoute 140 µl de diphosgène (1,16 mmoles).

En fin d'addition, on laisse revenir à température ambiante et on ajoute une solution de 840 µl de TEA dans 8 ml d'acétonitrile.

La solution est agitée pendant une nuit à température ambiante.

On évapore le solvant sous pression réduite et on redissout dans l'AcOEt, on lave à l'eau, on décante, on filtre et on sèche la phase organique sur sulfate de sodium.

On évapore le solvant sous pression réduite.

On obtient ainsi 860 mg d'une mousse jaune pâle que l'on purifie par chromatographie sur silice, en éluant avec un mélange acétone/dichlorométhane 1/9.

On recueille ainsi une fraction principale de 370 mg de [6-oxo-7-(2-propényloxy)-5,6,7,8-tétrahydro-5,8-méthano-4H-thiazolo[4,5-e][1,3]diazépin-2-yl]-carbamate de phénylméthyle de formule brute C₁₈H₁₈N₄O₄S (M = 386,43 g).

Le rendement correspondant est de 48%.

### Stade I

On procède comme indiqué au stade C de l'Exemple 7, avec 58 mg (0,15 mmole) du produit obtenu au stade H, 17 µl d'acide acétique, 86 mg de Pd[P(C₆H₅)₃]₄ et 72 mg de SO₃-pyridine.

On obtient ainsi 24 mg de sel de 1-propényltriphénylphosphonium de [6-oxo-7-(sulfooxy)-5,6,7,8-tétrahydro-5,8-méthano-4H-thiazolo[4,5-e][1,3]diazépin-2-yl]-carbamate de phénylméthyle de formule brute C₃₆H₃₃N₄O₇PS₂.

Le rendement correspondant est de 22%.

Après passage sur une colonne de résine DOWEX 50WX8 sous forme Na⁺ et lyophilisation on obtient 12,5 mg du sel de sodium attendu, de formule brute C₁₅H₁₃N₄O₇S₂ (M = 448,41 g).

Le rendement de cette étape d'échange est de 86 %.

### Spectre RMN du proton

Dans le DMSO d6, à 300 MHz, déplacements chimiques et multiplicité :
3,32 (d) et 3,55 (d) : N-CH₂-CH-C; 4,85 (d) : N-CH₂-CH-C ; 4,07 (d) et 4,28 (d) : N-CH₂-C= ; 5,24 (AB) : OC-OCH₂-C₆H₅ ; 7,40 (m) : C₆H₅

### Exemple 21

### Sel de sodium de 2-méthyl-5-(sulfooxy)-5,6,7,8-tétrahydro-4,7-méthano-4H-oxazolo[4,5-e][1,3]diazépin-6-one

### Stade A

Dans un ballon refroidi par un bain de glace et placé sous atmosphère d'argon, on introduit 5,44 g (23,9 mmoles) de p-carboxybenzène sulfonazide (M = 227,20 g), 55 ml d'acétonitrile et 3,33 ml de TEA.

On refroidit à 0-5°C et on ajoute en agitant 5,096 g de 3,5-dioxo-1-pipéridine-carboxylate de 1,1-diméthyléthyle de formule brute C₁₀H₁₅NO₄ (23,9 mmoles - M = 213,24 g) utilisé au stade A de l'exemple 5, 51 ml d'acétonitrile et 3,33 ml de TEA.

On laisse en contact pendant 30 minutes à 0-5°C, puis pendant 40 minutes à température ambiante.

On filtre, on rince le solide à l'AcOEt, puis on dilue le filtrat avec 400 ml d'AcOEt.

On lave ensuite successivement avec une solution aqueuse saturée d'hydrogénocarbonate de sodium, puis avec une solution aqueuse d'acide tartrique à 10% et avec de l'eau saturée en chlorure de sodium.

On décante la phase organique et on la sèche sur du sulfate de magnésium, puis on évapore le solvant sous pression réduite.

On obtient ainsi un produit huileux brun que l'on purifie par chromatographie sur silice en éluant avec un mélange dichlorométhane/AcOEt 90/10.

On recueille 4,13 g de 4-azo-3,5-dioxo-1-pipéridinecarboxylate de 1,1-diméthyléthyle de formule brute C₁₀H₁₃N₃O₄ (M= 239,23 g).

Le rendement correspondant est de 72,3%.

### Stade B

Dans un ballon placé sous atmosphère d'argon, on introduit 3,07 g (12,83 mmoles) de la diazocétone obtenue au stade A et 40 ml de acétonitrile, puis 153 mg de tétraacétate de rhodium.

On chauffe à 60°C pendant 9 heures.

On évapore le solvant sous pression réduite et on purifie le résidu par chromatographie sur silice en éluant avec un mélange dichlorométhane/AcOEt 9/1.

On recueille 742 mg de 6,7-dihydro-2-méthyl-7-oxo-oxazolo[5,4-c]pyridine-5(4H)-carboxylate de 1,1-diméthyléthyle de formule brute C₁₂H₁₆N₂O₄ (M = 252,27 g).

Le rendement correspondant est de 22,9%.

### Stade C

On procède comme indiqué au stade A de l'Exemple 6 avec 740 mg (2,93 mmoles) du produit obtenu au stade B, 321 mg de chlorhydrate de O-allylhydroxylamine et 0,7 ml de pyridine.

On obtient ainsi 760 mg de 6,7-dihydro-2-méthyl-7-[(2-propényloxy)imino]-oxazolo[5,4-c]pyridine-5(4H)-carboxylate de 1,1-diméthyléthyle de formule brute C₁₅H₂₁N₃O₄ (M = 307,35 g).

### Stade D

On procède comme indiqué au stade B de l'Exemple 6 avec 600 mg (1,95 mmoles) du produit obtenu au stade C, 1,84 g de cyanoborohydrure de sodium et 2,96 ml d'éthérate de trifluorure de bore.

On obtient ainsi 386 mg de 6,7-dihydro-2-méthyl-7-[(2-propényloxy)amino]-oxazolo[5,4-c]pyridine-5(4H)-carboxylate de 1,1-diméthyléthyle de formule brute C₁₅H₂₃N₃O₄ (M = 309,37 g).

Le rendement correspondant est de 63,9%.

### Stade E

On procède comme indiqué aux stades J et K de l'Exemple 3 avec 410 mg (1,32 mmoles) du produit obtenu au stade D, 3,1 ml de solution de chlorure d'hydrogène dans l'AcOEt.

On obtient ainsi 239 mg de 2-méthyl-N-(2-propényloxy)-4,5,6,7-tétrahydro-oxazolo[5,4-c]pyridin-7-amine de formule C₁₀H₁₅N₃O₂ (M = 209,25 g).

Le rendement correspondant est de 86,2%.

### Stade F

On procède comme indiqué au stade L de l'Exemple 3 avec 204 mg (0,975 mmoles) du produit obtenu au stade E, 680 µl de TEA et 59 µl de diphosgène.

On obtient ainsi 109 mg de 2-méthyl-5-(2-propényloxy)-4,5,7,8-tétrahydro-4,7-méthano-6H-oxazolo[4,5-e][1,3]diazépin-6-one de formule brute C₁₁H₁₃N₃O₃ (M = 235,24 g).

Le rendement correspondant est de 41,1 %.

### Stade G

On procède comme indiqué au stade C de l'Exemple 7 avec 39 mg (0,166 mmoles) du produit obtenu au stade F, 19 µl d'acide acétique, 96 mg de Pd[P(C₆H₅)₃]₄, 79 mg de complexe SO₃-pyridine.

On obtient ainsi 53 mg de sel de 1-propényltriphénylphosphonium de 2-méthyl-5-(sulfooxy)-4,5,7,8-tétrahydro-4,7-méthano-6H-oxazolo[4,5-e][1,3]diazépin-6-one de formule brute C₈H₈N₃O₆S⁺,⁻PC₂₁H₂₀ (M = 577,6 g).

Le rendement correspondant est de 55,4%.

Après passage du produit sur la colonne de résine DOWEX 50WX8 sous forme Na⁺, on recueille 56 mg de sel de sodium attendu, de formule brute C₈H₈N₃O₆SNa (M = 297,22 g).

Le rendement correspondant à cette étape d'échange est de 84,3%.

### Spectre RMN du proton

Dans le DMSO d6, à 300 MHz, déplacements chimiques et multiplicité :
2,39 (s): CH₃-C=N ; 3,24 (d) et 3,50 (dd) : N-CH₂-CH; 4,19 et 4,37 (AB) : N-CH₂-C= ; 4,63 (d) : N-CH₂-CH
IR (CHCl₃) : 1761, 1648, 1569
SM (Electrospray négatif) m/z : [2M-H]⁻= 549 ; [M]⁻= 274

### Exemple 22

### Sel de pyridinium de 1-propyl-5-(sulfooxy)-4,5,7,8-tétrahydro-4,7-méthano-imidazo[4,5-e][1,3]diazépin-6(1H)-one

### Stade A

On introduit 15 g (0,089 mole) de 5-formyl-1H-imidazole-4-carboxylate d'éthyle de formule brute C₇H₈N₂O₃ (M = 168,15 g, préparé selon une méthode analogue à celle décrite dans J. Chem. Soc. Perkin Trans. 1, 495-505 (1980), en remplaçant le butanol par de l'éthanol) dans du DMF.

On ajoute 12,31 g de carbonate de potassium, puis goutte à goutte 7,5 ml de bromure d'allyle.

On agite pendant 1 heure 30, puis on verse dans de l'eau, on extrait trois fois avec un mélange heptane/AcOEt 2/8, puis on lave à l'eau la phase organique, on la sépare et on la sèche sur du sulfate de magnésium.

On filtre et on évapore le solvant sous pression réduite.

Le résidu est purifié sur silice en éluant avec un mélange dichlorométhane/acétone 95/5.

On obtient ainsi 10,1 g d'un premier isomère de 5-formyl-1-(2-propényl)-1H-imidazole-4-carboxylate d'éthyle, de formule brute C₁₀H₁₂N₂O₃ (M = 208,22 g) sous la forme de cristaux blancs (10,1 g) et 4,5 g d'un second isomère sous la forme d'une huile jaune (4,5 g).

### Stade B

On dissout 9,5 g (0,045 mole) des cristaux blancs obtenus au stade A dans 100 ml d'éthanol.

On refroidit à 0°C, puis on ajoute 0,52 g de NaBH₄, puis on laisse réagir pendant une heure à 0°C.

Ensuite, on ajoute de l'AcOEt et on verse dans une solution saturée de chlorure de sodium, on extrait, on sèche la phase organique sur du sulfate de magnésium et on évapore le solvant sous pression réduite.

On obtient ainsi 10,1 g de 5-(hydroxyméthyl)-1-(2-propényl)-1H-imidazole-4-carboxylate d'éthyle de formule brute C₁₀H₁₄N₂O₃ (M = 210,23 g).

Le rendement correspondant est quantitatif.

### Stade C

On dissout 10,1 g (0,048 mole) de cristaux blancs obtenus au stade B dans 100 ml de trichlorométhane.

On refroidit à -10°C, on ajoute 17,5 ml de SOCl₂, puis on laisse réagir pendant 30 minutes à -10°C et on laisse le milieu réactionnel revenir à la température ambiante.

Ensuite, on ajoute 300 ml de toluène et on évapore le solvant sous pression réduite.

On obtient ainsi 12,8 g de 5-(chlorométhyl)-1-(2-propényl)-1H-imidazole-4-carboxylate d'éthyle de formule brute C₁₀H₁₃N₂O₂Cl,HCl.

Le rendement correspondant est quantitatif.

### Stade D

On dissout 12,8 g (0,048 mole) des cristaux blancs obtenus au stade C dans de l'acétonitrile.

On ajoute à température ambiante 29,83 g de carbonate de potassium et 16,4 g de glycinate de t-butyle.

On laisse réagir une nuit à température ambiante.

On filtre l'insoluble, puis on évapore le solvant sous pression réduite.

On solubilise le résidu dans 200 ml de mélange AcOEt/heptane 2/1, puis on lave avec une solution de NaH₂PO₄ 1 M, on sépare la phase organique et on la sèche sur du sulfate de magnésium.

On évapore le solvant sous pression réduite.

On obtient ainsi 14,1 g de 5-[[[[(1,1-diméthyléthoxy)carbonyl]méthyl]-mino]éthyl]-1-(2-propényl)-1H-imidazole-4-carboxylate d'éthyle de formule brute C₁₆H₂₅N₃O₄ (M =323,40 g).

Le rendement correspondant est est de 91 %.

### Stade E

Dans un ballon placé sous atmosphère d'argon on introduit 14,1 g (0,043 mole) de l'huile jaune obtenue au stade D dans 150 ml de THF.

On ajoute à température ambiante, 10,2 g de (BOC)₂O et on laisse réagir une heure, puis on évapore le solvant sous pression réduite.

On solubilise le résidu dans un mélange heptane/AcOEt 10/20, on lave la phase organique avec une solution aqueuse de NaH₂PO₄ 1M, et on sèche phase organique sur du sulfate de magnésium.

On purifie ensuite le produit brut obtenu sur silice en éluant avec un mélange heptane/AcOEt 2/1.

On recueille ainsi 15,07 g de 5-[[[(1,1-diméthyléthoxy)carbonyl][[(1,1-diméthyléthoxy)carbonyl]méthyl]amino]méthyl]-1-(2-propényl)-1H-imidazole-4-carboxylate d'éthyle de formule brute C₂₁H₃₃N₃O₆ (M = 423,51 g).

Le rendement correspondant est de 82%.

### Stade F

On dissout 6,5 g (0,015 mole) de l'huile incolore obtenue au stade E dans 400 ml de THF.

On ajoute 0,65 g de catalyseur Pd/C à 10% en poids. On place sous atmosphère d'hydrogène et on laisse réagir à température ambiante pendant une heure 30 minutes.

On filtre le catalyseur et on évapore sous pression réduite pour recueillir 6,6 g de 5-[[[(1,1-diméthyléthoxy)carbonyl][[(1,1-diméthyléthoxy)carbonyl]méthyl]-mino]éthyl]-1propyl-1H-imidazole-4-carboxylate d'éthyle de formule brute C₂₁H₃₇N₃O₆ (M = 427,55 g).

Le rendement correspondant est quantitatif.

### Stade G

Dans un ballon placé sous atmosphère d'argon et refroidi par un bain de glace, on introduit 6,6 g (0,0174 mole) de la gomme obtenue au stade F dans 100 ml de THF.

On ajoute ensuite 3,85 g de t-BuOK et on laisse réagir pendant 1 heure à 0°C.

On dilue ensuite avec un mélange heptane/AcOEt 10/20, on lave avec une solution d'hydrogénophosphate de sodium 1M, on sépare la phase organique et on la sèche sur du sulfate de magnésium.

On évapore le solvant sous pression réduite.

On obtient ainsi 5,62 g de 7-hydroxy-3-propyl-3H-imidazo[4,5-c]pyridine-5(4H),6-dicarboxylate de 5,6-di(1,1-diméthyléthyle) d'une huile de formule brute C₁₉H₂₉N₃O₅ (M = 379,46 g).

Le rendement correspondant est quantitatif.

### Stade H

On dissout 5,62 g (0,0174 mole) de l'huile obtenue au stade G dans 60 ml d'acide trifluoroacétique à température ambiante.

On laisse réagir à température ambiante pendant 45 minutes, puis on ajoute du toluène et on évapore le solvant sous pression réduite.

On solubilise ensuite le résidu dans 120 ml de THF. On ajoute 7,5 ml de TEA, puis 4,55 g de (Boc)₂O.

On laisse réagir pendant 1 heure à température ambiante, puis on extrait avec un mélange heptane/AcOEt 10/20, on lave avec une solution de NaH₂PO₄ 1M, on sépare la phase organique et on la sèche sur du sulfate de magnésium.

On évapore le solvant sous pression réduite.

On obtient ainsi 5,2 g de 7-oxo-3-propyl-6,7-dihydro-3H-imidazo[4,5-c]pyridine-5(4H)-carboxylate de 1,1-diméthyléthyle de formule brute C₁₄H₂₁N₃O₃ (M = 279,34 g).

Le rendement correspondant est quantitatif.

### Stade I

On procède comme indiqué au stade A de l'Exemple 6 avec 5,2 g (0,0146 mole) du produit obtenu au stade H et 2,56 g de chlorhydrate de 0-benzylhydroxylamine.

On obtient ainsi 4,1 g de 7-[(phénylméthoxy)imino]-3-propyl-6,7dihydro-3H-imidazo[4,5-c]pyridine-5(4H)-carboxylate de 1,1-diméthyléthyle de formule brute C₂₁H₂₈N₄O₃ (M = 384,48 g).

Le rendement correspondant est de 73%.

### Stade J

On dissout 4,1 g (0,0106 mole) du produit obtenu au stade I dans 8 ml d'acide acétique.

On refroidit à 0°C et on ajoute 1,98 g de cyanoborohydrure de sodium par portions de 0,33 g toutes les 30 minutes, puis on laisse réagir à température ambiante pendant 30 minutes.

On neutralise ensuite le milieu réactionnel avec une solution saturée de bicarbonate de sodium, on extrait avec un mélange AcOEt/heptane, on sèche la phase organique sur sulfate de magnésium et on évapore le solvant sous pression réduite.

On purifie le résidu par chromatographie sur silice en éluant avec un mélange dichlorométhane/acétone 90/10.

On recueille ainsi 3,5 g de 7-[(phénylméthoxy)amino]-3-propyl-6,7dihydro-3H-imidazo[4,5-c]pyridine-5(4H)-carboxylate de 1,1-diméthyléthyle de formule brute C₂₁H₃₀N₄O₃ (M = 386,5 g).

Le rendement correspondant est de 81 %.

### Stade K

On introduit 3,5 g (0,009 mole) du produit obtenu au stade J dans 7 ml d'AcOEt.

On refroidit à 0°C et on ajoute 17,5 ml de chlorure d'hydrogène en solution 5,3 N dans l'AcOEt.

On laisse réagir sous agitation pendant une heure et demie à température ambiante.

On évapore le solvant sous pression réduite.

Le produit cristallisé obtenu est solubilisé dans 900 ml d'acétonitrile, puis on refroidit à 0°C et on ajoute 5,22 ml de TEA.

On ajoute ensuite goutte à goutte, 0,55 ml de diphosgène, on agite pendant 45 minutes. On ajoute 1,3 ml de TEA, on agite 30 minutes et on évapore à sec. On ajoute du dichlorométhane et on lave avec une solution de d'hydrogénophosphate de sodium, puis on sèche la phase organique sur du sulfate de magnésium.

Ensuite, on évapore le solvant sous pression réduite.

On purifie le produit brut obtenu par chromatographie sur silice en éluant avec un mélange toluène/isopropanol 80/20.

On recueille ainsi 0,46 g de 5-(phénylméthoxy)-1-propyl-4,5,7,8-tétrahydro-4,7-méthano-imidazo[4,5-e][1,3]diazépin-6(1H)-one de formule brute C₁₇H₂₀N₄O₂ (M = 312,37 g).

Le rendement correspondant est de 16%.

### Stade L

On procède comme indiqué au stade A de l'Exemple 11 avec 0,46 g du produit obtenu au stade K et 0,9 g de catalyseur Pd/C à 10% en poids et en utilisant de l'acide acétique à la place de l'éthanol.

On obtient ainsi 0,32 g de 5-hydroxy-1-propyl-4,5,7,8-tétrahydro-4,7-méthano-imidazo[4,5-e][1,3]diazépin-6(1H)-one de formule brute C₁₀H₁₄N₄O₂ (M = 222,25 g).

Le rendement correspondant est de 98%.

### Stade M

On procède comme indiqué au stade J de l'Exemple 17 avec 0,314 g (0,0014 mole) du produit obtenu au stade L, 10 ml de pyridine et 0,675 g de complexe SO₃-pyridine.

On obtient 0,215 g du sel de pyridine attendu de formule brute C₁₀H₁₄N₄O₅S (M = 302,31 g).

Le rendement correspondant est de 50,8%.

### Spectre RMN du proton

Dans le DMSO d6, à 300 MHz, déplacements chimiques et multiplicité :
0,84 (t) : CH₃-CH₂-CH₂-N; 1,73 (m) : CH₃-CH₂-CH₂-N; 4,03(m) : CH₃-CH₂-CH₂-N; 3,34 (d) et 3,63 (dd) : N-CH₂-CH-N; 4,92 (d) : N-CH₂-CH-N; 4,40 et 4,47 (AB) : N-CH₂-C=; 8,99 (s) : N=CH
IR (Nujol) : 1766, 1615, 1520 cm⁻¹
SM Electrospray négatif) m/z : [2M+H]⁻ = 603 ; [M]⁻ = 301

### Exemple 23

### Sel de sodium de 6,8-diméthoxy-3-oxo-4-(sulfooxy)-2,3,4,5-tétrahydro-2,5-méthano-1H-pyrido[3,4-e][1,3]diazépine-1-carboxylate d'éthyle

### Stade A

On dissout 2,9 g (17,35 mmole) de 3,5-diméthoxy-4-pyridine carboxaldéhyde de formule brute C₈H₉NO₃ (M = 167,166 g, décrit dans J. Heterocycl. Chem., 11, 251, (1974)), 30 ml de dichlorométhane et 2,3 ml de cyanure de triméthylsilyle.

On amène à 0°C, puis on ajoute 0,120 ml de TEA.

On agite pendant 15 minutes, puis on évapore le solvant sous pression réduite.

On redissout le résidu dans 30 ml d'éthanol et 30 ml d'acide chlorhydrique concentré. On porte au reflux pendant 1 heure.

On ramène le milieu réactionnel à la température ambiante, on dilue avec de la glace, on ajoute de l'AcOEt, puis de l'ammoniaque, tout en refroidissant. La phase organique décantée est lavée 3 fois à l'eau puis séchée sur du sulfate de sodium et filtrée. Après évaporation du solvant sous pression réduite, on obtient 2,34 g de 2,6-diméthoxy-alpha-hydroxy-4-pyridineacétate d'éthyle de formule brute C₁₁H₁₅NO₅ (M =241,24 g).

Le rendement correspondant est de 55,9%.

### Stade B

Dans un ballon placé sous atmosphère d'azote, on introduit 5,34 g (17,35 mmole) du produit obtenu au stade A dans 55 ml de dichlorométhane.

On ajoute 4ml de TEA, 0,270 g de DMAP.

On refroidit à 0°C puis on introduit 1,85 ml de chlorure de mésyle. On agite 1 heure à 0°C.

On verse ensuite le milieu réactionnel sur un mélange d'eau et de dichlorométhane.

On extrait 2 fois avec du dichlorométhane, on lave 2 fois à l'eau, on réunit les phases organiques et on les sèche sur du sulfate de sodium, puis on évapore le solvant sous pression réduite.

On obtient ainsi 7,34 g de 2,6-diméthoxy-alpha-[(méthylsulfonyl)oxy]-4-pyridineacétate d'éthyle de formule brute C₁₂H₁₇NO₇S (M = 319,34 g).

### Stade C

Dans un ballon placé sous atmosphère d'azote, on introduit 8,38 g (24,4 mmole) du produit obtenu au stade B et 40 ml de DMF. On ajoute 3,6 ml de 2,6-lutidine et 6,5 ml de tertbutyl glycinate.

On porte à 80°C pendant 6 heures.

On laisse revenir à température ambiante, puis on ajoute 1,3 ml de terbutyl glycinate et on porte à 80°C pendant 4 heures 30.

On laisse revenir à température ambiante et on verse dans un mélange de glace et d'éther sulfurique. On extrait une fois à l'éther.

On lave 4 fois à l'eau la phase éthérée.

On sèche la phase organique sur du sulfate de sodium, puis on la filtre et on évapore le solvant sous pression réduite.

On entraîne au toluène.

Le produit brut est repris à l'AcOEt, on lave avec une solution aqueuse d'acide tartrique à 10% puis deux fois à l'eau et ensuite avec une solution aqueuse saturée d'hydrogénocarbonate de sodium.

On sèche la phase organique sur du sulfate de sodium, on filtre et on évapore le solvant sous pression réduite.

On recueille 7,65 g de 2,6-diméthoxy-alpha-[[[(1,1-diméthyl-éthoxy)carbonyl]méthyl]amino]-4-pyridineacétate d'éthyle de formule brute C₁₇H₂₆N₂O₆ (M = 354,41 g).

Le rendement correspondant est de 97,6%.

### Stade D

Dans un ballon refroidi par un bain de glace, on introduit 4,68 g (13,21 mmole) du produit obtenu au stade C, 2,2 ml de triéthylamine et 60 ml de dichlorométhane.

On refroidit à 0°C et on ajoute et 2,4 ml d'anhydride trifluoroacétique.

On laisse en contact pendant 2 heures 30.

On verse ensuite le milieu réactionnel sur un mélange glace/ammoniaque/dichlorométhane. On lave à l'eau, extrait au dichlorométhane. On sèche les phases organiques sur du sulfate de sodium. On filtre et on évapore le solvant sous pression réduite.

On obtient ainsi 5,86 g de 2,6-diméthoxy-alpha-[[[(1,1-diméthyléthoxy)-carbonyl]méthyl] (trifluoroacétyl)amino]-4-pyridineacétate d'éthyle de formule brute C₁₉H₂₅F₃N₂O₇ (M = 450,42 g).

Le rendement correspondant est de 98,5%.

### Stade E

Dans un ballon placé sous atmosphère d'azote, on introduit 5,86 g (13 mmoles) du produit obtenu au stade D et 40 ml de dichlorométhane.

On refroidit à 0°C et on introduit rapidement 40 ml d'acide trifluoroacétique.

On laisse remonter à température ambiante puis on laisse sous agitation pendant 4 heures.

On évapore le solvant sous pression réduite.

Le produit est dissout dans de l'AcOEt, lavé successivement par une solution diluée d'ammoniaque puis par une solution aqueuse saturée en NaH₂PO₄.

On sèche ensuite les phases organiques sur du sulfate de sodium, on filtre et on évapore le solvant sous pression réduite.

On obtient ainsi 4,54 g d'une huile qui est purifiée par passage par un sel de cyclohexylamine. Après retour à l'acide attendu, on obtient 3,84 g de alpha-[(carboxyméthyl)(trifluoroacétyl)amino]-2,6-diméthoxy-4-pyridineacétate d'éthyle sous la forme de cristaux incolores fondant à 134-136°C, de formule brute C₁₅H₁₇F₃N₂O₇ (M = 394,31 g).

Le rendement correspondant est de 74,9%.

### Stade F

Dans un ballon placé sous atmosphère d'azote, on introduit 1,97 g (5 mmoles) du produit obtenu au stade E, 10 ml de dichlorométhane et 77 µl de DMF.

On refroidit à 0°C et on introduit une solution de 480 µl de chlorure d'oxalyle dans 2 ml de dichlorométhane.

Après la fin du dégagement gazeux, on laisse revenir à température ambiante. Après la fin du second dégagement gazeux, on évapore le solvant sous pression réduite.

On obtient ainsi l'alpha-[(2-chloro-2-oxoéthyl)(trifluoroacétyl)amino]-2,6-diméthoxy-4-pyridineacétate d'éthyle.

### Stade G

Dans un ballon placé atmosphère d'azote, on introduit le chlorure d'acide préparé au stade F, préalablement mis en solution dans 30 ml de chlorobenzène.

On porte à 90°C, puis on ajoute rapidement 12 ml d'une solution de trichlorure de bore 1 M dans du dichlorométhane.

On maintient au reflux pendant 5 minutes puis on verse le milieu réactionnel sur un mélange glace/AcOEt, on extrait deux fois à l'AcOEt et on lave à l'eau salée. Les phases organiques sont réunies, séchées sur du sulfate de sodium, filtrées puis on évapore le solvant sous pression réduite.

On obtient 1,96 g de produit brut que l'on purifie par chromatographie sur silice en éluant avec un mélange dichlorométhane/isopropanol 95/5.

On recueille ainsi 1,41 g de 5-hydroxy-7-méthoxy-4-oxo-1,2,3,4-tétrahydro-2-(trifluoroacétyl)-2,6-naphthyridine-1-carboxylate d'éthyle, de formule brute C₁₄H₁₃F₃N₂O₆ (M = 362,26 g).

Le rendement correspondant est de 78%.

### Stade H

On procède comme au stade A de l'Exemple 6 avec 1,81 (5 mmoles) du produit obtenu au stade G, 0,603 g (5,5 mmoles) de chlorhydrate d'O-allylhydroxylamine et 1,2 ml de pyridine.

On obtient 2,14 g de 5-hydroxy-7-méthoxy-4-[(2-propényloxy)imino]-1,2,3,4-tétrahydro-2-(trifluoroacétyl)-2,6-naphthyridine-1-carboxylate d'éthyle de formule brute C₁₇H₁₈F₃N₃O₆ (M = 417,34 g).

Le rendement correspondant est quantitatif.

### Stade I

Dans un ballon refroidi par un bain de glace, on introduit 24 ml de méthanol, puis 500 mg de NaBH₄.

On introduit en maintenant la température à O°C, 2,14 g (5 mmoles) du produit préparé au stade H préalablement mis en solution dans 26 ml de dichlorométhane et 4 ml de méthanol.

Lorsque le dégagement gazeux a cessé, on dilue à l'AcOEt, on lave avec une solution saturée de NaH₂PO₄, on extrait deux fois à l'AcOEt, puis on lave les phases organiques à l'eau salée et on les sèche sur du sulfate de sodium, on les filtre et on évapore le solvant sous pression réduite.

On obtient 1,86 g de 5-hydroxy-7-méthoxy-4-[(2-propényloxy)imino]-1,2,3,4-tétrahydro-2,6-naphthyridine-1-carboxylate d'éthyle de formule brute C₁₅H₂₁N₃O₅ (M = 325,35 g).

### Stade J

On dissout 1,86 g du produit obtenu au stade I dans 20 ml de THF.

On refroidit à -10°C, puis on ajoute 2,18 g de (BOC)₂O et 1,5 ml de TEA.

On agite pendant 1 heure 30 en maintenant en dessous de 0°C, puis on rajoute 1,09 de diterbutylcarbonate et 0,75 ml de TEA.

On répète l'opération encore deux fois et on agite pendant 2 heures à -10°C.

On dilue ensuite le milieu réactionnel à l'AcOEt, puis on le lave avec une solution saturée de chlorure de sodium.

On sépare et sèche la phase organique sur du sulfate de sodium, on filtre et on évapore le solvant sous pression réduite.

On obtient ainsi 6,52 g d'une huile que l'on purifie par chromatographie sur silice en éluant avec un mélange dichlorométhane/AcOEt 9/1.

On recueille 1,66 g de 5-hydroxy-7-méthoxy-4-[(2-propényloxy)imino]-3,4-dihydro-2,6-naphthyridine-1,2(1H)-dicarboxylate de 1-éthyle et de 2-(1,1-diméthyléthyle) de formule brute C₂₀H₂₇N₃O₇ (M = 421,25 g).

Le rendement correspondant est de 78,8%.

### Stade K

On dissout 1,66 g du produit obtenu au stade J dans 33 ml de méthanol et on additionne un excès de diazométhane en solution dans le dichlorométhane jusqu'à disparition du produit de départ.

On ajoute au milieu réactionnel de la silice jusqu'à la fin du dégagement gazeux. Ensuite, on filtre et on évapore le solvant sous pression réduite. Le produit brut obtenu est purifié par chromatographie sur silice en éluant avec un mélange AcOEt/hexane 3/7.

On recueille 1,18 g de 5,7-diméthoxy-4-[(2-propényloxy)imino]-3,4-dihydro-2,6-naphthyridine-1,2(1H)-dicarboxylate de 1-éthyle et de 2-(1,1-diméthyléthyle)de formule brute C₂₁H₂₉N₃O₇ (M = 435,48 g).

Le rendement correspondant est de 69%.

### Stade L

On dissout 399 mg (0,92 mmoles) du produit obtenu au stade K, dans 5 ml de méthanol et on ajoute 288 mg de NaBH₃CN.

On ajuste le pH à environ 2 à l'aide d'une solution de chlorure d'hydrogène dans le méthanol. On agite deux heures température ambiante.

On dilue ensuite le milieu réactionnel au dichlorométhane, on ajoute une solution aqueuse d'hydrogénocarbonate de sodium pour ramener le pH vers 9, puis on lave à l'eau salée, on extrait les phases aqueuses au dichlorométhane.

On sépare et sèche les phases organiques sur du sulfate de sodium, on les filtre et on évapore le solvant sous pression réduite.

On obtient ainsi 388 mg de 5,7-diméthoxy-4-[(2-propényloxy)amino]-3,4-dihydro-2,6-naphthyridine-1,2(1H)-dicarboxylate de 1-éthyle et de 2-(1,1-diméthyléthyle) de formule brute C₂₁H₃₁N₃O₇ (M = 437,50 g).

Le rendement correspondant est de 96%.

### Stade M

On dissout 690 mg (1,58 mmoles) du produit obtenu au stade L dans 4 ml de dichlorométhane.

On refroidit à 0°C, puis on ajoute 24 ml de chlorure d'hydrogène en solution dans l'AcOEt à 4 moles/l. On laisse en contact pendant 3 heures.

On évapore le solvant sous pression réduite. On reprend à l'AcOEt, on ajoute de l'hydrogénocarbonate de sodium, puis de l'eau. On sépare et sèche la phase organique sur du sulfate de sodium, on filtre, puis on évapore le solvant sous pression réduite.

On obtient ainsi 0,49 g de 5,7-diméthoxy-4-[(2-propényloxy)amino]-1,2,3,4-tétrahydro-2,6-naphthyridine-1-carboxylate d'éthyle de formule brute C₁₆H₂₃N₃O₅ (M= 337,38 g).

Le rendement correspondant est de 92%.

### Stade N

On procède comme indiqué au stade L de l'Exemple 3 avec 102 mg (0,3 mmoles) du produit obtenu au stade M, 168 µml de TEA et 20µl de diphosgène.

On obtient 70 mg de 6,8-diméthoxy-3-oxo-4-(2-propényloxy)-2,3,4,5-tétrahydro-2,5-méthano-1H-pyrido[3,4-e][1,3]diazépine-1-carboxylate d'éthyle de formule brute C₁₇H₂₁N₃O₆ (M = 363,37 g).

Le rendement correspondant est de 63,6%.

### Stade O

On procède comme indiqué au stade C de l'Exemple 7 avec 94 mg (0,26 mmole) du produit obtenu au stade N, 44µl d'acide acétique, 149 mg de Pd[P(C₆H₅)₃]₄, 123 mg de complexe SO₃-pyridine.

On obtient 53 mg de sel de 1-propényltriphénylphosphonium de 6,8-diméthoxy-3-oxo-4-(sulfooxy)-2,3,4,5-tétrahydro-2,5-méthano-1H-pyrido[3,4-e][1,3]diazépine-1-carboxylate d'éthyle.

Le rendement correspondant est de 29%.

Après passage sur une colonne de résine DOWEX 50WX8 sous forme Na⁺ on obtient 26 mg du sel de sodium attendu, de formule brute C₁₄H₁₆N₃NaO₉S (M = 425,35 g).

Le rendement correspondant à cette échange est de 81 %.

### Spectre RMN du proton

Dans le CDCl₃, à 300 MHz, déplacements chimiques et multiplicité :
1,24 (t) : CH₃-CH₂-O-C=O ; 4,21 (q) : CH₃-CH₂-O-C=O ; 3,38 (d) et 3,50 (dd) : N-CH₂-CH-N; 4,93 (d) : N-CH₂-CH-N; 3,89 (s) et 3,86 (s) : C(O)O-CH₃ ; 4,99 (s) : N-CH-C=O ; 6,33 (s) : CH₃-O-C(N)=CH.

### Exemple 24

### Trans-6-oxo-5-(2-propényloxy)-5,6,7,8-tétrahydro-4,7-méthano-4H-thiéno[2,3-e][1,3]diazépine-8-carboxylate de méthyle

### Stade A

On dissout 48,14 g (0,281 mole) d'alpha-amino-2-thiophèneacétate de méthyle de formule brute C₇H₁₉NO₂S (préparé à partir de l'acide alpha-aminothiophène acétique commercial selon une technique analogue à celle décrite dans J. Med. Chem., 26, 1267-1277 (1983)) dans 930 ml d'acétonitrile.

On ajoute 38,8 g de carbonate de potassium (0,281 mole) puis 55,5 ml de BrCH₂CO₂tBu (0,337 mole).

On chauffe à 70°C pendant 6 heures et demie, puis on laisse revenir à 20°C et on élimine les insolubles par filtration. On concentre partiellement sous pression réduite, on reprend avec 550 ml d'AcOEt, on lave avec de l'eau, puis avec une solution saturée de chlorure de sodium. On sèche la phase organique sur du sulfate de sodium, on filtre et on évapore le solvant sous pression réduite.

On obtient ainsi 90 g de alpha-[[[(1,1-diméthyléthoxy)carbonyl]-méthyl]amino]-2-thiophéneacétate de méthyle de formule brute C₁₃H₁₉NO₄S (M = 285,36 g).

Le rendement correspondant est quantitatif.

### Stade B

On procède comme indiqué au stade B de l'Exemple 3 avec 90 g d'ester obtenu au stade A, 61,7 ml (0,354 mole) de diisopropyléthylamine et 25,2 ml (0,326 mole) de chloroformiate de méthyle.

On recueille 70,9 g de alpha-[[[(1,1-diméthyléthoxy)carbonyl]-méthyl](méthoxycarbonyl)amino]-2-thiophéneacétate de méthyle de formule brute C₁₅H₂₁NO₆S (M = 343,40 g).

Le rendement correspondant aux stades A et B est de 73,4%.

### Stade C

On procède comme indiqué au stade C de l'Exemple 3 avec 70 g (0,203 mole) de l'ester butylique obtenu au stade B et une solution de 450 ml d'acide trifluoroacétique dans 450 ml de dichlorométhane.

On obtient ainsi 75 g de produit brut.

Ce produit brut est purifié de la manière suivante.

On introduit les 75 g de produit brut dans 300 ml d'éther, puis on ajoute à 20°C, goutte à goutte, 33 ml de cyclohexylamine (0,29 mole).

Le sel ayant précipité est filtré et lavé 2 fois avec 50 ml d'éther.

Le produit obtenu est redissout dans 200 ml d'eau, puis on ajoute à 20°C, goutte à goutte, 36 ml d'acide chlorhydrique 6N, puis on décante, on extrait la phase aqueuse 2 fois avec 300 ml d'AcOEt.

On réunit les phases aqueuses et on les lave avec de l'eau, puis avec une solution de chlorure de sodium saturé.

On filtre et on sèche la phase organique sur sulfate de magnésium.

On évapore le solvant sous pression réduite.

On obtient ainsi 59,95 g de alpha-[(carboxyméthyl)-(méthoxycarbonyl)-amino]-2-thiophéneacétate de méthyle de formule brute C₁₁H₁₃NO₆S (M = 287,29 g).

Le rendement correspondant est quantitatif.

### Stade D

On procède comme indiqué au stade D de l'Exemple 3 avec 49,76 g (0,173 mole) de l'acide obtenu au stade C et 57 ml de chlorure de thionyle.

On obtient ainsi 44,50 g de 2,5-dioxo-alpha-(2-thiényl)-3-oxazolidineacétate de méthyle de formule brute C₁₀H₉NO₅S (M = 255,25 g).

Le rendement correspondant est quantitatif.

### Stade E

On procède comme indiqué au stade E de l'exemple 3 avec 44,5 g (0,173 mole) de l'anhydride brut obtenu au stade D, 92,3 g de trichlorure d'aluminium et en remplaçant le traitement à la soude par un traitement à l'acide tartrique et à l'ammoniaque.

On obtient ainsi 32,5 g de 4-oxo-4,5,6,7-tétrahydro-thiéno[2,3-c]pyridine-7-carboxylate de méthyle de formule brute C₉H₉NO₃S (M = 211,24 g).

Le rendement correspondant est de 88%.

### Stade F

On procède comme indiqué au stade F de l'Exemple 3 avec 30 g (0,142 mole) du produit obtenu au stade E et 93 g de (BOC)₂O.

On obtient ainsi 27,91 g de 4,5-dihydro-4-oxo-thiéno[2,3-c]pyridine-6(7H),7-dicarboxylate de 6-(1,1-diméthyléthyle) et de 7-méthyle de formule brute C₁₄H₁₇NO₅S (M = 311,36 g).

Le rendement correspondant est de 63%.

### Stade G

On procède comme indiqué au stade A de l'Exemple 6 avec 6 g (19,3 mmoles) du produit obtenu au stade F et 2,11 g (19,3 mmoles) de chlorhydrate de O-allyl-hydroxylamine.

On obtient ainsi 7,26 g de 4,5-dihydro-4-[(2-propényloxy)imino]-thiéno[2,3-c]pyridine-6(7H),7-dicarboxylate de 6-(1,1-diméthyléthyle) et de 7-méthyle de formule brute C₁₇H₂₂N₂O₅S (M = 366,44 g).

Le rendement correspondant est quantitatif.

### Stade H

On procède comme indiqué au stade B de l'exemple 6 avec 7,25 g (0,0198 mole) de l'oxime obtenu au stade G, 32,3 g de cyanoborohydrure de sodium et 53,7 ml de trifluorure de bore dans l'éther.

On obtient ainsi 7,28 g de cis-4,5-dihydro-4-[(2-propényloxy)amino]-thiéno[2,3-c]pyridine-6(7H),7-dicarboxylate de 6-(1,1-diméthyléthyle) et de 7-méthyle de formule brute C₁₇H₂₄N₂O₅S (M = 368,45 g).

Le rendement correspondant est quantitatif.

### Stade I

On procède comme indiqué aux stades J et K de l'Exemple 3 avec 7,26 g (19,7 mmole) du produit obtenu au stade H et 65 ml de solution 4,3 M de chlorure d'hydrogène dans l'AcOEt puis 20 ml de soude 2N.

On obtient ainsi 4,87 g de cis-4-[(2-propényloxy)amino]-4,5,6,7-tétrahydrothiéno[2,3-c]pyridine-7-carboxylate de méthyle de formule brute C₁₂H₁₆N₂O₃S (M = 268,33 g).

Le rendement correspondant est de 92%.

### Stade J

On procède comme indiqué au stade L de l'Exemple 3 avec 2,16 g (8,04 mmoles) du produit obtenu au stade 1, 2,24 ml de TEA, 535 µl de diphosgène et 98 mg de DMAP.

On obtient 1,227 g de trans-6-oxo-5-(2-propényloxy)-5,6,7,8-tétrahydro-4,7-méthano-4H-thiéno[2,3-e][1,3]diazépine-8-carboxylate de méthyle de formule brute C₁₃H₁₄N₂O₄S (M = 294,33 g)

Le rendement correspondant est de 51 %.

### Spectre RMN du proton

Dans CDCl₃, à 300 MHz, déplacements chimiques et multiplicité :
2,53 (d), 3,65 (dd) : N-CH₂-CH ; 3,85 (s) : CH₃-O ; 4,42 (m) : N-CH₂-CH ; 4,43 (m) : O-CH₂-CH=CH₂ ; 5,31 (dl) et 5,36 (dl) : O-CH₂-CH=CH₂; 5,34 (s) : CH-C(O)-O ; 6,02 (m) : O-CH₂-CH=CH₂ ; 6,95 (d) et 7,25 (d) : thiophène
SM (EI) m/z : [M]⁺ = 294, 266, 238, 235, 199, 41

### Exemple 25

### Sel de sodium de trans-6-oxo-5-(sulfooxy)-5,6,7,8-tétrahydro-4,7-méthano-4H-thiéno[2,3-e][1,3]diazépine-8-carboxamide

### Stade A

On procède comme indiqué au stade A de l'Exemple 7 avec 1,55 g (5,26 mmoles) de l'ester méthylique obtenu au stade J de l'Exemple 24 et 5,3 ml de soude 1N.

On obtient ainsi 1,357 g d'acide trans-6-oxo-5-(2-propényloxy)-5,6,7,8-tétrahydro-4,7-méthano-4H-thiéno[2,3-e][1,3]diazépine-8-carboxylique de formule brute C₁₂H₁₂N₂O₄S (M = 280,30 g).

Le rendement correspondant est de 92%.

### Stade B

On procède comme indiqué au stade B de l'Exemple 10 avec 327 mg (1,16 mmoles) de l'acide obtenu au stade A, 774 mg de BOP, 236 mg de HOBt, 125 mg de chlorure d'ammonium et 0,81 ml de N,N diisopropyléthylamine.

On obtient 305 mg de trans-6-oxo-5-(2-propényloxy)-5,6,7,8-tétrahydro-4,7-méthano-4H-thiéno[2,3-e][1,3]diazépine-8-carboxamide de formule brute C₁₂H₁₃N₃O₃S (M = 279,32 g).

Le rendement correspondant est de 93,7%.

### Stade C

On procède comme indiqué au stade C de l'Exemple 7 avec 481 mg (1,722 mmoles) de l'amide obtenu au stade B, 196 µl d'acide acétique, 1 g de Pd[P(C₆H₅)₃]₄ et 1 g de complexe SO₃-pyridine.

On obtient ainsi 464 mg de sel de 1-propényltriphénylphosphonium de trans-6-oxo-5-(sulfooxy)-5,6,7,8-tétrahydro-4,7-méthano-4H-thiéno[2,3-e][1,3]diazépine-8-carboxamide de formule brute C₃₀H₂₈N₃O₆PS₂ (M = 621,67 g).

Le rendement correspondant est de 43%.

Ce produit est transformé en sel de sodium par passage sur une colonne de résine DOWEX 50WX8 sous forme Na⁺.

On obtient ainsi 164 mg du sel de sodium de trans-6-oxo-5-(sulfooxy)-5,6,7,8-tétrahydro-4,7-méthano-4H-thiéno[2,3-e][1,3]diazépine-8-carboxamide, de formule brute C₉H₈N₃O₆S₂Na (M = 341,30 g).

Le rendement correspondant à cette étape d'échange est de 85%.
Spectre RMN du proton

Dans D₂O, à 300 MHz, déplacements chimiques et multiplicité :
3,46 (d), 3,80 (dd) : N-CH₂-CH; 4,96 (d) : N-CH₂-CH ; 5,36 (s) : CH-CO-NH₂ ; 7,15 (d) et 7,51 (d) : S-CH=CH-
SM (SIMS) m/z : [M+Na]⁺= 364

### Exemple 26

### Sel de sodium de trans-6-oxo-5-(sulfooxy)-5,6,7,8-tétrahydro-4,7-méthano-4H-thiéno[2,3-e][1,3]diazépine-8-carboxylate de méthyle

On procède comme indiqué au stade C de l'Exemple 7 avec 255 mg (0,866 mmoles) de l'ester méthylique obtenu au stade J de l'Exemple 24, 100 µl d'acide acétique, 505 mg de Pd[P(C₆H₅)₃]₄ et 484 mg de complexe SO₃-pyridine.

On obtient ainsi 395 mg de sel de 1-propényltriphénylphosphonium de trans-6-oxo-5-(sulfooxy)-5,6,7,8-tétrahydro-4,7-méthano-4H-thiéno[2,3-e][1,3]diazépine-8-carboxylate de méthyle de formule brute C₃₁H₂₉N₂O₇PS₂ (M = 636,69 g).

Le rendement correspondant est de 71 %.

Ce produit est transformé en sel de sodium par passage sur une colonne de résine DOWEX 50WX8 sous forme Na⁺.

On obtient ainsi 182 mg du sel de sodium attendu, de formule brute C₁₀H₉N₂O₇S₂Na (M = 356,31 g).

Le rendement correspondant à cette étape d'échange est de 74%.

### Spectre RMN du proton

Dans D₂O, à 300 MHz, déplacements chimiques et multiplicité:
3,60 (d), 3,79 (dd) : N-CH₂-CH; 3,89 : COOCH₃ ; 4,96 (d) : N-CH₂-CH ; 5,58 (s1) : N-CH-C=O ; 7,14 (dl) et 7,51 (d1) : S-CH=CH-
SM (Electrospray négatif) m/z : [M]⁻= 333

### Exemple 27

### Sel de sodium de trans-6-oxo-N-(phénylméthyl)-5-(sulfooxy)-5,6,7,8-tétrahydro-4,7-méthano-4H-thiéno[2,3-e][1,3]diazépine-8-carboxamide

### Stade A

On procède comme indiqué au stade B de l'Exemple 7 avec 102 mg (0,36 mmoles) du produit obtenu au stade A de l'Exemple 25, 46 µl de diméthylpyridine, 50 µl de chloroformiate d'isobutyle et 44µl (0,396 mole) de benzylamine.

On obtient ainsi 100 mg de trans-6-oxo-N-(phénylméthyl)-5-(2-propényloxy)-5,6,7,8-tétrahydro-4,7-méthano-4H-thiéno[2,3-e][1,3]diazépine-8-carboxamide de formule brute C₁₉H₁₉N₃O₃S (M = 369,45 g).

Le rendement correspondant est de 83%.

### Stade B

Dans un ballon placé sous atmosphère d'argon, on introduit 150 mg (0,406 mmoles) du produit obtenu au stade B dans 2 ml de dichlorométhane.

On ajoute 46 µl d'acide acétique, puis 237 mg de Pd[P(C₆H₅)₃]₄.

On laisse réagir pendant 10 minutes.

Ensuite, on évapore les solvants sous pression réduite.

On reprend le résidu dans du dichlorométhane et on lave à l'eau. On sépare et sèche la phase organique sur du sulfate de magnésium, on filtre, on évapore le solvant sous pression réduite.

On purifie le résidu par chromatographie sur silice en éluant avec un mélange dichlorométhane/acétone 95/5 contenant 0,1% de TEA, puis avec un mélange dichlorométhane/Acétone 90/10 contenant 0, 1 % de TEA.

On obtient ainsi 95 mg de trans-5-hydroxy-6-oxo-5,6,7,8-tétrahydro-4,7-méthano-4H-thiéno[2,3-e][1,3]diazépine-8-carboxylate de méthyle de formule brute C₁₆H₁₅N₃O₃S (M = 329,38 g) contenant 63% en poids d'oxyde de triphénylphosphine.

Ce produit est utilisé tel quel au stade suivant.

### Stade C

Dans un ballon placé sous atmosphère inerte, on introduit 51 mg (0,057 mmole) du produit obtenu au stade B dans 1 ml de pyridine.

On ajoute 71 mg (0,446 mmole) de complexe SO₃-pyridine.

On agite pendant 3 heures.

Ensuite, on filtre le mélange réactionnel, on rince avec du dichlorométhane et on évapore le solvant sous pression réduite.

On reprend le résidu avec 9 ml d'une solution de KH₂PO₄ dans l'eau (0,5 M).

On agite pendant 15 minutes à température ambiante et on lave 3 fois avec de l' AcOEt.

On ajoute 47 mg de tétrabutylammonium et on extrait 8 fois avec de l'AcOEt.

On rassemble les phases organiques et on les sèche sur du sulfate de magnésium, on filtre et on évapore le solvant sous pression réduite.

On dépose le résidu sur une colonne de résine DOWEX 50WX8 sous forme Na⁺, et on élue avec de l'eau contenant 10% de THF.

On lyophilise le produit recueilli pour obtenir 8,5 mg du sel de sodium attendu, de formule brute C₁₆H₁₄N₃O₆S₂Na (M = 431,43 g).

Le rendement correspondant est de 34%.
SM (Electrospray négatif) m/z : [M]⁻= 408

### Exemple 28

### Sel de sodium de 5-(sulfooxy)-5,6,7,8-tétrahydro-4,7-méthano-4H-furo[2,3-e][1,3]-diazépin-6-one

### Stade A

Dans un ballon refroidi par un bain de glace, on introduit 4,34 ml (30 mmoles) de chloroacétaldéhyde à 45% dans l'eau, 2,52 g d'hydrogénocarbonate de sodium et 20 ml d'eau.

On ajoute ensuite une suspension de 5,33 g (25 mmoles) de 3,5-dioxo-1-pipéridinecarboxylate de 1,1-diméthyléthyle de formule brute C₁₀H₁₅NO₄ (utilisé comme produit de départ au stade A de l'exemple 5) dans 100 ml de THF.

On laisse revenir à la température ambiante et on laisse une nuit sous agitation.

On verse ensuite le milieu réactionnel dans un mélange de glace et de 30 ml d'acide chlorhydrique 1N, puis on extrait 6 fois avec de l'AcOEt, on lave les phases organiques avec une solution saturée de chlorure de sodium, on les sèche sur du sulfate de sodium, on les filtre et on évapore le solvant sous pression réduite.

On obtient ainsi 6,78 g d'une mousse jaune que l'on purifie par chromatographie sur silice en éluant avec du dichlorométhane contenant 3% de méthanol.

On recueille ainsi 2,86 g de 2-hydroxy-2,3,4,7-tétrahydro-4-oxo-furo[2,3-c]pyridine-6(5H)-carboxylate de 1,1-diméthyléthyle de formule C₁₂H₁₇NO₅ (M = 255,27 g) qui est utilisé tel quel dans l'étape suivante.

### Stade B

On dissout les 2,86 g obtenus au stade A (11 mmoles) dans 40 ml de toluène.

On ajoute 418 mg d'acide p-toluène sulfonique monohydraté et on porte à reflux pendant 1 heure.

On verse ensuite dans un mélange de soude 1 N et de glace et on extrait 3 fois avec 50 ml d'AcOEt et on relave les phases organiques avec une solution saturée de chlorure de sodium.

On rassemble les phases organiques et on les sèche sur du sulfate de sodium, on les filtre et on évapore le solvant sous pression réduite.

La résine jaune obtenue est purifiée par chromatographie sur silice, en éluant avec un mélange AcOEt/hexane 2/8.

On recueille ainsi 1,23 g de 4,7-dihydro-4-oxo-furo[2,3-c]pyridine-6(5H)-carboxylate de 1,1-diméthyléthyle de formule brute C₁₂H₁₅NO₄ (M = 237,26 g).

Le rendement correspondant aux stades A et B est de 20%.

### Stade C

On procède comme indiqué au stade A de l'Exemple 6 avec 6,12 g du produit obtenu au stade B (25,8 mmoles), 2,90 g (25,9 mmoles) de chlorhydrate de O-allylhydroxylamine et 2,12 ml de pyridine.

On obtient ainsi 6,8 g de 4,7-dihydro-4-[(2-propényloxy)imino]-furo[2,3-c]pyridine-6(5H)-carboxylate de 1,1-diméthyléthyle de formule brute C₁₅H₂₀N₂O₄ (M = 292,34 g).

Le rendement correspondant est de 96%.

### Stade D

On procède comme indiqué au stade B de l'Exemple 6 avec 6,50 g du produit obtenu au stade C (23 mmoles) et 22,11 g de cyanoborohydrure de sodium et 33,5 ml d'éthérate de trifluorure de bore.

On obtient ainsi 5,9 g de 4,7-dihydro-4-[(2-propényloxy)amino]-furo[2,3-c]pyridine-6(5H)-carboxylate de 1,1-diméthyléthyle de formule brute C₁₅H₂₂N₂O₄ (M = 294,35 g).

Le rendement correspondant est de 87%.

### Stade E

On procède comme indiqué aux stades J et K de l'Exemple 3 avec 5,98 g du produit obtenu au stade D (19,6 mmoles) et 42,9 ml de chlorure d'hydrogène 5M dans l'AcOEt et en remplaçant la soude par de l'ammoniaque.

On obtient ainsi 3,54 g de N-(2-propényloxy)-4,5,6,7-tétrahydro-furo[2,3-c]pyridin-4-amine de formule brute C₁₀H₁₄N₂O₂ (M = 194,24 g).

Le rendement correspondant est de 93%.

### Stade F

On procède comme indiqué au stade L de l'Exemple 3 avec 1,10 g du produit obtenu au stade E (6,19 mmoles), 1,58 ml de TEA et 0,34 ml de diphosgène.

On obtient ainsi 709 mg de 5-(2-propényloxy)-5,6,7,8-tétrahydro-4,7-méthano-4H-furo[2,3-e][1,3]diazépin-6-one de formule brute C₁₁H₂₂N₂O₃ (M = 220,23 g).

Le rendement correspondant est de 52%.

### Stade G

On procède comme indiqué au stade C de l'Exemple 7 avec 418 mg du produit obtenu au stade F (1,9 mmoles), 0,217 ml d'acide acétique, 1,10 g (0,95 mmole) de Pd[P(C₆H₅)₃]₄ et 1,2 g de complexe SO₃-pyridine.

On obtient ainsi 251 mg de sel de 1-propényltriphénylphosphonium de 5-(sulfooxy)-5,6,7,8-tétrahydro-4,7-méthano-4H-furo[2,3-e][1,3]diazépin-6-one de formule brute C₂₉H₂₇N₂O₆PS (M = 562,59 g).

Le rendement correspondant est de 24%.

Après passage d'une solution de la mousse brune sur une colonne de résine DOWEX 50WX8 sous forme Na⁺ on recueille 92 mg du sel de sodium attendu, de formule brute C₈H₇N₂O₆SNa (M = 282,21 g).

Le rendement correspondant à cette étape d'échange est de 73%.

### Spectre RMN du proton

Dans le DMSO d6, à 300 MHz, déplacements chimiques et multiplicité :
3,20 (d) et 3,45 (dd) : N-CH₂ -CH; 4,65 (d) : N-CH₂-CH ; 4,15 et 4,35 (AB) : N-CH₂-C=; 6,52 (d) et 7,55 (d) : O-CH=CH-
SM (Electrospray négatif) m/z : [M]⁻= 259, 96

### Exemple 29

### Trans-6-oxo-5-(2-propényloxy)-5,6,7,8-tétrahydro-4,7-méthano-4H-furo[2,3-e][1,3]diazépine-8-carboxylate de méthyle

### Stade A

Dans un ballon placé sous atmosphère d'azote, on dissout à température ambiante 24,9 g (0,177 mole) d'acide α-oxo 2-furan-acétique dans 500 ml de méthanol.

Puis, on ajoute goutte à goutte, à température ambiante en 5 minutes, 3,5 ml de chlorure de thionyle.

On porte au reflux pendant 2 heures, puis on évapore le solvant sous pression réduite, on ajoute du bicarbonate de sodium, on extrait à l'AcOEt, on lave à l'eau salée, on sèche, puis on évapore le solvant sous pression réduite.

Le résidu est dissous à température ambiante sous atmosphère d'azote dans 280 ml d'éther, puis on ajoute 100 d'acide chlorhydrique 6 M.

On agite pendant 45 minutes à température ambiante, on décante, on lave au bicarbonate de sodium, à l'eau salée, on sépare la phase organique, on la sèche, on la filtre et on évapore le solvant sous pression réduite.

On obtient ainsi 24,45 g de cristaux d'alpha-oxo-2-furanacétate de méthyle de formule brute C₇H₆O₄ (M = 154,12 g).

Le rendement correspondant est de 89%.

### Stade B

Dans un ballon placé sous atmosphère d'azote, on introduit 10,65 g (69,1 mmol) du produit obtenu au stade A, 9,6 g de chlorhydrate d'hydroxylamine, 50 ml d'éthanol et 50 ml de pyridine.

On porte au reflux pendant 55 minutes, puis on évapore le solvant sous pression réduite.

On dissout le résidu dans 200 ml de dichlorométhane, on le lave avec une solution aqueuse d'acide tartrique à 10%, puis à l'eau, on sépare, sèche et filtre la phase organique et on évapore le solvant sous pression réduite.

On obtient ainsi 11,43 g d'alpha-(hydroxyimino)-2-furanacétate de méthyle de formule brute C₇H₇NO₄ (M = 161,14 g).

Le rendement correspondant est de 98%.

### Stade C

Dans un ballon placé sous atmosphère d'azote, on dissout 25,86 g (0,152 mole) du produit obtenu au stade B dans 100 ml d'éthanol.

Puis on ajoute 100 ml d'eau et 100 ml d'acide formique à 99%.

Ensuite, on ajoute en 5 heures à 15-20°C (bain d'eau glacée), 37 g (0,566 mole) de zinc en poudre 325 mesh.

On agite pendant 5 heures 45, puis on filtre, on lave au méthanol et à l'AcOEt, on évapore le solvant sous pression réduite.

Ensuite, on alcalinise avec une solution de carbonate de potassium à 10%, on extrait deux fois avec 500 ml d'un mélange AcOEt/THF 1/1, on lave à l'eau salée, on sépare la phase organique, on la sèche, on la filtre et on évapore le solvant sous pression réduite.

On obtient ainsi 21,13 g d'alpha-amino-2-furanacétate de méthyle de formule brute C₇H₉NO₃ (M = 155,16 g).

Le rendement correspondant est de 90%.

### Stade D

On procède comme indiqué au stade A de l'Exemple 3 avec les 21,13 g (0,136 mole) du produit obtenu au stade C, 31 ml de TEA et 38 ml (0,24 mole) de bromoacétate de t-butyle.

On obtient ainsi 21,65 g d'alpha-[[[(1,1-diméthyléthoxy)carbonyl]-méthyl]amino]-2-furanacétate de méthyle de formule bruteC₁₃H₁₉NO₅ (M = 269,30 g).

Le rendement correspondant est de 59%.

### Stade E

On procède comme indiqué au stade B de l'Exemple 3 avec 26,31 g (0,0977 mole) du produit obtenu au stade D, 22,19 ml de N-éthyldiisopropylamine et 9,31 ml (0,117 mole) de chloroformiate de méthyle.

On obtient ainsi 31,87 g d'alpha-[[[(1,1-diméthyléthoxy)carbonyl]méthyl]-(méthoxycarbonyl)amino]-2-furanacétate de méthyle de formule brute C₁₅H₂₁NO₇ (M = 327,34 g).

Le rendement correspondant est quantitatif.

### Stade F

On procède comme indiqué au stade C de l'Exemple 3 avec les 31,87 g (0,0977 mole) d'ester ter-butylique obtenu au stade E et 127 ml d'acide trifluoroacétique.

On obtient ainsi 26,45 g d'alpha-[(carboxyméthyl)(méthoxycarbonyl)amino]-2-furanacétate de méthyle de formule brute C₁₁H₁₃NO₇ (M = 271,23 g).

Le rendement correspondant est quantitatif.

### Stade G

On dissout 10 g (0,037 mole) du produit obtenu au stade F dans 13 ml de chlorure de thionyle.

On porte à 70°C pendant 5 heures.

Ensuite, on ajoute du dichlorométhane et on évapore le solvant sous pression réduite.

On obtient ainsi 9,99 g d'un résidu huileux.

On introduit dans un ballon 26 g de trichlorure d'aluminium, et 1,2 litre de dichlorométhane.

On ajoute goutte à goutte durant une heure, le résidu huileux obtenu précédemment, en solution dans 800 ml de dichlorométhane.

On porte au reflux pendant 1 heure 30 et on laisse reposer toute la nuit à température ambiante.

On verse ensuite dans 1,4 1 d'une solution molaire de tartrate double de potassium et de sodium contenant de la glace et de l'ammoniaque.

On extrait ensuite 3 fois avec 700 ml de dichlorométhane et on lave avec une solution saturée de chlorure de sodium.

On sépare la phase organique, on la sèche sur du sulfate de magnésium, on filtre et on évapore le solvant sous pression réduite.

On obtient ainsi 3,681 g de 4-oxo-4,5,6,7-tétrahydro-furo[2,3-c]pyridine-7-carboxylate de méthyle de formule brute C₉H₉NO₄ (M = 195,17 g).

Le rendement correspondant est de 51 %.

### Stade H

On procède comme indiqué au stade F de l'Exemple 3 avec 3,68 g (0,019 mole) du produit obtenu au stade G et 14,61 g de (BOC)₂O.

On obtient ainsi 1,77 g de 4,7-dihydro-4-oxo-furo[2,3-c]pyridine-6(5H),7-dicarboxylate de 6-(1,1-diméthyléthyle) et de 7-méthyle de formule brute C₁₄H₁₇NO₆ (M = 295,29 g).

Le rendement correspondant est de 31%.

### Stade I

On procède comme indiqué au stade A de l'Exemple 6 avec 3,36 g (11,34 mmoles) du produit obtenu au stade H, 1,27 g de chlorhydrate d'O-allylhydroxylamine (11,62 mmoles) et 2,75 ml de pyridine.

On obtient ainsi 3,97 g de 4,7-dihydro-4-[(2-propényloxy)imino]-furo[2,3-c]pyridine-6(5H),7-dicarboxylate de 6-(1,1-diméthyléthyle) et de 7-méthyle de formule brute C₁₇H₂₂N₂O₆ (M = 350,37 g).

Le rendement correspondant est de 99%.

### Stade J

On procède comme indiqué au stade B de l'Exemple 6 avec 3,70 g (0,0105 mole) du produit obtenu au stade I, 9,92 g de cyanoborohydrure de sodium et 16 ml d'ethérate de trifluorure de bore dans l'éther.

On obtient ainsi 3,83 g de produit qui contient un mélange de produit de départ et de produit réduit. Ce produit brut est remis en réaction dans les mêmes conditions que précédemment.

On obtient ainsi 3,92 g d'une huile jaune que l'on purifie par chromatographie en éluant avec un mélange éthanol/AcOEt 8/2.

On recueille ainsi 3,36 g de cis-4,7-dihydro-4-[(2-propényloxy)amino]-furo[2,3-c]pyridine-6(5H),7-dicarboxylate de 6-(1,1-diméthyléthyle) et de 7-méthyle de formule brute C₁₇H₂₄N₂O₆ (M = 352,29 g).

Le rendement correspondant est de 90%.

### Stade K

On procède comme indiqué aux stades J et K de l'Exemple 3 avec 3,36 g (0,0095 mole) du produit obtenu au stade J et 36 ml d'une solution 4 M de chlorure d'hydrogène dans l'AcOEt puis de la soude 2N.

On obtient ainsi 1,81 g de cis-4-[(2-propényloxy)amino]-4,5,6,7-tétrahydrofuro[2,3-c]pyridine-7-carboxylate de méthyle de formule brute C₁₂H₁₆N₂O₄ (M = 252,27 g).

Le rendement correspondant est de 75%.

### Stade L

On procède comme indiqué au stade L de l'exemple 3 avec 0,842 g (3,33 mmoles) du produit obtenu au stade K, 1,2 ml de TEA et 0,2 ml de diphosgène.

Le produit obtenu est ensuite épimérisé de la façon suivante.

On ajoute 0,1 ml de DBU et on agite pendant 40 minutes.

On ajoute ensuite 50 ml d'AcOEt et on lave avec 20 ml d'une solution aqueuse d'acide tartrique à 10%, puis avec 20 ml d'une solution de tampon phosphate de pH 7 et avec 50 ml d'une solution de chlorure de sodium.

La phase organique est séchée sur du sulfate de magnésium. On filtre puis on évapore le solvant sous pression réduite.

On purifie le résidu par chromatographie sur silice, en éluant avec un mélange dichlorométhane/AcOET 97/3 contenant 0,1 % de TEA.

On recueille ainsi 0,431 g d'un produit légèrement jaune de formule brute C₁₃H₁₄N₂O₅ (M = 278,27 g).

Le rendement correspondant est de 46%.

### Spectre RMN du proton

Dans le CDCl₃, à 300 MHz, déplacements chimiques et multiplicité :
3,48 (dl) et 3,54 (dd) : N-CH₂-CH-N; 4,39 (d) : N-CH₂-CH-N; 4,40 et 4,47 : O-CH₂-CH=CH₂; 6,01 (m): O-CH₂-CH=CH₂; 5,31 (m) et 5,36 (m): O-CH₂-CH=CH₂; 3,89 (s) : CH₃-O-C=O; 6,45 (d) et 7,34 (d) : O-CH=CH-
SM (Electrospray positif) m/z : [MH]⁺ = 279

### Exemple 30

### Sel de sodium de trans-6-oxo-5-(sulfooxy)-5,6,7,8-tétrahydro-4,7-méthano-4H-furo[2,3-e][1,3]diazépine-8-carboxylate de méthyle

On procède comme indiqué au stade C de l'Exemple 7 avec 0,120 g (0,43 mmole) du produit obtenu au stade L de l'Exemple 29, 0,05 ml d'acide acétique, 0,25 g (0,21 mmole) de Pd[P(C₆H₅)₃]₄ et 0,20 g (1,29 mmole) de complexe SO₃-pyridine.

On obtient ainsi 0,133 g de sel de 1-propényltriphénylphosphonium de trans-6-oxo-5-(sulfooxy)-5,6,7,8-tétrahydro-4,7-méthano-4H-furo[2,3-e] [1,3]diazépine-8-carboxylate de méthyle sous la forme d'une huile.

Le rendement correspondant est de 49%.

Après passage de l'huile jaune sur la colonne de résine DOWEX 50WX8 sous forme Na⁺ on recueille 54 mg du sel de sodium attendu, de formule brute C₁₀H₉N₂O₈SNa (M = 340,245 g).

Le rendement correspondant est de 74%.

### Spectre RMN du proton

Dans D₂O, à 300 MHz, déplacements chimiques et multiplicité :
3,53(d) et 3,71(dd): N-CH₂-CH; 3,88(s): OCH₃; 4,91 (d): N-CH₂-CH; 5,42 (s) : CH-C(O); 6,66 (d) et 7,54 (d) : O-CH=CH
SM (Electrospray négatif) m/z : [M]⁻= 317

### Exemple 31

### Sel de sodium de trans-6-oxo-5-(sulfooxy)-5,6,7,8-tétrahydro-4,7-méthano-4H-furo[2,3-e][1,3]diazépine-8-carboxamide

### Stade A

On procède comme indiqué au stade A de l'Exemple 7 avec 0,718 g (0,026 mole) du produit obtenu au stade N de l'Exemple 29 et 2,84 ml de soude 1N.

On obtient ainsi 0,538 g d'acide trans-6-oxo-5-(2-propényloxy)-5,6,7,8-tétrahydro-4,7-méthano-4H-furo[2,3-e][1,3]diazépine-8-carboxylique de formule brute C₁₂H₁₂N₂O₅ (M = 264,24 g).

### Stade B

On procède comme indiqué au stade B de l'Exemple 10 avec 0,284 g (0,0011 mole) du produit obtenu au stade A, 0,71 g de BOP, 0,22 g de HOBt, 0,12 g (0,0022 mole) d'hydrochlorate d'ammonium et 0,77 ml de N,N diisopropyléthylamine.

On obtient ainsi 0,173 g de cristaux de trans-6-oxo-5-(2-propényloxy)-5,6,7,8-tétrahydro-4,7-méthano-4H-furo[2,3-e][1,3]diazépine-8-carboxamide de formule brute C₁₂H₁₃N₃O₄ (M = 263,26 g).

Le rendement correspondant est de 75%.

### Stade C

On procède comme indiqué au stade C de l'Exemple 7 avec 0,173 g (0,66 mmole) du produit obtenu au stade B, 0,075 ml d'acide acétique, 0,38 g (0,33 mmoles) de Pd[P(C₆H₅)₃]₄ et 0,31 g (1,97 mmoles) de complexe SO₃-pyridine dans 6 ml de pyridine.

On agite à 20°C durant 5 heures, puis on rajoute 0,104 g (0,66 mmole) de complexe SO₃-pyridine.

On obtient ainsi 0,177 g de sel de 1-propényltriphénylphosphonium de trans-6-oxo-5-(sulfooxy)-5,6,7,8-tétrahydro-4,7-méthano-4H-furo[2,3-e][1,3]diazépine-8-carboxamide de formule brute C₃₀H₂₈N₃O₇PS (M = 605,61 g).

Le rendement correspondant est de 44%.

Après passage de la mousse blanche sur la colonne de résine DOWEX 50WX8 sous forme Na⁺ on recueille 87 mg du sel de sodium attendu, de formule brute C₉H₈N₃O₇SNa (M = 325,234 g).

Le rendement correspondant à cette étape d'échange est de 91%.

### Spectre RMN du proton

Dans D₂O, à 300 MHz, déplacements chimiques et multiplicité :
3,41 (d) et 3,70 (dd) : N-CH₂-CH; 4,90 (d) : N-CH₂-CH; 5,26 (s) : CH-C(O)-NH₂; 6,69 (m) et 7,54 (d) : O-CH=CH-
SM(Electrospray négatif) m/z : [M]⁻= 302

### Exemple 32

### 6,8-diméthoxy-4-hydroxy-3-oxo-2,3,4,5-tétrahydro-2,5-méthano-1H-pyrido[3,4-e][1,3]diazépine-1-carboxamide

### Stade A

On procède comme indiqué au stade A de l'Exemple 7 avec 135 mg (0,37 mole) du produit obtenu au stade N de l'Exemple 23 et 0,4 ml de soude 1N.

On obtient ainsi 103 mg d'acide 6,8-diméthoxy-3-oxo-4-(2-propényloxy)-2,3,4,5-tétrahydro-2,5-méthano-1H-pyrido[3,4-e][1,3]diazépine-1-carboxylique de formule brute C₁₅H₁₇N₃O₆ (M = 335,32 g).

Le rendement correspondant est de 83%.

### Stade B

On procède comme indiqué au stade B de l'Exemple 10 avec 29 mg (0,1 mmole) du produit obtenu au stade A, 66 mg de BOP, 20 mg de HOBt, 11 mg (0,2 mmoles) de chlorure d'ammonium et 70 µl de N,N-diisopropyléthylamine.

On obtient ainsi 15 mg de 6,8-diméthoxy-3-oxo-4-(2-propényloxy)-2,3,4,5-tétrahydro-2,5-méthano-1H-pyrido[3,4-e][1,3]diazépine-1-carboxamide de formule brute C₁₅H₁₈N₄O₅ (M = 334,33 g).

Le rendement correspondant est de 45%.

### Stade C

On procède comme indiqué au stade B de l'Exemple 8 avec 41 mg (0,12 mmole) du produit obtenu au stade B, 21 µl d'acide acétique, 71 mg (0,06 mmole) de Pd[P(C₆H₅)₃]₄ et 58,5 mg de complexe SO₃-pyridine et 41 mg d'hydrogénosulfate de tétrabutylammonium.

Après le passage sur la colonne de résine DOWEX 50WX8 sous forme Na⁺ et la lyophilisation, on recueille 5 mg du sel de sodium attendu, de formule brute C₁₂H₁₃N₄O₈SNa (M = 396,31 g).

Le rendement correspondant à cette étape d'échange est de 10,4%.

### Spectre RMN du proton

Dans le DMSO d6, à 300 MHz, déplacements chimiques et multiplicité :
3,42 (dd) et 3,65 (d) : N-CH₂-CH; 4,90 (d) : N-CH₂-CH; 4,77 (s) : N-CH-C(O)-NH₂; 7,45 et 7,89 : N-CH-C(O)-NH₂; 6,24 (s) : CH= aromatique ; 3,84 (s) et 3,85 (s) : OCH₃
SM (Electrospray négatif) m/z : [2M+Na]⁻= 769 ; [M]⁻=373

### Exemple 33

### Sel de sodium de 5-(sulfooxy)-5,6,7,8-tétrahydro-4,7-méthano-4H-furo[3,4-e][1,3]-diazépin-6-one

### Stade A

Dans un ballon placé sous atmosphère d'argon, on introduit 850 mg (5 mmoles) de 3,4-furandicarboxylate de 4-méthyle de formule brute C₇H₆O₅ (M = 170,12 g) et 25 ml de THF anhydre.

On refroidit à 0°C et on ajoute à l'aide d'une seringue 0,728 ml de N-méthylmorpholine et 0,778 ml de chloroformiate d'isobutyle.

On refroidit à -70°C, puis on introduit 9 ml de méthanol et on ajoute 700 mg (17,5 mmoles) de borohydrure de sodium (NaBH₄) à 95%.

On laisse réagir pendant 4 heures puis on ajoute, toutjours à -70°C, 7,5 ml d'acide acétique glacial.

On laisse revenir à la température ambiante, puis on verse dans 100 ml d'une solution saturée de bicarbonate de sodium, on extrait avec du dichlorométhane, on lave avec une solution de chlorure de sodium et on sèche sur du sulfate de magnésium.

On filtre et on évapore le solvant sous pression réduite.

On purifie le résidu par chromatographie sur silice en éluant avec un mélange dichlorométhane/méthanol 97/3.

On recueille ainsi 675 mg de 4-(hydroxyméthyl)-3-furancarboxylate de méthyle de formule brute C₇H₈O₄ (M = 156,14 g).

Le rendement correspondant est de 86%.

### Stade B

Dans un ballon placé sous atmosphère d'azote, on introduit 0,77 g (4,9 mmoles) du produit obtenu au stade A et 16 ml de dichlorométhane.

On abaisse la température à -25°C, puis on introduit 2,04 g (6,2 mmoles) de tétrabromométhane.

On refroidit à -40°C, puis on introduit 1,6 g (6,2 mmoles) de triphénylphosphine.

On laisse réagir 15 minutes à -40°C, puis on laisse la température remonter à 0°C.

Après 30 minutes de réaction, on évapore le solvant sous pression réduite.

On purifie le résidu par chromatographie sur silice en éluant au dichlorométhane.

On recueille 960 mg de 4-(bromométhyl)-3-furancarboxylate de méthyle de formule brute C₇H₇BrO₃ (M = 219,04 g).

Le rendement est quantitatif.

### Stade C

Dans un ballon placé sous atmosphère d'azote, on introduit 960 mg (4,4 mmoles) du produit obtenu au stade B, 18 ml de DMF, 1,82 g de carbonate de potassium et 1,2 ml de glycinate de t-butyle.

On laisse sous agitation pendant 30 minutes.

On filtre pour récupérer le précipité que l'on lave à l'AcOEt. On verse le filtrat dans une solution saturée d'hydrogénophosphate de sodium, on extrait 3 fois avec 50 ml d'AcOEt, on lave 2 fois avec 50 ml d'eau, on sèche, on filtre et on évapore le solvant sous pression réduite.

On purifie le résidu par chromatographie sur silice en éluant avec un mélange dichlorométhane/AcOEt 80/20.

On recueille ainsi 570 mg de 4-[[[[(1,1-diméthyléthoxy)carbonyl]méthyl]-amino]méthyl]-3-furancarboxylate de méthyle de formule brute C₁₃H₁₉NO₅ (M = 269,30 g).

Le rendement correspondant est de 48%.

### Stade D

On procède comme indiqué au stade E de l'Exemple 22 avec 660 mg (2,45 mmoles) du produit obtenu au stade C et 0,588 g (2,7 mmoles) de (BOC)₂O.

On obtient ainsi 1,01 g de 4-[[[(1,1-diméthyléthoxy)carbonyl][[(1,1-diméthyl-éthoxy)carbonyl]méthyl]amino]méthyl]-3-furancarboxylate de méthyle de formule brute C₁₈H₂₇NO₇ (M = 369,42 g).

Le rendement correspondant est quantitatif.

### Stade E

On procède comme indiqué au stade G de l'Exemple 22 avec 185 mg (0,5 mmoles) du produit obtenu au stade D et 1 ml de solution de t-butylate de potassium 1M dans le THF.

On obtient ainsi 150 mg de cristaux de 4,7-dihydro-7-oxo-furo[3,4-c]pyridine-5(6H),6-dicarboxylate de bis-(1,1-diméthyléthyle) de formule brute C₁₇H₂₃NO₆ (M = 337,38 g).

Le rendement correspondant est de 89%.

### Stade F

On procède comme indiqué au stade H de l'Exemple 22 avec 540 mg (1,6 mmoles) du produit obtenu au stade E et 5,4 ml d'acide trifluoroacétique.

Dans un deuxième temps, le produit intermédiaire est mis à réagir avec 418 mg de (BOC)₂O et 0,67 ml de TEA.

On obtient ainsi 345 mg de 4,7-dihydro-7-oxo-furo[3,4-c]pyridine-5(6H)-carboxylate de 1,1-diméthyléthyle de formule brute C₁₂H₁₅NO₄ (M = 237,26 g).

Le rendement correspondant est de 90%.

### Stade G

On procède comme indiqué au stade A de l'Exemple 6 avec 345 mg (1,45 mmoles) du produit obtenu au stade G, et 174 mg de chlorhydrate d'O-allylhydroxylamine.

On obtient ainsi 320 mg de 4,7-dihydro-7-[(2-propényloxy)imino]-furo[3,4-c]pyridine-5(6H)-carboxylate de 1,1-diméthyléthyle de formule brute C₁₅H₂₀N₂O₄ (M = 292,34 g).

Le rendement correspondant est de 75%.

### Stade H

Dans un ballon placé sous atmosphère d'azote, on introduit 118 mg (0,403 mmoles) du produit obtenu au stade H et 1 ml d'acide acétique glacial.

On refroidit à 10°C et on ajoute environ 183 mg de cyanoborohydrure de sodium.

On laisse revenir à température ambiante et on laisse réagir pendant 5 heures.

On reprend avec 20 ml d'AcOEt, on verse dans 50 ml de soude 1N, on décante, on extrait plusieurs fois à l'AcOEt, on lave à nouveau à la soude 1N, puis à l'eau puis avec une solution de chlorure de sodium.

On sèche la phase aqueuse sur du sulfate de magnésium, on filtre et on évapore le solvant sous pression réduite.

On purifie le résidu par chromatographie sur silice en éluant avec un mélange dichlorométhane/AcOEt 97/3.

On recueille ainsi 80 mg de 4,7-dihydro-7-[(2-propényloxy)amino]-furo[3,4-c]pyridine-5(6H)-carboxylate de 1,1-diméthyléthyle de formule brute C₁₅H₂₂N₂O₄ (M = 294,35 g).

Le rendement correspondant est de 67%.

### Stade I

On procède comme indiqué aux stades J et K de l'Exemple 3 avec 180 mg (0,61 mmoles) du produit obtenu au stade I et 1,5 ml d'une solution d'acide chlorhydrique dans l'AcOEt 5,5 M et 1 ml de soude 2N.

On obtient ainsi 100 mg de N-(2-propényloxy)-4,5,6,7-tétrahydro-furo[3,4-c]pyridin-7-amine de formule brute C₁₀H₁₄N₂O₂ (M = 194,24 g).

Le rendement correspondant est de 84%.

### Stade J

On procède comme indiqué au stade H de l'Exemple 20 avec 1,037 g (5,38 mmoles) du produit obtenu au stade J, 0,333 ml de diphosgène, 2,222 ml de TEA et 656 mg de DMAP.

On obtient ainsi 690 mg de 5-(2-propényloxy)-5,6,7,8-tétrahydro-4,7-méthano-4H-furo[3,4-e][1,3]dlazépin-6-one de formule brute C₁₁H₁₂N₂O₃ (M = 220,23 g).

### Le rendement correspondant est de 58%.

### Stade K

On procède comme indiqué au stade C de l'Exemple 7 avec 310 mg (1,4 mmoles) du produit obtenu au stade K, 0,790 g de catalyseur de Pd[P(C₆H₅)₃]₄ (0,7 mmole) et 0,650 g (4,2 mmoles) de complexe SO₃-pyridine.

On obtient ainsi 320 mg de sel de 1-propényltriphénylphosphonium de 5-(sulfooxy)-5,6,7,8-tétrahydro-4,7-méthano-4H-furo[3,4-e][1,3]diazépin-6-one de formule brute C₂₉H₂₇N₂O₆PS (M = 562,59 g).

Le rendement correspondant est de 41%.

Après passage de la résine sur la colonne de résine DOWEX 50WX8 sous forme Na⁺ on recueille 60 mg du sel de sodium attendu, de formule brute C₈H₇N₂O₆SNa (M = 282,21 g).

Le rendement correspondant à cette étape d'échange est de 70%.

### Spectre RMN du proton

Dans D₂O, à 300 MHz, déplacements chimiques et multiplicité :
3,38 (d) et 3,82 (dd) : N-CH₂-CH; 5,02 (d) : N-CH₂-CH; 4,36 et 4,46 (AB) : N-CH₂-C=; 7,35 (sl) et 7,63 (sl) : C=CH-O-CH=C
SM (electrospray négatif) m/z : [M]⁻= 259

### Exemple 34

### 5-(phénylméthoxy)-2-[2-(phénylthio)éthyl]-5,6,7,8-tétrahydro-4,7-méthano-4H-[1,2,3]-triazolo[4,5-e][1,3]diazépin-6(2H)-one

### Stade A

On introduit 19,88 g d'acide 1H-1,2,3-triazole-4,5-dicarboxylique complexé avec du 1H-benzotriazole et obtenu selon Chem. Heterocycl. Compd. (Engl. Transl.), 17, 510-515, (1981), dans 600 ml d'eau, on ajoute 148 ml de soude 1N.

On extrait à trois reprises avec à chaque fois 600 ml d'acétate d'éthyle.

On acidifie la phase aqueuse par ajout de 163 ml d'acide chlorhydrique aqueux 1N (163 mmoles), puis on évapore à sec, on reprend avec 200 ml de toluène, que l'on évapore ensuite sous vide. On répète deux fois cette opération.

On obtient ainsi 19,86 g de produit brut.

On ajoute à ce produit brut 245 ml de méthanol saturé en acide chlorhydrique et on agite pendant 20 heures à température ambiante.

On dilue le mélange réactionnel avec 490 ml de méthanol, on agite pendant 10 minutes la suspension, on filtre, on lave à trois reprises avec 20 ml de méthanol.

On évapore le solvant sous pression réduite.

On obtient un produit que l'on reprend avec 650 ml d'AcOEt, on lave trois fois avec 100 ml d'une solution aqueuse saturée de chlorure de sodium, on sèche la phase organique sur du sulfate de magnésium, on filtre et on évapore le solvant sous pression réduite.

On obtient un résidu que l'on recristallise dans le toluène.

Après séchage sous pression réduite, on obtient 10,92 g de 1H-1,2,3-triazole-4,5-dicarboxylate de méthyle de formule brute C₆H₇N₃O₄ (M = 185,41 g).

Le rendement correspondant est de 81,9%.

### Stade B

Dans un ballon équipé placé sous atmosphère d'argon, on introduit 16,9 ml de 2-hydroxyéthylphénylsulfide (0,125 moles), 21,21 g (0,114 moles) du produit obtenu au stade B et 672 ml de THF anhydre.

On ajoute ensuite goutte à goutte, 60,11 g (0,209 moles) de triphénylphosphine.

On refroidit à 3,5°C, puis on ajoute 19,6 ml (125,91 mmoles) d'azodicarboxylate de diéthyle, puis on laisse le milieu réactionnel revenir à la température ambiante.

On évapore le solvant sous pression réduite, puis on reprend le résidu avec 368 ml de dichlorométhane, on filtre, puis évapore le solvant sous pression réduite.

On obtient un produit brut auquel on ajoute 200 ml de mélange dichlorométhane/AcOEt 97,5/2,5, on filtre pour éliminer le précipité.

Le filtrat est purifié par chromatographie sur silice en éluant avec un mélange dichlorométhane/AcOEt 97,5/2,5.

On recueille 15,07 g d'un isomère A, le 1-[2-(phénylthio)éthyl]-1H-1,2,3-triazole-4,5-dicarboxylate de diméthyle et 10,64 g d'un isomère B de ce produit, le 2-[2-(phénylthio)éthyl]-2H-1,2,3-triazole-4,5-dicarboxylate de diméthyle ainsi que 5,66 g d'un mélange de ces deux isomères A et B.

On chromatographie le mélange sur silice en éluant avec un mélange dichlorométhane/AcOET 97,5/2,5, pour obtenir encore 2,84 g d'isomère A et 1,97 g d'isomère B.

Le rendement total en isomère A est donc de 37% et le rendement total en isomère B est de 46%.

### Stade C

Dans un ballon équipé placé sous atmosphère d'argon, on introduit 17,04 g du diester B obtenu au stade B dans 341 ml de méthanol, puis 55,7 ml de soude 1N.

On agite à température ambiante pendant 15 heures, puis on ajoute 61,3 ml d'acide chlorhydrique 1N.

On évapore le méthanol sous pression réduite, puis on ajoute au résidu 250 ml d'eau, on extrait à l'AcOEt, on lave à l'eau, puis avec une solution aqueuse saturée en chlorure de sodium, on sèche sur du sulfate de magnésium, on filtre et on évapore le solvant sous pression réduite.

On obtient, 15,97 g de 2-[2-(phénylthio)éthyl]-2H-1,2,3-triazole-4,5-dicarboxylate de 4-méthyle de formule brute C₁₃H₁₃N₃O₄ (M = 307,33 g).

Le rendement correspondant est de 98%.

### Stade D

On procède comme indiqué au stade A de l'Exemple 33 avec 15,97 g (51,963 mmoles) du monoester obtenu au stade C, 6,3 ml de N-méthyl morpholine et 7,1 ml de chloroformiate d'isobutyle.

On obtient ainsi 10,39 g de 5-(hydroxyméthyl)-2-[2-(phénylthio)éthyl]-2H-1,2,3-triazole-4-carboxylate de méthyle de formule brute C₁₃H₁₅N₃O₃S (M = 293,34 g).

Le rendement correspondant est de 68,1 %.

### Stade E

On procède comme indiqué au stade B de l'Exemple 33 avec les 10,39 g (35,41 mmoles) du produit obtenu au stade D, 14,68 g (44,27 mmloes) de tétrabromométhane et 11,61 g de triphénylphosphine (44,27 mmoles).

On obtient ainsi 10,04 g de 5-(bromométhyl)-2-[2-(phénylthio)éthyl]-2H-1,2,3-triazole-4-carboxylate de méthyle de formule brute C₁₃H₁₄N₃O₂SBr (M = 356,25 g).

Le rendement correspondant est de 79,6%.

### Stade F

On procède comme indiqué au stade C de l'Exemple 33 avec 9,71 g (27,25 mmoles) du produit obtenu au stade E et 7,45 ml (54,51 mmoles) de glycinate de t-butyle et en remplaçant le DMF par de l'acétonitrile.

On obtient ainsi 7,47 g de 5-[[[[(1,1-diméthyl-éthoxy)carbonyl]méthyl]amino]méthyl]-2-[2-(phénylthio)éthyl]-2H-1,2,3-triazole-4-carboxylate de méthyle de formule brute C₁₉H₂₆N₄O₄S (M = 406,50 g).

Le rendement correspondant est de 67,4%.

### Stade G

On procède comme indiqué au stade E de l'Exemple 22 avec 7,57 g (18,62 mmoles) du produit obtenu au stade F et 5,28 g (24,20 mmoles) de (BOC)₂O.

On obtient ainsi 10,66 g de 5-[[[(1,1-diméthyléthoxy)carbonyl][[(1,1-diméthyléthoxy)carbonyl]méthyl]amino]méthyl]-2-[2-(phénylthio)éthyl]-2H-1,2,3-triazole-4-carboxylate de méthyle de formule brute C₂₄H₃₄N₄O₆S (M = 506,62 g).

Le rendement correspondant est quantitatif.

### Stade H

On procède comme indiqué au stade G de l'Exemple 22 avec 10,66 g (18,62 mmoles) du produit obtenu au stade G et 41 ml d'une solution 1M de t-butylate de potassium dans le THF.

On obtient ainsi 5,35 g de 4,7-dihydro-7-oxo-2-[2-(phénylthio)éthyl]-2H-[1,2,3]-triazolo[4,5-c]pyridine-5(6H),6-dicarboxylate de bis(1,1-diméthyléthyle) de formule brute C₂₃H₃₀N₄O₅S (M = 474,58 g).

Le rendement correspondant est de 60,5%.

### Stade I

On introduit dans un ballon placé sous atmosphère d'argon 5,35 g du produit obtenu au stade H et 54 ml d'acide trifluoroacétique.

Après 45 minutes de contact à température ambiante, on évapore le solvant sous pression réduite.

Le résidu est dissout dans 116ml de THF, on ajoute 4,7 ml de TEA, puis 2,95 de (BOC)₂O.

On laisse réagir à température ambiante pendant 1 heure 30 puis on dilue avec 700 ml d'AcOEt, on lave avec une solution aqueuse d'hydrogénophosphate de sodium 1 M, puis avec unse solution aqueuse saturée de chlorure de sodium, on sèche la phase organique sur du sulfate de magnésium, on filtre et on évapore le solvant sous pression réduite.

On obtient ainsi un produit brut que l'on purifie par chromatographie sur silice en éluant avec un mélange dichlorométhane/AcOEt 97,5/2,5.

On obtient ainsi 4,05 g de 4,7-dihydro-7-oxo-2-[2-(phénylthio)éthyl]-2H-[1,2,3]-triazolo[4,5-c]pyridine-5(6H)-carboxylate de 1,1-diméthyléthyle de formule brute C₁₈H₂₂N₄O₃S (M = 374,46 g).

Le rendement correspondant est de 95,9%.

### Stade J

On procède comme indiqué au stade A de l'Exemple 6 avec 3,03 g (8,09 mmoles) du produit obtenu au stade I et 1,42 g (8,90 mmole) de chlorhydrate d'O-benzylhydroxylamine.

On obtient ainsi 3,82 g de 4,7-dihydro-7-[(phénylméthoxy)imino]-2-[2-(phénylthio)éthyl]-2H-[1,2,3]-triazolo[4,5-c]pyridine-5(6H)-carboxylate de 1,1-diméthyléthyle de formule brute C₂₅H₂₉N₅O₃S (M = 479,60 g).

Le rendement correspondant est de 98,4%.

### Stade K

On procède comme indiqué au stade B de l'Exemple 6 avec 3,82 g (7,96 mmoles) du produit obtenu au stade J, 7,51 g de cyanoborohydrure et 12,1 ml d'étherate de trifluorure de bore.

On obtient ainsi 3,29 g de 4,7-dihydro-7-[(phénylméthoxy)aminol-2-[2-(phénylthio)éthyl]-2H-[1,2,3]-triazolo[4,5-c]pyridine-5(6H)-carboxylate de 1,1-diméthyléthyle de formule brute C₂₅H₃₁N₅O₃S (M = 481,62 g).

Le rendement correspondant est de 85,7%.

### Stade L

On procède comme indiqué aux stades J et K de l'Exemple 3 avec 3,26 g (6,8 mmoles) du produit obtenu au stade K et 29,7 ml (216,71 mmoles) d'acide chlorhydrique 7,3 M dans l'AcOEt, puis 13,6 ml de soude 1N.

On obtient ainsi 2,46 g de 4,5,6,7-dihydro-N-(phénylméthoxy)-2-[2-(phényl-thio)éthyl]-2H-[1,2,3]-triazolo[4,5-c]pyridin-7-amine de formule brute C₂₀H₂₃N₅O₅ (M = 381,50 g).

Le rendement correspondant est de 95%.

### Stade M

On procède comme indiqué au stade H de l'Exemple 30 avec 2,44 g (6,41 mmoles) du produit obtenu au stade L, 425 µl (3,52 mmoles) de diphosgène, 2,68 ml (19,23 mmoles) de TEA et 783 mg de 4-DMAP.

On recueille ainsi 1,66 g de 5-(phénylméthoxy)-2-[2-(phénylthio)éthyl]-5,6,7,8-tétrahydro-4,7-méthano-4H-[1,2,3]-triazolo[4,5-e][1,3]diazépin-6(2H)-one de formule brute C₂₁H₂₁N₅O₂S (M = 407,49 g).

Le rendement correspondant est de 63,4%.

On dissout 1,64 g de l'huile obtenue dans un ballon placé sous atmosphère d'argon avec 66 ml de dichlorométhane.

Puis, on ajoute à température ambiante, 2,38 g d'acide métachloroperbenzoïque à 70%, on agite pendant 1 heure.

Ensuite, on dilue avec 210 ml d'AcOEt et on verse le milieu réactionnel dans une solution aqueuse saturée de bicarbonate de sodium, on extrait à l'AcOEt, on lave avec une solution aqueuse de Na₂S₂O₃, avec une solution aqueuse saturée de bicarbonate de sodium et avec une solution aqueuse saturée de chlorure de sodium.

On sèche la phase organique sur sulfate de magnésium, on évapore le solvant sous pression réduite.

On purifie la mousse blanche obtenue par chromatographie sur silice en éluant avec un mélange dichlorométhane/méthanol 97,5/2,5 contenant 0,1% de TEA.

On recueille ainsi 1,32 g de 5-(phénylméthoxy)-2-[2-(phénylsulfonyl)éthyl]-5,6,7,8-tétrahydro-4,7-méthano-4H-[1,2,3]-triazolo[4,5-e][1,3]diazépin-6(2H)-one, de formule brute C₂₁H₂₁O₄N₅S (M = 439,496).

Le rendement correspondant est de 74,4%.

### Spectre RMN du proton

Dans le CDCl₃, à 300 MHz, déplacements chimiques et multiplicité :
2,90 (d) et 3,59 (dd) : N-CH₂-CH; 4,19 (d) : N-CH₂-CH; 3,78 (tl): S-CH₂-CH₂-N; 4,12 et 4,43 (AB): N-CH₂-C=; 4,73 (m): S-CH₂-CH₂-N; 4,88 et 4,98 (AB) : O-CH₂-C₆H₅; 7,82 (dl), 7,65 (tl), 7,53 (tl) : SO₂-C₆H₅; de 7,34 à 7,46 (m) : O-CH₂-C₆-H₅
SM (Electrospray positif) m/z : [2M+H]⁺ = 879; [M+H+CH₃CN]⁺= 481; [M+Na]⁺= 462 ; [M+H]⁺ = 440,332,291,142

### Stade N

On procède comme indiqué au stade A de l'Exemple 11 avec 419 mg (0,953 mmole) du produit obtenu au stade M et 419 mg de catalyseur Pd/C à 10% en poids.

On obtient 303 mg de 5-hydroxy-2-[2-(phénylsulfonyl)éthyl]-5,6,7,8-tétrahydro-4,7-méthano-4H-[1,2,3]-triazolo[4,5-e][1,3]diazépin-6(2H)-one de formule brute C₂₄H₁₅N₅O₄S (M = 349,37 g).

Le rendement correspondant est de 88,7%.

### Stade O

Dans un ballon équipé placé sous atmosphère d'argon, on introduit 303 mg (0,867 mmole) du produit obtenu au stade N.

On entraîne 3 fois au toluène, puis on ajoute 3,45 ml de pyridine et 414,4 mg (2,60 mmoles) de complexe SO₃-pyridine.

On agite la suspension blanche obtenue à température ambiante pendant 18 heures, puis on ajoute 0,5 ml d'eau, on agite 5 minutes et on évapore sous pression réduite.

On purifie ensuite le résidu par chromatographie sur silice en éluant avec un mélange dichlorométhane/éthanol 70/30 contenant 0,5% de TEA.

On recueille ainsi 377 mg de sel de triéthylammonium de 2-[2-(phénylsulfonyl)éthyl]-5-(sulfooxy)-5,6,7,8-tétrahydro-4,7-méthano-4H-[1,2,3]-triazolo[4,5-e][1,3]diazépin-6(2H)-one de formule brute C₂₀H₃₀N₆O₇S₂ (M = 530,62 g).

Le rendement correspondant est de 81,8%.

On dissout les 377 mg du sel obtenu dans 3 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 288 mg de sel de sodium de 2-[2-(phénylsulfonyl)éthyl]-5-(sulfooxy)-5,6,7,8-tétrahydro-4,7-méthano-4H-[1,2,3]-triazolo[4,5-e][1,3]diazépin-6(2H)-one, de formule brute C₁₄H₁₄N₅O₇S₂Na (M = 451,41 g).

Le rendement correspondant à cette opération d'échange est de 73,6%.

### Spectre RMN du proton

Dans D₂O à 300 MHz, déplacements chimiques et multiplicité :
3,22 (d) et 3,86 (dd) : N-CH₂-CH; 4,19(m) et 4,88(m) : O-CH₂-CH₂-SO₂-C₆H₅; 4,29 (s1) : N-CH₂-C= ; 4,96 (d) : N-CH₂-CH; 7,58 (m) et 7,72 (m) : -SO₂-C₆H₅
SM (Electrospray négatif) m/z : [M+HCO₂H]⁻=474 ; [M]⁻=428

### Exemple 35

### Sel de bis(triéthylammonium) de 5-(sulfooxy)-5,6,7,8-tétrahydro-4,7-méthano-4H-[1,2,3]-triazolo[4,5-e][1,3]diazépin-6(1H)-one

Dans un ballon placé sous atmosphère d'argon, on dissout 97,7 mg (0,216 mmole) du produit obtenu au stade C de l'Exemple 36 dans 2,5 ml de DMF.

On refroit à -10°C, puis on ajoute 12,4 mg d'hydrure de sodium à 50% dans l'huile, puis on agite pendant 1 heure à -10°C.

Ensuite, on ajoute 50µl d'acide acétique (0,873 mmoles), on évapore à sec sous pression réduite.

Le résidu est purifié par chromatographie sur silice, en éluant avec un mélange dichlorométhane/méthanol 70/30 contenant 0,1% de TEA.

On recueille ainsi 33,6 mg du produit attendu, de formule brute C₁₈H₃₇N₇O₅ (M = 463,604 g).

Le rendement correspondant est de 42,8%.

### Spectre RMN du proton

Dans D₂O, à 300 MHz, déplacements chimiques et multiplicité:
1,29 (tl) : CH₃-CH₂-N; 3,21 (ql): CH₃-CH₂-N; 3,50(d) et 3,94 (dd) : N-CH₂-CH; 4,60 (sl): N-CH₂-C=; 5,19 (d) : N-CH₂-CH
IR (nujol): 1765, 1695, 1560, 1540 cm⁻¹
SM (Electrospray négatif) m/z : [M²⁻+H]⁻= 260

### Exemple 36

### Sel de sodium de 2-méthyl-5-(sulfooxy)-4,5,7,8-tétrahydro-4,7-méthano-4H-[1,2,3]-triazolo[4,5-e][1,3]diazépin-6(2H)-one

### Stade A

Dans un ballon équipé placé sous atmosphère d'argon, on dissout 1,23 g (2,81 mmole) du produit obtenu au stade N de l'Exemple 34 dans 41 ml de THF.

On refroidit la solution à -40°C, puis on ajoute 3,36 ml d'une solution de tbutylate de potassium 1M dans le THF.

On agite à -40°C pendant 25 minutes, puis on verse dans une solution aqueuse saturée de chlorure d'ammonium, on extrait à l'AcOEt, on lave avec une solution aqueuse 1M d'hydrogénophosphate de sodium, puis avec une solution aqueuse saturée de chlorure de sodium.

On sèche la phase organique sur du sulfate de magnésium et on évapore le solvant sous pression réduite.

On purifie le résidu par chromatographie sur silice en éluant avec un mélange dichlorométhane/méthanol 95/5.

On recueille ainsi 623 mg de 5-(phénylméthoxy)-5,6,7,8-tétrahydro-4,7-méthano-4H-[1,2,3]-triazolo[4,5-e][1,3]diazépin-6(1H)-one de formule brute C₁₃H₁₃N₅O₂ (M = 271,28 g).

Le rendement correspondant est de 81,8%.

### Stade B

Dans un ballon placé sous atmosphère d'argon, on dissout 200 mg (0,737 mmole) du produit obtenu au stade A dans 6,7 ml de DMF.

On refroidit à O°C, puis on ajoute 140 µl d'iodure de méthyle (2,21 mmole), 46 mg d'hydrure de sodium à 50% dans l'huile.

On laisse sous agitation pendant 1 heure, puis on verse dans une solution aqueuse saturée de chlorure d'ammonium, on extrait à l'AcOEt, on lave avec une solution aqueuse 1M de NaH₂PO₄, ensuite avec une solution aqueuse saturée de chlorure de sodium.

On sèche la phase organique sur sulfate de magnésium, on filtre, on évapore le solvant sous pression réduite.

On purifie le résidu par chromatographie sur silice en éluant avec un mélange dichlorométhane/méthanol 97,5/2,5.

On recueille ainsi 82 mg d'un premier isomère, le 2-méthyl-5-(phénylméthoxy)-5,6,7,8-tétrahydro-4,7-méthano-4H-[1,2,3]-triazolo[4,5-e][1,3]diazépin-6(2H)-one et 57 mg d'un mélange des deux autres isomères, le 1-méthyl-5-(phénylméthoxy)-5,6,7,8-tétrahydro-4,7-méthano-4H-[1,2,3]-triazolo[4,5-e][1,3]diazépin-6(1H)-one et le 3-méthyl-5-(phénylméthoxy)-5,6,7,8-tétrahydro-4,7-méthano-4H-[1,2,3]-triazolo[4,5-e][1,3]diazépin-6(3H)-one.

Le rendement correspondant au premier isomère est de 34%.

Le rendement correspondant au mélange d'isomères est de 24%.

### Stade C

On procède comme indiqué au stade A de l'Exemple 11 avec 73,1 mg (0,256 mmole) du premier isomère obtenu au stade B et 146 mg de catalyseur Pd/C à 10% en poids.

On obtient ainsi 47,5 mg de 5-hydroxy-2-méthyl-5,6,7,8-tétrahydro-4,7-méthano-4H-[1,2,3]-triazolo[4,5-e][1,3]diazépin-6(2H)-one de formule brute C₇H₉N₅O₂ (M = 195,18 g).

Le rendement correspondant est de 95%.

### Stade D

On procède comme indiqué aux stades O et P de l'Exemple 34 avec 47,5 mg (0,2433 mmole) du produit obtenu au stade C, et 116,2 mg (0,730 mmole) de complexe SO₃-pyridine.

On obtient ainsi 66,3 mg de sel de triéthylammonium de 2-méthyl-5-(sulfooxy)-5,6,7,8-tétrahydro-4,7-méthano-4H-[1,2,3]-triazolo[4,5-e][1,3]diazépin-6(2H)-one de formule brute C₁₃H₂₄N₆O₅S (M = 376,41 g).

Le rendement correspondant est de 72,4%.

Après le passage sur la colonne de résine DOWEX 50WX8 sous forme Na⁺, on obtient 48,7 mg du sel de sodium attendu, de formule brute C₇H₈N₅O₅SNa (M = 297,22 g).

Le rendement correspondant est de 93%.

### Spectre RMN du proton

Dans D₂O, à 400 MHz, déplacements chimiques et multiplicité:
3,51 (d) et 3,94 (dd) : N-CH₂-CH; 4,17 (s) : N-CH₃; 4,55 (s) : N-CH₂-C=N; 5,14 (d) : N-CH₂-CH
SM (Electrospray négatif) m/z : [M]⁻= 274

### Exemple 37

### Sel de sodium de 1-méthyl-5-(sulfooxy)-5,6,7,8-tétrahydro-4,7-méthano-4H-[1,2,3]-triazolo[4,5-e][1,3]diazépin-6(1H)-one

On procède comme indiqué aux stades 0 et P de l'Exemple 34 avec 55 mg (0,192 mmole) du mélange d'isomères obtenu au stade B de l'Exemple 36 et 110 mg de catalyseur Pd/C à 10% en poids et 92 mg (0,578 mmole) de complexe SO₃-pyridine.

On obtient ainsi 16,6 mg d'un premier isomère, le sel de triéthylammonium de 1-méthyl-5-(sulfooxy)-5,6,7,8-tétrahydro-4,7-méthano-4H-[1,2,3]-triazolo[4,5-e][1,3]diazépin-6( 1 H)-one
et 17,3 mg d'un second isomère, le sel de triéthylammonium de 3-méthyl-5-(sulfooxy)-5,6,7,8-tétrahydro-4,7-méthano-4H-[1,2,3]-triazolo[4,5-e] [1,3]diazépin-6(3H)-one.

Le rendement correspondant au premier isomère est de 22,9%.

Le rendement correspondant au second isomère est de 23,8%.

On dissout les 17,3 mg du second isomère dans 3 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 11,3 mg du sel de sodium attendu, le sel de sodium de 1-méthyl-5-(sulfooxy)-5,6,7,8-tétrahydro-4,7-méthano-4H-[1,2,3]-triazolo[4,5-e][1,3]diazépin-6(1H)-one, de formule brute C₇H₈N₅O₅SNa (M = 297,22 g).

Le rendement correspondant est de 82,7%.

### Spectre RMN du proton

Dans D₂O, à 400 MHz, déplacements chimiques et multiplicité :
3,41 (d) et 3,91 (dd) : N-CH₂-CH; 3,97 (s): N-CH₃; 4,61 (s): N-CH₂-C=; 5,15 (d) : N-CH₂-CH
SM (Electrospray négatif) m/z : [2M+Na]⁻= 571 ; [M]⁻= 274

### Exemple 38

### Sel de sodium de 3-méthyl-5-(sulfooxy)-5,6,7,8-tétrahydro-4,7-méthano-4H-[1,2,3]-triazolo[4,5-e][1,3]diazépin-6(3H)-one

On dissout les 16,6 mg du premier isomère obtenus au stade B de l'Exemple 37 dans 1 ml d'eau contenant 10% de THF.

On fait passer la solution obtenue sur une colonne de résine DOWEX 50WX8 sous forme Na⁺, en éluant avec de l'eau contenant 10% de THF.

On lyophilise le produit recueilli pour obtenir 11,9 mg du sel de sodium attendu, de formule brute C₇H₈N₅O₅SNa (M = 297,22 g).

Le rendement correspondant est de 90,8%.

### Spectre RMN du proton

Dans D₂O, à 400 MHz, déplacements chimiques et multiplicité :
3,48 (d) et 3,93 (dd) : N-CH₂-CH; 4,11 (s) : N-CH₃ ; 4,56 [AB] : N-CH₂-C= ; 5,34 (d) : N-CH₂-CH;
SM (Electrospray négatif) m/z : [2M+Na]⁻= 571 ; [M]⁻ = 274

### Exemple 39

### Trans-1,2,3,5-tétrahydro-3-oxo-2-(phénylméthoxy)-8-(2-propényloxy)-1,4-méthano-4H-2,4-benzodiazépine-5-carboxylate d'éthyle.

### Stade A

### Etape 1

Dans une solution de 48g (0,253 mole) de chlorhydrate de D,L-Norphenylephrine dans 94 ml de méthanol chauffée à reflux, on introduit à chaud 51,72g d'une solution de glyoxalate d'éthyle à 50 % dans le toluène.

Après 30 minutes de reflux, le chlorhydrate du produit attendu précipite. La suspension est laissée à nouveau 30 minutes à reflux avant d'être refroidie par un bain de glace afin de faire cristalliser le chlorhydrate attendu.

Après avoir ajouté 50 ml d'éther le précipité filtré, lavé à l'éther donne 46 g de 1,2,3,4-tetrahydro-4,6-dihydro-1-isoquinoleinecarboxylate d'éthyle.

### Etape 2

On ajoute 25 ml de TEA à une suspension refroidie à 0°C de 44 g (0,160 mole) du composé obtenu à l'étape précédente dans 500 ml de THF. Puis après changement d'aspect de la suspension, on ajoute 38,7 g (0,177 mole) de (BOC)₂O. On agite ensuite durant 2 heures à 20°C avant de verser le milieu réactionnel sur une solution aqueuse de hydrogénosulfate de sodium à 10 %.

Après extraction au THF et à l'acétate d'éthyle, la phase organique est lavée à nouveau par une première solution de hydrogénosulfate de sodium puis une deuxième solution de dihydrogénophosphate de sodium à 1 molaire. On sèche la phase organique sur du sulfate de magnésium puis on filtre et on évapore le solvant sous pression réduite pour obtenir 60,2 g de 3,4-dihydro-4,6-dihydroxy- 1,2(1H)-isoquinoléinedicarboxylate de 2-(1,1-diméthyléthyle) et de 1-éthyle de formule brute C₁₇H₂₃NO₆ (M = 337,38 g)

### Stade B

On introduit 60 g (1,177 mole) du composé obtenu au stade A dans 600 ml d'acétone. Puis on ajoute 49,4 g de carbonate de potassium et goutte à goutte 29 ml de bromure d'allyle. On chauffe à reflux pendant 2 heures 30 puis on filtre les sels, on évapore l'acétone.

Le résidu est dissout dans un mélange heptane/ACOEt. On lave ensuite la phase organique avec une solution saturée de bicarbonate de sodium, on sèche sur sulfate de magnésium, puis on évapore le solvant sous pression réduite.

On obtient 66 g de 3,4-dihydro-4-hydroxy-6-(2-propényloxy)-1,2(1*H*)-isoquinoléinedicarboxylate de 2-(1,1-diméthyléthyle) et de 1-éthyle de formule brute C₂₀H₂₇NO₆ (M = 377,44 g).

Le rendement correspondant est de 98 %.

### Stade C

On introduit 57,5 g de chlorochromate de pyridinium et 120 g de tamis moléculaire dans 1 litre de dichlorométhane. Puis on introduit à 0°C une solution de 65,5 g (0,178 mole) du composé obtenu au stade B dans 300 ml de dichlorométhane. On agite la solution durant 1 heure 30 en la laissant revenir à température ambiante puis on filtre sur 1 kg de Florisil en éluant avec du dichlorométhane.

Après évaporation sous pression réduite du solvant on obtient 49,92 g de 3,4-dihydro-4-oxo-6-(2-propényloxy)-1,2(1*H*)-isoquinoléinedicarboxylate de 2-(1,1-diméthyléthyle) et de 1-éthyle de formule brute C₂₀H₂₅NO₆ (M = 375,40 g).

Le rendement correspondant est de 78 %.

### Stade D

On introduit 49,9 g du composé obtenu au stade C dans 500 ml de pyridine puis on ajoute 23,3 g de PhCH₂ONH₂,HCl et on agite le milieu réactionnel pendant 1 heure.

On évapore le solvant sous pression réduite puis le résidu est dissous dans un mélange de solvant heptane/ACOEt-1/2

On lave la phase organique 3 fois avec une solution de hydrogénosulfate de sodium à 10 % puis on sèche sur sulfate de magnésium.

On obtient 57 g sous forme d'huile de 3,4-dihydro-4-[(phénylméthoxy)-imino]-6-(2-propényloxy)-1,2(1*H*)-isoquinoléinedicarboxylate de 2-(1,1-diméthyléthyle) et de 1-éthyle de formule brute C₂₇H₃₂N₂O₆ ( M = 480,57 g).

Le rendement correspondant est de 89 %.

### Stade E

On procède comme indiqué au H de l'Exemple 33 avec 56 g du produit obtenu au stade D , 44g de cyanoborohydrure de sodium et 500 ml d'acide acétique glacial

On obtient 22 g de 3,4-dihydro-4-[(phénylméthoxy)amino]-6-(2-propényloxy)-1,2(1*H*)-isoquinoléinedicarboxylate de 2-(1,1-diméthyléthyle) et de 1-éthyle de formule brute C₂₇H₃₄N₂O₆ (M=482,58 g).

Le rendement correspondant est de 40 %.

### Stade F

On dissout 22g du produit obtenu au stade E dans 22 ml d'l'acétate d'éthyle 0°C puis on ajoute 95 ml d'une solution 4,59M d'acide chlorhydrique dans l'ACOEt.

On agite 1h à 20°C puis on filtre les cristaux obtenus.

On introduit ensuite les cristaux obtenus dans 180 ml de dichlorométhane et on ajoute 45 ml de soude 2N.

Après agitation 45 minutes à 20°C, on décante puis la phase organique est séchée sur sulfate de magnésium.

Puis après évaporation du solvant sous pression réduite on obtient 16,5 g de 1,2,3,4-tétrahydro-4-[(phénylméthoxy)amino]-6-(2-propényloxy)-1-isoquinoléine-carboxylate d'éthyle de formule brute C₂₂H₂₆N₂O₄ (M = 382,46 g).

Le rendement correspondant est quantitatif.

### Stade G

On introduit 16,4 g (0,043 mole) du produit obtenu au stade F dans 3,5 litres d'acétate d'éthyle puis on ajoute à 0°C 14,2 ml de TEA et enfin on ajoute goutte à goutte 4,2 g de diphosgène.

On agite 3 heures à 20°C puis on évapore l'acétate d'éthyle et on ajoute du dichlorométhane. La phase organique est lavée avec une solution de sulfate de sodium à 10% puis séchée sur sulfate de magnésium et enfin le solvant est évaporé sous pression réduite.

Le résidu obtenu est solubilisé dans 500 ml de dichlorométhane puis isomérisé par agitation en présence de 1 équivalent de DBU pendant 1 heure.

On purifie le produit brut obtenu par chromatographie sur colonne de silice en éluant avec un mélange de heptane/ acétate d'éthyle -1/1.

On obtient 3,5 g de Trans-1,2,3,5-tétrahydro-3-oxo-2-(phénylméthoxy)-8-(2-propényloxy)-1,4-méthano-4*H*-2,4-benzodiazépine-5-carboxylate d'éthyle de formule brute C₂₃H₂₄N₂O₅ (M = 408 g).

Le rendement correspondant est de 20 %.

### Spectre RMN du proton

Dans le CDCl₃, à 300 MHz, déplacement chimiques et multiplicité :
1,31(t) :CH₃-CH₂-O ; 4,25(m): CH₃-CH₂-O ; 3,47(dd) et 3,63(d): N-CH₂-CH ; 3,68(d) : N-CH₂-CH ; 4,50(dt) : O-CH₂-CH ; 4,93(AB) : O-CH₂-Ph; 5,03 (s) : N-CH-CO; 5,30(qd) et 5,40(qd): CH₂-CH ; 6,03(m); CH₂-CH; 6,53(d) et 6,84(dd): H₂ et H₆ aromatiques en ortho de C-O-; 7,27(m) : H₅ en méta de C-O-et 7,44(m) : H aromatique de O-CH₂-Ph
SM (Electrospray positif) m/z :[MH]⁺ = 409, [MNa]⁺ = 431.
IR CHCl₃) : 1755, 1746, 1649, 1617, 1611, 1573, 1495 cm⁻¹

### Exemple 40

### Sel de sodium de trans-1,2,3,5-tétrahydro-3-oxo-8-propoxy-2-(sulfooxy)-1,4-méthano-4H-2,4-benzodiazépine-5-carboxylate d'éthyle

### Stade A

On procède comme indiqué au stade A de l'Exemple 11 avec 0,15 g du produit obtenu à l'exemple 39, 35 mg de Pd/C à 10% en poids et 3 ml d'éthanol.

On obtient 0,12 g de 1,2,3,5-tétrahydro-2-hydroxy-3-oxo-8-propoxy-1,4-méthano-4*H*-2,4-benzodiazépine-5-carboxylate d'éthyle de formule brute C₁₆H₂ON₂O₅ (M = 320,35 g).

Le rendement est quantitatif.

### Stade B

On procède comme indiqué au stade B de l'Exemple 11 avec 0,12 g du produit obtenu à l'étape précédente A et 0,172 g du complexe SO₃-pyridine et 1,5 ml de pyridine.

On obtient 0,106 g de sel de sodium de trans-1,2,3,5-tétrahydro-3-oxo-8-propoxy-2-(sulfooxy)-1,4-méthano-4*H*-2,4-benzodiazépine-5-carboxylate d'éthyle de formule brute C₁₆H₁₉N₂O₈S.Na (M = 436,42 g).

Le rendement correspondant est de 93 %.

### Spectre RMN du proton

Dans DMSO-d₆, à 300 MHz, déplacements chimiques et multiplicité :
0,97(t) : CH₃-CH₂-CH₂-O-C=; 1,73(m) :CH₃-CH₂-CH₂-O-C=; 3,91(t) : CH₃-CH₂-CH₂-O-C=; 1,24(t): CH₃-CH₂-O-C=; 4,20(q) : CH₃-CH₂-O-C=; 3,51 (m): N-CH₂-CH-N; 4,63(d) N-CH₂-CH(-N)(C=); 4,92(s) : =C-CH(N-)(-C=); 6,67(d) et 6,94(dd) : H₂ et H6 aromatiques en ortho de C-O; 7,23(d) :H₅ aromatique en méta de C-O.
SM (electrospray négatif) m/z : (2M+Na)⁻ = 821,3 ; M⁻= 399,1
IR (CHCl₃): 1745, 1611,1575, 1499 cm-¹

### Exemple 41

### Sel de sodium de trans-1,2,3,5-tétrahydro-8-[(2-méthoxyéthoxy)méthoxy]-3-oxo-2-(sulfooxy)-1,4-méthano-4H-2,4-benzodiazépine-5-carboxamide

### Stade A

On dissout 3,5 g du produit obtenu à l'Exemple 39 dans 35 ml de toluène. On introduit sous argon à O°C 0,191 g de Pd(PPh₃)₄ puis 0,52 g d'acide acétique et enfin goutte à goutte 2,89 g de Bu₃SnH.

On agite 45 minutes à 0°C puis on évapore le solvant sous pression réduite et le résidu est purifié par chromatographie sur silice avec un mélange heptane/AcOEt-1/1.

On obtient 2,92 g sous forme de cristaux blancs de 1,2,3,5-tétrahydro-3-oxo-8-hydroxy-2-(phénylméthoxy)-1,4-méthano-4*H*-2,4-benzodiazépine-5-carboxylate d'éthyle de formule brute C₂₀H₂₀N₂O₅(M = 368 g). Le rendement correspondant est de 96 %.

### Stade B

On dissout 3,68 g du produit obtenu au stade A dans 40 ml de dichlorométhane puis on ajoute 2,72 g de CIMEM et goutte à goutte 2,84 g de DIEA.

On agite 1 heure à 0°C puis on verse le milieu réactionnel dans de l'eau et ensuite la phase organique est lavée avec une solution de dihydrogénophosphate de sodium à 1 molaire

La phase organique extraite est ensuite évaporée sous pression réduite puis le résidu est purifié sur silice en éluant avec une solution de dichlorométhane à 5 % d'acétone.

On obtient 2,8 g de 1,2,3,5-tétrahydro-8-[(2-méthoxyéthoxy)méthoxy]-3-oxo-2-(phénylméthoxy)-1,4-méthano-4*H*-2,4-benzodiazépine-5-carboxylate d'éthyle de formule brute C₂₄H₂₈N₂O₇ (M = 456,50 g).

Le rendement correspondant est de 63%.

### Stade C

On dissout 2,8 g (0,0061 mole) du produit obtenu au stade B dans 30 ml de dioxane et 30 ml d'eau puis on ajoute goutte à goutte 6,13 ml de soude N sans dépasser 15°C.

On agite la solution 45 minutes puis on ajout 15 ml d'eau et on extrait la phase organique basique avec 100 ml d'acétate d'éthyle.

La phase aqueuse est acidifiée à pH = 4, puis on extrait la phase organique avec 5 x 100 ml d'AcOEt.

On évapore le solvant sous pression réduite et on obtient 2,18 g d' Acide 1,2,3,5-tétrahydro-8-[(2-méthoxyéthoxy)méthoxy]-3-oxo-2-(phénylméthoxy)-1,4-méthano-4*H*-2,4-benzodiazépine-5-carboxylique de formule C₂₂H₂₄N₂O₇ (M = 428,45 g).

Le rendement correspondant est de 86 %.

### Stade D

On procède comme au stade B de l'exemple 10 avec 1,83 g (4,27 mmoles) du produit obtenu au stade précédent C, 2,71 g de BOP, 0,865 g de HOBt, 0,456 g de NH₄Cl, 2,97 ml de DIEA et 30 ml de DMF.

On obtient 1,22 g de 1,2,3,5-tétrahydro-8-[(2-méthoxyéthoxy)méthoxy]-3-oxo-2-(phénylméthoxy)-1,4-méthano-4*H*-2,4-benzodiazépine-5-carboxamide de formule C₂₂H₂₅N₃O₆ (M = 427,46 g).

Le rendement correspondant est de 67 %.

### Stade E

On procède comme au stade A de l'Exemple 11 avec 0,15 g du produit obtenu au stade précédent D avec 20 mg de Pd/C à 10% en poids et 2 ml d'acide acétique.

On obtient 0,12 g sous forme de cristaux blancs de 1,2,3,5-tétrahydro-2-hydroxy-8-[(2-méthoxyéthoxy)méthoxy]-3-oxo-1,4-méthano-4H 2,4-benzodiazépine-5-carboxamide de formule brute C₁₅H₁₉N₃O₆ (M= 337,34 g).

Le rendement est quantitatif.

### Stade F

On procède comme au stade B de l'Exemple 11 avec 0,12 g du produit obtenu au stade E précédent, 0,170 g du complexe SO₃-pyridine et 1,5 ml de pyridine.

On obtient 0,12 g sous forme de poudre beige de sel de sodium de trans-1,2,3,5-tétrahydro-8-[(2-méthoxyéthoxy)méthoxy]-3-oxo-2-(sulfooxy)-1,4-méthano-4*H*-2,4-benzodiazépine-5-carboxamide de formule brute C₁₅H₁₈N₃O₉S.Na.

Le rendement correspondant est de 78 %.

### Spectre RMN du proton.

Dans DMSO d₆, à 300 MHz, déplacements chimiques et multiplicité :
3,23(s) : CH₃-O-(CH₂)₂-O-CH₂-O-; 3,46(m) et 3,72(m) : CH₃-O-(CH₂)₂-O-CH₂-O-; 5,23(se) : CH₃-O-(CH₂)₂-O-CH₂-O-; 3,42(dd) et 3,74(m) : N-CH₂-CH-N; 4,59(d) N-CH₂-CH-N; 6,76(d) et 7,01(dd),H₂ et H6 aromatiques en ortho de C-O; 7,17(d) :H₅ aromatique en méta de C-O; 7,41 et 7,88 NH₂-C(=O)CH; 4,74(s) : NH₂-C(=O)CH.
SM (electrospray négatif) m/z : M⁻= 416,1.

### Exemple 42

### Sel de triethylammonium de trans-1,2,3,5-tétrahydro-8-hydroxy-3-oxo-2-(sulfooxy)-1,4-méthano-4H-2,4-benzodiazépine-5-carboxamide

On dissout 90 mg du produit obtenu à l'Exemple 41 dans 2 ml d'un mélange dichlorométhane/acide trifluoroacétique-1/1 contenant 0,4 ml d'anisole.

On agite pendant 30 minutes à 20°C puis on introduit du toluène et on évapore le solvant sous pression réduite.

Le résidu repris par de l'éther pour éliminer l'anisole cristallise sous forme de cristaux verts.Puis on purifie sur plaque de silice 0,5 mm en éluant avec un mélange dichlorométhane/ méthanol 70/30 et 0,1% de triéthylamine

On obtient 20 mg de sel de triéthylamonium de trans-1,2,3,5-tétrahydro-8-hydroxy-3-oxo-2-(sulfooxy)-1,4-méthano-4*H*-2,4-benzodiazépine-5-carboxamide de formule brute C₁₁H₁₀N₃O₇S.C₆H₁₆N (M = 328 g).

Le rendement correspondant est de 30 %

### Spectre RMN du proton.

Dans DMSO d₆, à 300 MHz, déplacements chimiques et multiplicité :
3,38(m) et 3,75(d) : N-CH₂-CH-N; 4,50(d) N-CH₂-CH-N; 4,67(s) : CH-C(=O)-NH₂; 7,36(sl) et 7,83(sl) : CH-C(=O)-NH₂
6,55(d) et 6,71(dd) :H₂ et H6 aromatiques en ortho de C-O ; 7,03 (d) :H₅ aromatique en méta de C-O ; 9,52(s) : Ph-OH
SM (electrospray négatif) m/z : (2M⁻+Na)⁻= 678.9, M⁻= 328,1.

### Exemple 43

### Sel de sodium de trans-7-(acétylamino)-1,2,3,5-tétrahydro-8-hydroxy-3-oxo-2-(sulfooxy)-1,4-méthano-4H-2,4-benzodiazépine-5-carboxamide.

### Stade A

On dissout 3,69 g du produit obtenu au stade A de l'Exemple 41 dans 40 ml d'éthanol et 40 ml de dichlorométhane. Cette solution est ajoutée à une solution contenant 0,84 g de NaNO₃ dans 12 ml d'eau et 7,9 ml d'acide chlorhydrique concentré.

Après ajout de 200 µl de Ac₂O, on agite pendant 5 heures à 20°C.

Le milieu réactionnel est dilué avec du chlorure de méthylène et une solution 1M de NaH₂PO_{4.} .Puis on décante et la phase organique est séchée sur sulfate de magnésium. Le solvant est évaporé sous pression réduite puis l'isomère attendu cristallise dans un mélange ether/dichlorométhane-1/1.

On obtient 1,24 g de 1,2,3,5-tétrahydro-8-hydroxy-7-nitro-3-oxo-2-(phényl-méthoxy)-1,4-méthano-4*H*-2,4-benzodiazépine-5-carboxylate d'éthyle de formule brute C₂₀H₁₉N₃O₇ ( M= 413 g)

Le rendement correspondant est de 30 %.

### Stade B

On procède comme au stade B de l'Exemple 41 avec 0,71 g du produit obtenu au stade précédent A, 0,218 ml de C1MEM, 0,313 ml de DIEA et 12 ml de dichlorométhane.

On obtient 0,88 g de 1,2,3,5-tétrahydro-8-[(2-méthoxyéthoxy)méthoxy]-7-nitro-3-oxo-2-(phénylméthoxy)-1,4-méthano-4*H*-2,4-benzodiazépine-5-carboxylate d'éthyle de formule brute C₂₄H₂₇N₃O₉ ( M= 501 g).

Le rendement correspondant est quantitatif.

### Stade C

On procède comme au stade A de l'Exemple 7 avec 0,88 g du produit obtenu au stade précédent B, 1,8 ml de soude 1N, 25 ml d'un mélange dioxane/eau-15m1/10 ml

On obtient 0,688 g d'acide 1,2,3,5-tétrahydro-8-[(2-méthoxyéthoxy)-méthoxy]-7-nitro-3-oxo-2-(phénylméthoxy)-1,4-méthano-4*H*-2,4-benzodiazépine-5-carboxylique de formule brute C₂₂H₂₃N₃O₉ ( M = 473 g).

### Stade D

On procède comme au stade C de l'Exemple 10 avec 0,68 g du produit obtenu au stade précédent C, 0,911 g de BOP, 0,28 g de HOBT et 0,153 de NH₄Cl et 0,995 ml de DIEA.

On obtient 0,290 g sous forme de cristaux jaunes de 1,2,3,5-tétrahydro-8-[(2-methoxyethoxy)methoxy]-7-nitro-3-oxo-2-(phénylméthoxy)-1,4-méthano-4*H*-2,4-benzodiazépine-5-carboxyamide, de formule brute C₂₂H₂₄N₄O₈, (M = 472 g).

### Stade E

On dissout 0,265 g (0,56 mmole) du produit obtenu au stade précédent D dans 6,5 ml d'un mélange éthanol/eau 4:1. On chauffe à 60°C puis on ajoute 0,66 g de dithionite de sodium, puis on chauffe à 60°C pendant 45 minutes. Ensuite, on verse la solution sur H₂O et on l'extrait plusieurs fois à l'acétate d'éthyle en saturant la phase aqueuse par NaCl.

On sèche sur sulfate de magnésium et le solvant est évaporé sous pression réduite. On obtient 0,125 g sous forme de cristaux blancs de 7-amino-1,2,3,5-tétra-hydro-8-[(2-méthoxyéthoxy)méthoxy]-3-oxo-2-(phénylméthoxy)-1,4-méthano-4*H* 2,4-benzodiazépine-5-carboxamide, de formule brute C₂₃H₂₈N₃O₆ (M = 442 g).

### Stade F

On dissout 80 mg du produit obtenu au stade précédent E dans la pyridine puis on ajoute 8 mg de DMAP et à 0°C un équivalent d'anhydride acétique puis on agite pendant 30 minutes. Ensuite, on évapore sous pression réduite la pyridine et on solubilise le résidu dans un mélange acétate d'éthyle/THF. La solution est lavée avec une solution 1M de NaH₂PO₄ puis celle-ci est séchée sur MgSO₄ avant d'être évaporée sous pression réduite.Le brut est empaté dans l'éther

On obtient 88 mg sous forme de cristaux blancs de 7-(acétylamino)-1,2,3,5-tétrahydro-8-[(2-méthoxyéthoxy)méthoxy]-3-oxo-2-(phénylméthoxy)-1,4-méthano-4*H*-2,4-benzodiazépine-5-carboxamide, de formule brute C₂₄H₂₈N₄O₇ (M = 484 g).

Le rendement correspondant est quantitatif.

### Stade G

On procède comme au stade A de l'Exemple 11 avec 88 mg du produit obtenu au stade précédent E, 20 mg de palladium sur charbon à 10 % en poids et avec 2 ml d'acide acétique.

On obtient le 7-(acétylamino)-1,2,3,5-tétrahydro-2-hydroxy--8-[(2-méthoxyéthoxy)méthoxy]-3-oxo-1,4-méthano-4H 2,4-benzodiazépine-5-carboxamide, de formule brute C₁₇H₂₂N₄O₇ (M = 394 g).

### Stade H

On procède comme au stade Bl'Exemple 11 avec 65 mg du produit obtenu au stade précédent G, 1,5 ml de pyridine, 80 mg du complexe pyridine-SO₃.

On obtient 61 mg sous forme de cristaux jaunes pâles de sel de sodium de 7-(acétylamino)-1,2,3,5-tétrahydro-8[(2-méthoxyéthoxy)méthoxy]-3-oxo-2-(sulfooxy)-1,4-méthano-4H-2,4-benzodiazépine-5 carboxamide de formule brute C₁₇H₂₁N₄O₁₀S.Na (M = 473 g, M = 496 g).

Le rendement correspondant est de 75 %.

### Stade I

On procède comme indiqué à l'Exemple 42 avec 60 mg du produit obtenu au stade précédent H, et avec 0,4 ml d'anisole, 1ml d'acide trifluoroacétique et 1 ml de dichlorométhane.

On obtient 40 mg de sel de sodium de trans-7-(acétylamino)-1,2,3,5-tétra-hydro-8-hydroxy-3-oxo-2-(sulfooxy)-1,4-méthano-4H-2,4-benzodiazépine-5-carboxamide, de formule brute C₁₃H₁₃N₄O₈S.Na (M = 409 g).

Le rendement correspondant est de 81 %.

### Spectre RMN du proton

Dans le DMSO-d₆ à 300 MHz, déplacements chimiques et multiplicité :
3,35 (m) et 3,85 (d) : N-CH₂-CH ; 4,50 (d) : N-CH₂-CH ; 4,67 (s) : CH-C=O-NH₂ ; 7,36 (se) et 7,89 (se) : CH=CO=NH₂ ; 2,07 (se) : CH₃C=ON ; 6,65 (s) et 7,63 (s) pour les H aromatiques ; 9,26 (s) et 9,94 (s) : NH-C=O et Ph-OH.
SM (électrospray négatif) m/z M⁻ = 385 g ; 2 M- + Na (-) = 793 g.

### Exemple 44

### Sel de sodium de 4,5,6,8-tétrahydro-6-oxo-5-(sulfooxy)-1H-4,7-méthanoimidazo [4,5-e][1,3]diazépine-1-caraboxylate de 1,1-diméthyléthyle

### Stade A

On solubilise dans 100 ml de DMF, 20 g du composé 1H-imidazole-4,5-dicarboxylate d'éthyle, 26 g de K₂CO₃, 42,4 g de NaI puis on ajoute goutte à goutte 27,6 ml du 2-chloroéthyle phenyl sulfure. On chauffe ensuite la solution à 60°C et on laisse réagir pendant une nuit. On extrait avec un mélange acétate d'éthyle/heptane 2:1 et du phosphate. Puis, on lave la phase organique et on la sèche sur sulfate de magnésium. Après filtration, on évapore le solvant sous pression réduite et on purifie sur silice en éluant avec un mélange heptane/acétate d'éthyle 1:1. Après évaporation du solvant, on obtient 29,84 g de 1-[2-(phénylthio)éthyl]-1*H*-imidazole-4,5-dicarboxylate de diéthyle, de formule brute C₁₇H₂₀N₂O₄S (M = 348,42 g).

Le rendement correspondant est de 91 %.

### Stade B

On procède comme indiqué au stade Cde l'Exemple 34 avec 33,84 g (0,0971 mole) du produit obtenu au stade précédent A, 102 ml de soude et 280 ml d'éthanol. On obtient 23 g d'un mélange des composés de 1-[2-(phénylthio)éthyl]-1*H*-imidazole-4,5-dicarboxylate de 4-éthyle et de 1-[2-(phénylthio)éthyl]-1*H*-imidazole-4,5-dicarboxylate de 5-éthyle, de formule brute C₁₅H₁₆N₂O₄S (M = 320,37 g).

Le rendement est de 93 %.

### Stade C

On procède comme indiqué au stade A de l'Exemple 33 avec 10,1 g (0,031 mole) du composé obtenu au stade précédent B avec 0,902 g de N-méthyl-morpholine, 4,52 g de chloroformiate d'isobutyle, 3,5 g de borohydrure de sodium, 60 ml de méthanol et 170 ml de THF.

On obtient 10,5 g de 5-(hydroxyméthyl)-1-[2-(phénylthio)éthyl]-1*H*-imidazole-4-carboxylate d'éthyle, de formule brute C₁₅H₂₀N₂O₃S (M = 308,40 g).

Le rendement est quantitatif.

### Stade D

On procède comme indiqué au stade C de l'Exemple 22 avec 18,1 g du produit obtenu au stade précédent C, 21,7 ml de SOCl₂ et 300 ml de chloroforme.

On obtient 23,5 g sous forme de cristaux blancs de 5-(chlorométhyl)-1-[2-(phénylthio)éthyl]-1*H*-imidazole-4-carboxylate d'éthyle, de formule C₁₅H₁₉ClN₂O₂S (M = 326,85 g).

Le rendement est quantitatif.

### Stade E

On procède comme indiqué au stade C de l'Exemple 33 avec 23 g (63,66 mmoles) du composé obtenu au stade C précédent, avec 35,4 g de K₂CO₃, 17,4 ml de glycinate de tert-butyle avec 250 ml d'acétonitrile.

On obtient 29 g sous forme d'huile de 5-[[[2-(1,1-diméthyléthoxy)-2-oxo-éthyl]amino]méthyl]-1-[2-(phénylthio)éthyl]-1H imidazole-4-carboxylate d'éthyle, de formule brute C₂₁H₃₁N₃O₄S (M = 421,56 g).

Le rendement est quantitatif.

### Stade F

On procède comme indiqué au stade E de l'Exemple 22, avec 29 g du composé obtenu au stade précédent E, 16,5 g (BOC)₂O et 300 ml de THF.

On obtient 20,8 g sous forme d'huile de 5-[[[(1,1-diméthyléthoxy)carbonyl]-[2-(1,1-diméthyléthoxy)-2-oxoéthyl]amino]méthyl]-1-[2-(phénylthio)éthyl]-1*H-*imidazole-4-carboxylate d'éthyle, de formule brute C₂₆H₃₉N₃O₁₀S (M = 521,68 g).

### Stade G

On procède comme indiqué au stade G de l'Exemple 22, avec 20,2 g du produit obtenu au stade précédent F, deux équivalents de tert-butylate de potassium et 400 ml de THF.

On obtient 13,9 g sous forme de cristaux blancs de 3,4,6,7-tétrahydro-7-oxo-3-[2-(phénylthio)éthyl]-5*H*-imidazo[4,5-*c*]pyridine-5,6-dicarboxylate de bis (1,1-diméthyléthyle), de formule brute C₂₄H₃₁N₃O₅S (M = 473,60 g).

Le rendement correspondant est de 76 %.

### Stade H

On procède comme indiqué au stade H de l'Exemple 22, avec 13,9 g (0,029 mole) du composé obtenu au stade précédent G et avec 140 ml d'acide trifluoroacétique, 7,58 g de (BOC)₂O, 12,4 ml de triéthylamine et 300 ml de toluène et 130 ml de tétrahydrofurane.

On obtient 10,5g sous forme de cristaux beiges de 3,4,6,7-tétrahydro-7-oxo-3-[2-(phénylthio)éthyl]-5*H*-imidazo[4,5-*c*]pyridine-5,6-carboxylate de 1,1-diméthyléthyle, de formule brute C₁₉H₂₃N₃O₃S (M = 373,48 g).

Le rendement correspondant est de 96 %.

### Stade I

On procède comme indiqué au stade A de l'Exemple 6 avec 10,4 g (0,0278 mole) du produit obtenu au stade précédent H et avec 30 ml de pyridine et 4,88 g de chlorhydrate de 2-O-benzylhydroxylamine.

On obtient 11,1 g sous forme de cristaux blancs de 3,4,6,7-tétrahydro-7-[(phénylméthoxy)imino]-3-[2-(phénylthio)éthyl]-5*H*-imidazo[4,5-*c*]pyridine-5-carboxylate de 1,1-diméthyléthyle, de formule brute C₂₆H₃₀N₄O₃S (M = 478,62 g).

Le rendement correspondant est de 84 %.

### Stade J

On procède comme indiqué au stade J de l'Exemple 22 avec 11 g du produit obtenu au stade précédent 1, 2,1 g de cyanoborohydrure de sodium, 30 ml d'acide acétique.

On obtient 9,5g sous forme d'huile de 3,4,6,7-tétrahydro-7-[(phényl-méthoxy)amino]-3-[2-(phénylthio)éthyl]-5*H*-imidazo[4,5-*c*]pyridine-5-carboxylate de 1,1-diméthyléthyle de formule brute C₂₆H₃₂N₄O₃S (M 480,63 g).

Le rendement est de 82 %.

### Stade K

On dissout dans 20 ml d'acétate d'éthyle à 0°C, 9,5 g du produit obtenu au stade précédent J puis on ajoute 46 ml d'une solution d'acide chlorhydrique dans l'acétate d'éthyle à 4,3 M. On agite pendant 45 minutes à 20°C et on refroidit à 0°C. Le précipité obtenu est filtré rapidement puis lavé à l'éther et séché sous vide sous P₂O₅.

On obtient 8,33 g sous forme de cristaux blancs de chlorhydrate de 4,5,6,7-tétrahydro-7-[(phénylméthoxy)amino]-3-[2-(phénylthio)éthyl]-3*H*-imidazo[4,5-*c*]pyridine, de formule brute C₂₁H₂₄N₄OS (M = 417,02 g).

Le rendement est quantitatif.

### Stade L

On dissout 8,1 g (0,018 mole) du produit obtenu au stade précédent K dans 1,7 litres d'acétonitrile puis on ajoute à 0°C 9 ml de triéthylamine. On ajoute ensuite goutte à goutte 1,75 ml de diphosphogène. Puis on agite pendant deux heures à température ambiante., On ajoute ensuite deux équivalents de triéthylamine et on agite la solution pendant 45 mn. Puis on évapore le solvant sous pression réduite, le résidu est solubilisé dans du dichlorométhane contenant 6 % de méthanol. Puis on lave la phase organique avec une solution de NaH₂PO₄ 1M. La phase organique est séchée sur MgSO₄ et le solvant est évaporé sous pression réduite.

On obtient un résidu qui est purifié sur silice en éluant avec un mélange de toluène/alcool isopropylique à 20 %. Ensuite, les solvants sont évaporés sous pression réduite et la gomme est cristallisée dans un mélange acétate d'éthyle/éther.

On obtient 1,5 g sous forme de cristaux de 1,4,5,8-tétrahydro-5-(phényl-méthoxy)-1-[2-(phénylthio)éthyl]-6*H*-4,7-méthanoimidazo[4,5-*e*][1,3]diazépin-6-one, de formule brute C₂₂H₂₂N₄O₂S (M = 406,51 g).

Le rendement est de 20 %.

### Stade M

On dissout 1,5 g du produit obtenu au stade L dans du dichlorométhane. Puis on ajoute à température ambiante 1,85 g d'acide méthachloroperbenzoïque et on agite la solution durant 3 heures. Après avoir ajouté 50 ml de dichlorométhane à la solution, celle-ci est lavée avec une solution aqueuse de bisulfite de sodium, puis de NaHCO₃. La phase organique est séchée sur sulfate de magnésium et le solvant est évaporé sous pression réduite. Le produit brut est purifié sur silice en éluant avec un mélange de dichlorométhane à 3 % dans le méthanol.

On obtient 1,33 g sous forme de mousse cristalline blanche de 1,4,5,8-tétra-hydro-5-(phénylméthoxy)-1-[2-(phénylsulfonyl)éthyl]-6*H*-4,7-méthanoimidazo [4,5-*e*][1,3]diazépin-6-one, de formule brute C₂₂H₂₂N₄O₄S (M = 438,51 g).

Le rendement correspondant est de 84 %.

### Stade N

On dissout 1,1 g du produit obtenu au stade précédent M dans 15 ml de DMF anhydre. Puis on ajoute sous argon et à -10°C, 0,11 g d'hydrure de sodium. Après 45 minutes, on ajoute 0,4 ml d'acide acétique et 50 ml de toluène. On élimine les solvants sous pression réduite, le résidu obtenu est purifié sur silice en éluant avec un mélange toluène/alcool isopropylique à 30 %.

On obtient 0,41 g de 1,4,5,8-tétrahydro-5-(phénylméthoxy)-6H-4,7-métha-noimidazo[4,5-e][1,3]diazépin-6-one, de formule brute C₁₄H₁₄N₄O₂ (M = 270,29 g).

Le rendement correspondant est de 61 %.

### Stade 0

On dissout 0,385 g du produit obtenu au stade précédent N dans 10 ml de THF puis on ajoute 0,31 g de (BOC)₂O. On ajoute ensuite 0,2 ml de triéthylamine puis on agite pendant 2 heures 30. On évapore les solvants sous pression réduite et le résidu est purifié sur silice avec un mélange chlorure de méthylène/acétone à 5 %.

On obtient 0,44 g sous forme d'huile de 4,5,6,8-tétrahydro-6-oxo-5-(phényl-méthoxy)-1*H*-4,7-méthanoimidazo[4,5-*e*][1,3]diazépine-1-carboxylate de 1,1-diméthyléthyle, de formule brute C₁₉H₂₂N₄O₄ (M = 370,41 g).

Le rendement est quantitatif.

### Stade P

On procède comme indiqué au stade A de l'Exemple 11, avec 0,44 g du produit obtenu au stade précédent 0, 12 ml d'éthanol, 60 mg de palladium sur charbon à 10 % en poids.

On obtient 0,3 g sous forme de cristaux blancs de 4,5,6,8-tétrahydro-5-hydroxy-6-oxo-1*H*-4,7-méthanoimidazo[4,5-*e*][1,3]diazépine-1-carboxylate de 1,1-diméthyléthyle, de formule brute C₁₂H₁₆N₄O₄ (M = 280,29 g).

Le rendement correspondant est de 91 %.

### Stade Q

On procède comme indiqué au stade B de l'Exemple 11 avec 0,3 g du produit obtenu au stade précédent P, 3 ml de pyridine et 3 équivalents du complexe SO₃-pyridine.

On obtient 0,196 g sous forme de cristaux jaunes de sel de sodium de 4,5,6,8-tétrahydro-6-oxo-5-(sulfooxy)-1H-4,7-méthanoimidazo[4,5-*e*][1,3]diazépine-1-carboxylate de 1,1-diméthyléthyle, de formule brute C₁₂H₁₅N₄O₇S.Na (M = 382,33 g).

Le rendement correspondant est de 50 %.

### Spectre RMN du proton

Dans DMS-d₆ à 300 MHz, déplacements chimiques et multiplicité :
1,56 (s) : BOC ; 3,22 (d) et 3,24 (d) et 3,52 (dd) et 3,53 (dd) : N-CH₂-CH ; 4,62 (d) et 4,71 (d) : N-CH₂-CH ; 4,11 et 4,24, 4,28, 4,31 : N-CH₂-C= ; 7,66 (s) et 8,08 (s) : N=CH-N.
SM (électrospray négatif) m/z :
(2M+Na)⁻ = 741 ; (2M+H)⁻ = 719 ; (M)- = 359

### Exemple 45

### Sel de sodium de 1,4,5,8-tétrahydro-5-(sulfooxy)-6H-4,7-méthanoimidazo [4,5-e][1,3]diazépin-6-one

On dissout 0,155 g du produit obtenu à l'Exemple 44, dans 2 ml d'acide trifluoroacétique. On agite 5 minutes puis on évapore le solvant sous pression réduite en l'entraînant avec 2 x 10 ml de toluène. Après séchage, le résidu est cristallisé dans l'acétone.

On obtient 120 mg sous forme de cristaux beiges de sel de sodium et de trifluoroacétate de 1,4,5,8-tétrahydro-5-(sulfooxy)-6*H*-4,7-méthanoimidazo [4,5-e][1,3]diazépine-6-one, de formule brute C₇H₇N₄O₅S.Na.CF₃CO₂H (M = 282,21 g).

Le rendement est quantitatif.
SM (électrospray négatif) m/z :
(2M+Na)⁻ = 541 ; (2M+H)⁻ =519 ; (M)⁻ = 259.

### Exemple 46

### Sel de sodium de trans-1,2,3,5-tétrahydro-3-oxo-9-propoxy-2-(sulfooxy)-1,4-méthano-4H-2,4-benzodiazépine-5-carboxylate d'éthyle

### Stade A

On dissout 50 g de 3-méthoxy-benzaldéhyde dans 245 ml d'acide acétique. A 0°C, on ajoute goutte à goutte 22 ml de brome, on agite à température ambiante pendant 4 heures, puis on laisse une nuit à température ambiante. On ajoute 300 ml d'eau à la solution et le produit attendu cristallise. Après filtration et lavage à l'eau et séchage, on obtient 68 g de 2-bromo-5-méthoxy-benzaldéhyde, de formule brute C₈H₇Br.O₂ (M = 114 g).

Le rendement correspondant est de 87 %.

### Stade B

On dissout 30 g du produit obtenu à l'étape précédente B dans 200 ml de dichlorométhane. On ajoute à 0°, 0,4 g de ZnI₂ puis goutte à goutte 20,86 ml de TMSCN. On agite pendant 1h30, puis on verse le mélange dans une solution aqueuse saturée en bicarbonate de sodium. Après décantation, la phase organique est séchée sur sulfate de magnésium et le solvant est évaporé sous pression réduite. Le produit brut est solubilisé dans 9 ml d'éthanol et 6 ml d'acide chlorhydrique concentré. Après chauffage à reflux pendant 1 h, on verse le milieu réactionnel dans une solution saturée en NaHC0₃, puis on extrait avec de l'acétate d'éthyle. Après évaporation du solvant sous pression réduite, on obtient 41 g de 2-bromo-α-hydroxy-5-méthoxy-benzèneacétate d'éthyle, de formule brute C₁₁H₁₃BrO₄ (M = 288 g).

Le rendement correspondant est de 63 %.

### Stade C

On procède comme indiqué au stade B de l'Exemple 23 avec 23,5 g du produit obtenu à l'étape précédente C, 100 ml de pyridine, 6,31 ml de chlorure de mésyle.

On obtient 32 g sous forme d'huile du composé attendu de formule brute C₁₇H₂₄N.BrO₅ (M = 401 g).

### Stade D

On procède comme indiqué au stade C de l'Exemple 23 avec 30 g du produit obtenu au stade précédent C, 130 ml de DMF, 16 g de glycinate de tert-butyle et 9,66 ml de lutidine.

On obtient 15,3 g sous forme d'huile de 2-bromo-α-[[2-(1,1-diméthyléthoxy)-2-oxoéthyl]amino]-5-méthoxy-benzèneacétate d'éthyle, de formule brute C₁₇H₂₄BrNO₅ (M = 402,29 g).

Le rendement correspondant est de 47 %.

### Stade E

On procède comme indiqué au stade D de l'Exemple 23 avec 6 g du produit obtenu au stade précédent D, 60 ml de dichlorométhane, 1,2 équivalent de triéthylamine, 1,3 équivalent d'anhydride trifluoroacétique.

On obtient 6,05 g de 2-bromo-α-[[2-(1,1-diméthyléthoxy)-2-oxoéthyl]-(trifluoroacétyl)amino]-5-méthoxy-benzèneacétate d'éthyle, de formule brute C₁₉H₂₃ BrF₃NO₆ (M = 498,30 g).

Le rendement correspondant est de 81 %.

### Stade F

On procède comme indiqué au stade E de l'Exemple 23 avec 6,05 g du produit obtenu au stade précédent E, 30 ml de dichlorométhane, 30 ml d'acide trifluoroacétique.

On obtient 5,2 g sous forme de cristaux blancs de 2-bromo-α-[((carboxy-méthyl)(trifluoroacétyl)amino]-5-méthoxy-benzèneacétate d'éthyle, de formule brute C₁₅H₁₅BrF₃NO₆ (M = 442,19 g).

Le rendement correspondant est de 97 %.

### Stade G

### Etape 1 - Préparation du chlorure d'acide

61 g du produit obtenu au stade F est solublisé dans 120 ml de SOCl₂. On chauffe à 80° C pendant 2h puis on évapore à sec.

### Etape 2

Le chlorure d'acide est solubilisé dans 300 ml de CH₃NO₂. Puis on ajoute par fractions 76 g de chlorure d'aluminium et on agite une nuit à température ambiante. Puis on verse le milieu réactionnel dans un mélange hepthane/acétate d'éthyle et on lave la phase organique avec NaH₂PO₄ 1M. Après séchage de la phase organique sur MgSO₄ et évaporation du solvant sous pression réduite, on purifie le résidu obtenu par chromatographie sur silice en éluant avec un mélange heptane/acétate éthyle 4 :1. On obtient un résidu qui est cristallisé dans un mélange pentane/éther.

On obtient 31 g de 8-bromo-1,2,3,4-tétrahydro-5-hydroxy-4-oxo-2-(trifluoroacétyl)-1-isoquinoléinecarboxylate d'éthyle, de formule brute C₁₄H₁₁BrF₃NO₅ (M = 409,15 g).

Le rendement correspondant est de 55 %.

### Stade H

On dissout 30 g du produit obtenu au stade précédent G dans 250 ml d'éthanol. Puis on ajoute 3 g de palladium sur charbon à 10 % en poids et 21,1 ml de triéthylamine. Le milieu est mis sous pression d'hydrogène. Après 1h30, on filtre le catalyseur puis le filtrat est versé dans un mélange heptane/acétate d'éthyle, la phase organique est lavée avec une solution aqueuse de NaH₂PO₄ 1M, puis séchée sur sulfate de magnésium et le solvant est évaporé sous pression réduite. Le produit brut obtenu est purifié sur silice en éluant avec un mélange heptane/acétate d'éthyle 4/1.

On obtient 22,2 g de 1,2,3,4-tétrahydro-5-hydroxy-4-[(phényl-méthoxy)imino]-2-(trifluoroacétyl)-1-isoquinoléinecarboxylate d'éthyle de formule brute C₁₄H₁₂F₂NO₅ (M = 331,25 g).

Le rendement correspondant est de 92 %.

### Stade I

On procède comme indiqué au stade A de l'Exemple 6 avec 2 g du produit obtenu au stade précédent H, 1,05 g de chlorhydrate de O-benzylhydroxylamine. On agite pendant 1h30 à température ambiante puis on verse la solution dans un mélange acétate d'éthyle/heptane 2/1. On lave la phase organique à l'eau puis avec une solution aqueuse de NaH₂PO₄ 1M. Après séchage sur sulfate de magnésium et évaporation du solvant sous pression réduite, on obtient 2,95 g d'un composé brut qui est cristallisé dans un mélange éther/pentane.

On obtient 2,85 g sous forme de cristaux beiges de 1,2,3,4-tétrahydro-5-(2-propenyloxy)-4-[(phénylméthoxy)imino]-2-(trifluoroacétyl)-1-isoquinoléine-carboxylate d'éthyle, de formule brute C₂₁H₁₉F₃N₂O₅ (M = 436,39 g).

Le rendement correspondant est de 94 %.

### Stade J

On solubilise 2,45 g (5,61 mmoles) du produit obtenu au stade 1 précédent dans 30 ml d'acétone puis on ajoute 9,16 g de K₂CO₃ et 3,5 équivalents de bromure d'allyle. On chauffe une nuit à reflux puis on verse la solution dans l'eau et on extrait la phase organique avec un mélange acétate d'éthyle/heptane. On lave la phase organique avec une solution aqueuse de NaH₂PO₄ 1M puis après séchage sur sulfate de magnésium et évaporation du solvant sous pression réduite, on obtient un produit brut qui est purifié par chromatographie sur silice en éluant avec un mélange heptane/acétate d'éthyle 4/1.

On obtient 2,35 g sous forme d'huile jaune de 1,2,3,4-tétrahydro-4-[(phényl-méthoxy)imino]-5-(2-propényloxy)-2-(trifluoroacétyl)-1-isoquinoléinecarboxylate d'éthyle, de formule brute C₂₄H₂₃F₃N₂O₅ (M = 476,47 g).

Le rendement correspondant est de 88 %.

### Stade K

On solubilise 25,2 g du produit obtenu au stade précédent J dans 300 ml d'éthanol. On fait barboter à 0°C NH₃ gazeux pendant 5 minutes dans la solution. Puis, on agite à température ambiante pendant 3 heures. La solution est ensuite versée sur de l'acétate d'éthyle et la phase organique lavée avec une solution aqueuse de NaH₂PO₄ puis séchée sur sulfate de magnésium. Le solvant est évaporé sous pression réduite avec un entraînement au toluène.

On obtient 21,2 g sous forme d'huile jaune de 1,2,3,4-tétrahydro-4-[(phényl-méthoxy)imino]-5-(2-propényloxy)-1-isoquinoléinecarboxylate d'éthyle, de formule brute C₂₂H₂₄N₂O₄ (M = 300,45 g).

Le rendement obtenu est quantitatif.

### Stade L

On procède comme indiqué au stade J de l'Exemple 23 avec 22,5 g du produit obtenu au stade précédent K, et 1,1 équivalent de (Boc)₂O et 300 ml de THF.

On obtient 19,3 g sous forme de cristaux blancs du 3,4-dihydro-4-[(phényl-méthoxy)imino]-5-(2-propényloxy)-1,2(1*H*)-isoquinoléinedicarboxylate de 2-(1,1-diméthyléthyle) et de 1-éthyle, de formule brute C₂₇H₃₂N₂O₆ (M = 480,57 g).

Le rendement est de 67%.

### Stade M

On procède comme indiqué au stade B de l'Exemple 6 avec 19,3 g du produit obtenu au stade précédent L, 250 ml de méthanol, 40,5 g de cyanoborohydrure de sodium, 59 ml d'éthérate de trifluorure de bore.

On obtient 21 g de 3,4-dihydro-4-[(phénylméthoxy)amino]-5-(2-propényl-oxy)-1,2(1*H*)-isoquinoléinedicarboxylate de 2-(1,1-diméthyléthyle) et de 1-éthyle, de formule brute C₂₇H₃₄N₂O₆ (M = 482,58 g).

Le rendement correspondant est de 79 %.

### Stade N

On procède comme indiqué au stade C de l'Exemple 6 avec 15,3g du produit obtenu au stade précédent M, 83 ml d'une solution d'acide chlorhydrique à 140 g/l solubilisé dans 10 mld"acétate d'éthyle ,35 ml de soude 2N et 130 ml de chlorure de méthylène..

On obtient 12,1 g sous forme d'huile incolore de 1,2,3,4-tétrahydro-4-[(phénylméthoxy)amino]-5-(2-propényloxy)-1-isoquinoléinecarboxylate d'éthyle, de formule brute C₂₂H₂₆N₂O₄ (M = 382,46 g).

### Stade O

On procède comme indiqué au stade N de l'Exemple 3 avec 12,1 g du produit obtenu au stade précédent N, 3 l d'acétonitrile, 1,92 ml de diphosgène et 9 ml de triéthylamine.

On obtient 7 g de 1,2,3,4-tétrahydro-3-oxo-2-(phénylméthoxy)-9-(2-propé-nyloxy)-1,4-méthano-4*H*-2,4-benzodiazépine-5-carboxylate d'éthyle, de formule brute C₂₃H₂₄N₂O₅ (M = 408,48 g)

### Spectre RMN du proton

Dans CDCl₃ à 300 MHz, déplacements chimiques et multiplicité :
3,10 (d) et 3,60 (dd) : N-CH₂-CH-φ; 4,80 (d) : N-CH₂-CH φ; 4,26 et 4,41: N-CH₂-φ; 4,56 (m) : O-CH₂-CH=CH₂; 6,05 (m) : O-CH₂-CH=CH₂; 5,31 (qd) et 5,41 (qd) : O-CH₂-CH=CH₂; 4,92 et 4,96 : O-CH₂ φ; 6,61 (d) et 6,71 (d) et 7,19 (t) pour les trois hydrogènes aromatiques ; 7,35 (m) et 7,44 (m) pour les hydrogènes du groupe phényle.
IR (CHCl₃): 1755, 1645, 1600, 1583, 1497 cm⁻¹

### Exemple 47

### Trans-1,2,3,5-tétrahydro-3-oxo-9-(phénylméthoxy)-2-(sulfooxy)-1,4-méthano-4H-2,4-benzodiazépine-5-carboxylate de méthyle

### Stade A

On procède comme indiqué au stade A de l'Exemple 6 avec 21,9 g du produit obtenu à l'Exemple 46, 7,25 g de chlorhydrate de O-allylhydroxylamine, et 200 ml de pyridine.

On obtient 25,5g de 1,2,3,4-tétrahydro-5-hydroxy-4-[(2-propényloxy)imino]-2-(trifluoroacétyl)-1-isoquinoléinecarboxylate d'éthyle, de formule brute C₁₇H₁₇F₃N₂O₅ (M = 386,33 g).

Le rendement obtenu est quantitatif.

### Stade B

On procède comme indiqué au stade J de l'exemple 46 avec 29,4 g du produit obtenu au stade précédent A , 126 g de K₂CO₃, 32 ml de bromure de benzyle et 450 ml d'acétone.

On obtient 34,75 g sous forme d'huile jaune de 1,2,3,4-tétrahydro-5-(phényl-méthoxy)-4-[(2-propényloxy)imino]-2-(trifluoroacétyl)-1-isoquinoléinecarboxylate d'éthyle, de formule brute C₂₄H₂₃F₃N₂O₅ (M = 476 g).

Le rendement correspondant est de 85 %.

### Stade C

On procède comme indiqué au stade B de l'Exemple 6 avec 2 g du produit obtenu au stade précédent B, 3,97 g de cyanoborohydrure de sodium, 6,17 ml d'éthérate de trifluorure de bore et 100 ml de méthanol.

On obtient 1,42 g sous forme d'huile de 1,2,3,4-tétrahydro-5-(phényl-méthoxy)-4-[(2-propényloxy)amino]-2-(trifluoroacétyl)-1-isoquinoléinecarboxylate d'éthyle, de formule brute C₂₄H₂₅F₃N₂O₅ (M = 478,47 g).

Le rendement correspondant est de 70 %.

### Stade D

Dans un ballon maintenu à 0°C sous atmosphère d'argon, on introduit 1,4 g du produit obtenu au stade précédent C, dans 25 ml d'éthanol. Puis, on ajoute deux fois 120 mg de borohydrure de sodium. Après 20 minutes, la réaction est arrêtée par ajout à 0°C d'un mélange heptane/acétate d'éthyle (1/1). Puis on ajoute une solution aqueuse saturée de NaHCO₃. On agite 5 minutes puis on décante et on extrait de nouveau avec un mélange heptane/acétate d'éthyle (1/1). La phase organique est ensuite séchée sur sulfate de magnésium et le solvant évaporé sous pression réduite.

On obtient 1,18 g sous forme d'huile de 1,2,3,4-tétrahydro-5-(phényl-méthoxy)-4-[(2-propényloxy)amino]-1-isoquinoléinecarboxylate d'éthyle, de formule brute C₂₂H₂₆N₂O₄ (M = 382,46 g).

Le rendement obtenu est quantitatif.

### Stade E

On procède comme indiqué au stade C de l'Exemple 3, avec 1,15 g du produit obtenu au stade précédent D, 18 ml d'acétonitrile, 0,2 ml de diphosgène et 0,88 ml de triéthylamine.

On obtient 0,41 g de 1,2,3,5-tétrahydro-3-oxo-9-(phénylméthoxy)-2-(2-propényloxy)-1,4-méthano-4*H*-2,4-benzodiazépine-5-carboxylate de méthyle, de formule brute C₂₃H₂₄N₂O₅ (M = 408,46).

Le rendement correspondant est de 33 %.

### Stade F

On dissout sous atmosphère d'argon 0,524 g du produit obtenu au stade précédent E dans 50 ml de dichlorométhanne. Puis on ajoute 0,307 ml d'acide acétique et 741 mg de Pd(PPh₃)₄. Après 10 minutes d'agitation, on évapore le solvant sous pression réduite et le produit brut obtenu est purifié sur silice en éluant avec un mélange chlorure de méthylène/acétone (9/1).

On obtient 0,4 g de 1,2,3,5-tétrahydro-2-hydroxy-3-oxo-9-(phénylméthoxy)-1,4-méthano-4*H*-2,4-benzodiazépine-5-carboxylate d' éthyle, de formule brute C₂₀H₂₀N₂O₅ (M = 368,39 g).

Le rendement correspondant est de 83 %.

### Stade G

On dissout 0,090 g du produit obtenu au stade précédent F dans 4 ml de pyridine en présence de tamis moléculaire. Puis on ajoute sous argon, 3 équivalents du complexe pyridine-SO₃ et on agite pendant deux heures à température ambiante. Puis on évapore la pyridine et le produit brut obtenu est purifié sur silice en éluant avec un mélange acétone/acétate d'éthyle/eau (5/4/0,5).

On obtient 32 mg de trans-1,2,3,5-tétrahydro-3-oxo-9-(phénylméthoxy)-2-(sulfooxy)-1,4-méthano-4*H*-2,4-benzodiazépine-5-carboxylate de méthyle, de formule brute C₂₀H₂₀N₂O₈S (M = 448,48 g).

### RMN du proton

Dans CDCl₃ à 300 MHz, déplacements chimiques et multiplicité :
1,20 (t) : CH₃-CH₂-O-C=O ; 4,17 (q) : CH₃-CH₂-O-C=O ; 3,34 (dl) et 3,71 (t1) : N-CH₂-CH ; 5,30 (s1) : N-CH₂-CH ; 4,99 et 5,13 : φ- CH₂-O ; 5,09 (s1) : CH-CO₂-Et ; 6,88 (d), 6,75 (d), 7,07 (t) : pour les 3H aromatiques ; 7,16 (t), 7,29 (d), 7,45 (d) : pour les 5H aromatiques.
SM (électrospray négatif) m/z : (M-H)⁻ = 447.
Infrarouge (CHCl₃) : 1745, 1646, 1601, 1582, 1496 cm⁻¹.

### Exemple 48

### Trans-1,2,3,5-tétrahydro -9-hydroxy -3-oxo 2-(sulfooxy)-1,4-méthano-4H-2,4-benzodiazépine-5-carboxylate d' éthyle

On dissout 0,1g du produit obtenu à l'Exemple 47 dans 4 ml de THF. Puis on ajoute 25 mg de palladium sur charbon à 10 % et on met la solution sous atmosphère d'hydrogène. On agite pendant 5 heures à température ambiante puis on filtre le catalyseur, on évapore le solvant et le résidu est recristallisé dans l'éther.

On obtient 77 mg sous forme de cristaux gris de trans-1,2,3,5-tétrahydro-9-hydroxy-3-oxo-2-(sulfooxy)-1,4-méthano-4*H*-2,4-benzodiazépine-5-carboxylate de méthyle, de formule brute C₁₄H₁₆N₂O₅ (M = 292,09 g).

Le rendement est quantitatif.

### RMN du proton

Dans DMSO-d₆ à 300 MHz, déplacements chimiques et multiplicité :
1,25 (t) : CH₃-CH₂-O-C=O; 4,20 (q) : CH₃-CH₂-O-C=O; 3,52 (m) : N-CH₂-CH ; 5,10 (d) : N-CH₂-CH; 4,88 (s) : CH-CO₂-Et; 6,71 (d), 6,74 (d) et 7,13 (t) pour les 3H aromatiques ; 9,56 (sl) H mobile
SM (électrospray négatif) m/z: (M⁻= 357.

### Exemple 49

### Sel de sodium de trans-1,2,3,5-tétrahydro-3-oxo-9-propoxy-2-(sulfooxy)-1,4-méthano-4H-2,4-benzodiazépine-5-carboxylate d'éthyle

### Stade A

On procède comme indiqué au stade A de l'Exemple 11 avec 150 mg (0,367 mmole) du produit obtenu à l'Exemple 46, 6 ml de THF, 30 mg de palladium sur charbon à 10 % en poids.

On obtient 120 mg de produit brut 1,2,3,5-tétrahydro-2-hydroxy-3-oxo-9-propoxy-1,4-méthano-4*H*-2,4-benzodiazépine-6-carboxylate d'éthyle, de formule brute C₁₆H₂₀N₂O₅ (M = 320,35 g).

### Stade B

On procède comme indiqué au stade B de l'Exemple 11 avec 117,6 mg du produit obtenu au stade précédent A avec 2 ml de pyridine et 175,2 mg de complexe pyridine-SO₃.

On obtient 143 mg de sel de sodium de trans-1,2,3,5-tétrahydro-3-oxo-9-propoxy-2-(sulfooxy)-1,4-méthano-4*H*-2,4-benzodiazépine-5-carboxylate d'éthyle, de formule brute C₁₆H₁₉N₂NaO₈S (M = 422,39 g).

Le rendement correspondant est de 93 %.

### Spectre RMN du proton

Dans le CDCl₃ à 300 MHz, déplacements chimiques, multiplicité :
0,98 (t): CH₃=CH=CH₂-O; 1,78 (m) CH₃=CH₂=CH₂-O; 3,89 (m) CH₃=CH₂-CH₂-O ; 1,26 (r) : CH₃-CH₂ ; 4,22 (q) : CH₃-CH₂ ; 3,25 (sl) et 3,71 (sl) : N-CH₂; 5,13 (singulet), 5,19 (singulet) : les CH du cycles ; 6,72 (d) et 6,86 (d) et 7,08 (r) : les 3H du cycle aromatique.
SM (SIMS) m/z : (M+Na)⁺= 445.
IR CHCl₃: 1746, 1639, 1602, 1584 cm⁻¹.

### Exemple 50

### Sel de sodium de -5-fluoro-1,2,3,5-tétrahydro-3-oxo-2-(sulfooxy)-1,4-méthano-4H-2,4-benzodiazépine-5-carboxylate de méthyle

### Stade A

On dissout 1g du composé préparé au stade L de l'Exemple 3 dans 15 ml de THF anhydre. On ajoute à une température de -78°C, 6,9 ml d'une solution 0,5 M de bis-triméthyl silylamide de potassium. Puis après 10 minutes de contact,on ajoute en une seule fois 1,09 g de N-fluorobenzènesulfonimide. Après 30 minutes d'agitation, on verse le milieu réactionnel dans un mélange heptane/acétate d'éthyle 1/2 et on lave la phase organique avec une solution aqueuse de NaH₂PO₄ 1M. Après séchage sur sulfate de magnésium et évaporation du solvant sous pression réduite, on obtient 1,3g de produit brut qui est purifié sur silice en éluant avec du dichlorométhane. Après recristallisation dans l'éther, on obtient 0,1 g du sel de sodium de 5-fluoro-1,2,3,5-tétrahydro-3-oxo-2-(2-propényloxy)-1,4-méthano-4*H*-2,4-benzodiazépine-5-carboxylate de méthyle, de formule brute C₁₅H₁₅F.N₂O₄ (M = 306,30 g).

Le rendement correspondant est de 62 %.

### Stade B

On procède comme indiqué au stade C de l'Exemple 7 avec 0,4 g du produit obtenu au stade précédent A, 148 µl d'acide acétique, 756 mg de Pd[PPh₃]₄, 8 ml de pyridine et 832 mg de complexe SO₃-pyridine.

On obtient 0,21 g de sel de sodium de -5-fluoro-1,2,3,5-tétrahydro-3-oxo-2-(sulfooxy)-1,4-méthano-4*H*-2,4-benzodiazépine-5-carboxylate de méthyle, de formule brute C₁₂H₁₀FN₂O₇S.Na (M = 368,28 g).

Le rendement correspondant est de 23 %.

### Spectre RMN du proton

Dans DMSO-d₆ à 300 MHz, déplacements chimiques et multiplicité :
3,65 (s1) : N-CH₂-CH; 4,78 (sl) : N-CH₂-CH ; 3,72 (s) : CH₃-O-C=O- ; de 7,21 à 7,68 (m, 4H aromatique).
IR (Nujol) : 1761, 1632 cm⁻¹
SM (electrospray négatif) m/z : M⁻ = 345.

### Exemple 51

### Sel de sodium de 1,2,3,5-tétrahydro-5-(méthylthio)-3-oxo-2-(sulfooxy)-1,4-méthano-4H-2,4-benzodiazépine-5-carboxylate de méthyle

### Stade A

On dissout 1 g du produit préparé au stade L de l'Exemple 3 dans 15 ml de THF. Puis, on ajoute 6,9 ml goutte à goutte de bis-triméthyl silylamide de potassium et on agite 10 minutes à -78°C. On ajoute en une fois 437 mg de CH₃SO₂SCH₃. On agite la solution pendant 30 minutes puis on verse dans un mélange heptane/acétate d'éthyle, 1/2. Le milieu est lavé avec une solution 1N NaH₂PO₄, puis on sèche la phase organique sur MgSO₄ et le solvant est évaporé sous pression réduite. Le produit brut obtenu est purifié par passage sur colonne de silice en éluant avec un mélange dichlorométhane/acétone 99/1. Puis après recristallisation dans l'éther, on obtient 0,65 g de 1,2,3,5-tétrahydro-5-(méthylthio)-3-oxo-2-(2-propényloxy)-1,4-méthano-4*H*-2,4-benzodiazépine-5-carboxylate de méthyle, de formule brute C₁₆H₁₈N₂O₄S (M = 334,40 g).

Le rendement correspondant obtenu est de 56 %.

### Stade B

On procède comme indiqué au stade C de l'Exemple 7 avec 0,443 g du produit obtenu au stade précédent A, 10 ml de dichlorométhane, 0,151 mg d'acide acétique, 0,765 mg de Pd (PPh₃)₄ et 10 ml de pyridine et 0,845 g du complexe SO₃-pyridine.

On obtient 0,250 g de sel de sodium de -1,2,3,5-tétrahydro-5-(méthylthio)-3-oxo-2(sulfooxy)-1,4-méthano-4*H*-2,4-benzodiazépine-5-carboxylate de méthyle, de formule brute C₁₃H₁₃N₂O₇S₂.Na (M = 396,38 g).
SM (électrospray négatif) m/z : M⁻= 373.

### RMN du proton

Dans le DMSO-d₆ à 300 MHz, déplacements chimiques et multiplicité :
2,31 (s) CH₃-S ; 3,56 (dd) et 4,10 (d) : N-CH₂-CH ; 4,70 (d) N-CH₂-CH ; 3,73 (s) CH₃-O-C=O; 7,15 (dl) et 7,31 (m) et 7,39 (tl) : 4H aromatique.
IR (Nujol) : 1760, 1736, 1635, 1576 cm⁻¹.

### Exemple 52

### Sel de triéthylammonium de trans -4,5,6,8-tétrahydro-8-méthyl-6-oxo-5-(sulfooxy)-4,7-méthano-7H-thiéno[2,3-e][1,3]diazépine-8-carboxylate de méthyle

### Stade A

On procède comme indiqué au stade A de l'Exemple 6 à partir de 4,5 g du produit préparé au stade F de l'Exemple 24, 19 ml de pyridine et 22,87 g de PhCH₂ONH₂, HCl.

On obtient 6,89 g de 4,7-dihydro-4-[(phénylméthoxy)imino]-thiéno[2,3-*c*]pyridine-6,7(*5H*)-dicarboxylate de 6-(1,1-diméthyléthyle) et de 7-méthyle, de formule brute C₂₁ H₂₄N₂O₅S (M = 416,50 g).

Le rendement correspondant est de 88 %.

### Stade B

Dans un ballon sous atmosphère inerte, on introduit 6,89 g du produit obtenu au stade A précédent dans 70 ml de DMF. On refroidit le milieu réactionnel à -5°C et on ajoute 1,03 ml de CH₃I, puis 785 mg de NaH à 50% dans l'huile. Puis, on agite pendant une heure à 20°C et on ajoute un mélange heptane/acétate d'éthyle, 1/ 2 au milieu réactionnel. On lave la phase organique à l'eau plusieurs fois ainsi qu'avec une solution 1N de NaH₂P0₄. La phase organique est séchée sur sulfate de magnésium, filtrée et le solvant évaporé sous pression réduite. Le produit brut est purifié par chromatographie sur silice en éluant avec un mélange heptane/acétate d'éthyle, 4/1.

On obtient 6,14 g de 4,7-dihydro-7-méthyl-4-[(phénylméthoxy)imino]-thiéno [2,3-*c*]pyridine-6,7(5H)-dicarboxylate de 6-(1,1-diméthyléthyle) et de 7-méthyle, de formule brute C₂₂H₂₆N₂O₅S (M = 430,53 g).

Le rendement correspondant est de 56 %.

### Stade C

On procède comme indiqué au stade B de l'Exemple 6 avec 6,14 g du produit obtenu au stade précédent B, 14,4 g de cyanoborohydrure de sodium, 22 ml d'éthérate de trifluorure de bore et 60 ml de méthanol.

On obtient 4,72 g de mélange cis et trans-4,7-dihydro-7-méthyl-4-[(phényl-méthoxy)amino]-thiéno[2,3-*c*]pyridine-6,7(5*H*)-dicarboxylate de 6-(1,1-diméthyléthyle) et de 7-méthyle, de formule brute C₂₂H₂₈N₂O₅S (M = 432,54 g).

### Stade D

On procède comme indiqué au stade C de l'Exemple 6 avec 4,62 g du produit obtenu au stade précédent C, 10 ml d'acétate d'éthyle, et d'une solution 4,3N 25 ml d'acide chlorhydrique dans l'acétate d'éthyle.

On obtient 4,29 g sous forme de cristaux blancs d'un mélange des chlorhydrates cis et trans.

Le chlorhydrate est libéré de manière analogue au stade C de l'exemple 6 puis les 2 isomères cis et trans sont séparés par chromatographie sur silice en éluant par un mélange heptane/Acétonitrile: 1/2.

On obtient 0,7g de trans-4,5,6,7-tétrahydro-7-méthyl-4-[(phénylméthoxy)-amino]-thiéno[2,3-*c*]pyridine-7-carboxylate de méthyle, et 2,36g de cis-4,5,6,7-tétrahydro-7-méthyl-4-[(phénylméthoxy)amino]-thiéno[2,3-*c*]-pyridine-7-carboxylate de méthyle de formule brute C₁₇H₂₀N₂O₃S (M = 332,42 g).

Le rendement global est de 87%.

### Stade E

On procède comme indiqué au stade D de l'Exemple 6 avec 0,7 g du produit obtenu au stade précédent D, 175 ml d' acétonitrile, 0,607 ml de triéthylamine et 0,126 ml de diphosgène.

On obtient 0,52 g de trans-4,5,6,8-tétrahydro-8-méthyl-6-oxo-5-(phényl-méthoxy)-4,7-méthano-7*H*-thiéno[2,3-*e*][1,3]diazépine-8-carboxylate de méthyle, de formule brute C₁₈H₁₈N₂O₄S (M = 358,42 g).

Le rendement correspondant est de 67 %.

### Stade F

On procède comme indiqué au stade A de l'Exemple 11 avec 0,1 g du produit obtenu au stade précédent E, 0,1 g de palladium sur charbon à 30 % en poids, et 2 ml de méthanol et 1 ml de THF.

On obtient 81 mg de trans-4,5,6,8-tétrahydro-5-hydroxy-8-méthyl-6-oxo-4,7-méthano-7*H*-thiéno[2,3-*e*][1,3]diazépine-8-carboxylate de méthyle, de formule brute C₁₁H₁₂N₂O₄S (M = 268,29 g).

Le produit est utilisé tel que à l'étape suivante.

### Stade G

On procède comme indiqué au stade B de l'Exemple 11 avec le produit brut obtenu au stade précédent F, 0,133 g du complexe pyridine-SO₃ et 1 ml de pyridine. Après purification du produit sur silice, on obtient 0,015 g sous forme de cristaux blancs de sel de triéthylammonium de trans-4,5,6,8-tétrahydro-8-méthyl-6-oxo-5-(sulfooxy)-4,7-méthano-7*H*-thiéno[2,3-e][1,3]diazépine-8-carboxylate de méthyle, de formule brute C₁₁H₁₁N₂O₇S_{2..} C₆H₁₆N (M = 347,35 g , 102,21 g).

Le rendement obtenu est de 15%.
SM (électrospray négatif) m/z : M- = 347.

### Exemple 53

### Sel de triéthylammonium du cis-4,5,6,8-tétrahydro-8-méthyl-6-oxo-5-(sulfooxy)-4,7-méthano-7H-thiéno[2,3-e][1,3]diazépine-8-carboxylate de méthyle Stade A

On introduit 2,35g de cis -4,5,6,7-tétrahydro-7-méthyl-4-[(phénylméthoxy)-amino]-thiéno[2,3-*c*]pyridine-7-carboxylate de méthyle préparé au stade D de l'exemple 52

Et on procède comme au stade D de l'exemple 46 avec 600ml d'acétonitrile, 3,0 ml de TEA et 0,42 ml de diphosgène.

On obtient le cis-4,5,6,8-tétrahydro-8-méthyl-6-oxo-5-(phénylméthoxy)-4,7-méthano-7*H*-thiéno[2,3-*e*][1,3]diazépine-8-carboxylate de méthyle, de formule brute C₁₈H₁₈N₂O₄S (M = 358,42).

### Stade B

On procède comme indiqué au stade F de l'exemple 52 avec 1,1 g du produit obtenu au stade précédent A , 2ml de méthanol et 100mg de palladium sur charbon à 10% en poids.On obtient 75 mg de cis-4,5,6,8-tétrahydro-5-hydroxy-8-méthyl-6-oxo-4,7-méthano-7*H*-thiéno[2,3-*e*][1,3]diazépine-8-carboxylate de méthyle, de formule brute C₁₁H₁₂N₂O₄S (M = 268,29 g).

Le rendement est quantitatif.

### Stade C

On procède comme indiqué au stade G de l'exemple 52 avec 75 mg du produit obtenu au stade précédent B, 1 ml de pyridine et 0,33 g du complexe SO₃-pyridine

On obtient 48 mg sous forme de cristaux blancs du sel de triéthylammonium du cis-4,5,6,8-tétrahydro-8-méthyl-6-oxo-5-(sulfooxy)-4,7-méthano-7*H*-thiéno[2,3-e][1,3]diazépine-8-carboxylate de méthyle de formule brute C₁₁H₁₁N₂O₇S₂ (M = 347,35 g).

Le rendement obtenu correspondant est de 50%.

### RMN du proton

Dans le DMSO-d₆ à 300 MHz, déplacements chimiques et multiplicité :
1,75 (s) CH₃-C ; 3,21 (dd) et 3,50 (dd) : N-CH₂-CH-C= ; 4,73 (d) : N-CH₂-CH-C=; 3,74(s) : CH₃-O-C=O ; 6,92 (d) et 7,53(d): H thiophène.
SM (électrospray négatif) m/z : M⁻ = 347

### Exemple 54

### Sel de sodium de trans-1,5-dihydro-5-[(phénylméthoxy)méthyl]-2-(sulfooxy)-1,4-méthano-4H-2,4-benzodiazépin-3(2H)-one

### Stade A

On solubilise 1,55 g du produit préparé au stade A de l'Exemple 7 dans 20 ml de THF. On ajoute goutte à goutte à 0°C, 0,69 ml de N-méthylmorpholine, puis, 0,89 ml de chloroformiate d'isobutyle. Le mélange réactionnel est ensuite refroidi à - 78°C et on ajoute 7 ml de méthanol. Puis, on ajoute en une fois 0,45 g de NaBH₄. On agite 1 heure à -78°C. Une fois la réaction terminée, on ajoute de l'acétate d'éthyle et une solution aqueuse saturée en NaHCO₃. Après extraction, séchage et évaporation, on obtient 1,65 g de produit brut qui est purifié par chromatographie sur silice en éluant avec un mélange heptane/acétate d'éthyle, 1/4.

On obtient 0,95 g de 1,5-dihydro-5-(hydroxyméthyl)-2-(2-propényloxy)-1,4-méthano-4*H*-2,4-benzodiazépin-3(2*H*)-one, de formule brute C₁₄H₁₇N₂O₂ (M = 245,30 g).

Le rendement obtenu correspondant est de 69 %.

### Stade B

On solubilise 0,4 g du produit obtenu au stade précédent A dans 10 ml de DMF, puis à 20°C on ajoute 1,2 équivalent de bromure de benzyle et 1 équivalent d'hydrure de sodium. On agite à -20°C et après deux heures, on verse la solution sur un mélange heptane/acétate d'éthyle, 1/2. Après lavage de la phase organique avec une solution aqueuse de dihydrogénophosphate de sodium 1 M, puis séchage sur MgSO₄ et filtration, on évapore le solvant sous pression réduite. On obtient 0,6 g de produit brut qui est purifié par chromatographie sur silice en éluant avec un mélange heptane/acétate d'éthyle, 3/1.

On obtient 0,42 g sous forme d'huile incolore de 1,5-dihydro-5-[(phényl-méthoxy)méthyl]-2-(2-propényloxy)-1,4-méthano-4*H*-2,4-benzodiazépin-3(2*H*)-one, de formule brute C₂₁H₂₂N₂O₃ (M = 350,42 g).

Le rendement obtenu est de 74 %.

### Stade C

On procède comme indiqué au stade C de l'Exemple 7 avec 0,4 g du composé obtenu au stade précédent B, 0,136 ml d'acide acétique, 5 ml de dichlorométhane, 0,69 g de Pd(PPh₃)₄, 0,57 g du complexe pyridine-SO₃ et 5 ml de pyridine.

On obtient 0,130 g sous forme de cristaux beiges de sel de sodium de trans-1,5-dihydro-5-[(phénylméthoxy)méthyl]-2-(sulfooxy)-1,4-méthano-4*H*-2,4-benzodiazépin-3(2*H*)-one, de formule brute C₁₈H₁₇N₂O₆S.Na (M = 389,41 g, 22,99 g).

### RMN du proton

Dans DMSO-d₆ à 300 MHz, déplacements chimiques et multiplicité :
3,40 (dd) et 3,60 (d) : N-CH₂-CH-N; 4,61 (d) : N-CH₂-CH-N ; 3,87 (dd) et 3,96 (dd) : O-CH₂CH-N; 4,46 (dd) : O-CH₂-CH-N; 4,56 :OCH₂Ph et de 7,10 à 7,40 pour les 4 hydrogènes aromatiques.
SM (électrospray négatif) m/z : M- = 389.
Infrarouge (Nujol): 1755, 1608, 1494 cm⁻¹.

### Exemple 55

### Sel de sodium de trans-1,5-dihydro-5-(hydroxyméthyl)-2-(sulfooxy)-1,4-méthano-4H-2,4-benzodiazépin-3(2H)-one

On solubilise dans 2 ml de méthanol 0,110 g du produit obtenu à l'Exemple 54 puis on ajoute 0,020 g de palladium sur charbon à 10 % en poids. Après 6 heures de réaction sous atmosphère d'hydrogène, on ajoute alors 2 équivalents d'acide acétique et on agite à température ambiante pendant 12 heures. Après filtration sur ?? du milieu réactionnel, on évapore le solvant sous pression réduite. Le résidu est ensuite solubilisé dans 2 ml de méthanol. On ajoute alors à nouveau 100 mg de palladium sur charbon à 10 % et on réintroduit de l'hydrogène pendant 4 heures. Après filtration du catalyseur et lavage au méthanol et au THF, on évapore à sec la solution, le résidu est cristallisé dans l'éther et on isole 25 mg de sel de sodium de trans-1,5-dihydro-5-(hydroxyméthyl)-2-(sulfooxy)-1,4-méthano-4*H*-2,4-benzo-diazépin-3(2*H*)-one, de formule brute C₁₁H₁₁N₂O₆S.Na (M = 299,28 g, 22,99 g).

Le rendement correspondant est de 30 %.

### Spectre RMN du proton

Dans DMSO-d₆ à 300 MHz, déplacements chimiques et multiplicité :
3,35 (dd) et 3,55 (d) : =CN-CH₂-CH-N ; 4,25 (dd) : =C-N-CH₂-CH-N ; 4,60 (d) : C-N-CH(C=)-CH₂(OH ; 3,82 (m) : C-N-CH(C=)-CH₂OH ; 5,04 (d) : C-N-CH(C=)-CH₂OH ; 7,10 (dl) et 7,20 (m) et 7,3 (dt) : les 4H aromatiques.
SM (électrospray négatif) m/z : M⁻ = 299.

### Exemple 56

### Sel de sodium de trans-5-[[(aminocarbonyl)oxy]méthyl]-1,5-dihydro-2-(sulfooxy)-1,4-méthano-4H-2,4-benzodiazépin-3(2H)-one

### Stade A

Dans un ballon placé sous argon, on introduit 0,4 g du produit préparé au stade A de l'Exemple 54 dans 8 ml de dichlorométhane en présence de tamis moléculaire. Puis, on refroidit à 0°C et on ajoute 0,239 g de DMAP et 0,39 g de 4-nitro-phénylchloroformate. On agite pendant 45 minutes, puis on évapore le solvant sous pression réduite et on ajoute 4 ml de DMF. On obtient une suspension visqueuse dans laquelle on fait barboter de l'ammoniac gazeux à 0°C pendant 20 secondes. Puis on ajoute de l'acétate d'éthyle et une solution aqueuse de NaHCO₃. On extrait de nouveau la phase aqueuse avec du THF puis les phases organiques rassemblées sont lavées plusieurs fois avec une solution aqueuse saturée en NaHCO₃. Après séchage sur sulfate de magnésium, filtration et évaporation du solvant sous pression réduite, on obtient 0,650 g de produit brut qu'on purifie sur une colonne de silice en éluant avec du dichlorométhane contenant 10 % d'acétone.

On obtient 0,365 g sous forme de mousse incolore du 5-[[(aminocarbonyl)oxy]méthyl] 1,5-dihydro-2-(2-propényloxy)-1,4-méthano-4*H*-2,4-benzodiazépin-3(2*H*)-one, de formule brute C₁₅H₁₇N₃O₄ (M = 303,32 g).

Le rendement correspondant est de 72 %.

### Stade B

On procède comme indiqué au stade C de l'Exemple 7 avec 0,33 g du produit obtenu au stade A précédent, 0,13 ml d'acide acétique, 0,63 g de Pd(PPh₃)₄, 0,517 g de complexe pyridine-SO₃ et 4 ml de pyridine.

On obtient 188 mg sous forme de cristaux blancs de sel de sodium de trans-5-[[(aminocarbonyl)oxy]méthyl]- 1,5-dihydro-2-(sulfooxy)-1,4-méthano-4*H*-2,4-benzodiazépin-3(2*H*)-one, de formule brute C₁₂H₁₂N₃O₇S.Na (M = 342,31 g, 22,99 g).

### RMN du proton

Dans DMSO-d₆ à 300 MHz, déplacements chimiques et multiplicité :
3,38 (dd) et 3,57 (d) : =C-N-CH₂-CH-N; 4,62 (d) : =C-N-CH₂-CH-N; 4,32 (dd) et 4,44 (dd): COO-CH₂-CH(-N)-C=; 4,35 (t): COO-CH₂-CH(-N)-C=; 7,13 (dl) et 7,24 (tl) et 7,27 (dl) et 7,35 (tl) : pour les 4H aromatiques ; 6,52(sl) et 6,80(sl) : NH₂ absorptions mobiles
SM (électrospray négatif) m/z : M- = 342.

### Exemple 57

### Sel de sodium de [[trans-4,5,6,8-tétrahydro-6-oxo-5-(sulfooxy)-4,7-méthano-7H-thiéno[2,3-e][1,3]diazépin-8-yl]méthyl]-carbamate de 1,1-diméthyléthyle

### Stade A

On procède comme indiqué au stade G de l'Exemple 3 avec 50 g (160,58 mmoles) de produit préparé au stade F de l'exemple 24 , 1500 ml de méthanol, 225 ml de CH₂Cl₂, 1,6g de NaBH₄ à 95 %, on obtient 51,4 g de 4,7-dihydro-4-hydroxy-thiéno[2,3-c]pyridine-6,7(5H)-dicarboxylate de 6-(1,1-diméthyléthyle) et de 7-méthyle, de formule brute C₁₄H₁₅NO₅S (M = 313,38 g).

### Stade B

On procède comme indiqué aux stades H et I de l'Exemple 3 avec 59,7 g de produit obtenu du stade A, 583 ml de CH₂Cl₂, 39,3 ml de triéthylamine, 48,7 g de (CH₃SO₂)₂O, 68,9 g de benzyl-O-NH₂.

On obtient 47,0 g de 4,7-dihydro-4-[(phénylméthoxy)amino]-thiéno[2,3-*c*]pyridine-6,7(5*H*)-dicarboxylate de 6-(1,1-diméthyléthyle) et de 7-méthyle, de formule chimique C₂₁H₂₆O₅N₂S (M = 418,515 g).

Le rendement correspondant est de 60,2 %.

### Stade C

On procède comme indiqué au stade J de l'Exemple 3, avec 47, g du produit obtenu du stade B, 79 ml d'acétate d'éthyle, 261 ml d'une solution saturée de HCl gazeux dans l'acétate d'éthyle.

On obtient 44,12 g de chlorhydrate de 4,5,6,7-tétrahydro-4-[phényl-méthoxy)amino]-thiéno[2,3-c]pyridine-7-carboxylate de méthyle, de formule brute C₁₆H₂₀N₂O₃S₂Cl₂ (M = 391,318 g).

### Stade D

On procède comme indiqué au stade K de l'Exemple 3 avec 44,1 g (du produit obtenu du stade C, 250 ml d'eau, 1000 ml de CH₂Cl₂, 35 ml d'une solution d'ammoniaque concentrée.

On obtient 34,6 g de 4,5,6,7-tétrahydro-4-[(phénylméthoxy)amino]-thiéno[2,3-*c*]pyridine-7-carboxylate de méthyle, de formule brute C₁₆H₁₈N₂O₃S (M = 318,4 g).

Le rendement correspondant est de 96,7 %.

### Stade E

On procède comme indiqué au stade L de l'Exemple 3, avec 34,1 g du produit obtenu du stade D, 8,8 1 de CH₃CN, 30,8 ml de TEA, 6,5 ml de diphosgène.

On obtient 37,2 g de trans 4,5,6,8-tétrahydro-6-oxo-5-(phénylméthoxy)-4,7-méthano-7*H*-thiéno[2,3-*e*][1,3]diazépine-8-carboxylate de méthyle, de formule brute C₁₇H₁₆N₂O₄S (M = 344,39 g).

Le rendement correspondant est de 80 %.

### Stade F

On procède comme indiqué au stade A de l'Exemple 7, avec 3,35 g du produit obtenu au stade E, 10,7 ml de NaOH 1N, 20 ml de dioxanne, 16 ml d'H₂O. On obtient 2,25 g de trans 4,5,6,8-tétrahydro-6-oxo-5-(phénylméthoxy)-4,7-méthano-7*H* thiéno[2,3-*e*][1,3]diazépine-8-carboxylate de méthyle de formule chimique brute C₁₆H₁₄N₂O₄S (M = 330,36 g).

Le rendement est de 71 %.

### Stade G

On procède comme indiqué au stade A de l'Exemple 54, avec 1 g du produit obtenu du stade F, 1,1 équivalent de N-méthylmorpholine, 1,05 équivalent de chloro-formate d'isobutyle, 4 ml de méthanol, 0,23 g de NaBH₄ ( et 15 ml de THF.

On obtient 0,7365 g de 4,8-dihydro-8-(hydroxyméthyl)-5-(phénylméthoxy)-4,7-méthano-7*H*-thiéno[2,3-*e*][1,3]diazépin-6(5*H*)-one, , de formule brute C₁₆H₁₆N₂O₃S (M = 316,38 g).

Le rendement correspondant est de 77 %.

### Stade H

On procède comme indiqué au stade B de l'Exemple 23, avec 0,635 g (0,2 mmole) du produit obtenu du stade G, 10 ml de CH₂Cl₂, 0,324 ml de TEA, 0,171 ml de chlorure de mésyle (1,1 équivalent).

On obtient 0,790 g de 4,8-dihydro-8-[[(méthylsulfonyl)oxy]méthyl]-5-(phénylméthoxy)-4,7-méthano-7*H*-thiéno[2,3-*e*][1,3]diazépin-6(5*H*)-one 8, de formule brute C₁₇H₁₈N₂O₅S₂ (M = 394,47 g).

Le rendement correspondant est quantitatif.

### Stade I

On met en solution 0,780 g du produit obtenu au stade H dans 8 ml de DMF, on ajoute 0,320 g de NaN₃ (2,5 équivalents) et on chauffe le milieu réactionnel à 60°C. Après 15 heures de chauffage, on ajoute de nouveau 2,5 équivalents de NaN₃ et on poursuit le chauffage à 60°C pendant encore 8 heures. On verse le milieu réactionnel dans le CH₂Cl₂, on lave avec une solution de NaH₂PO₄ puis avec H₂O. On sèche sur MgSO₄ puis on concentre par évaporation du solvant sous vide.

On obtient 0,61 g de-(azidométhyl)-4,8-dihydro-5-(phénylméthoxy)-4,7-méthano-7*H*-thiéno[2,3-*e*][1,3]diazépin-6(5*H*)-one de formule brute C₁₆H₁₅N₅O₂S (M = 341,39 g).

Le rendement correspondant est de 82 %.

### Stade J

On dissout 0,736 g du produit obtenu du stade I dans 10 ml de toluène, puis on ajoute à 20°C goutte à goutte 2,26 ml d'une solution 1M de Me₃P dans le THF. Au bout de 60 minutes, la réaction est terminée. On refroidit alors le milieu réactionnel à 0°C et on ajoute 0,256 g de BOC-ON (1,05 éq.). Au bout de 1 heure à 20°C, on verse le mélange réactionnel dans un mélange heptane/acétate d'éthyle (1/1) et on lave avec une solution de NaH₂PO₄ à 1 M. Puis on sèche sur MgSO₄ et on évapore à sec. Le solide brut est purifié sur silice en éluant avec un mélange heptane/acétate d'éthyle (2/1).

On obtient 0,66 g de [[4,5,6,8-tétrahydro-6-oxo-5-(phénylméthoxy)-4,7-méthano-7H thiéno[2,3-e][1,3]diazépin-8-yl]méthyl]-carbamate de 1,1-diméthyléthyle, de formule brute C₁₁H₂₅N₃N₄S (M = 415,52 g).

### Stade K

On procède comme indiqué au stade A de l'Exemple 11 avec 0,09 g du produit obtenu au stade J précédent, 100 mg de palladium sur charbon à 30 % et 2 ml de méthanol.

On obtient 79 mg de produit brut ,[[4,5,6,8-tétrahydro-5-hydroxy-6-oxo-4,7-méthano-7*H*-thiéno[2,3-e][1,3]diazépin-8-yl]méthyl]-carbamate de 1,1-diméthyléthyle utilisé tel que dans l'étape suivante, de formule brute C₁₄H₁₉O₃N₄S (M = 325,39 g).

Le rendement est quantitatif.

### Stade L

On procède comme indiqué au stade B de l'Exemple 11 avec 0,51 g du produit obtenu au stade K précédent, 5 ml de pyridine, 0,75 g du complexe pyridine-SO₃.

On obtient 0,250 g sous forme de cristaux beiges du sel de sodium de [[trans-4,5,6,8-tétrahydro-6-oxo-5-(sulfooxy)-4,7-méthano-7*H*-thiéno[2,3-*e*][1,3]diazépin-8-yl]méthyl]-carbamate de 1,1-diméthyléthyle.

Le rendement correspondant est de 54 %.

### Spectre RMN du proton

Dans DMSO-d₆ à 300 MHz, déplacements chimiques et multiplicité :
3,32 (m) : NH-CH₂-CH ; 4,47 (t1) : NH-CH₂-CH ; 7,19 (tl) : NH-CH₂-CH ; 3,31 (m) : N-CH₂-CH ; 4,70 (s1) : N-CH₂-CH ; 6,96 (d) et 7,43 (d) : les hydrogènes du thiophène ; 1,41 (s) : OC(CH₃)₃.
SM (électrospray négatif) m/z : M⁻ = 404.

### Exemple 58

### Sel de sodium et de trifluoroacétate de trans-8-(aminométhyl)-4,8-dihydro-5-(sulfooxy)-4,7-méthano-7H-thiéno[2,3-e][1,3]diazépin-6(5H)-one

On procède comme indiqué selon l'Exemple 45 avec le produit obtenu à l'Exemple 57, stade L, dans 0,5 ml d'acide trifluoroacétique.

On obtient 32 mg sous forme de cristaux beiges de sel de sodium et de trifluoroacétate de trans-8-(aminométhyl)-4,8-dihydro-5-(sulfooxy)-4,7-méthano-7H thiéno[2,3-*e*][1,3]diazépin-6(5*H*)-one.

Le rendement est quantitatif.

### Spectre RMN du proton

Dans le DMSO-d₆ à 300 MHz, déplacements chimiques et multiplicité :
3,25 (dd) et 3,38 (m) : NH₂-CH₂-CH ; 4,71 (ed) : NH₂-CH₂-CH; 3,39 (dl) et 3,51 (d) : N-CH₂-CH; 4,77 (d) : N-CH₂-CH; 7,01 (d) et 7,52 (d) : les 2H du thiophène ; 7,94 (sl): NH₂.
Spectre SM (électrospray négatif) m/z : M⁻ = 304.

### Exemple 59

### Sel de sodium de N-[[trans-4,5,6,8-tétrahydro-6-oxo-5-(sulfooxy)-4,7-méthano-7H thiéno[2,3-e][1,3]diazépin-8-yl]méthyl]-acétamide

On solubilise dans 1 ml de pyridine 100mg du produit obtenu à l'Exemple 58 puis on ajoute 1,5 equivalent d'anhydride acétique et 5 mg de DMAP à 0°C puis on agite à température ambiante. On évapore ensuite les solvants sous pression réduite et on solubilise le résidu dans un mélange chlorure de méthylène/éthanol/triéthylamine, 70/30/05. Le produit est ensuite échangé par passage sur résine Dowex 50WX8sous forme Na⁺, puis la solution est lyophilisée.

On obtient 35 mg de sel de sodium de N-[[trans-4,5,6,8-tétrahydro-6-oxo-5-(sulfooxy)-4,7-méthano-7*H*-thiéno[2,3-*e*][1,3]diazépin-8-yl]méthyl]-acétamide, de formule brute C₁₁H₁₂N₃O₆S₂ (M = 346,36 g).

### Spectre RMN du proton

Dans du DMSO-d₆ à 300 MHz, déplacements chimiques et multiplicité :
3,38 (m) et 3,51 (m) : NH-CH₂-CH-N ; 4,49 (dd) : NH-CH₂-CH-N ; 8,10 (tl) : NH-CH₂-CH-N ; 3,33 (m) : N-CH₂-CH ; 4,73 (dd) : N-CH₂-CH ; 6,96 (d) et 7,40 (d) pour les H du thiophène ; 1,89 (s) : CH₃-CO.
SM (électrospray négatif) m/z : M⁻ = 346.

### Exemple 60

### Sel de sodium de trans-4,5,6,8-tétrahydro-N-méthyl-6-oxo-5-(sulfooxy)-4,7-méthano-7H-thiéno[2,3-e][1,3]diazépine-8-carboxamide

### Stade A

On procède comme indiqué au stade B de l'Exemple 10, avec 0,15 g du produit préparé au stade F de l'exemple 57, 0,285 g de BOP, 0,091g de HOBT, 60 mg de NH₂Me,HCl, 0,313 ml de DIEA.

On obtient 0,115 g de 4,5,6,8-tétrahydro-N-méthyl-6-oxo-5-(phényl-méthoxy)-4,7-méthano-7*H*-thiéno[2,3-*e*][1,3]diazépine-8-carboxamide, de formule brute C₁₇H₁₇N₃O₃S (M = 343,41 g).

Le rendement correspondant est de 73 %.

### Stade B

On procède comme indiqué au stade A de l'Exemple 11 avec 0,15 g du produit obtenu au stade précédent A, 20 mg de palladium sur charbon à 30 % et 2 ml d'éthanol et 1 ml d'acide acétique.

On obtient 90 mg de 4,5,6,8-tétrahydro-5-hydroxy-N-méthyl-6-oxo-4,7-méthano-7*H*-thiéno[2,3-*e*][1,3]diazépine-8-carboxamide, de formule brute C₁₀H₁₁O₃S (M = 253,28 g).

Le rendement correspondant est de 90 %.

### Stade C

On procède comme indiqué au stade B de l'Exemple 11 avec 90 mg du produit obtenu au stade précédent B, 170 mg du complexe SO₃-pyridine et 2 ml de pyridine.

On obtient 30m g sous forme de cristaux jaune pâle de sel de sodium de trans-4,5,6,8-tétrahydro-N-méthyl-6-oxo-5-(sulfooxy)-4,7-méthano-7*H*-thiéno[2,3-*e*][1,3]diazépine-8-carboxamide, de formule brute C₁₀H₁₀N₃O₆S₂.Na (M = 332,34 g, 22,99 g).

Le rendement correspondant est de 24 %.

### RMN du proton

Dans le DMSO-d₆ à 300 MHz, déplacements chimiques et multiplicité :
2,67 (dl) : CH₃-NH-CO-CH ; 8,35 (ql) : CH₃-NH-CO-CH ; 4,94 (s) : CH₃-NH-CO-CH ; 3,36 (d) et 3,48 (dd) : N-CH₂-CH ; 4,73 : N-CH₂-CH ; 6,94 (d) et 7,45 (dl) pour les hydrogènes du thiophène.
SM (électrospray négatif) m/z : M⁻ = 332.

### Exemple 61

### Trans-4,5,6,8-tétrahydro-6-oxo-5-(phénylméthoxy)-1H-4,7-méthanopyrazolo[3,4-e] [1,3]diazépine-8-carboxylate de méthyle

### Stade A : Préparation du [2-(phénylthio)éthyl]hydrazine.

### Etape 1

Dans un ballon placé sous atmosphère d'azote, on introduit 100 g de 2-bromoéthylphényl sulfure dissous dans 11. d'éthanol et on ajoute 184,2 g d'hydrate d'hydrazine. On chauffe à 100°C toute une nuit. Puis, une fois la réaction terminée, on distille le solvant sous pression réduite à 80°C- 90°C. Puis, on ajoute au milieu 65g de carbonate de potassium et 1 1. de chlorure de méthylène. On agite pendant 15 minutes, puis on extrait la phase organique avec 2 x 500 ml d'eau, puis la phase organique est séchée sur sulfate de magnésium, filtrée et évaporée sous pression réduite. On reprend ensuite le résidu dans 750 ml d'un mélange éthanol/eau auquel on ajoute goutte à goutte 12 ml d'acide sulfurique concentré. Le produit cristallise et le précipité est filtré puis rincé avec une solution éthanol/eau, 80/20 puis à l'éther.Le produit est ensuite séché sous pression réduite.

On obtient 81,84 g de sel d'hemisulfate de [2-(phénylthio)éthyl]-hydrazine, de formule brute, C₈H₁₂N₂S +1/2 de H₂SO₄. (M = 217,3 g).

Le rendement obtenu est de 81 %.

### Etape 2

On dissout 79,7 g du produit obtenu à l'étape 1 précédente, dans 1,9 1 de dichlorométhane. Puis on ajoute 400 ml de soude 1N, et on agite fortement. On extrait ensuite la phase organique au dichlorométhane, puis on sèche la phase organique sur MgSO₄ et l'on évapore sous pression réduite.

On obtient avec un rendement quantitatif le [2-(phenylthio)éthyl]-hydrazine de formule brute C₈H₁₂N₂S (M = 168,26 g).

Le rendement obtenu est de 89 %.

### Stade B

On procède comme indiqué au stade B de l'Exemple 5 avec 79 g du produit préparé au stade A de l'exemple 5, 54,5 g de [2-(phénylthio)éthyl]-hydrazine et 1 1. de méthanol.

On obtient le composé 1,4,5,7-tétrahydro-4-oxo-1-[2-(phénylthio)éthyl]-6*H-*pyrazolo[3,4-c]pyridine-6-carboxylate de 1,1-diméthyléthyle, de formule brute C₁₉H₂₃N₃O₃S (M = 373,48 g).

Le rendement obtenu correspondant est de 57,6 %.

### Stade C

On procède comme indiqué au stade A de l'Exemple 18 avec 74,5g du produit obtenu au stade précédent B, 7,58 g de borohydrure de sodium et 372,5 ml de méthanol.

On obtient 72,5 g du composé 1,4,5,7-tétrahydro-4-hydroxy-1-[2-(phényl-thio)éthyl]-6*H*-pyrazolo[3,4-c]pyridine-6-carboxylate de 1,1-diméthyléthyle, de formule brute C₁₉H₂₅N₃O₃S (M = 375,49 g).

Le rendement correspondant est de 97 %.

### Stade D

On dissout 75 g du produit obtenu au stade précédent C dans 1 1. de dichlorométhane à 0°C. On ajoute 118 g d'acide méthachloroperbenzoïque à 70 %, puis on agite 1h30 à température ambiante. On ajoute au milieu réactionnel 1,5 1. de dichlorométhane et 1,61. de thiosulfate de sodium 0,5N. Après extraction de la phase organique, on la relave avec 1 1. de thiosulfate de sodium puis avec 1,5 1. de NaHCO₃ et enfin avec 1,5 1. d'eau. Les phases aqueuses sont alors à nouveau réextraites avec du dichlorométhane, puis les différentes phases organiques sont rassemblées, séchées sur sulfate de magnésium, filtrées, puis le solvant est évaporé sous pression réduite.

Le produit brut est recristallisé dans l'éther isopropylique pour donner 81 g de composé 1,4,5,7-tétrahydro-4-hydroxy-1-[2-[1-propénylsulfonyl]éthyl]-6*H-*pyrazolo [3,4-*c*]pyridine-6-carboxylate de 1,1-diméthyléthyle de formule brute C₁₉H₂₅N₃O₅S (M = 407,49 g).

Le rendement correspondant est de 99 %.

### Stade E

Dans un ballon placé sous atmosphère inerte et à une température de -30°C, on dissout 57,2 g du produit obtenu au stade précédent D, dans 572 ml de THF anhydre. Puis, on ajoute 337 ml d'une solution de tert-butylate de potassium 1M dans le THF. On agite pendant 1 heure puis on ajoute au milieu réactionnel 20 ml d'acide acétique. On ajoute ensuite une solution aqueuse de bicarbonate de NaHCO₃ et NaCl, puis on extrait la phase organique par de l'acétate d'éthyle plusieurs fois. Les phases organiques sont séchées sur sulfate de magnésium, filtrées et le solvant évaporé sous pression réduite. On récupère 57,4g de produit brut qui est ensuite purifié sur silice en éluant avec un mélange dichlorométhane/acétone, 4/6.

On obtient après évaporation du solvant 38,6 g de 1,4,5,7-tétrahydro-4-hydroxy-6H pyrazolo[3,4-c]pyridine-6-carboxylate de 1,1-diméthyléthyle, de formule brute C₁₁H₁₇N₃O₃ (M = 239,28 g).

Le rendement correspondant est de 84 %.

### Stade F

Dans un ballon placé sous atmsophère d'azote, on dissout 35 g du produit obtenu au stade précédent E, dans 890 ml de dichlorométhane. On ajoute 49,77 g de (PPh₃)CCl puis on refroidit la solution à -30°C avec un mélange carboglace/acétone. On ajoute ensuite 24,7 ml de triéthylamine, et on agite le milieu réactionnel en laissant revenir à température ambiante pendant 4h30. Puis, après évaporation du solvant sous pression réduite, on verse le résidu dans 1,61. d'acétate d'éthyle et on lave avec 1,8 1. d'eau. La phase organique est ensuite séchée sur sulfate de magnésium, filtrée, et le solvant évaporé sous pression réduite. Le résidu est repris dans l'éther éthylique et le précipité obtenu est lavé dans du pentane. Après séchage, on obtient 62,5 g de 2,4,5,7-tétrahydro-4-hydroxy-2-(triphénylméthyl)-6*H*-pyrazolo [3,4-*c*]pyridine-6-carboxylate de 1,1-diméthyléthyle, de formule brute C₃₀H₃₁N₃O₃ (M = 481,60 g).

Le rendement est de 87 %.

### Stade G

On procède comme indiqué au stade B de l'Exemple 18 avec 26,6 g du produit obtenu au stade F et 81 ml de tert-butyllithium, 1,7 mole/l dans le pentane, 230 ml de THF et du gaz carbonique. Le mélange réactionnel est hydrolysé par addition de 100 ml d'eau et 300 ml d'acétate d'éthyle puis acidifié à pH=4 par addition d'acide formique. La phase aqueuse est extraite pluisieurs fois à l'acétate d'éthyle puis la phase organique est séchée sur MgSO₄, filtrée et le solvant évaporé sous pression réduite pour donner 29,8 g de produit brut. Ce dernier est dissout dans 300ml d'éther puis extrait par 3x200 ml d'une solution saturée de NaHCO₃ La phase aqueuse est acidifiée à pH=4 par addition d'acide formique puis extrait par l'acétate d'éthyle. Après séchage sur MgS04, filtration et évaporatin sous pression réduite on obtient 14,8 g d'acide de formule bruteC₃₁H₃₁N₃O₅ (M = 525,61g).

Les 15 g du composé obtenu sont ensuite estérifiés en présence de 3,7 g de K₂CO₃ et 4,9 ml de sulfate diméthylique. On agite pendant 1 heure à température ambiante puis on ajoute 7,4 ml de triéthylamine. Après 40 minutes, on ajoute 300 ml d'acétate d'éthyle et 200 ml d'eau. Après agitation, on décante puis on réextrait la phase aqueuse à l'acétate d'éthyle. Les phases organiques sont lavées avec une solution d'eau saturée en NaCl. Les phases organiques sont ensuite séchées sur sulfate de magnésium et le solvant est évaporé sous pression réduite.

On obtient 11,23 g de 2,4,5,7-tétrahydro-4-hydroxy-2-(triphénylméthyl)-6*H-*pyrazolo[3,4-*c*]pyridine-6,7-dicarboxylate de 6-(1,1-diméthyléthyle) et de 7-méthyle, de formule brute C₃₂H₃₃N₃O₅ (M = 739,64 g).

Le rendement correspondant sur les deux étapes est de 42 %.

### Stade H

Dans un ballon sous atmosphère d'argon, on solubilise 1g du produit obtenu au stade précédent G dans 50ml de dichlorométhane. On ajoute 2,8g de triéthylamine puis 4,8 g de CH₃SO₂ )dilués dans 1 ml de dichlorométhane. On agite une heure à - 70°C puis on ajoute 0,68 g de O-benzylhydroxylamine. On agite encore 10 minutes à -78°C, 1h20 à -50°C, et enfin à 0°C pendant toute la nuit. On laisse encore une heure à 20°C, puis on ajoute du dichlorométhane et on lave la phase organique avec une solution d'acide tartrique puis une solution aqueuse de NaCl et enfin d'eau pure. On sèche la phase organique sur MgSO₄, on filtre et on évapore le solvant sous pression réduite. On obtient 11,9 g de produit qui est purifié sur silice en éluant avec un mélange d'éther de pétrole/acétate d'éthyle, 8/2.

On obtient 8,9 g de 2,4,5,7-tétrahydro-4-[(phénylméthoxy)amino]-2-(triphénylméthyl)-6*H* pyrazolo[3,4-*c*]pyridine-6,7-dicarboxylate de 6-(1,1-diméthyléthyle) et de 7-méthyle, de formule brute C₃₉H₄₀N₄O₅ (M = 644,78 g).

Le rendement correspondant est de 75 %.

### Stade I

Dans un ballon maintenu à 0°C, on dissout 10g du produit obtenu au stade précédent H dans 70 ml d'acétate d'éthyle. On ajoute 35 ml d'une solution saturée d'acide chlorhydrique dans l'acétate d'éthyle puis on agite pendant 3 heures. Après évaporation du solvant, on reprend dans l'eau et on lave la phase aqueuse avec de l'acétate d'éthyle. La phase aqueuse est ensuite amenée à pH=10 avec une solution d'ammoniaque à 20 % puis extraite trois fois avec de l'acétate d'éthyle. Après séchage sur sulfate de magnésium, filtration, et évaporation du solvant sous pression réduite, on obtient 4,21 g de 4,5,6,7-tétrahydro-4-[(phénylméthoxy)amino]-2H-pyrazolo[3,4-*c*]pyridine-7-carboxylate de méthyle, de formule brute C₁₅H₁₈N₄O₃ (M = 302,34 g).

Le rendement correspondant est de 89,7 %.

### Stade J

On procède comme indiqué au stade L de l'Exemple 3 avec 9,24 g du produit obtenu au stade précédent J, 12,3 g de triéthylamine, 1,85 mlde diphosgène et 3 l d'acétonitrile.

On obtient 9,6 g de trans-4,5,6,8-tétrahydro-6-oxo-5-(phénylméthoxy)-1H 4,7-méthanopyrazolo[3,4-e][1,3]diazépine-8-carboxylate de méthyle, de formule brute C₁₆H₁₆N₄O₄ (M = 328,33 g).

Le rendement correspondant est de 95 %.

### RMN du proton

DMSO-d₆ à 300 MHz à 60°C, déplacements chimiques et multiplicité :
3,37 (dd) et 3,42 (d) : N-CH₂-CH ; 4,54 : N-CH₂-CH; 3,74 (s) CH₃-O-CO ; 4,90 (s1) : O-CH₂-φ ; 7,29 à 7,44 (m) : 5H aromatiques ; 5,02 (s) : CH-COO ; 7,69 (s) : N=CH ; 7,83 (sl) et 12,7 (l): H mobile.

### Exemple 62

### Sel de triéthylammonium de trans-1-éthyl-4,5,6,8-tétrahydro-6-oxo-5-(sulfooxy)-1H-4,7-méthanopyrazolo[3,4-e][1,3]diazépine-8-carboxylate de méthyle

### Stade A

Dans un ballon maintenu à 0°C et sous atmosphère d'azote, on introduit 0,1 g du produit obtenu à l'Exemple 61 dans 0,8ml de DMF. On ajoute 0,052 g de iodo-éthane et 0,015 g de NaH à 50% dans l'huile, puis on agite 15 minutes à 0 °C et on laisse revenir le milieu réactionnel à température ambiante. On agite 1h30, puis on ajoute de l'acétate d'éthyle et on lave la phase organique avec une solution aqueuse de NH₄Cl puis on extrait la phase aqueuse avec de l'acétate d'éthyle. Après séchage de la phase organique sur sulfate de magnésium et filtration, on évapore le solvant sous pression réduite. On obtient 102 mg de produit qui est purifié sur colonne de silice en éluant avec un mélange heptane/acétate d'éthyle 1/1 à 1,1% de triéthylamine.

On obtient 22,4 mg de l-éthyl-4,5,6,8-tétrahydro-6-oxo-5-(phénylméthoxy)-1*H*-4,7-méthanopyrazolo [3,4-e][1,3]diazépine-8-carboxylate de méthyle et 29 mg de 2-éthyl-4,5,6,8-tétrahydro-6-oxo-5-(phénylméthoxy)-4*H*-4,7-méthanopyrazolo[3,4-*e*][1,3]diazépine-8-carboxylate de méthyle, de formule brute C₁₈H₂₀N₄O₄ (M = 358,38 g).

### Stade B

On procède comme au stade A de l'Exemple 11 avec 22m g de 1-éthyl-4,5,6,8-tétrahydro-6-oxo-5-(phénylméthoxy)-1*H*-4,7-méthanopyrazolo[3,4-e][1,3]diazépine-8-carboxylate de méthyle dans 1 ml. de méthanol et avec 0,0132 g de palladium sur charbon à 10 % en poids.

On obtient 15,8 mg de 1-éthyl-4,5,6,8-tétrahydro-5-hydroxy-6-oxo-1*H*-4,7-méthanopyrazolo[3,4-*e*][1,3]diazépine-8-carboxylate de méthyle, de formule brute C₁₁H₁₄N₄O₄ (M = 266,26 g).

Le rendement correspondant est de 88 %.

### Stade C

Dans un ballon placé sous atmosphère inerte on introduit 0,0158 g du produit obtenu au stade précédent B dans 1 ml de pyridine, puis on ajoute 0,028 g du complexe pyridine-SO₃... On agite une nuit à température ambiante, puis on filtre, on dilue la solution de chlorure de méthylène puis on lave à l'eau et on évapore le solvant sous pressio réduite et on obtient un produit brut que l'on purifie sur silice en éluant avec un mélange dichlorométhane/éthanol 95/5. à 0,1 % de triéthylamine

On obtient 11 mg de sel de triéthylammonium de trans-1-éthyl-4,5,6,8-tétrahydro-6-oxo-5-(sulfooxy)-1*H*-4,7-méthanopyrazolo[3,4-*e*][1,3]diazépine-8-carboxylate de méthyle, de formule brute C₁₁H₁₃N₄O₇S ,C₆H₁₆N(M = 447,51 g. 102,20g).

Le rendement est de 41,4%

### LC/MS : Conditions générales :

Colonne kromasil C18 4,6 x 250 mm, 5 µ Four à 30°C
Débit = 1 ml/minute. Vᵢₙⱼ = 15 µl
Détection λ= 200-400 mm
SM/ESP mode+/- CV = 50 V
- Eluant :: A = H₂O (0,1 % HCO₂H)
B = CH₃ CN

| Gradient : | temps. | A % | B % |
|---|---|---|---|
| | 0,00 | 80,0 | 20,0 |
| | 15,00 | 50,0 | 50,0 |
| | 25,00 | 20,0 | 80,0 |
| | 40,00 | 80,0 | 20,0 |
| | 50,00 | 80,0 | 20,0 |

| | | | |
|---|---|---|---|
| LC/ESP m/z= (2M+Na)⁻ = 713; (M)⁻ =345; (M-CH₃OH)⁻ = 313 | | | |

### Exemple 63

### Sel de triéthylammonium de trans-2-éthyl-4,5,6,8-tétrahydro-6-oxo-5-(sulfooxy)-4H-4,7-méthanopyrazolo[3,4-e][1,3]diazépine-8-carboxylate de méthyle

### Stade A

On procéde comme indiqué au stade A de l'Exemple 11 avec 0,029 g de 2-éthyl-4,5,6,8-tétrahydro-6-oxo-5-(phénylméthoxy)-4*H*-4,7-méthanopyrazolo-[3,4-*e*][1,3]diazépine-8-carboxylate de méthyle, 0,5 ml de méthanol et 0,0174 g de palladium sur charbon.

On obtient 20,1 mg de 2-éthyl-4,5,6,8-tétrahydro-5-hydroxy-6-oxo-4*H*-4,7-méthanopyrazolo[3,4-*e*][1,3]diazépine-8-carboxylae de méthyle, de formule brute C₁₁H₁₄N₄O₄ (M = 266,26 g).

Le rendement correspondant est de 85 %.

### Stade B

On procède comme indiqué au stade C de l'Exemple 62 avec 0,02 g du produit obtenu au stade précédent A, 0,036 g du complexe pyridine-SO₃ et 1 ml de pyridine.

On obtient 21 mg de sel de triéthylammonium de trans-2-éthyl-4,5,6,8-tétra-hydro-6-oxo-5-(sulfooxy)-4*H*-4,7-méthanopyrazolo[3,4-*e*][1,3]diazépine-8-carboxylate de méthyle, de formule brute C₁₁H₁₃N₄O₇S + C₆H₁₆N (M = 345,31 g + 102,20 g).

Le rendement correspondant est de 62 %.
LC/ESP : voir conditions générales exemple 62
TR = 5,01 mn. m/z : M⁻ = 345.

### Exemple 64

### Sel de sodium de N-[4,6,7,8-tétrahydro-6-oxo-7-(sulfooxy)-5,8-méthano-5H-thiazolo[4,5-e][1,3]diazépin-2-yl]-acétamide

### Stade A

Dans un ballon placé sous atmosphère d'azote, on introduit 2,69 g du produit préparé au stade C de l'Exemple 20 dans 50 ml de THF. Puis, on ajoute 1,22g de DMAP et on refroidit la solution à 0°C. On ajoute ensuite 0,94 ml d'anhydride acétique puis on agite pendant 1h30 en laissant revenir le mélange réactionnel à température ambiante. Une fois la réaction terminée, on ajoute à la solution de l'acétate d'éthyle et de la glace. La phase organique est lavée trois fois à l'eau puis séchée sur sulfate de magnésium et le solvant est évaporé sous pression réduite. On isole 2,95 g de produit brut qui est recristallisé dans l'acétonitrile. Après essorage et séchage sous vide, on obtient 2,39 g de 2-(acétylamino)-6,7-dihydro-7-oxo-thiazolo [4,5-*c*]pyridine-5(4*H*)-carboxylate de 1,1-diméthyléthyle, de formule brute C₁₃H₁₇N₃O₄S (M = 311,36 g).

Le rendement obtenu est de 77 %.

### Stade B

On procède comme indiqué au stade A de l'Exemple 18 avec 2,39 g du produit obtenu au stade précédent A, 290 mg de NaBH₄, 15 ml de méthanol et 15 ml de THF.

On obtient 2,024 g sous forme de cristaux incolores de 2-(acétylamino)-6,7-dihydro-7-hydroxy-thiazolo[4,5-c]pyridine-5(4H)-carboxylate de 1,1-diméthyléthyle, de formule brute, C₁₃H₁₉N₃O₄S (M = 313,38 g).

Le rendement obtenu est de 84 %.

### Stade C

On procède comme indiqué au stade H de l'Exemple 61, avec 900 mg du produit obtenu au stade précédent B, 750 mg de (CH₃SO₂)₂O, 15 ml de DMF, 600 µl de triéthylamine et 1,061 g de O-benzylhydroxylamine.

On obtient 0,617 mg du sel de pyridinium de 2-(acétylamino)-6,7-dihydro-7-[(phénylméthoxy)amino]-thiazolo[4,5-c]pyridine-5(4H)-carboxylate de 1,1-diméthyléthyle, de formule brute C₂₀H₂₆N₄O₄S (M = 418,51 g).

Le rendement est de 25 %.

### Stade D

Dans un ballon, on introduit 559 mg du produit obtenu au stade précédent C, dans 15 ml de dichlorométhanne en présence de 293 µl d'anisole. Puis la solution est refroidie à 0°C et on additionne rapidement 14 ml d'acide trifluoroacétique. On agite deux heures la solution en laissant revenir à température ambiante, puis on évapore le solvant sous pression réduite. Le milieu réactionnel est dilué à l'acétate d'éthyle et on lave deux fois à l'eau puis on extrait les phases aqueuses à l'acétate d'éthyle. Les phases organiques réunies sont séchées sur sulfate de sodium puis évaporées sous pression réduite. Le produit brut obtenu est repris dans l'acétate d'éthyle et extrait deux fois avec une solution d'acide chlorhydrique 1M. La phase aqueuse est ensuite lavée à l'acétate d'éthyle puis la phase aqueuse est amenée à pH 12 avec une solution de soude 2M. On extrait à nouveau deux fois l'acétate d'éthyle et on lave à l'eau. Les phases organiques sont ensuite réunies afin d'être séchées sur sulfate de sodium, filtrées puis le solvant est évaporé sous pression réduite. On isole 184 mg de *N-*[4,5,6,7-tétrahydro-7-[(phénylméthoxy)amino]thiazolo[4,5-*c*]pyridin-2-yl]-acétamide, de formule brute C₁₅H₁₈N₄O₂s (M = 318,40 g).

Le rendement est de 43 %.

### Stade E

On procède comme indiqué au stade L de l'Exemple 3 avec 184 mg du produit obtenu au stade précédent D, 241 µl de triéthylamine, 38 µl de diphosgène et 125 ml d'acétonitrile.

On obtient 70 mg du *N*-[4,6,7,8-tétrahydro-6-oxo-7-(phényméthoxy)-5,8méthano-5*H*-thiazolo [4,5-*e*][1,3]diazépin-2-yl]-acétamide, de formule brute C₁₆H₁₆N₄O₃S (M = 344,39 g).

Le rendement correspondant est de 35 %.

### Stade F

Dans un ballon, on introduit 43 mg du composé obtenu au stade précédent E dans 4,5 ml de méthanol puis on ajoute en plusieurs fois 215 mg de palladium sur charbon à 30 %. On maintient le milieu sous hydrogène pendant plusieurs heures. Une fois la réaction terminée, on filtre le catalyseur et on évapore le solvant sous pression réduite. On obtient 20 mg de produit attendu N-(4,6,7,8-tétrahydro-7-hydroxy-6-oxo-5,8-méthano-5*H*-thiazolo[4,5-*e*][1,3]diazépin-2-yl)-acétamide, de formule brute C₉H₁₀N₄O₃S (M = 254,26 g).

### Stade G

On utilise le produit brut obtenu au stade précédent F que l'on dissout dans 0,4 ml de pyridine. Puis on ajoute 41 mg du complexe pyridine-SO₃. On agite une nuit à température ambiante, puis on évapore la pyridine sous vide. On reprend le résidu avec de l'acétate d'éthyle en présence d'une solution aqueuse de NaH₂PO₄ saturé. On extrait deux fois à l'acétate d'éthyle et avec une solution aqueuse de NaH₂PO₄. Enfin on ajoute à la phase aqueuse 25,7 mg de dihydrogénosulfate tétrabutylammonium et on extrait à l'acétate d'éthyle. La phase organique est séchée sur Na₂SO₄ puis filtrée et évaporée sous pression réduite. On isole 17 mg de produit brut qui sont purifiés sur silice en éluant avec un mélange chloroforme/acétonitrile (4/6). Après évaporation des fractions, on recueille 8 mg de sel de tétrabutylammonium de *N*-[4,6,7,8-tétrahydro-6-oxo-7-(sulfooxy)-5,8-méthano-5*H*-thiazolo[4,5-e][1,3]diazépin-2-yl]-acétamide qui sont transformés en sel de sodium de *N*-[4,6,7,8-tétrahydro-6-oxo-è7-(sulfooxy)-5,8-méthano-5*H*-thiazolo[4,5-*e*][1,3]diazépin-2-yl]-acétamide, de forme brute C₉H₉NaO₆S₂.Na+ (M = 333,23 g) par passage de résine Dowex 50WX8 sous forme Na+.

Le rendement obtenu sur les deux étapes F et G est de 6,7 %.
SM (électrospray négatif) m/z : M⁻ = 333.

### Exemple 65

### Sel de sodium de 7,8-dihydro-7-(sulfooxy)-5,8-méthano-5H-thiéno[2,3-e][1,3]-diazépin-6(4H)-one

### Stade A

On dissout 3,9 g (22,89 mmoles) de 3-formyle 2-thiophène carboxylate de méthyle dans 39 ml de méthanol. On refroidit dans un bain d'eau glacée et on ajoute en cinq fois et en cinq minutes, 305 mg (7,66 mmoles) de NaBH₄ à 95 %. On agite une heure à 2°C. Puis on ajoute 39 ml d'acétate d'éthyle et 39 ml d'une solution aqueuse M de NaH₂PO₄. Après décantation, on extrait la phase organique par 20 ml d'acétate d'éthyle puis on lave par 20 ml d'eau saturée au demi par HCl.

Après séchage sur sulfate de magnésium, filtration et évaporation du solvant sous vide, on obtient 3,9 g de 3-(hydroxyméthyl)-2-thiophènecarboxylate de méthyle, de formule brute C₇H₈O₃S (M = 172,20 g).

Le rendement obtenu est quantitatif.

### Stade B

On procède comme indiqué au stade C de l'Exemple 22 avec 3,9 g (22,65 mmoles) du produit obtenu au stade précédent A, 39 ml de dichlorométhane et 7,8 ml (107,56 mmoles) de SOCl₂.

On obtient 3,85 g de 3-(chlorométhyl)-2-thiophènecarboxylate de méthyle, de formule brute C₇H₇ClO₂S (M = 190,65 g).

Le rendement est de 89 %.

### Stade C

On procède comme indiqué au stade D de l'Exemple 22 avec 3,84 g (20,4 mmoles) du produit chloré obtenu au stade précédent B, 41,5 ml d'acétonitrile, 11,15 g de K₂CO₃ et 8,45 ml de glycinate de tert-butyle..

On obtient après purification 3,76g de 3-[[[(1,1-diméthyléthoxy)carbonyl]-amino]méthyl]-2-thiophènecarboxylate de méthyle, de formule brute C₁₃H₁₉NO₄S (M = 285,36 g).

Le rendement correspondant est de 65 %.

### Stade D

On procède comme indiqué au stade E de l'Exemple 22 avec 3,73 g (13,07 mmoles) du produit obtenu au stade précédent C, 37 ml de THF et 3,17 g (14,38 mmoles) de (BOC)₂O à 99 %.

On obtient 5,35 g de 3-[[[(1,1-diméthyléthoxy)carbonyl] [2-(1,1-diméthyl-éthoxy)-2-oxoéthyl] amino]méthyl]-2-thiophènecarboxylate de méthyle, de formule brute C₁₈H₂₇NO₆S (M = 385,48 g).

Le rendement obtenu est quantitatif.

### Stade E

On procède comme indiqué au stade F de l'Exemple 22, avec 5,3 g (12,95 mmoles) du produit obtenu au stade précédent D, 21 ml de THF anhydre, 2,97 g (25,9 mmoles) de tert-butylate de potassium à 98%.

On obtient 3,74 g sous forme de cristaux jaunes de 6,7-dihydro-7-oxo-thiéno [3,2-*c*]pyridine-5,6(6*H*)-dicarboxylate de bis(1,1-diméthyléthyle), de formule brute C₇H₂₀NO₅S (M = 353,44 g).

Le rendement correspondant est de 82 %.

### Stade F

On procède comme indiqué au stade H de l'Exemple 22, avec 3,65 g (10,33 mmoles) du produit obtenu au stade précédent E, 29 ml d'acide trifluoroacétique.

On obtient 2,76 g sous forme de cristaux jaunes de sel de trifluoroacétique de 4,5,6,7-tétrahydro-7-oxo-thiéno[3,2-*c*]pyridinium, de formule brute C₇H₇NO₅S, CF₃CO₂H (M = 267,23 g).

Le rendement correspondant est de 82,6 %.

On met en suspension 2,24 g (8,38 mmoles) du produit obtenu précédemment, dans 22,5 ml de THF. Puis on ajoute à température ambiante 1,4 ml (10 mmoles) de triéthylamine. Après dissolution des composants, on introduit 2,03 g (9,22 mmoles) de di-tert-butyl carbonate. On agite une heure à température ambiante, puis on introduit 30 ml d'une solution aqueuse saturée aux deux tiers de NaCl, on extrait par 15 ml d'acétate d'éthyle, on lave la phase organique par 15 ml d'une solution aqueuse de NaH₂PO₄ 0,1M et 10 ml d'une solution d'eau salée. Après séchage sur sulfate de magnésium, filtration et évaporation des solvants sous pression réduite, on obtient 2,3 g sous forme de résidus jaunes pâles de 6,7-dihydro-7-oxo-thiéno[3,2-*c*]pyridine-5(4*H*)-carboxylate de 1,1-diméthyléthyle, de formule brute C₁₂H₁₅N₃S (M = 253,32 g).

Le rendement correspondant est de 92 %.

### Stade G

On procède comme indiqué au stade I de l'Exemple 22 avec 253 mg du produit obtenu au stade F précédent, 181 mg de chlorhydrate de O-alkylhydroxyl-amine, 243 µl de pyridine et 5 ml d'éthanol.

On obtient 328 mg de 6,7-dihydro-7-[(2-propényloxy)imino]-thiéno[3,2-*c*]pyridine-5(4*H*)-carboxylate de 1,1-diméthyle, de formule brute C₁₅H₂₀N₂O₃S (M = 308,40 g).

Le rendement est quantitatif.

### Stade H

On procède comme indiqué au stade B de l'Exemple 6, avec 1 g (3,24 mmoles) du produit obtenu au stade G, 32 ml de méthanol, 3,26 g (51,84 mmoles) de cyanoborohydrure de sodium et 4,93 ml (38,88 mmoles) d'étherate de trifluorure de bore.

On obtient 664 mg sous forme d'huile jaune de 6,7-dihydro-7-[(2-propényloxy)amino]-thiéno[3,2-*c*]pyridine-5(4*H*)-carboxylate de 1,1-diméthyléthyle, de formule brute C₁₅H₂₂N₂O₃S (M = 310,4182 g).

Le rendement correspondant est de 66 %.

### Stade I

On procède comme indiqué au stade D de l'Exemple 64 avec 588 mg du produit obtenu au stade précédent H, 20 ml de chlorure de méthylène, 0,38 ml d'anisole et 19 ml d'acide trifluoroacétique.

On obtient 326 mg sous forme d'huile incolore de 4,5,6,7-tétrahydro-7-[(2-propényloxy)amino]-thiéno[3,2-c]pyridine, de formule brute C₁₀H₁₄N₂OS (M = 210,29 g).

Le rendement correspondant est de 82 %.

### Stade J

On procède comme indiqué au stade L de l'Exemple 3 en prenant 307 mg du produit obtenu au stade précédent I, 609 µl de TEA, 97 µl de diphosgène et 150 ml d'acétonitrile.

On obtient 128mg sous forme d'huile de 7,8-dihydro-7-(2-propényloxy)-5,8-méthano-5*H*-thiéno[3,2-*e*][1,3]diazépin-6(4*H*)-one, de formule brute C₁₁H₁₂N₂O₂S (M = 236,29 g).

Le rendement correspondant est de 37 %.

### Stade K

On procède comme indiqué à l'Exemple C de l'Exemple 7 avec 128 mg du produit obtenu au stade précédent J, 2 ml de dichlorométhane, 93 µl d'acide acétique, 313 mg de Pd(PPh₃)₄, 295 mg du complexe pyridine-SO₃ et 2 ml de pyridine..

On obtient 58 mg de sel de sodium de 7,8-dihydro-7-(sulfooxy)-5,8-méthano-5*H*-thiéno[2,3-*e*][1,3]diazépin-6(4*H*)-one, de formule brute C₈H₇N₂NaO₅S₂ (M = 298,27g).

Le rendement est de 36 %.

### RMN du proton

Dans DMSO-d₆ à 300 MHz, déplacements chimiques et multiplicité :
3,30 (d), 3,60 (dd) : N-CH₂-CH ; 4,78 (d) : N-CH₂-CH ; 4,19 et 4,28 : N-CH₂-C= ; 6,88 (d), 7,45 (d), les H du thiophène.
SM (électrospray négatif) :m/z : (2M+Na)⁻ =573 ; (2M+H)⁻ = 551 ; (M)⁻ =275

### Exemple 66

### Sel de sodium de 4,8-dihydro-5-(sulfooxy)-4,7-méthano-7H-isoxazole[4,5-e][ 1,3]diazépin-6(5H)-one

### Stade A

Dans un ballon, on introduit dans 144 ml de méthanol 12 g de 4-[(diméthylamino)méthylène]-3,5-dioxo-1-pipéridinecarboxylate de 1,1-diméthyléthyle avec 7,82 (112 mmoles) de chlorhydrate d'hydroxylamine, on agite ensuite 16 heures à 20°C et ensuite on évapore le solvant sous pression réduite. Le mélange est versé dans 10 ml d'eau, ensuite on procède à l'extraction à l'acétate d'éthyle (3 x 10 ml). On rassemble les phases organiques obtenues, on les sèche sur Na₂SO₄, on les filtre puis on évapore le solvant sous pression réduite.Le produit brut est purifié par chromatographie sur silice en éluant par CH₂CL₂/isopropanol 97/3. On obtient 3,93g de 4,7-dihydro-4-(hydroxylmino)-isoxazolo[5,4-*c*]pyridine-6(5H)-carboxylate de 1,1-diméthyléthyle, de formule brute C₁₁H₁₅N₃O₄ (M = 253,26 g).

Le rendement est de 35%.

### Stade B

Dans un ballon placé sous atmosphère d'azote, on place 3,5g (14 mmoles) du composé obtenu au stade A dans 60cm³ de DMF. Après dissolution, on refroidit la solution à 0°C puis on additionne 2,26ml de bromure d'allyleet 1,01gde NaH à 50% dans l'huile, on agite à 20°C pendant 1 heure puis on arrête la réaction en versant la suspension sur un mélange glace-eau.. On ajoute de l'acétate d'éthyle et on laisse décanter. On extrait à nouveau 3 fois par de l'acétate d'éthyle, on rassemble les phases acétate d'éthyle et on les lave deux fois à l'eau déminéralisée. On rassemble les phases organiques et on les sèche sur MgSO₄, on les filtre, on les rince puis on les évapore sous pression réduite. On obtient 6,57 g d'huile jaune que l'on purifie par chromatographie sous pression sur silice en utilisant comme éluant un mélange hexane/acétate d'éthyle (6 /4).

On obtient 3,43 g de 4,7-dihydro-4-(hydroxyimino)-isoxazolo[5,4-*c*]pyridine-6(5*H*)-carboxylate de 1,1-diméthyléthyle, de formule brute C₁₄H₁₉N₃O₄ (M = 293,33 g).

Le rendement correspondant est de 77,3 %.

### Stade C

Dans un tricol, on place 2,71 g (9,24 mmoles) du composé obtenu au stade B et 38 ml de méthanol. On refroidit le mélange à -5°C -10°C, puis on ajoute 9,14 g (0,138 mmole) de NaBH₃CN. On ajoute ensuite goutte à goutte 14,05 cm³ (0,111 mole) de d'éthérate de triflorure de bore. On agite pendant 5 minutes à -5°C, puis à 20°C pendant 1 heure, ensuite on verse le milieu réactionnel sur un mélange NaHC0₃ saturé/acétate d'éthyle, on agite 10 minutes. Après décantation, on procède à une extraction par 4 x 150 cm³ d'acétate d'éthyle, on lave les phases organiques par 1 x 150 cm³ d'une solution de NaOH 2N, puis par 2 x 150 cm³ d'eau (jusqu'à un pH neutre). On sèche la phase organique sur MgSO₄, on rince à l'acétate d'éthyle puis on évapore le solvant sous pression réduite pour obtenir 4,2136 g d'une huile jaune claire qui est purifiée par chromatographie sur silice en utilisant un mélange éluant hexane/acétate d'éthyle (8/2).

On obtient 1,11g g de 4,7-dihydro-4-[(2-propényloxy)amino]-isoxazolo[5,4-*c*]pyridine-6(*5H* )-carboxylate de 1,1-diméthyléthyle, de formule brute C₁₄H₂₁N₃O₄ (M = 295,34 g).

Le rendement correspondant est de 41 %.

### Stade D

Dans un tricol de 50 cm³ muni d'une agitation magnétique, d'une sonde de température, on place 1,24 g du composé obtenu au stade C (4,21 mmoles) et 8,7 cm³ d'acétate d'éthyle. On refroidit la solution à 0 °C puis on ajoute goutte à goutte 8,7 cm³ d'une solution saturée d'HCl gazeux dans l"acétate d'éthyle. On laisse agiter 5 minutes à 0°C puis on ramène la température de la solution à 20°C. Au bout de deux heures d'agitation à 20°C, on évapore le solvant sous pression réduite pour obtenir une poudre blanche à laquelle on ajoute un mélange acétate d'éthyle/eauet on ajoute une solution d'ammoniaque à 20%. Après dissolution et agitation pendant 10 minutes, on procède à une décantation, puis on extrait de nouveau par 3 x avec l'acétate d'éthyle. On sèche la phase organique sur MgSO₄, on filtre, et on rince à l'acétate d'éthyle. Enfin, on évapore le solvant sous pression réduite pour obtenir 0,740 g de 4,5,6,7-tétrahydro-4-[(2-propényloxy)amino]-isoxazolo[5,4-*c*]pyridine, de formule brute C₉H₁₃N₃O₂ (M = 195,22 g).

Le rendement correspondant est de 90 %.

### Stade E

Dans un tricol placé sous atmosphère d'azote, on place 0,661 g (3,38 mmoles) du composé obtenu au stade D et 227 ml de CH₃CN ainsi que 1,24 ml (8,8 mmoles) de TEA. On ajoute à -5°C/-10°C en une fois 0,209 ml (1,72 mmoles) de diphosgène au milieu réactionnel. On agite ensuite pendant 5 minutes à cette température, puis on maintient à 20°C. Le milieu réactionnel est alors versé sur 150 ml d'acétate d'éthyle puis on procède au lavage par 100 ml d'eau déminéralisée. Deux autres lavages à l'eau déminéralisée sont effectués. Après décantation, on extrait les phases aqueuses dans 1 x 100 ml d'acétate d'éthyle puis on laisse décanter. On rassemble les phases organiques puis on les sèche sur MgSO₄ on les rince à l'acétate d'éthyle puis on évapore le solvant sous pression réduite pour obtenir 0,894 g de cristaux blancs que l'on purifie par chromatographie sur silice à l'aide d'un éluant CH₂Cl₂/acétone (97/3).

On obtient 0,5573 g de 4,8-dihydro-5-(2-propényloxy)-4,7-méthano-7*H-*isoxazolo [4,5-*e*][1,3]diazépin-6(5H)-one, de formule brute C₁₀H₁₁N₃O₃ (221,22 g).

Le rendement correspondant est de 74,8 %.

### Stade F

On procède comme indiqué au stade C de l'Exemple 7 avec 250 mg du produit obtenu au stade E, 4, ml de CH₂Cl₂, 0,038 ml d'éthanol, 0,653 g de Pd(PPh₃)₄ ainsi que, 4,8 ml de pyridine, et 566,5 mg de p-sulfate de pyridinium 4 pour obtenir 0,133 g de sel de sodium de 4,8-dihydro-5-(sulfooxy)-4,7-méthano-7*H* -isoxazolo [4,5-*e*][1,3]diazépin-6(5*H*)-one, de formule brute C₇H₆N₃Na.O₆S (283,20 g).

Le rendement correspondant est de 41 %.

### Spectre RMN du proton.

Dans D₂O, à 300 MHz, déplacements chimiques et multiplicité:
3,40 (d) et 3,88 (dd) : N-CH₂-O-CH ; 5,15 (d) : N-CH₂-CH ; 4,60 et 4,66 : N-CH₂-C= ; 8,77 (s) : HC=
SM (electrospray négatif) m/z : (M)=260

### Exemple 67

### Sel de sodium de 5,9-dihydro-6-(sulfooxy)-5,8-méthano-8H-pyrido[2,3-e][1,3] - diazépin-7(6H)-one]

### Stade A

On dissout 8 ml (51,6 mmoles) de 2-méthylnicotinate d'éthyle dans 93 ml de tétrachlorure de carbone. Puis on ajoute 2,9 ml d'acide acétique, 17,73 g de N-bromosuccinimide et 0,185g d'AIBN. On place le mélange sous une lampe de 500 W pendant 4 heures. Puis on verse la solution rouge et la gomme obtenue dans une solution glacée de bicarbonate de sodium. On extrait la phase aqueuse par dichlorométhane, on sèche et on évapore le solvant sous pression réduite. On obtient 14,74 g d'huile rouge contenant le dérivé bromé attendu. Puis l'huile obtenue est dissoute dans 90 ml de THF anhydre en présence de 6 ml de triéthylamine et 15g (72 mmoles) de N-benzyl-glycinate de tert-butyle. On agite pendant 24 heures à température ambiante, on dilue à l'eau puis on extrait à l'acétate d'éthyle. La phase organique est séchée sur sulfate de magnésium puis le solvant est évaporé sous pression réduite. On obtient 25 g de produit sous forme de résine que l'on purifie par chromatographie sur silice en éluant avec un mélange cyclohexane/acétate d'éthyle (80/20). Après évaporation du solvant, on obtient 14,18 g sous forme d'huile orangée du 2-[[[2-(1,1-diméthyléthoxy)-2-oxoéthyl](phénylméthyl)amino]méthyl]-3-pyridinecarboxylate d'éthyle, de formule brute C₂₁H₂₆N₂O₄ (M = 370,45 g).

Le rendement obtenu est de 71,6 %.

### Stade B

On dissout 0,740 g du produit obtenu au stade A précédent dans 8 ml de THF et on place sous athmosphère inerte. Puis on refroidit cette solution à -70°C et on introduit en 15 minutes une solution contenant 0,448 g de tert-butylate de potassium dans 4 ml de THF. On agite deux heures à -78°C puis la solution brune est versée sur une solution saturée de phosphate monosodique. On extrait à l'acétate d'éthyle. On sèche la phase organique sur sulfate de magnésium puis les solvants sont évaporés sous pression réduite. On obtient 0,664 g sous forme d'huile jaune de 7,8-dihydro-5-hydroxy-7-(phénylméthyl)-1,7-naphthyridine-6-carboxylate de 1,1 -diméthyléthyle, de formule brute C₂₀H₂₂N₂O₃ M - 338,41 g).

Le rendement obtenu est de 98 %.

### Stade C

On dissout 4,26 g de produit obtenu comme décrit au stade précédent D dans 6 ml de dichlorométhane puis on refroidit la solution à 0°C. On ajoute lentement 20 ml d'acide trifluoroacétique. On agite une demi-heure à 0°C et pendant 1 heure à 20°C et on évapore le solvant sous pression réduite. Le résidu obtenu est dissous dans 20 ml de méthanol puis on introduit lentement dans cette solution du borohydrure de sodium jusqu'à saturation et on laisse agiter une nuit à 0°C. On dilue ensuite la solution par une solution aqueuse de phosphate monosodique, on extrait au dichlorométhane. On obtient 2,742 g de produit brut que l'on purifie par chromatographie sur silice en éluant avec un mélange dichlorométhane /méthanol (95/5). On obtient 1,99 g de produit 7,8-dihydro-7-(phénylméthyl)-1,7-naphthyridin-5-(6*H*)-ol, de formule brute C₁₅H₁₆N₂O(M = 240,31 g).

Le rendement correspondant est de 65,8 %.

### Stade D

On place sous athmosphère d'hydrogène pendant 4 heures un mélange contenant 0,980 g du produit obtenu au stade précédent, 30 ml d'éthanol, 8,16 ml d'acide chlorhydrique 2N et 0,445 g de palladium sur charbon à 10 %. Puis après 4 heures, on filtre le catalyseur, on lave par de l'éthanol et une solution d'HCl 0,1N et on évapore les solvants sous pression réduite. Puis on effectue à nouveau une hydrogénolyse dans les mêmes conditions que précédemment.

Le produit brut obtenu 5,6,7,8-tétrahydro-1,7-naphthyridin-5-ol est utilisé sans purification dans l'étape suivante.

### Stade E

On dissout le produit brut obtenu au stade précédent D dans 3 ml de THF, 18 ml de triéthylamine et on ajoute 1,520 g de di-tert-butyl carbonate. On agite pendant 72 heures à 0°C. Le mélange réactionnel est ensuite concentré sous pression réduite et le résidu est purifié par chromatographie sur silice en éluant avec un mélange dichlorométhane/méthanol (95/5). On obtient après évaporation des solvants 0,771 g sous forme d'huile brune de 5,8-dihydro-5-hydroxy-1,7-naphthyridine-7(6*H*)-carboxylate de 1,1-diméthyléthyle, de formule brute C₁₃H₁₈N₂O₃ (M = 250,30 g).

Le rendement correspondant est de 75 %.

### Stade F

On refroidit à -70°C sous atmosphère inerte une solution contenant 0,5 g du produit obtenu au stade précédent E, 10 ml de dichlorométhane, 2 ml d'acétonitrile et 3 ml de triéthylamine. On introduit ensuite en une fois 0,52g d'anhydride mésylique. On agite pendant une demi-heure à -70°C puis on introduit 1 ml de O-allyl hydroxylamine. On laisse revenir à température ambiante et on poursuit l'agitation pendant une nuit. On ajoute du DMAP et on porte la solution à 30°C pendant 5 heures après avoir éliminé le solvant sous pression réduite. On dilue ensuite avec du dichlorométhane, on lave avec une solution d'HCl diluée puis on lave à l'eau. La phase organique une fois séchée sur sulfate de magnésium et les solvants évaporés sous pression réduite, on obtient 0,6 g de produit brut qui sont purifiés par chromatographie sur silice en éluant avec un mélange cyclohexane/acétate d'éthyle (5/5). On obtient 0,35 g de 5,8-dihydro-5-[(2-propényloxy)amino]-1,7-naphthyridine-7(6*H*)-carboxylate de 1,1-diméthyléthyle, de formule brute C₁₆H₂₃N₃O (M = 305,38 g).

Le rendement correspondant est de 57 %.

### Stade G

On dissout 0,330 g du produit obtenu au stade précédent F dans 4 ml d'acétate d'éthyle. On ajoute 7 ml d'une solution saturée d'HCl gazeux dans l'acétate d'éthyle. On agite pendant deux heures la suspension blanche. Puis, on ajuste la suspension à pH alcalin par de l'ammoniaque. On dilue à l'eau puis on extrait à l'acétate d'éthyle et la phase organique est évaporée sous pression réduite et on obtient 0,176 g de produit brut qui sont purifiés par chromatographie sur silice en éluant avec un mélange dichlorométhane/méthanol (95/5). On obtient 0,156 g de 5,6,7,8-tétrahydro-5-[(2-propényloxy)amino]-1,7-naphthyridine. Le rendement obtenu correspondant est de 70 %.

### Stade H

On refroidit à 0°C sous atmosphère inerte une solution contenant 0,114g (0,55 mmole) du produit obtenu au stade précédent G, 5 ml d'acétonitrile. On ajoute lentement 0,034 ml de diphosgène puis on agite pendant une demi-heure à 0°C. On introduit ensuite goutte à goutte 0,16 ml de triéthylamine et on l'agite pendant une nuit. On dilue le milieu réactionnel par de l'eau puis on extrait au dichlorométhane. La phase organique est séchée puis évaporée sous pression réduite. On obtient 0,189 g sous forme d'huile qui est reprise dans 1 ml de pyridine et une quantité catalytique de DMAP. Cette solution est portée à 30°C pendant une demi-heure, puis la pyridine est évaporée sous pression réduite et le résidu est dilué par de l'eau et extrait au dichlorométhane. Après séchage et évaporation du solvant, on obtient 0,1 g de résine qui est purifiée par chromatographie sur silice en éluant avec un mélange dichlorométhane/méthanol (95/5). On récupère 0,085 g de 5,9-dihydro-6-(2-propényloxy)-5,8-méthano-8*H*-pyrido[2,3-*e*][1,3]diazépin-7(6*H*)-one, de formule brute C₁₂H₁₃N₃O₂ (M = 231,26 g).

Le rendement correspondant est de 66 %.

### Stade I

On procède comme indiqué au stade M de l'Exemple 3 avec 0,039 g du produit obtenu au stade précédent H, 0,6 ml de dichlorométhane, 0, 1g de palladium tétrakistriphénylphosphine, 0,081 g du complexe SO₃-pyridine et 0,6 ml de pyridine, et résine Dowex 50W8. On obtient 0,030 g de sel de sodium de 5,9-dihydro-6-(sulfooxy)-5,8-méthano-8*H*-pyrido[2,3-*e*][1,3]diazépin-7(6*H*)-one, de formule brute C₉H₈N₃NaO₅S (M = 293,24 g).

Le rendement correspondant est de 48 %.

### Spectre RMN du proton

Dans DMSO-d₆ à 300 MHz, déplacements chimiques et multiplicité :
3,57 (d) et 3,76 (dd) : N-CH₂--CH ; 4,94 (d) : N-CH₂-CH ; 4,31 et 4,58 : N-CH₂-C= ; 7,44 (dd) et 7,72 (dd) et 8,67 (dd) pour les 3H aromatiques.

### Exemple 68

### Sel de sodium de trans-2,5,6,8-tétrahydro-2-méthyl-6-oxo-5-sulfooxy)-4H-4,7-méthanopyrazolo [3,4-e][1,3]diazépine-8-carboxylate de méthyle

### Stade A

On procède comme indiqué au stade A de l'Exemple 62 avec 600 mg (1,83 mmole) du produit obtenu de l'Exemple 61, 80 mg de NaH à 50 % dans l'huile, 174 µl de sulfate diméthylique pour obtenir 72 mg de 2,5,6,8-tétrahydro-2-méthyl-6-oxo-5-(phénylméthoxy)-4*H*-4,7-méthanopyrazole[3,4-*e*][1,3]diazépine-8-carboxylate de méthyle, de formule brute C₁₇H₁₈N₄O₄ (M = 342,36 g).

Le rendement correspondant est de 11,5 %.

### Stade B

On procède comme indiqué au stade A de l'Exemple 11 avec 52 mg (0,155 mmole) de produit obtenu du stade A, 1 ml de mélange CH₃OH / THF : 2/1, 10 mg de Pd/C à 30 %, pour obtenir 37 mg de 2,5,6,8-tétrahydro-5-hydroxy-2-méthyl-6-oxo-4*H*-4,7-méthanopyrazolo[3,4-*e*][1,3]diazépine-8-carboxylate de méthyle, de formule brute C₁₀H₁₂N₄O₄ (M = 252,23 g).

Le rendement correspondant est de 96,6 %.

### Stade C

On introduit 37 mg (0,146 mmole) du composé obtenu au stade B dans 0,75 ml de pyridine, puis on ajoute 70 mg de complexe pyridine -SO₃. On agite une nuit à 20°C. On ajoute 0,15 ml d'eau déminéralisée, puis on agite pendant 10 minutes, on évapore le solvant sous pression réduite. Le produit brut est purifié sur une couche de résine XAD₄ en éluant par H₂O à 5 % d'acétone, on évapore l'acétone des fractions contenant le produit et on lyophilise. La solution de sel de pyridinium est échangée sur résine Dowex 5W8 sous forme Na⁺. Après lyophilisation des fractions sulfate et sel de sodium, on recueille 18 mg de sel de sodium de 2,5,6,8-tétrahydro-2-méthyl-6-oxo-5-(sulfooxy)-4*H*-4,7-méthanopyrazolo[3,4-*e*][1,3]diazépine-8-carboxylate de méthyle, de formule brute C₁₀H₁₁N₄O₇S.Na (M = 354,27 g).

Le rendement correspondant est de 34,8 %.
Spectre RMN du proton dans D20 à 400 MHz, déplacements chimiques et multiplicité :
3,54 (d) et 3,79 (dd) : N-CH₂-CH ; 4,97 (d) : N-CH₂-CH ; 3,88 (s) : N-CH₃ ; 3,89 (s) : O-CH₃ ; 5,43 (s) : N-CH-CO ; 7,73 (s) : N-CH=C.
SM ( electrospray négatif) m/z : (M)⁻ = 331.

### Exemple 69

### Sel de triéthylammonium de trans-2-éthyl-4,5,6,8-tétrahydro-6-oxo-5-(sulfooxy)-4,7-méthano-7H-thiéno[2,3-e][1,3]diazépine-8-carboxylate de méthyle

### Stade A

Dans un ballon de 60 ml muni d'une agitation magnétique, on dissout sous argon, 5,09 g (14,7 mmoles) de 4,5,6,8-tétrahydro-6-oxo-5-(phénylméthoxy)-4,7-méthano-7*H*-thiéno[2,3-*e*][1,3]diazépine-8-carboxylate de méthyle préparé au stade E de l'exemple 57 dans 100 ml CCl₄, on refroidit au bain de glace, puis on ajoute 1,87 g (7,4 mmoles) d'iode, 3,813 g de PhI(OCOCF₃)₂. On laisse revenir à 20°C. Après 2 à 3 h d'agitation à 20°C, on verse la solution dans une solution aqueuse 0,5N de thiosulfate de sodium, on extrait à l'acétate d'éthyle, puis on lave de nouveau avec une solution aqueuse de thiosulfate de sodium à 0,5N, on lave avec une solution aqueuse saturée de chlorure de sodium, puis avec une solution de tampon phosphate, pH = 7,0, et enfin avec une solution aqueuse saturée de chlorure de sodium. Après séchage sur MgSO₄ et évaporation du solvant sous pression réduite, on obtient le produit brut qui est purifié par chromatographie sur silice en éluant avec un mélange CH₂Cl₂/acétate d'éthyle (95/5), TEA = 0,1 %.

On obtient 4,88 g de 4,5,6,8-tétrahydro-2-iodo-6-oxo-5-(phénylméthoxy)-4,7-méthano-7*H*-thiéno[2,3-*e*][1,3]diazépine-8-carboxylate de méthyle, de formule brute C₁₇H₁₅N₂IO₄S (M = 470,289 g).

Le rendement est de 70%.

### Stade B

Dans un ballon placé sous athmosphère d'argon, on dissout 300 mg (0,6379 mmole) de produit obtenu du stade A, 12 ml de DMF préalablement dégazés à l'argon, puis on ajoute 372,5 µl (1,2758 mmole) de vinyl-tributylstanane, puis 36,7 mg (0,0637 mmole) de chlorure de -bis-1,2-diphénylphosphino éthane palladium. Après agitation de la solution pendant 17 heures, on élimine le solvant par évaporation, on dilue avec 60 ml d'acétate d'éthyle, on ajoute 60 ml d'une solution aqueuse de KF et on agite énergiquement pendant 30 minutes, filtre, et on réextrait la phase aqueuse à l'acétate d'éthyle, enfin on lave les extraits organiques avec une solution aqueuse saturée de chlorure de sodium. Après séchage sur MgSO₄ et évaporation du solvant sous pression réduite et purification par chromatographie sur silice, en éluant initialement avec le mélange CH₂Cl₂ à 0,1 % de TEA et ensuite avec le mélange CH₂Cl₂/acétate d'éthyle (95/5), TEA à 0,1 %, on obtient 208,7 mg de 2-éthényl-4,5,6,8-tétrahydro-6-oxo-5-(phénylméthoxy)-4,7-méthano-7*H*-thiéno[2,3-*e*][1,3]diazépine-8-carboxylate de méthyle, de formule brute, C₁₉H₁₈N₂O₄S (M = 370,43 g).

### Stade C

Dans un ballon de 30 ml, on dissout 51,1 mg (0,1379 mmole) du produit obtenu au stade B, dans 5 ml de méthanol, puis on ajoute 51,1 mg de palladium à 30 % sur charbon actif, on place sous athmosphère d'hydrogène pendant 20 heures. Après dilution de la suspension réactionnelle par ajout de 5 ml de CH₂Cl₂, on agite 10 minutes, puis on filtre. On extrait avec le mélange CH₂Cl₂/méthanol (1/1) puis on évapore à sec sous vide. On obtient 37,1 mg de 2-éthyl-4,5,6,8-tétrahydro-5-hydroxy-6-oxo-4,7-méthano-7H thiéno[2,3-e][1,3]diazépine-8-carboxylate de méthyle, de formule brute C₁₂H₁₄N₂O₄S (M = 282,321 g).

Le rendement correspondant est de 95,2 %.

### Stade D

### Etape 1

On procède comme indiqué au stade C de l'Exemple 62 avec 37,1 mg (0,1314 mmole) du produit obtenu à partir du stade C, 0,85 ml de pyridine, et 62,7 mg (0,3942 mmole) de complexe SO₃-pyridine.

On obtient 7,3 mg de sel de triéthylammonium de 2-éthyl-4,5,6,8-tétrahydro-6-oxo-5-(sulfooxy)-4,7-méthano-7H-thiéno[2,3-e][1,3]diazépine-8-carboxylate de 8-méthyle, de formule brute C₁₈H₂₉O₇N₃S₂ (M = 463,57 g).
SM (electrospray négatif) m/z : (M⁻) = 361

### Exemple 70

### Sel de triéthylammonium de trans-4,5,6,8-tétrahydro-2-méthyl-6-oxo-5-(sulfooxy)-4,7-méthano-7H-thiéno[2,3-e][1,3]diazépine-8-carboxylate de méthyle

### Stade A

Dans un ballon placé sous atmosphère d'argon, on dissout 330mg (0,70 mmole) de produit obtenu à l'étape A de l'Exemple 69 dans 12 ml de DMF préalablement dégazés à l'argon, puis on ajoute 972 µl de tétraméthylétain (7 mmoles), puis on ajoute 49,5 mg de chlorure de bis-(triphénylphosphéno)palladium (0,07 mmole). On porte le mélange à 75°C, pendant 6 heures, puis on évapore le solvant sous pression réduite.On dilue l'extrait sec avec 10 ml d'acétate d'éthyle, on ajoute 10 ml d'une solution aqueuse saturée de KF et on agite énergiquement pendant 30 minutes. Après filtration, on réextrait la phase aqueuse à l'acétate d'éthyle, on lave les extraits organiques dans une solution aqueuse saturée de chlorure de sodium, on sèche sur MgSO₄ et on évapore sous vide. Après chromatographie sur colonne de silice en éluant avec le mélange CH₂Cl₂/ acétate d'éthyle (95/5), TEA = 0,1%, on obtient 40,8 mg de 4,5,6,8-tétradhydro-2-méthyl-6-oxo-5-(phénylméthoxy)-4,7-méthano-7*H*-thiéno[2,3-*e*][1,3]diazépine-8-carboxylate de méthyle, de formule brute C₁₈H₁₈N₂O₄S (M = 358,419 g).

Le rendement correspondant est de 16,2 %.

### Stade B

On procède comme indiqué dans le stade A de l'Exemple 11 avec 52,2 mg (0,1456 mmole), 5 ml de méthanol, 52,2 mg de produit obtenu de l'étape A.

On obtient 34,2 mg de 4,5,6,8-tétrahydro-5-hydroxy-2-méthyl-6-oxo-4,7-méthano-7*H*-thiéno [2,3-*e*][1,3]diazépine-8-carboxylate de méthyle, de formule brute C₁₁H₁₂N₂O₄S (M = 268,294 g).

Le rendement correspondant est de 87,5 %.

### Stade C

On procède comme indiqué dans le stade B de l'Exemple 62 avec 34,2 mg de produit du stade B, 0,8 ml de pyridine conservé sur tamis, 60,9 mg de SO₃-pyridine complexe (0,3824 mmole). Après purification sur colonne de silice, en utilisant comme éluant un mélange CH₂Cl₂/méthanol (80/20), TEA = 0,5 %, on obtient 8,5 mg de sel de triéthylammonium de trans-4,5,6,8-tétrahydro-2-méthyl-6-oxo-5-(sulfooxy)-4,7-méthano-7*H*-thiéno[2,3-*e*][1,3]diazépine-8-carboxylate de méthyle, de formule brute C₁₇H₂₇O₇N₃S₂ (449,55 g).

Le rendement correspondant est de 14,5 %.
SM ( électrospray négatif) m/z : (M)⁻ = 347

### Exemple 71

### Trans-4,5,6,8-tétrahydro-6-oxo-5-[(phénylsulfonyl)oxy]-4,7-méthano-7H-thiéno[2,3-e]-[1,3]diazépine-8-carboxamide

### Stade A

On procède comme indiqué dans le stade A de l'Exemple 11 avec 40 mg (0,12 mmole) de 4,5,6,8-tétrahydro-6-oxo-5-(phénylméthoxy)-4,7-méthano-7*H-*thiéno[2,3-*e*][1,3]diazépine-8-carboxamide obtenu suivant le stade A de l'exemple 60 en remplaçant NH₂Me,HCl par NH₄CL, 2,1 ml d'éthanol, 40 mg de palladium à 30 % de charbon actif.

On obtient 30 mg du produit mentionné ci-dessus.

Le rendement correspondant est de 46%.

### Stade B

Le produit obtenu au stade A est dissout dans 1 ml de pyridine avec 15 µl de PhSO₂Cl à 20°C pendant 1 heure. On dilue avec CH₂Cl₂ et avec uns solution aqueuse d'acide tartrique à 10 %. Puis après évaporation du solvant sous pression réduite, on procède à une purification par chromatographie sur silice en éluant par CH₂Cl₂/acétate d'éthyle à 20 %.

On obtient 20,9 mg de trans-4,5,6,8-tétrahydro-6-oxo-5-[(phénylsulfonyl)oxy]-4,7-méthano-7*H*-thiéno[2,3-*e*][1,3]diazépine-8-carboxamide, de formule brute C₁₅H₁₃N₃O₅S₂ (M = 371,42 g).

Le rendement correspondant est de 46 %.

### Spectre RMN du proton

Dans le DMSO, à 300 MHz, déplacements chimiques et multiplicité :
3,47 (dd) et 3,53 (d) : N-CH₂-CH ; 4,45 (d) : CH₂-CH; 5,04 (s) : N-CH-CO-; 7,61 (sl) et 7,84 (sl): CO-NH₂; 7,01 (d) et 7,53 (d) : les 2 hydrogènes du thiophène; 7,74 (dl), 7,88 (tl) et 8,03 (dl) pour les 5 hydrogènes aromatiques.
SM (électrospray positif) m/z : [2M+H]⁺= 759[M+H]+ = 380.

### Exemple 72

### Sel de sodium de N-[5,6,7,9-tétrahydro-7-oxo-6-(sulfooxy)-5,8-méthano-8H-pyrimido-[4,5-e][1,3]diazépin-2-yl]-acétamide

### Stade A

On porte à reflux pendant 1h30 un mélange réactionnel comprenant 5 g (18,6 mmoles) de composé préparé au stade A de l'exemple 5 en présence de 4,9 g d'acétyl guanidine et 150 ml de toluène. Puis on concentre sous pression réduite et on purifie le résidu obtenu par chromatographie sur silice en éluant avec mélange dichlorométhane/méthanol (95/5). O obtient ainsi 11% de produit attendu et 63% de produit déacétyle. 3,07 g de produit désacétyle intermédiaire obtenu sont repris dans 2 ml d'anhydride acétique, plongé dans un bain d'huile 130° ou 140°C pendant 20 minutes puis refroidi et concentré sous pression réduite. Le résidu est repris par une solution de bicarbonate de sodium, extrait au dichlorométhane, séché.sur Na₂SO₄ On évapore le solvant sous pression réduite puis on purifie par chromatographie. On obtient ainsi au total 3,49 g de 2-(acétylamino)-5,8-dihydro-5-oxo-pyrido[3,4-*d*]pyrimidine-7(6*H*)-carboxylate de 1,1-diméthyléthyle, de formule brute C₁₄H₁₈N₄O₄ (M = 306,32 g).

### Le rendement global est de 61 %.

### Stade B

On refroidit à 0°C sous atmosphère inerte une solution contenant 0,65 g du produit obtenu au stade précédent A dans 15 ml d'éthanol. On introduit lentement 0,088 g de borohydrure de sodium puis on agite pendant une demi-heure à 0°C. On verse la solution sur une solution glacée saturée par du phosphate monosodique puis on extrait au dichlorométhane. Après séchage et évaporation des solvants sous pression réduite, on obtient 0,546 g de 2-(acétylamino)-5,8-dihydro-5-hydroxy-pyrido[3,4-*d*]pyridine-7(6*H*)-carboxylate de 1,1-diméthyléthyle, de formule brute C₁₄H₂₀N₄O₄ (M = 308, 34 g).

Le rendement correspondant est de 84 %.

### Stade C

On refroidit à -20°C sous atmosphère inerte une solution contenant 1,54 g de l'alcool obtenu au stade précédent B, 15 ml de dichlorométhane et 0,86 ml de triéthylamine. On introduit ensuite lentement 0,42 ml de chlorure de mésyle. On laisse revenir à 0°C puis on ajoute 15 ml de O-allyl-hydroxylamine. On agite encore le mélange pendant 4 jours à 0°C. On évapore les solvants sous pression réduite, puis le résidu est purifié par chromatographie sur silice en éluant avec un mélange dichlorométhane/méthanol (95/5).

On obtient 0,870 g de 2-(acétylamino)-5,8-dihydro-5-[(2-propényloxy)-amino]-pyrido[3,4-*d*]pyrimidine-7(6*H*)-carboxylate de 1,1-diméthyléthyle, de formule brute C₁₇H₂₅N₅O₄.C₃H₇NO (M = 363,42 g, 73,10 g).

Le rendement correspondant est de 50 %.

### Stade D

On dissout 1,368 g (3,76 mmoles) du composé obtenu au stade précédent C dans 5 ml d'acétate d'éthyle. On introduit en 5 minutes à la solution précédente 10 ml d'acétate d'éthyle saturé par HCl gazeux. On agite pendant une demi-heure puis on concentre à faible volume avant d'ajouter 5 ml d'eau, 2 ml d'ammoniac puis on sature la solution par NaCl et on extrait au dichlorométhane.

On obtient 0,991 g de résidu que l'on purifie par chromatographie sur silice en éluant par un mélange dichlorométhane/méthanol/ammoniac (90/10/1).

On obtient 0,313 g de *N*-[5,6,7,8-tétrahydro-5-[(2-propényloxy)amino]pyrido [3,4-d]pyrimidin-2-yl]-acétamide, de formule brute C₁₂H₁₇N₅O₂ (M = 263,30 g).

Le rendement obtenu correspondant est de 32 %.

### Stade E

On dissout 0,413 g (1,58 mmole) du composé obtenu au stade précédent D dans 14 ml d'acétonitrile. On refroidit à 0°C et on place sous atmosphère inerte avant d'additionner 0,07 ml de diphosgène. On laisse revenir à température ambiante et on introduit 0,33 ml de triéthylamine. Puis on agite pendant 4 heures. On élue de l'acétate d'éthyle et on lave à l'eau Après séchage de la phase organique et évaporation sous pression réduite du solvant, on purifie le résidu par chromatographie sur silice en éluant par un mélange dichlorométhane/méthanol (90/10).

On obtient 0,127 g *N*-[5,6,7,9-tétrahydro-7-oxo-6-(2-propényloxy)-5,8-méthano-8*H*-pyrimido[4,5-*e*][1,3]diazépin-2-yl]-acétamide, de formule brute C₁₃H₁₅N₅O₃ (M = 289,30 g).

Le rendement correspondant est de 28 %.

### Stade F

On place sous atmosphère inerte 0,12 g du produit obtenu au stade précédent E, 1,4 ml de dichlorométhane, 0,05 ml d'acide acétique, 0,240 g de tétrakis-(triphénylphosphine)-palladium. Après 30 minutes de réaction, on introduit 1 ml de pyridine et 0,196 g du complexe SO₃-pyridine. On poursuit l'agitation pendant 20 heures puis on hydrolyse par de la glace et On évapore ensuite les solvants sous pression réduite On obtient 630 mg de produit brut que l'on purifie par chromatographie sur silice en éluant avec un mélange de dichlorométhane/acétone/triéthylamine (60/40/1). On récupère 68 mg du produit attendu avec un rendement de 26 %.Ce produit est ensuite transformé en sel de sodium par passage sur résine Dowex 50WX8 sous forme Na+. On recueille 28 mg du sel de sodium de N-[5,6,7,9-tétra-hydro-7-oxo-6-(sulfooxy)-5,8-méthano-8*H*-pyrimido[4,5-*e*][1,3]dlazépin-2-yl]-acétamide, de formule brute C₁₀H₁₀N₅O₆S.Na (M = 328,29 g, 22,99 g).

Le rendement correspondant est de 76 %.

### RMN du proton

Dans DMSO-d₆ à 300 MHz, déplacements chimiques et multiplicité :
2,16 (sl) : CH₃-CO-NH ; 3,40 (d) et 3,60 (dd) : N-CH₂-CH ; 4,04 et 4,40 : N-CH₂=C ; 4,79 (d) : N-CH₂-CH ; 8,34 (s) : N-CH₂=C; 10,63 : CH₃-CO-NH.
SM (électrospray négatif) m/z : M⁻ = 328.

### Exemple 73

### Sel de triéthylammonium de trans-2-bromo-4,5,6,8-tétrahydro-6-oxo-5-(sulfooxy)-4,7-méthano-7H thiéno[2,3-e][1,3]diazépine-8-carboxamide

Dans un ballon de 5 ml sous agitation magnétique, on mélange 21,4 mg (6,27 10⁻² mmoles) de produit obtenu à partir du stade C de l'Exemple 25, dans 1 ml d'H₂O, on ajoute 6 mg de NaHCO₃, puis 1 équivalent de Br₂ en solution dans 0,1 ml de CH₂Cl₂. Après 2 heures, on évapore le solvant sous pression réduite puis on lyophilise, on extrait le sel de sodium à deux reprises par 12 ml d'acétone et on évapore sous pression réduite. Le résidu est dissous dans 1 ml H₂O et purifié sur plaque préparative en éluant avec CH₂Cl₂/acétone 2/8 à 0,1% de TEA. On obtient 16 mg de sel de triéthylammonium de trans-2-bromo-4,5,6,8-tétrahydro-6-oxo-5-(sulfooxy)-4,7-méthano-7*H*-thiéno[2,3-*e*][1,3]diazépine-8-carboxamide, de formule brute C₉H₇BrN₃O₆S₂.Na (M = 420,20 g).

Le rendement correspondant est de 50 %.

### Spectre RMN du proton

Dans le D₂O, à 300 MHz , déplacements chimiques et multiplicité:
1,29 (t) : CH₃-CH₂; 3,21 (q) : CH₃-CH₂; 3,48 (d) et 3,79 (dd) : N-CH₂-CH; 5,29 (s) : CH-CO-NH₂; 7,20 (s) : HC=CBr.
SM (électrospray positif) m/z : [M]⁺ = 102
SM (électrospray négatif) m/z : [M]⁻= 396.

### Exemple 74

### Sel de triéthylammonium de trans-2,5,6,8-tétrahydro-6-oxo-5-(sulfooxy)-4H-4,7-méthanopyrazolo[3,4-e][1,3]diazépine-8-carboxylate de méthyle

### Stade A

Dans un ballon placé sous athmosphère d' argon à 0°C, on mélange 0,157 g (0,478 mmole) de composé préparé à l'exemple 61 -, 0,8 ml de CH₂Cl₂, 0,101 g (0,717 mmole) de C₆H₅COCl, et 0,073 g (0,717 mmole) de TEA. On laisse la température revenir à 20°C, puis après une heure, on évapore le mélange réactionnel sec sous pression réduite. On reprend le résidu de l'acétate d'éthyle, on lave avec H₂O puis on sèche la phase organique sur du MgSO₄, on filtre et on évapore les solvants sous pression réduite. Après purification par chromatographie sur silice, en éluant avec un mélange CH₂Cl₂/acétone 98/2 à 0,1 % de TEA , on obtient 58,4 mg de 2-benzoyl-2,5,6,8-tétrahydro-6-oxo-5-(phénylméthoxy)-4*H*-4,7-méthanopyrazolo[3,4-*e*][1,3]diazépine-8-carboxylate de méthyle, de formule brute C₂₃H₂₀N₄O₅ (M = 433,4 g).

Le rendement correspondant est de 60 %.

### Stade B

On procède comme indiqué au stade A de l'Exemple 11 avec 55 mg (0,127 mmole) du produit obtenu au stade A, 17 mg de palladium à 30 % de charbon actif, et 2 ml de THF.

On obtient 42 mg de 2-benzoyl-2,5,6,8-tétrahydro-5-hydroxy-6-oxo-4*H*-4,7-méthanopyrazolo[3,4-*e*][1,3]diazépine-8-carboxylate de méthyle, de formule chimique C₁₆H₁₄NaO₅ (M = 342,31 g).

Le rendement correspondant est de 97 %.

### Stade C

On procède comme indiqué au stade B de l'Exemple 62 avec 42 mg (0,123 mmole) du produit obtenu du stade B, 59 mg (0,368 mmole) de complexe pyridine/SO₃ et 2 ml de pyridine. Après purification par chromatographie sur colonne de silice, en éluant avec un mélange CH₂Cl₂/méthanol 85/15)à 0, 1 % de TEA on obtient 16,6 mg de sel de triéthylammonium de trans-2,5,6,8-tétrahydro-6-oxo-5-(sulfooxy)-4*H*-4,7-méthanopyrazolo[3,4-e][1,3]diazépine-8-carboxylate de méthyle, de formule chimique C₁₆H₁₃N₄O₃S.C₆H₁₆N. (M = 421,37, 102,20)

Le rendement correspondant est de 31 %.
LC/MS : voir conditions générales exemple 62
Résultats : TR = 3,71 min. : m/z : [2M+Na]⁻ = 657 , [M]⁻ = 317

### Exemple 75

### Sel de sodium de 1,4,5,8-tétrahydro-5-(sulfooxy)-6H-4,7-méthanopyrazolo[3,4-e][1,3]diazépin-6-one

### Stade A

6 g (19,33 mmoles) du produit obtenu au stade H de l'Exemple 17 sont mis en solution dans 180 ml de THF, 180 ml de tert-butanol et 60 ml d'eau. On introduit 3,92 g (29 mmoles) de N-oxyde de N-méthyle-morpholine puis 2,98 ml (0,579 mmole) de tétraoxyde d'osmium. On agite 54 heures à température ambiante. Après évaporation du THF, le milieu est repris avec une solution aqueuse 1M de NaH₂PO₄. On extrait avec un mélange acétate d'éthyle/heptane à 20 % puis au dichloro-méthane/chlorure de méthylène et THF. Après séchage de la phase organique sur MgSO₄ puis évaporation des solvants sous pression réduite, on obtient 6,16 g de 1-(2,3-dihydroxypropyl)-1,4,5,8-tétrahydro-5-(phénylméthoxy)-6H-4,7-méthanopyrazolo[3,4-*e*][1,3]diazépin-6-one, de formule brute C₁₇H₂₀N₄O₄ (M = 344,37 g).

Le rendement correspondant obtenu est de 93 %.

### Stade B

6,13 g (17,8 mmoles) du produit obtenu au stade précédent A est dissous dans 140 ml de THF. Puis on ajoute 45 ml de méthanol suivi de 45 ml d'eau. La solution obtenue est refroidie à 0 °C. Puis on ajoute 6,08 g de métapériodate de sodium. On agite pendant 2 heures en laissant remonter la température à 20°C. Au bout de 2 heures, on rajoute 1,52g de métapériodate de sodium et on agite à nouveau pendant 40 minutes. Une fois la réaction terminée, on ajoute 260 ml d'une solution aqueuse de NaH₂PO₄ 1M, puis on sature la solution avec du NaCl solide et on extrait au THF et avec un mélange acétate d'éthyle/heptane à 30 %. La phase organique est lavée avec une solution aqueuse de NaH₂PO₄ saturée puis séchée sur MgSO₄. Après évaporation du solvant sous pression réduite on obtient 9,98 g de 4,5,6,8-tétrahydro-6-oxo-5-(phénylméthoxy)-1H-4,7-méthanopyrazolo[3,4-e] [1,3]diazépine-1-acétaldéhyde, de formule brute C₁₆H₁₆N₄O₄ (M = 342,39 g).

Le rendement obtenu est quantitatif.

### Stade C

On dissout 5,6 g du produit obtenu au stade précédent A dans 100 ml d'éthanol puis on ajoute à 0°C 2,71 g de NaBH₄ par portions. On agite 2 heures à 0°C puis on évapore l'éthanol, on rajoute de la glace, du chlorure de méthylène et petit à petit une solution aqueuse 1M de NaH₂PO₄. Le dégagement gazeux est important. Puis on extrait la phase aqueuse avec du chlorure de méthylène et on procède à un lavage de la phase organique avec une solution de thiosulfate pour éliminer les résidus de NaIO₄. Après séchage de la phase organique sur MgSO₄, on évapore les solvants sous pression réduite.

On obtient un résidu solide qui est cristallisé dans un mélange d'éther éthylique et d'isopropanol. Après filtration, on obtient 3,45 g de 1,4,5,8-tétrahydro-1-(2-hydroxyéthyl)-5-(phénylméthoxy)-6H-4,7-méthano-pyrazolo[3,4-*e*][1,3]diazépin-6-one, de formule brute C₁₆H₁₈N₄O₃ (M = 314,35 g.

Le rendement correspondant est de 62 %.

### Stade D

On dissout 1,35 g (4,29 mmoles) du produit obtenu au stade précédent C dans 50 ml de THF. Puis on ajoute à température ambiante, 0,69 ml de pyridine, suivi de 1,46 g de triphénylphosphine. On ajoute 1,42 g d'iode par portions puis après 2 heures, on rajoute 200 mg d'iode, 220 mg de triphénylphosphine et 0,13ml de pyridine. On verse dans le milieu par une solution de NaH₂PO₄ puis on extrait par un mélange acétate d'éthyle/heptane et on lave la phase organique avec une solution aqueuse saturée de N_{A}Cl. Après évaporation des solvants sous pression réduite de la phase organique, on obtient 1,6 g de produit brut qui est purifié par chromatographie liquide en éluant avec un mélange dichlorométhane/acétonitrile à 10 %. On obtient 1,60 g du produit 1,4,5,8-tétrahydro-1-(2-iodoéthyl)-5-(phénylméthoxy)-6H-4,7-méthanopyrazolo[3,4-*e*][1,3]diazépin-6-one, de formule brute C₁₇H₁₇IN₄O₂ (M = 424,24 g).

### Le rendement obtenu est de 80 %.

### Stade E

On dissout 1,6 g (3,77 mmoles) du produit obtenu au stade précédent D dans 16 ml de DMF anhydre. On ajoute 260 mg de cyanure de potassium et on agite à température ambiante pendant 20 heures. On lave le milieu réactionnel à l'eau puis on extrait par un mélange acétate d'éthyle/heptane à 20 %. On sèche la phase organique sur sulfate de magnésium puis après évaporation des solvants sous pression réduite, on obtient un résidu brut qui est purifié par chromatographie sur silice en éluant avec tout d'abord du dichlorométhane puis un mélange dichlorométhane/méthanol à 10 %. Après évaporation des fractions contenant le produit attendu, on obtient 1,20 g de 4,5,6,8-tétrahydro-6-oxo-5-(phénylméthoxy)-1*H*-4,7-méthanopyrazolo[3,4-*e*][1,3]diazépine-1-propanenitrile, de formule brute C₁₇H₁₇N₅O₂ (M = 323,36 g).

Le rendement correspondant est de 98 %.

### Stade F

On dissout 500 mg (1,546 mmole) du produit obtenu au stade précédent E dans 5 ml de DMF. On refroidit la solution à 0°C puis on ajoute 68 mg d'hydrure de sodium. On agite pendant 3 heures à 0°C. Puis on verse le milieu réactionnel sur une solution aqueuse de NaH₂PO₄ et on extrait avec un mélange d'acétate d'éthyle/heptane. La phase organique est séchée sur sulfate de magnésium puis les solvants sont évaporés sous pression réduite. 448 mg de produit brut obtenu sont purifiés sur silice en éluant avec un mélange chlorure de méthylène/méthanol à 10 %. On obtient 177 mg de 1,4,5,8-tétrahydro-5-(phénylméthoxy)-6*H*-4,7-méthanopyrazolo[3,4-*e*][1,3]diazépin-6-one, de formule brute C₁₄H₁₄N₄O₂ (M = 270,29g).

Le rendement correspondant est de 42 %.

### Stade G

On dissout 163 mg du produit obtenu au stade précédent F dans 8 ml d'éthanol en présence d'une goutte d'acide acétique. On introduit ensuite 400 mg de palladium sur charbon à 10 % en poids puis on maintient le milieu sous atmosphère d'hydrogène pendant 20 minutes. Une fois la réaction terminée, on élimine le catalyseur par filtration puis on évapore le THF sous pression réduite en présence de toluène pour entraîner de façon azéotropique l'acide acétique restant. On obtient 92,8 mg sous forme de poudre blanche de 1,4,5,8-tétrahydro-5-hydroxy-6H-4,7-méthanopyrazolo[3,4-*e*][1,3]diazépin-6-one, de formule brute C₇H₈N₄O₂ (M = 180,17 g).

Le rendement correspondant est de 85 %.

### Stade H

On dissout 92 mg du produit obtenu au stade précédent G dans 2 ml de pyridine. Puis on ajoute 325 mg du complexe pyridine-SO₃. Après une nuit à 20°C, on évapore la pyridine avec un entraînement au toluène. Le produit est ensuite repris dans l'eau puis passé sur résine Dowex 50WX8 sous forme Na⁺. Après évaporation des fractions contenant le produit attendu, on reprend le résidu dans le méthanol puis après filtration et évaporation du solvant sur pression réduite, on obtient un résidu qui une fois concrétisé dans l'éther donne 140 mg de sel de sodium de 1,4,5,8-tétra-hydro-5-(sulfooxy)-6*H*-4,7-méthanopyrazolo[3,4-*e*][1,3]diazépin-6-one, de formule brute C₇H₇N₄O₅S.Na (M = 282,21 g).

Le rendement correspondant est de 97,3 %.

### Spectre RMN du proton

Dans DMSO-d₆, déplacements chimiques et multiplicité :
3,14 (d) et 3,50 (dd) : N-CH₂-CH; 4,71 (d) : N-CH₂-CH; 4,22 (m) : N-CH₂C= ; 7,68 (s1) : N=CH ; 12,64 (s1) H mobile
SM (électrospray négatif) m/z: [2M+Na]⁻ =541 ; [2M+H]⁻ = 519 ; [M+MeOH]⁻ = 291 ; [M]⁻ = 259

### ETUDE PHARMACOLOGIQUE DES PRODUITS DE L'INVENTION

### Activité in vitro, méthode des dilutions en milieu liquide

On prépare une série de tubes dans lesquels on répartit la même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits de l'invention suivants :
- le sel de sodium de trans-3-oxo-4-(sulfooxy)-2,3,4,5-tétrahydro-2,5-méthano-1H-2,4-benzodiazépine-1-carboxamide ;
- le sel de sodium de 3-méthyl-5-(sulfooxy)-5,6,7,8-tétrahydro-4,7-méthano-4H-[1,2,3]-triazolo[4,5-e][1,3]diazépine-6(3H)-one ;
- le sel de sodium de trans-1-méthyl-6-oxo-5-(sulfooxy)-4,5,6,8-tétrahydro-4,7-méthano-1H-pyrazolo[3,4-e][1,3]diazépine-8(7H)-carboxamide ;
- le sel de sodium de trans-N-méthyl-3-oxo-4-(sulfooxy)-2,3,4,5-tétrahydro-2,5-méthano-1H-2,4-benzodiazépine-1-carboxamide ;
- le sel de sodium de 5-(sulfooxy)-5,6,7,8-tétrahydro-4,7-méthano-4H-furo[2,3-e][1,3]diazépine-6-one ;
- le sel de pyridinium de 1 -propyl-5-(sulfooxy)-4,5,7,8-tétrahydro-4,7-méthano-imidazo[4,5-e][1,3]diazépine-6(1H)-one;
- le sel de sodium de trans-6-oxo-5-(sulfooxy)-5,6,7,8-tétrahydro-4,7-méthano-4H-furo[2,3-e][1,3]diazépine-8-carboxylate de méthyle ;
- le sel de sodium de 1-méthyl-5-(sulfooxy)-5,6,7,8-tétrahydro-4,7-méthano-4H-pyrazolo[3,4-e][1,3]diazépine-6(1H)-one ;
- le sel de sodium de trans-3-oxo-N-(4-pyridinylméthyl)-4-(sulfooxy)-2,3,4,5-tétrahydro-2,5-méthano-1 H-2,4-benzodiazépine-1-carboxamide ;
- le sel de sodium de trans-6-oxo-5-(sulfooxy)-5,6,7,8-tétrahydro-4,7-méthano-4H-thiéno[2,3-e][1,3]diazépine-8-carboxamide ;
- le sel de sodium de trans-6-oxo-5-(sulfooxy)-5,6,7,8-tétrahydro-4,7-méthano-4H-furo[2,3-e][1,3]diazépine-8-carboxamide.

Ces composés ont les activités regroupées dans le tableau suivant :

| **Gram-positif CMI µg/ml à 24 heures** | |
|---|---|
| S. aureus SG511 | 1,25 - 40 |
| S. aureus Exp54146 | 1,25 - 40 |
| S. pyogenes A561 | < 0,08 - 20 |

| **Gram-négatif CMI µg/ml à 24 heures** | |
|---|---|
| E. coli UC 1894 | < 0,15 - 10 |
| E. coli 250HT7 | < 0,15-20 |
| E. cloacae 1321E | < 0,15 - 40 |

Les composés selon l'invention montrent donc une activité anti-bactérienne.

### Exemple 76

Dans cet exemple, on a préparé une composition pharmaceutique pour injection.

Cette composition pharmaceutique renfermait :
- le composé de l'exemple 9 : 500 mg
- un excipient aqueux stérile: q.s.p. 10 ml.

## Revendications

1. Composé de formule générale (III) ou l'un de ses sels avec un acide, notamment son chlorhydrate et non trifluoroacétate: 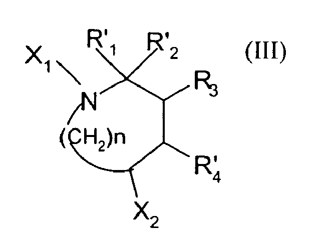 dans laquelle :
a) ou bien R'₁ représente un atome d'hydrogène, un radical CN, COOH protégé, COOR", (CH₂)_{n'} R'₅, CONR₆R₇ ou 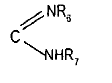 R" est choisi dans le groupe constitué par un radical alkyle renfermant de 1 à 6 atomes de carbone, éventuellement substitué par un radical pyridyle, un radical - CH₂-alkényle renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical NO₂, OH protégé, NH₂ protégé, alkyle renfermant de 1 à 6 atomes de carbone, alkoxy renfermant de 1 à 6 atomes de carbone ou par un ou plusieurs atomes d'halogène,
R'₅ est choisi dans le groupe constitué par un radical OH protégé, CN, NH₂ protégé, CO-NR₆R₇, COOH protégé, COOR", OR", OCOH, OCOR", OCOOR", OCONH₂, OCONHR", NHR" protégé, NHCOR", NHSO₂R", NH-COOR", NH-CONHR" ou NH-CONH₂, R" étant défini comme ci-dessus,
R₆ et R₇, identiques ou différents, sont choisis dans le groupe constitué par un atome d'hydrogène, un radical alkyle renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
n' est égal à 1 ou 2,
R₃ et R'₄ forment ensemble un phényl ou un hétérocycle à caractère aromatique à 5 ou 6 sommets renfermant de 1 à 4 héréroatomes choisis parmi l'azote, l'oxygène et le soufre, et éventuellement substitué par un ou plusieurs groupements R', R' étant choisi dans le groupe constitué par un atome d'hydrogène et les radicaux alkyles renfermant de 1 à 6 atomes de carbone éventuellement substitués par un ou plusieurs radicaux hydroxy, oxo, halogène, ou cyano ou par un radical nitro, alkényle renfermant de 2 à 6 atomes de carbone, halogèno, amino, OH, OH protégé, -OR, - NHCOH, -NHCOR, NHCOOR, COOH, -COOR, -C(C₆H₅)₃ et -CH₂-CH₂-S(O)ₘ-R, R étant tel que défini précédemment et m étant égal à 0, 1 ou 2,
b) ou bien R'₄ représente un atome d'hydrogène ou un groupement (CH₂)n'₁R₅, n'1 étant égal à 0, 1 ou 2 et R₅ étant tel que défini ci-dessus,
et R'₁ et R₃ forment ensemble un phényl ou un hétérocycle éventuellement substitué, tel que défini ci-dessus,
dans les deux cas a) et b)
R'₂ est choisi dans le groupe constitué par un atome d'hydrogène, un atome d'halogène et les radicaux R", S(O)ₘR", OR", NHCOH, NHCOR", NHCOOR" et NHSO₂R", R" et m étant tel que défini précédemment,
n est égal à 1 ou 2,
et soit X₁ est un atome d'hydrogène et X₂ représente un groupement -Z-CO-X₃, X₃ représentant le reste de l'agent de carbonylation, soit X₂ est un groupement - ZH et X₁ représente un groupement CO-X₃, X₃ étant défini comme ci-dessus ;
à l'exclusion du composé dans lequel n=l, R'₄ représente un atome d'hydrogène, R'₁ et R₃ forment ensemble un phényle, X₁ représente CO-X₃ avec X₃=O-tBu et X₂ représente HNR'₈-(CH₂)ₙ"- avec R'₈=CH₃ et n"=0.

2. Composé de formule (III) telle que définie à la revendication 1, dans laquelle lorsque R'₄ représente un atome d'hydrogène et n=1, R'₁ et R₃ forment ensemble un hétérocycle éventuellement substitué tel que défini pour R₃ et R₄, ainsi que leurs sels avec les acides et notamment leurs chlorhydrates et trifluoroacétates.

3. Composé de formule (II) ou l'un de ses sels avec un acide, notamment son chlorhydrate et son trifluoroacétate: 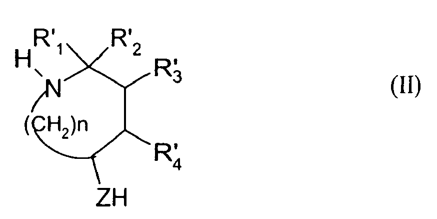 dans laquelle R'₁, R'₂, R₃, R'₄ et n ont les mêmes significations que dans la revendication 1,
ZH représente un groupement HO-(CH₂)ₙ", HNR'₈-(CH₂)ₙ"- ou HNR'₈-O-, n" est égal à 0 ou 1 et R'₈ représente un atome d'hydrogène, un radical R", OH protégé, OR", Y', OY', Y'₁, OY'₁, Y'₂, OY'₂, Y'₃, O-CH₂-CH₂-S(O)ₘ-R", SiRaRbRc et OSiRaRbRc, Ra, Rb et Rc représentant individuellement un radical alkyle linéaire ou ramifié renfermant de 1 à 6 atomes de carbone ou un radical aryle renfermant de 6 à 10 atomes de carbone et R" et m étant défini comme à la revendication 1 ;
Y' est choisi dans le groupe constitué par les radicaux COH, COR", COOR", CONH₂, CONHR", CONHSO₂R", CH₂COOR", CH₂tétrazole protégé, CH₂SO₂R", CH₂PO(OR")₂, CONHOH protégé, CH₂COOH protégé, CH₂CONHOH protégé, CH₂SO₃ protégé, CH₂PO(OR)(OH) protégé, CH₂PO(R)(OH) protégé et CH₂PO(OH)₂ protégé,
Y'₁ est choisi dans le groupe constitué par les radicaux SO₂R", SO₂NHCOH, SO₂NHCOR", SO₂NHCOOR", SO₂NHCONH₂ SO₂NHCONHR" et SO₃H protégé,
Y'₂ est choisi dans le groupe constitué par les radicaux PO(OR")₂, PO(OH)₂ protégé, PO(OH)(OR) protégé et PO(OH)(R) protégé,
Y'₃ est choisi dans le groupe constitué par les radicaux tétrazole protégé, tétrazole substitué par le radical R", squarate protégé, NH tétrazole protégé, NR" tétrazole protégé, NH protégé, NR" tétrazole substitué par le radical R", NHSO₂R" et NSO₂R", R" étant défini précédemment, ainsi que leurs dérivés dans lesquels l'azote est protégé.

4. Composés de formules (IV), (V) et (VI) ou l'un de leurs sels avec un acide, notamment leurs chlorhydrates et leurs trifluoroacétates : 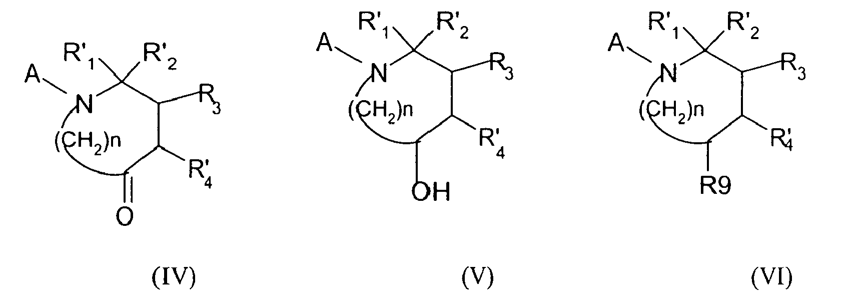 dans lesquelles R'₁, R'₂, R₃, R'₄ et n ont les mêmes significations que dans la revendication 1, R₉ représente un groupe partant de type sulfonate ou halogène et A représente un atome d'hydrogène ou un groupe protecteur de l'azote.

5. Composés de formules (VII) et (VIII) ou l'un de leurs sels avec un acide, notamment leurs chlorhydrates et leurs trifluoroacétates : 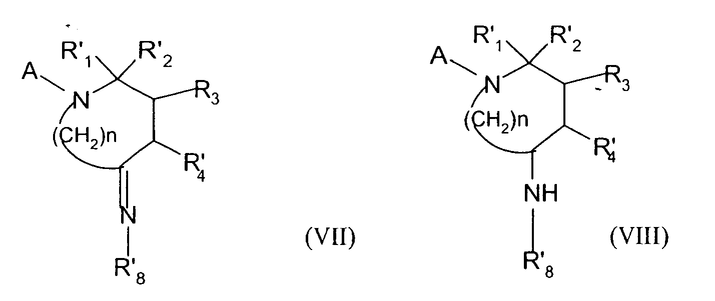 dans lesquelles R'₁, R'₂, R₃, R'₄ et n ont les mêmes significations que dans la revendication 1, A représente un atome d'hydrogène ou un groupe protecteur de l'azote et R'₈ a la même signification que dans la revendication 3.
